(19) Europäisches Patentamt
European Patent Office
Office européen des brevets

(11) EP 3 581 646 A1

(12) EUROPEAN PATENT APPLICATION

(43) Date of publication:
**18.12.2019 Bulletin 2019/51**

(51) Int Cl.:
*C12N 7/02* (2006.01) *C12N 15/86* (2006.01)

(21) Application number: **18178037.0**

(22) Date of filing: **15.06.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Themis Bioscience GmbH**
**1190 Vienna (AT)**

(72) Inventors:
- **Csar, Patrick**
  **3433 Königstetten (AT)**
- **Jungbauer, Alois**
  **1210 Wien (AT)**
- **Kort, Alexander**
  **1220 Wien (AT)**
- **Müllner, Matthias**
  **3441 Pixendorf (AT)**
- **Palmberger, Dieter**
  **1160 Wien (AT)**
- **Ramsauer, Katrin**
  **1220 Wien (AT)**
- **Schrauf, Sabrina**
  **7072 Mörbisch (AT)**
- **Steppert, Petra**
  **2432 Schwadorf (AT)**
- **Tauber, Erich**
  **3426 Muckendorf (AT)**

(74) Representative: **Eisenführ Speiser**
**Patentanwälte Rechtsanwälte PartGmbB**
**Postfach 31 02 60**
**80102 München (DE)**

# (54) INTEGRATED MANUFACTURING AND CHROMATOGRAPHIC SYSTEM FOR VIRUS PRODUCTION

(57) Provided is a method for producing and/or purifying measles virus (MV) particles from a sample, the method comprising in sequential order the following steps loading a sample containing MV particles and one or more impurities onto a stationary phase material for carrying out flow-through chromatography to bind at least a fraction of the impurities contained in the sample and to produce a flow-through comprising at least a fraction of the MV particles contained in the sample; carrying out filtration, preferably ultrafiltration, and obtaining a retentate having an increased MV titer relative to the MV titer comprised in the flow-through. Further provided is a system for producing and/or purifying MV particles, comprising at least one bioreactor; a clarification unit, preferably a dead end filter unit, downstream to the bioreactor; a flow through chromatography unit downstream to the clarification unit; and a filtration unit, downstream to the flow through chromatography unit.

EP 3 581 646 A1

## Description

### Technical Field

The present invention generally relates to the field of virology and specifically relates to chromatography based purification strategies of sterically demanding, i.e. large and pleomorphic, virus particles, particularly measles viruses (MVs) and more particularly viruses having a MV scaffold to yield fractions or compositions comprising virus particles in high yield and low content of impurities such as host cell DNA contaminants and/or host cell protein contaminants.

### Background of the Invention

Virus particles are used in a variety of different prophylactic and curative medical applications, e.g., for vaccination as well as for therapeutic purposes. Regulatory agencies demand the provision of specifically produced virus material to guarantee the safety of the respective material. The WHO and the responsible national and regional approval authorities, like the FDA or the EMEA, understandably impose high product and labelling requirements to a composition comprising a virus material used as vaccine or therapeutic to achieve the provision of safe biological products. The major hallmarks to be fulfilled by a vaccine candidate based on a virus or viral component are its safety, purity and potency. Therefore, there exists an ongoing need to provide suitable vaccines having an acceptable immunogenicity, safety and tolerability profile for vaccination.

Virus particles find also application in oncolytic virus (OV) therapy, which has been recognized as a promising and potentially break-through therapeutic approach for cancer treatment. OVs are genetically engineered or naturally occurring viruses that can selectively replicate in and kill cancer cells without, or at least to a less extent, harming benign tissues. In contrast to gene therapy where a virus is used as a mere carrier for transgene delivery, OV therapy uses the virus itself as an active ingredient. OVs implement a unique mode of action, tumor-restricted viral infection, replication, cell lysis and spread. Preclinical and clinical research has revealed their pleiotropic therapeutic activity: (i) viral tumor cell lysis triggers systemic antitumor immunity, (ii) the insertion of therapeutic genes can trigger bystander killing by different means, depending on the chosen gene, and (iii) endothelial cells specifically in tumor vessels are susceptible to OVs, resulting in vascular shut down and indirect destruction of tumor cells. Whereas numerous oncolytic viruses have been subjected to clinical trials, the common feature that is expected to play a major role in prolonging the survival of cancer patients is an induction of specific antitumor immunity in the course of tumor-specific viral replication (for a review, see Fukuhara et al., Cancer Sci. 2016 Oct; 107(10): 1373-1379).

In contrast to using virus particles as a vaccine, oncolytic activity as an advanced therapy medicinal product depends on high concentration of infectious particles. Product- and process-related impurities are an inherent problem when working with viruses due to the fact that a virus will have to be propagated on suitable host cell in a suitable manner. Though batch preparations meet the requirements for clinical trials, purity needs to be improved to bring an OV therapeutic product to the market. The ratio between total virus particles (vp) and infectious particles (ip) is a critical parameter for the quality of virus preparations. Although a certain vp/ip ratio is not specified by the regulatory agencies, a value of $\approx$50 in virus based preparations can be assumed on average. Residual DNAf rom host cells in the final product is one of the concerns in the manufacturing process of MV as an advanced therapy medicinal product. For viral vaccines, the standard limits adopted by various countries (U.S. Pharmacopoeia, European Pharmacopoeia and Pharmacopoeia of the People's Republic of China) for viral vaccines are mainly three grades: 10 ng/dose, 100 pg/dose and 10 pg/dose. But for the use of high doses of MV in cancer therapies (e.g., $10^9$ ip for intratumoral injections), the limit for expectedly higher amounts of residual host DNA in the final product still needs to be established (Mol Ther Methods Clin Dev. 2016; 3: 16018). Thus, there is a need for providing a method for the provision of virus based preparations suitable for OV therapy, which method allows the production of virus particles in high yield and high purity, and which can be carried out aseptically under GMP conditions throughout the whole process.

Viruses are a highly heterogeneous group of infectious agents. An enormous variety of possible genomic compositions for different virus groups can be observed. The major virus groups are double-stranded (ds) DNA viruses, single-stranded (ss) DNA viruses, dsRNA viruses, (+ strand) ssRNA viruses, (- strand) RNA viruses, ssRNA retroviruses and dsDNA retroviruses. In turn, viruses display a wide diversity of shapes and sizes, also called morphologies. This diversity strongly influences purification strategies, which have to be optimized individually. A complete virus particle or virion usually consists of nucleic acid (RNA or DNA) surrounded by a protective coat of protein called a capsid. Capsids are formed from identical protein subunits called capsomeres. Certain viruses can have a lipid "envelope" derived from the host cell membrane. The capsid comprises proteins encoded by the viral genome and its shape serves as the basis for morphological distinction. Viruses in the size range of between about 20 and 800 nm have been described, whereas some filoviruses may have a total length of up to 1,400 nm. A particular problem for purification approaches may be pleomorphic viruses, i.e., the appearance and shape of one and the same virus can vary within a given preparation.

Based on the nature of the different groups of viruses, the amount of residual cell-substrate DNA in a viral preparation, e.g., a vaccine, will depend on the degree to which the vaccine can be purified. For example, a protein vaccine or a subunit vaccine, such as an influenza vaccine, would usually have less contaminating DNA than an inactivated, whole virus vaccine, such as the inactivated poliovirus vaccine, and both of these would have less DNA than a live attenuated vaccine, such as the OPV (oral polio vaccine), MMR (measles - mumps - rubella), or varicella vaccines.

With regard to purification of virus particles, Nestola et al. (Biotechnology and Bioengineering, Vol. 112, No. 5, May, 2015) points out that viruses and viral vectors are complex biopharmaceutical products, which vary in size, shape, and surface structure. The isoelectric point (pi), surface hydrophobicity, presence or absence of an envelope, and particle lability can play important roles in the design of the DSP train. The virus surface defines its individual physicochemical characteristics including the charge magnitude and distribution, hydrophobic residues, and post-translational modifications (i.e., glycans) of surface proteins. Compared to classic monoclonal antibodies, further additional aspects have to be taken into consideration in virus production: the bioreaction and purification steps must maintain the immunogenicity and stability of the viruses or viral vectors throughout the entire production chain. Moreover, safety usually, biosafety level 2 or higher is required. In addition, the complexity of viruses requires refined analytical techniques for assessing the purity and quality attributes of the final product. Given this complexity, an effective and scalable purification process of the bioproduct can only be achieved through a targeted and fine-tuned combination of several unit operations which necessarily have to be modified for each different virus or virus particle.

Ultrafiltration (UF) is considered a key operation in all large-scale bioprocesses that produce large volumes of bulk, e.g., up to 2-kL for vaccines and 20 kL for mAbs, since they must be concentrated 10-100 times prior to being further purified by chromatography. The volumetric concentration and optional buffer exchange of the virus bulk is critical not only to obtain high titer stocks in the proper formulation buffer, but also to reduce the handled volume; the latter accelerates the downstream processing and keeps the scalability of the purification train at a manageable level (Nestola et al., *supra*).

One specific virus which is known to be difficult to purify is measles virus (MV), said virus being inherently pleomorphic. Despite the availability of first vaccine candidates based on MV particles, there exists an ongoing need to provide purification schemes for removing product-related impurities from virus preparations intended for therapy, particularly if derived from sterically demanding large viruses, for example, from the order *Mononegavirales,* particularly the family *Paramyxoviridae,* for example MV particles, simultaneously allowing the provision of an infectious and immunogenic virus population, which can be conducted under GMP conditions. Moreover, there exists an ongoing need to provide purification schemes for virus particles in high yields, in particular when the virus particles are intended to be used in OV therapy, which demands for much higher doses than vaccination. Moreover, there exists an ongoing need to provide virus particles in high purity to allow the provision of a safer medicament finding more acceptance and being faced with less concerns due to their impurities, in particular in case it has to be administered in high doses such as in OV therapy.

## Summary of the Invention

An object of the present invention is thus the provision of a method suitable for the purification of MVs, in particular recombinant MV but also applicable to wild-type structures, which allows the provision of a highly pure viral composition with high yields. Another object of the present invention was to establish a method suitable for the purification of MVs, which method can be carried out as a fully-integrated process. The process should be a closed process having end-to-end functionality to comply with sterility requirements throughout the whole process. Furthermore, it was an aim to establish a method suitable for the purification of MVs, which method can be precisely controlled.

These objects have been achieved by providing, in a first aspect, a method suitable for purifying MV particles from a sample, the method comprising in sequential order the following steps: loading a sample containing MV particles and one or more impurities onto a stationary phase material for carrying out flow-through chromatography to bind at least a fraction of the one or more impurities contained in the sample and to produce a flow-through comprising at least a fraction of the MV particles contained in the sample; and carrying out filtration, preferably ultrafiltration, and obtaining a retentate having an increased MV titer relative to the MV titer comprised in the flow-through.

The objects have been further achieved by providing in a second aspect a system suitable for purifying MV particles, the system comprising or consisting of: (i) at least one bioreactor; (ii) a clarification unit, preferably a dead end filter unit, downstream to the bioreactor; (iii) a flow through chromatography unit downstream to the clarification unit; and (iv) a filtration unit, preferably a tangential flow filtration unit, downstream to the flow through chromatography unit.

In a third aspect of the disclosure, the objects have been achieved by use of a flow-through chromatography unit as defined in the second aspect of the present invention for the purification of MV particles.

The first and second aspect of the present invention are particularly useful in providing a prophylactic and/or therapeutic composition. Hence, in a fourth aspect of the present disclosure, there is provided a prophylactic or therapeutic composition produced by the method of the first aspect of the invention or the system of the second aspect of the invention, wherein infectious MV particles are comprised as an active ingredient.

Preferred prophylactic and/or therapeutic compositions include immunogenic and/or vaccine compositions, which are suitable and/or intended to be used in a method of eliciting an immune response or in a method of prophylactic treatment of a subject for protecting the subject from infection with a virus. In such method, protection is achieved by exposing the subject to the MV particles comprised by the immunogenic composition or the vaccine composition. Further preferred prophylactic and/or therapeutic compositions include compositions, which are suitable and/or intended to be used for tumor therapy such as oncolytic tumor therapy based on infectious MV particles.

Hence, in a fifth aspect of the present disclosure, there is provided a composition according to the fourth aspect of the present invention for use in a method of therapeutic and/or prophylactic treatment. In one embodiment of the fifth aspect, a subject is exposed to the MV particles comprised by the composition for protecting the subject from infection with a virus and/or an immune response is elicited by exposing the subject to the MV particles comprised in the composition. In another embodiment of this aspect, a subject is exposed to the MV particles comprised by the composition for tumor therapy such as oncolytic tumor therapy and/or an immune response is elicited by exposing the subject to the MV particles comprised in the composition.

In a sixth aspect of the present disclosure, there is provided a method of prophylactic and/or therapeutic treatment, the method comprising exposing a subject to the MV particles comprised by the composition of the fourth aspect of the present disclosure. In one embodiment of this aspect, the method is used for protecting the subject from infection with a virus. In another embodiment of this aspect, the method is used for (oncolytic) tumor therapy. In both cases, the method may comprise exposing the subject to the MV particles comprised in the composition and/or elicit an immune response by exposing the subject to the MV particles comprised in the composition.

In a seventh aspect, the present disclosure relates to use of MV particles, obtained by the method according to the first aspect or the system according to the second aspect of the invention, for the manufacture of a medicament for therapeutic and/or prophylactic treatment, in particular as a vaccine or in OV therapy.

As the various embodiments and aspects encompassed by the present disclosure all relate to possible variations of the methods of the present invention, they can be used alone or in combination with each other all forming individual embodiments according to the various aspects according to the present disclosure, where reasonable for the skilled person having knowledge of the present disclosure. Especially, all embodiments disclosed for the first aspect according to the present invention likewise apply for the second aspect of the present application, as the system of the invention is suited and intended to be used for carrying out the various embodiments of the method according to the first aspect of the invention.

Further aspects and embodiments of the present invention can be derived from the subsequent detailed description and drawings, the sequence listing, as well as the attached set of claims.

**Brief description of the drawings**

Figure 1 show a process scheme of upstream manufacturing in accordance with an embodiment of the first aspect of the present invention. Arrows indicate: (A) inoculation of bioreactor; (B) infection of bioreactor; (C) harvest.

Figure 2 shows a process scheme of downstream manufacturing in accordance with a further embodiment of the first aspect of the present invention. Arrows indicate: (A) A: 1 M MgCl2, 500mM EDTA; B: A: 50 mM HEPES, 150 mM NaCl, pH 7.5, (B) 50 mM HEPES, 2M NaCl, pH 7.5; (C) 50 mM HEPES, 150 mM NaCl, pH 7.5; (D) 5 % Sorbit (w/v); (E) 1 M NaOH, 0.1 M NaOH, WFI, 20 % EtOH; (F) 1 M NaOH, WFI, 50 mM HEPES, 150 mM NaCl, pH 7.5; (G) 1 M NaOh, 0.1 M NaOH, WFI, 20 % EtOH.

Figure 3 shows attachment to and growth of host cells on microcarriers, which can be employed in certain embodiments of the first aspect of the present invention. In this specific embodiment, Vero cells and Cytodex I were used as host cells and microcarriers, respectively. Figure 3A is a photograph taken 1 h after inoculation. As outlined above, "inoculation" means combining host cells, or a host cells containing liquid, and microcarriers, or a microcarrier containing liquid, in a common mixture under condition which allows the host cells to adhere to the microcarriers. As can be observed, the host cells readily attached to the microcarriers. Figure 3B is a photograph taken 96 h after inoculation and demonstrates that the cells grow until confluence. At or around this point, the host cells are ready for virus infection, which time point will vary depending on the nature of the host cell.

Figure 4 show cell growth and metabolite consumption over time following inoculation. Figure 4A shows development of cell numbers. The solid line indicates counts of attached cells, whereas the dotted line indicates counts of suspended cells. As can be seen, upon attachment, cells immediately start to grow until confluence resulting in a cell density of up to 1.5 x $10^6$ cells/mL 96 h post inoculation. After a medium exchange, the culture was infected at 96 h post inoculation resulting in a growth arrest and finally leading to cell detachment (decrease of solid line; increase of dashed line) form the microcarrier and cell lysis. Figure 4B shows the course of various metabolites following inoculation. During a first growth phase lasting until 96 h post infection, cells metabolize nutrients provided by the medium thereby producing toxic by-products. In accordance with this, glucose (dashed line) and glutamine (dotted line) were consumed in relation to cell growth, whereas lactate (solid black line) and ammonium (solid grey line) were build up. In order to improve effectiveness of infection with and production of MV, the medium was renewed after at 96 h post inoculation.

Figure 5 shows the time-dependent increase of virus titer expressed by means of $TCID_{50}$ (median tissue culture infective/infectious dose) post infection. As a result of successful virus amplification, the virus titer steadily increases. A top level of 9.55 x $10^5$ $TCID_{50}$/mL is achieved at 144 h post infection. The bar on the right hand ("filt. Harvest") illustrates the virus titer determined after clarification using depth filtration. By comparing it with the respective virus titer at 144 h post infection, one could deduce that clarification results in an increase of the virus titer. This observation could be explained in that clarification and in particular filtration might lead to a break up of virus aggregates, thereby increasing the number of infectious virus particles.

Figure 6 shows on-line cell density measurements in accordance with an embodiment of the first aspect of the present invention. On-line signals were generated by permittivity measurements of live cells (solid line). Comparative off-line data were generated by measurement of cellular nuclei after citric acid release (dashed line). As can be seen, the on-line signal immediately reacts on the inoculation of the bioreactor and continuously increases upon confluence of the cells on the microcarrier and depletion of the medium. Off-line values are in good agreement with on-line counterparts and deviations can be explained by technical issues during off-line measurements. 96 h post inoculation the medium is exchanged, and the culture is infected with MV. This causes a stop in cell growth followed by a detachment and lysis of the cells.

Figure 7 shows data generated during two individual purification runs using flow-through chromatography in 1 mL scale in accordance with an embodiment of the first aspect of the present invention. In a first experiment, 35 CV (column volume(s)) of filtered cell culture supernatant was directly loaded on a 0.844 mL Capto Core 700 column. In a second experiment, 44 CV of filtered and endonuclease treated cell culture supernatant was directly loaded on a 0.903 mL Capto Core 700 column. Results of the first experiment are shown in Figures 7A to 7D. Results of the second experiment are shown in Figures 7E and 7G. More specifically, Figures 7A and 7E show chromatograms, Figures 7B and 7F show Western blot analyses of elution fractions using Measles NP (3E1) antibody, Figures 7C show a Coomassie-stained gel and Figures 7D and 7G show a silver-stained gel. The analyzed elution fractions are indicated in the respective chromatograms. The fractions are abbreviated as follows. L: Loading material; BH: Bulk harvest; FT: Flow-through; W: Wash; R: Regenerate. The Western blot analyses also show a sample, which was desorbed from the frit of the Tricorn column as outlined herein. This sample is indicated as F: Frit Tricorn column. It was thereby proven than the MV adsorbed to the frit, resulting in a low recovery.

Figure 8 shows on-line data (Figures 8A and 8E), Western blot analysis (Figures 8B and 8E) as well as silver-stained (Figures 8D and 8H) and Coomassie-stained SDS-PAGE gels (Figures 8C and 8G), which were generated during further flow-through chromatography runs, upscaled to 10.5 mL (Figures 8A to 8D) or 188.5 mL (Figures 8E to 8H) Capto Core 700 column, in accordance with an embodiment of the first aspect of the present invention. Elution fractions were collected according to the chromatogram and analyzed by Western blot detecting Measles NP (3E1). The following abbreviations were used for denoting the fractions. L: Loading material; BH: Bulk harvest; FT: Flow-through; W: Wash; R: Regenerate; FH: Filtered harvest

Figure 9 shows ultrafiltration of MVs material that has been purified by flow-through chromatography in accordance with an embodiment of the first aspect of the present invention. The purified MVs material was concentrated by a HF module (Figures 9A to 9D), a RC membrane (Figures 9E to 9H) or SC membrane (Figures 9I to 9L). All membranes providing 50 $cm^2$ filtration area and 300 kDa MWCO (Figures 9A to 9H) or 30 kDa MWCO (Figures 9I to 9L). The moving average of the permeate flux Samples of the feed (Figure 9F), the concentrate (Figure 9C), and permeate (Figure 9P) were collected during the filtration according to the labels in diagrams (Figures 9A, 9E and 9I) and were analyzed by Western blot detecting Measles NP (3E1) (Figures 9B, 9F, 9J) and SDS-PAGE analysis Coomassie -stained (Figures 9C, 9G, 9K) or silver stained (Figures 9D, 9H, 9L).

Figure 10 shows a process scheme according to an embodiment of the method of the present invention. Recoveries (yields) in terms of TCID50/mL as well as relative recoveries calculated from TCID50/mL are indicated for each step. Furthermore, relative amounts of impurities, specifically total protein content and dsDNA content, are indicated.

For example, according to this embodiment, the optional endonuclease treatment step results in an about 60 % reduction in the amount of dsDNA. Notably, the depletion factors are determined after they have been correlated to the respective volume, i.e., the total amount (conc.*volume) is 100% of virus, protein or DNA, which have been used for a specific step. The % values after each step thus indicate the amount (conc.*volume) which could be recovered.

Figure 11 shows the results of viscosity measurement of process fluid, measured after each process step.

Figure 12 shows results obtained from chromatographic purification of GFP-MV by Capto Core 700 in accordance with an embodiment of the first aspect of the present invention. The chromatogram (Figure 9A) shows inter alia an online GFP signal measured at Absorbance of 490 nm. Western blot analysis (Figure 9B) were carried out by detecting Measles NP (3E1). L: Loading material; BH: Bulk harvest; FT: Flow-through.

Figure 13 shows fluorescence microscopic pictures taken after infection of Vero host cells with MV-GFP in accordance with an embodiment of the first aspect of the present invention. Fig. 13A: 24 h post infection; Fig. 13B: 48 h post infection; Fig. 13C: 72 h post infection; Fig. 13D: 96 h post infection; Fig. 13E: 120 h post infection; and Fig. 13F: 144 h post infection. The largest image (Fig. 13A to F) always shows the increasing GFP expression (white/bright spots in black/and white drawings). The large image on the left is a merge of the upper image on the right and the lower image on the right. The lower image on the right shows the GFP-fluorescence channel (again, white/bright spots indicated GFP signals), whereas the upper image on the right shows the bright field image. As can be seen, the number of GFP expressing cells (bright spots in the respective image) on the microcarrier increased over time indicating success of infection and production of MV-GFP. With increasing duration of incubation following infection, spots were also visible in the culture broth as a result of cell lysis and release of MV.

## Definitions

When reference is made to "a" or "an" entity this means that one, or more than one, of that entity is meant, unless otherwise dictated by context. For example, "a nucleotide sequence," is understood to represent a first embodiment, wherein one nucleotide sequence is meant, and a second embodiment, wherein more than one nucleotide sequences are meant. As such, the terms "a" (or "an"), "one or more," and "at least one" can be used interchangeably herein.

The expression "and/or" as used herein shall be understood as specific disclosure of each of the two specified features or components with or without the other. Thus, the term "and/or" as used in a phrase such as "A and/or B" herein includes "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

The term "about" is used herein to mean approximately, roughly, around, or in the regions of. When the term "about" is used in conjunction with a numerical range, it modifies that range by extending the boundaries above and below the respective numerical values by a variance. When the term "about" is used in conjunction with a numerical value, it modifies that value above and below the stated value by a variance. In the context of the present invention, a variance is to be taken to include maximum values of 50 %, 40 %, 30 %, 20 % or 10 %.

An "adjuvant" in the field of immunology and as used herein refers to a substance or composition enhancing antigenicity of another substance. This is achieved by specifically stimulating and balancing mainly the Th1 and Th2 subsets of T-cells and thus their effector molecules. Usually, immunogenic compositions based on live-attenuated or killed viruses are highly immunogenic *per* se and thus there might not be the need for an additional adjuvant, whereas an additional adjuvant might still be favorable to balance the provoked immune response. Th1 cells secrete IFN-y, which activates macrophages and thus induces the production of opsonizing antibodies by B cells. The Th1 response therefore leads mainly to a cell-mediated immunity. Th2 cells mainly secrete cytokines, including IL-4, which induces B cells to make neutralizing antibodies. Th2 cells generally induce a humoral (antibody) response critical in the defense against extracellular pathogens (helminths, extracellular microbes and toxins).

The terms "amino acid molecule/sequence", "protein", or "peptide" or "polypeptide" are used interchangeably herein. The term "amino acid" or "amino acid sequence" or "amino acid molecule" comprises any natural or chemically synthesized protein, peptide, or polypeptide or a modified protein, peptide, polypeptide and enzyme, wherein the term "modified" comprises any chemical or enzymatic modification of the protein, peptide, polypeptide and enzyme.

The term "antigen" as used herein and as commonly used in the field of immunology refers to an "antibody generating" molecule, i.e. a substance, which can elicit an adaptive immune response. An antigen is thus a molecule binding to an antigen-specific receptor, either a T-cell or a B-cell receptor. An antigen is usually a (poly)peptide, but it can also be a polysaccharide or a lipid, possibly combined with a protein or polysaccharide carrier molecule. For the purpose of the various aspects and embodiments of the present invention, the antigen is a (poly)peptide, i.e. an amino acid sequence. In the case of binding to a T-cell receptor, the antigen is presented to the respective T-cell receptor via an antigen-presenting cell as an antigenic peptide bound to a histocompatibility molecule on the surface of the antigen presenting cell, wherein the antigenic peptide has been processed in advance by the antigen presenting cell. Thus, an "antigen" as used herein refers to a molecule, such as a protein or a polypeptide, containing one or more epitopes that will stimulate a host's immune system to make a humoral and/or cellular antigen-specific response. The term is also used interchangeably with the term "immunogen" and concerning the effect "immunogenic".

As used herein, the term "aseptic" denotes condition, under which contamination from living organisms prevented. In the context of the present invention, an aseptic operation results in a product having low bioburden or even being substantially sterile.

The term "at least a fraction" in the context of binding impurities includes embodiments, wherein at least 5 %, at least 10 %, at least 15 %, at least 20 %, at least 25 %, at least 30 %, at least 35 %, at least 40 %, at least 45 %, at least 50 %, at least 55 %, at least 60 %, at least 65 %, at least 70 %, at least 75 %, at least 80 %, at least 85 %, at least 90 %, at least 95 % or even 100 % of the one or more impurities bind to a chromatography material and is thereby separated from the product of interest.

The term "at least one host cell" may generally refer to one host cell, more than one host cell and preferably a plurality of host cells (herein sometimes also denoted a "cell population"). The term host cell encompasses non-recombinant cells, i.e. cells that were not immortalized or transformed or manipulated in a purposive manner, as well as recombinant host cells or immortalized host cells (spontaneous or immortalized in a targeted manner). To be suitable for the purposes of the present invention, the host cell(s) must be able to support the MV replication cycle, i.e. the cell(s) must be susceptible to MV or MV scaffold infection and suitable for the subsequent propagation or replication cycle, including replication, translation, encapsidation of the RNA of the virus and budding from the host cell to be released as virus particle. Preferred eukaryotic host cells that fulfil these requirements are disclosed herein.

The terms "attenuation" or "attenuated" as used herein in connection with a virus or a material derived therefrom refers to a virus weakened under laboratory conditions which is less vigorous than the respective wild-type virus, for example by deleting certain genes, for example a viral accessory protein of the virus. An attenuated virus may be used to make a vaccine that is capable of stimulating an immune response and creating immunity.

"Binding" a molecule such as an impurity to a chromatography material means exposing the molecule to chromatography material under appropriate conditions (e.g. pH/conductivity) such that the molecule is reversibly retained and/or immobilized in or on the chromatography resin by virtue of ligand-molecule interactions.

The term "cDNA" stands for a complementary DNA and refers to a nucleic acid sequence/molecule obtained by reverse transcription from an RNA molecule.

The term "clarifying" according to the present disclosure refers to a step for removing large impurities from a bulk product such as a cell culture supernatant to be clarified. The term removing also encompasses a reduction in the amount (e.g. concentration) of impurities. Clarification may rely on separation by size, molecular weight or density and the like. "Large impurities" is to be taken to preferably refer to a size bigger than the MV particles to be purified.

A "carrier" according to the present disclosure is a substance that aims at improving the delivery and effectiveness of a drug composition. Carrier materials may depend on the physical state of a drug composition to be administered. Typically, immunogenic or vaccine compositions are administered as liquid solution. Suitable substances include, large, slowly metabolized macromolecules, such as proteins, polysaccharides, polylactic acids, polyglycolic acids, polymeric amino acids, amino acid copolymers, and inactive virus particles. Pharmaceutically and veterinary acceptable salts can also be used in the immunological composition, for example, mineral salts such as hydrochlorides, hydrobromides, phosphates, or sulfates, as well as the salts of organic acids such as acetates, propionates, malonates, or benzoates. Immunological compositions can also contain liquids, such as water, saline, glycerol, and ethanol, as well as substances such as wetting agents, emulsifying agents, or pH buffering agents. Furthermore, nanocarriers, including liposomes, also can be used as carriers. Depending on the nature of the therapeutic or prophylactic composition used and dependent on the immune response, which is intended to be provoked, such a composition can additionally comprise an adjuvant and further pharmaceutically and/or veterinary acceptable carriers. Furthermore, a therapeutic or prophylactic composition according to the present disclosure can comprise more than one active ingredient in the form of an antigen.

The term "chromatography" shall be understood to refer to any kind of technique (preferably a preparative technique) which separates a virus particle of interest from other molecules present in a mixture by differential partitioning between a mobile phase and a stationary phase. The stationary phase is thereby preferably held in place, while the mobile phase moves in a definite direction. Partitioning is to be understood to occur repeatedly as the sample flows with the mobile phase along or through a dimension, e.g. length, of the stationary phase. As such, the term does not encompass a method, wherein a sample is and a stationary phase material are combined, mixed and the supernatant recovered ("batch adsorption"). In the context of the present invention column chromatography is preferred. "Column chromatography" as used herein is a technique in which a stationary phase material is contained in a container such as a tube or column having an inlet on one side of the container and an outlet on another side, preferably the opposite side. The stationary phase material may for example be comprised of particles of a solid stationary phase material or a support coated with a liquid stationary phase. It may fill the whole inside volume of the container (packed column) or be concentrated on or along the inside tube wall leaving an open, unrestricted path for the mobile phase in the middle part of the tube (open tubular column). In the context of the present invention, a packed column chromatography is most preferred.

It is understood that wherever aspects are described herein with the language "comprising", "having", "containing", "involving" and similar expressions otherwise analogous aspects described in terms of "consisting of and/or "consisting essentially of" are also provided.

The terms "derived", "derived from", "derivative" or "descendant" or "progenitor" as used herein in the context of either a host cell, a cell population or a virus particle according to the present invention relates to the descendants of such a host cell or virus particle which results from natural reproductive propagation including sexual and asexual propagation and, in the context of virus nucleic acids, the propagation of the virus genetic material by the host cell machinery. Propagation can lead to the introduction of mutations into the genome of an organism resulting from natural phenomena which results in a descendant or progeny, which is genomically different to the parental host cell, however, still belongs to the same genus/species and possesses the same characteristics as the parental host cell. A virus, derivative or descendant or progenitor can thus naturally possess one or more mutations. Such derivatives or descendants resulting from natural phenomena during reproduction or propagation are thus comprised by the term host cell or cell population according to the present disclosure and the skilled person can easily define, by means of molecular biology, microscopy or the like, that the derivative or descendant is indeed derived from a parental host cell of the same genus. These terms, therefore, do not refer to any arbitrary derivative, descendant or progenitor, but rather to a derivative, or descendant or progenitor phylogenetically associated with, i.e. based on, a parent cell or virus or a molecule thereof.

As used herein, the term "diafiltration" or "DF" is used to mean a specialized class of filtration in which the retentate is diluted with a (fresh) solution or buffer and re-filtered, to reduce the concentration of soluble permeate components. Diafiltration may or may not lead to an increase in the concentration of retained components, including, virus particles. For example, in continuous diafiltration, a solvent is continuously added to the retentate at the same rate as the filtrate is generated. In this case, the retentate volume and the concentration of retained components does not change during the process. On the other hand, in discontinuous or sequential dilution diafiltration, an ultrafiltration step is followed by the addition of solvent to the retentate side; if the volume of solvent added to the retentate side is not equal or greater to the volume of filtrate generated, then the retained components will have a high concentration. Diafiltration may be used to alter the pH, ionic strength, salt composition, buffer composition, or other properties of a solution or suspension of macromolecules.

The term "direct fluid communication" as referred to herein, implies that the fluid transported, e.g., between two unit operations, does not pass a section, e.g., another unit operation, where it is treated in such a way that its composition is substantially changed. In some embodiments of the present invention, a direct fluid communication is achieved through one or more flow lines, e.g., ducts arranged between two unit operations, which may optionally comprise bends, reservoirs, valves, and the like. Similarly, the term "direct" referred to in the context of subjecting a sample, liquid, mixture or the like to a unit operation, implies that the sample, liquid, mixture or the like does not pass a section, where it is treated in such a way that its composition is substantially changed.

An "excipient" is a substance included in a drug composition, including an immunological or a vaccine composition, which is added for the purpose of long-term stabilization, bulking up solid formulations that contain active ingredients, including, for example, infectious virus particles, or to confer a therapeutic enhancement on the active ingredient in the final dosage form, e.g. for improving the absorption, modifying the viscosity or for enhancing solubility.

The terms "flow-through mode" and "flow-through chromatography", as used interchangeably herein, refer to a separation technique in which at least one product of interest (herein a virus particle) contained in a sample is intended to pass a chromatography medium, while the contaminants (here the one or more impurities) bind to the chromatography medium. The product of interest does not or only to a little extent interact with the chromatography medium and can thus be recovered in the flow-through.

As used herein, a "flow-through chromatography unit" refers to an apparatus comprising a stationary phase material and optionally a control unit, wherein the stationary phase material under appropriate conditions is capable of binding one or more impurities but not MV particles and wherein the control unit, if present, is preferably programmed to operate the chromatographic purification in flow-through mode. The control unit may in particular include commands to recover the MV particles containing flow-through and/or isolate it from the one or more impurities bound to the stationary phase material.

The terms "genetically modified", "recombinant", "genetically engineered" or similar expression as used herein refer to a nucleic acid molecule or an amino acid molecule or a host cell comprising a targeted and purposive manipulation and/or modification achieved by means of molecular biology or protein engineering, e.g. by introducing a heterologous sequence into another host cell, by modifying a naturally occurring nucleic acid sequences and the like. Further modifications include, but are not limited to, one or more point mutation(s), one or more point mutation(s), e.g. for targeted protein engineering or for codon optimization, deletion(s), and one or more insertion(s) of at least one nucleic acid or amino acid molecule, modification of an amino acid sequence, or a combination thereof. The terms can also imply a sequence, which *per* se occurs in nature, but has been purposively treated by means of molecular biology.

Percentages of homology or identity of nucleic acid or amino acid sequences shall be understood to correspond to values determined by using the EMBOSS Water Pairwise Sequence Alignments (nucleotide) program (http://www.ebi.ac.uk/Tools/psa/emboss_water/nucleotide.html) for nucleic acids or the EMBOSS Water Pairwise Sequence Alignments (protein) program (http://www.ebi.ac.uk/Tools/psa/emboss_water/) for amino acid sequences. Those tools provided by the European Molecular Biology Laboratory (EMBL) European Bioinformatics Institute (EBI) for local sequence alignments use a modified Smith-Waterman algorithm (see http://www.ebi.ac.uk/Tools/psa/ and Smith, T.F. & Waterman, M.S. "Identification of common molecular subsequences" Journal of Molecular Biology, 1981 147 (1):195-197).

When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5.

As used herein, "impurities" and "contaminants" may interchangeably refer to undesired components in the viral preparation, which components may be present at any step during the purification process and particularly in the beginning of the downstream process. In some embodiments, impurities or contaminants may be host cells or fragments thereof, including host cell DNA and/or host cell proteins; viral fragments or viral nucleic acid; enzymes, such as BENZONASE® Nuclease, salts; and components of the cell culture medium.

An "immunogenic composition" as used herein refers to a composition which is able to induce an immune response in a subject. An immunogenic composition according to the present disclosure comprises at least one vaccine composition based on MV. A vaccine *per se* also is an immunological composition. However, it is well known to the skilled person that for being suitable to administration to an animal, an immunogenic composition can additionally comprise suitable pharmaceutically and/or veterinary acceptable carriers.

As used herein, the term "infectious" according to the present disclosure characterizes one or more virus particles that are able to (re-)infect a cell population, host cell or subject of interest, i.e. to enter a host and replicate therein and potentially spread to further cells or tissues.

"Load" as used herein denotes the part of a sample which is directly subjected to a chromatography step, i.e. loaded onto a chromatography unit.

The term "measles virus particles" (MV particles), as used herein, is to be understood as referring to any particle comprising or derived from MV including native (wild-type) or recombinant MV, either in live, attenuated, inactivated or killed form. Also covered by said term is a virion and a virus-like particle derived from, and thus comprising structural elements of, MV.

The term "MV titer" herein denotes the number of MV particles per volume. The various assays, which can be used for virus titer quantification include, but are not limited to, plaque assays, endpoint dilution assays, protein assays and transmission electron microscopy.

Plaque-based assays are the standard method used to determine virus concentration in terms of infectious dose. Viral plaque assays determine the number of plaque forming units (pfu) in a virus sample, which is one measure of virus quantity. Endpoint dilution assays report 50% Tissue culture Infective Dose (TCID50) as measure of infectious virus titer. The endpoint dilution assay quantifies the amount of virus required to kill 50% of infected hosts or to produce a cytopathic effect in 50% of inoculated tissue culture cells. Protein-based virus quantification assays quantify either the amount of all protein or the amount of a specific virus protein in the sample rather than the number of infected cells or virus particles. Quantification most commonly relies on fluorescence detection. Exemplary protein-based virus quantification methods include hemagglutination assays, bicinchoninic acid assays and single radial immunodiffusion assay. TEM is a specialized type of microscopy that utilizes a beam of electrons focused with a magnetic field to image a sample. TEM images can show individual virus particles and quantitative image analysis can be used to determine virus concentrations. These high resolution images also provide particle morphology information that most other methods cannot. Quantitative TEM results will often be greater than results from other assays as all particles, regardless of infectivity, are quantified in the reported virus-like particles per mL (vlp/mL) result. An alternative method for determining the TCID is quantitative PCR (qPCR). The $TCID_{50}$ as used herein refers to median tissue culture infective dose as defined above. When reference is made herein to specific values of viral titers these are preferably determined by endpoint limit dilution assay, e.g. on Vero cells, and $TCID_{50}$ is be calculated by using the Kärber method.

As used herein, the term "prophylactic treatment" as referred to herein in the context of vaccine compositions means a treatment which mediates a protective immune response in a subject vaccinated so that there are no or less severe symptoms, when the subject after having been vaccinated and after having developed an immune response to the vaccine composition will encounter an infection with the non-attenuated wild-type strain corresponding to the virus antigens present in the vaccine composition. Similarly, a "prophylactic composition" as referred to herein denotes a composition which, when administered, mediates a corresponding protective immune response.

The terms "protection", "protective immunity" and "protective immune response" refer herein to the ability of serum antibodies and cellular response induced during immunization to protect (partially or totally) against a virus of interest. Thus, an animal immunized by the compositions or vaccines of the invention will afterwards experience limited growth and spread if infected with the respective naturally occurring virus.

The term "purifying" as used herein refers to increasing the degree of purity of a product of interest (herein MV particles) from a mixture, composition or sample comprising the product and one or more impurities or contaminants. In some embodiments, the degree of purity of the product of interest is increased by removing (completely or partially) one or more impurities from the mixture, composition or sample.

The term "regulatory sequence" as used herein refers to a nucleic acid sequence which can direct and/or influence the transcription and/or translation of a target nucleic acid sequence of interest. The term thus refers to promoter and terminator sequences or to polyadenylation signals and the like.

The term "sample" herein implies a complex mixture or complex composition originating from a cell-based method, which mixture or composition comprises a product of interest (herein MV particles) and contaminants (herein one or more impurities). The cell-based method preferably includes at least one step of cultivating cells and a step of infecting the cells with MV. In some embodiments of the present invention, the sample is a cell culture supernatant or cell culture lysate directly obtained from a mixture comprising said supernatant and a residue containing cells and/or cell debris. In some embodiments, a sample results from clarification and/or a treatment with an agent having nucleic acid digesting activity of a cell culture supernatant or cell culture lysate directly obtained from a mixture comprising said supernatant and a residue containing cells and/or cell debris.

"Scaffold", "virus scaffold" and similar expressions made in the context of MV shall be understood to refer to a framework or backbone structure based on a MV sequence, in particular a recombinant, infective and/or replicative MV particle, optionally expressing one or more foreign antigens, i.e. antigens that are not naturally occurring in wildtype MV. Therefore, the basic sequence of a MV scaffold will originate of a MV, whereas the sequence encoding the MV or the MV scaffold can comprise further elements.

The terms "sequence(s)" and "molecule(s)" are used interchangeably herein when referring to nucleic acid or amino acid sequences/molecules.

The term "sequentially" herein denotes events, which occur one after another. That is, in a series of sequentially events, only one event takes place at the same time. For example, event B starts with the ending of event A. After end of event B, the series may be complete, or another event (c) or again event A may take place.

As used herein, "simultaneously" encompasses precisely simultaneous as well as nearly simultaneous events. For example, simultaneous events may begin at about the same time, end at about the same time, and/or take place over at least partially overlapping time periods.

The term "stationary phase material" is interchangeably used with the terms "chromatography material" and "chromatography medium" and refers to any kind of a substance fixed in place for chromatography procedure. It is capable of separating a virus particle of interest from other molecules, in the context of the present invention in particular the one or more impurities, present in a mixture. The stationary phase material can be classified according to its (primary) mode of interaction. Examples, without limitation, of such material comprise: size exclusion media, ion exchange media; anion exchange media; cation exchange media; hydroxyapatite media; hydrophobic interaction chromatography media; and mixed-mode media. Suitable stationary phase materials include, without limitation, a non-aqueous matrix comprising agarose, sepharose, glass, silica, polystyrene, collodion charcoal, sand, polymethacrylate, cross-linked poly(styrenedivinylbenzene), agarose with dextran surface extender or any other suitable material, which can optionally be functionalized.

As used herein, the term "ultrafiltration" or "UF" refers to any technique in which a liquid (e.g., a solution or suspension) is subjected to a semi-permeable membrane that retains macromolecules (herein MV particles) while allowing solvent and small solute molecules to pass through. Ultrafiltration may be used to increase the concentration of macromolecules in a liquid and/or to decrease the concentration of impurities, i.e. improve purity. It may employ membranes with molecular weight cut-off (MWCO) ranging from 0.5 to 1,000 kDa. The MWCO is defined as the minimum molecular weight at which 90% of the solute is retained by the membrane. The rejection profiles may be generally determined by polymer dextrans in the range of 1-2000 kDa. Ultrafiltration membranes may be composed by two main layers, e.g., a thick macroporous support that provides mechanical strength, and a thin skin layer that is responsible for membrane selectivity and permeability. UF membranes can be manufactured using different polymers, such as regenerated cellulose (RC), polysulfone (PS), modified polyethersulfone (mPES), and polyvinylidene fluoride (PVDF).

As used herein, the term "ultrafiltration/diafiltration" or "UF/DF" refer to a process, technique or combination of techniques that accomplishes ultrafiltration and diafiltration, either sequentially or simultaneously. In the context of the present invention, UF/DF preferably occurs in the same UF/DF unit.

The term "vector" or "plasmid vector" as used herein defines a system comprising at least one vector suitable for transformation, transfection or transduction of a host cell. A vector *per* se thus denotes a cargo for the delivery of a biomolecule into a host cell of interest, wherein the biomolecule includes a nucleic acid molecule, including DNA, RNA and cDNA, or, in the case of a transfection system as vector, an amino acid molecule, or a combination thereof. A preferred vector according to the present invention is a plasmid or expression vector. An expression vector can comprise one vector encoding at least one target molecule, preferably a nucleic acid molecule, to be introduced into a host cell. A vector of the vector system can also comprise more than one target molecules to be introduced. Alternatively, the vector system can be built from several individual vectors carrying at least one target molecule to be introduced. An expression vector additionally comprises all elements necessary for driving transcription and/or translation of a sequence of interest in a host cell, the expression vector is designed for. These elements comprise, *inter alia,* regulatory elements, which are involved in the regulation of transcription, including promoters and the like functional in the host cell of interest. Furthermore, an expression vector comprises an origin of replication and optionally depending on the type of vector and the intended use a selectable marker gene, a multiple cloning site, a tag to be attached to a sequence of interest, a chromosomal integration cassette and the like. The choice and possible modification of a suitable expression vector for use with a respective host cell and sequence of interest to be inserted into the expression vector is well within the capabilities of the person skilled in the art.

A "viral particle" or "virus particle" as used herein refers to a single particle derived from a viral nucleic acid, which is located outside a cell. As the viral particle may contain genetic information, it may be able to replicate, and/or propagate in a susceptible host cell. An "infectious virus particle" represent the mature and infectious form of a virus.

The term "viral vaccine" or "vaccine composition" as used herein refers to a virus particle, which is able to induce a protective immune response in a subject.

A "virion" as used herein refers to a single particle derived from a viral nucleic acid, which is located outside a cell containing nucleic acids and thus being able to replicate or to be transcribed in a suitable host cell.

A "virus-like particle" or "VLP" as used herein refers to at least one virus particle, which does not contain nucleic acid. VLPs can thus be used for vaccination or inducing an immunogenic reaction in a subject. Due to the absence of nucleic acids they will, however, not be able to replicate in a host cell and are thus non-replicative.

A "virus sample", "virus material" or the like refers to a material comprising at least one of a (recombinant infectious) virus particle and/or a virion and/or a VLP.

The term virus stock refers to a seed stock comprising at least one recombinant infectious virus particle derived from a MV scaffold suitable to infect a host cell of interest. Given the fact that a virus should be provided in a certain amount to efficiently infect a host cell of interest, the term virus stock usually implies a stock comprising more than one infectious virus particle, as depending on the host cell of interest, the infectious virus particle of interest and the intended multiplicity of infection (MOI) used for infection. The MOI usually depends on the Tissue culture infective dose (TCID). An appropriate MOI/$TCID_{50}$ can be determined following common tests, e.g. the Kärber method or the Reed Muench method. A virus stock as used herein preferably refers to a recombinant virus stock.

"Viscosity" is a measure of a fluid's resistance to flow and can be measured at 25°C by DV-II + Pro viscometer (Brookfield Engineering Laboratories, Middleboro, MA, USA). Shear stress and corresponding shear rate data can be used for evaluation of viscosity by power-law according to Ostwald-de Waele. Viscosities measured at a shear rate of 525 1/sec can be selected for comparison. Whenever the present disclosure refers to viscosity values these shall be understood to be determined in accordance with the aforementioned test unless otherwise stated.

By "recombinant virus (particle)" is meant a virus that has been genetically altered, e.g., by the addition or insertion of a heterologous nucleic acid construct into the particle. A recombinant virus does preferably not include a virus as they exist in nature. Still, obtaining a cDNA from a naturally occurring virus and making this sequence recombinantly available also shall imply a recombinant virus (particle) according to the present disclosure.

"Recovery" is interchangeably used herein with the term "yield" and refers to the percentage recovered from a unit operation, relative to amount subjected to said unit operation. For example, a recovery of 100 % TCID50 obtained for flow-through chromatography indicates that the flow-through has the same infectious dose as the load.

As used herein, all numerical values or numerical ranges include integers within such ranges and fractions of the values or the integers within ranges unless the context clearly indicates otherwise. Thus, to illustrate, reference to at least 1-10%% identity, includes 1%, 2%, 3%,4%, 5%, 6%, 7%, 8%, 9%, 10%, as well as 1.1%, 1.2%, 1.3% 1.4%, 1.5%, etc., 2.1%, 2.2%, 2.3%, 2.4%, 2.5%, etc., and so forth.

Reference to a number with more (greater) or less than includes any number greater or less than the reference number, respectively. Thus, for example, a reference to less than 40,000, includes 39,999, 39,998, 39,997, etc. all the way down to the number zero; and less than 100, includes 99, 98, 97, etc. all the way down to the number zero.

As used herein, all numerical values or ranges include fractions of the values and integers within such ranges and fractions of the integers within such ranges unless the context clearly indicates otherwise. Thus, to illustrate, reference to a numerical range, such as 2,000-40,000 includes 2,000; 3,000; 4, 000; 5,000, 6,000, etc. as well as 2,100; 3,100; 4,100; 5,100; 6,100; etc., and so forth. Reference to a range of 20-100 therefore includes 21, 22, 23, 24, 25, 26, 27, 28, 29, 30, 31, 32, 33, 34, 35, 36, 37, 38, 39, 40, etc., up to and including 100, as well as 21.1, 21.2, 21.3, 21.4, 21.5, etc., 22.1, 22.2, 22.3, 22.4, 22.5, etc., and so forth.

Units, prefixes, and symbols are denoted in their Systeme International de Unites (SI) accepted form. Numeric ranges are inclusive of the numbers defining the range. Unless otherwise indicated, amino acid sequences are written left to right in amino to carboxy orientation. The headings provided herein are not limitations of the various aspects of the disclosure, which can be had by reference to the specification as a whole. Accordingly, the terms defined immediately below are more fully defined by reference to the specification in its entirety.

Unless defined otherwise or dictated otherwise by context, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

## Detailed Description

It is a common hurdle in the field of virology and vaccination that viruses possess highly different and divergent biological and biochemical properties and therefore purification schemes must be established specifically for each virus particle. Virus genomes generally show a high degree of variability. In general, they can be composed of DNA, single or double stranded, or RNA, as plus or minus strand or ambisense, the genome can be linear, circular or segmented and there can be an envelope (composed of lipids and proteins) or not. Besides that, the genome size can vary a lot from about 1.7 kilobase (kb) (e.g. *Circoviridae* or Hepatitis delta virus) to about 2.5 megabase (Mb) (e.g. *Pandoravirus salinus*). With respect to purification, especially the size, diameter and chemical reactivity of the exposed surface of a virus particle, especially of the envelope, if present, are factors to be taken into consideration. The challenges in producing a virus particles-containing composition for pharmaceutical use not only imply the provision of a suitable vaccine vector and propagation strategies as detailed further below, but further demand the provision of improved methods for downstream processing of the particular virus material of interest to achieve a significant increase in purity of the resulting material without a loss in functional, i.e. immunogenic, virus particles.

Clinical batches of MV may be produced in Vero cells or other suitable cell lines known to the skilled person adapted to serum-free growth in cell factory multilayer vessels, resulting in 50% of the virus in the supernatant and 50% staying associated with the cells. The supernatant is clarified by filtration and treated with Benzonase to digest contaminating nucleic acids. MV particles are then concentrated and purified using ultrafiltration and diafiltration, followed by a final passing through a clarifying filter. Especially regarding purity considerations, vaccine candidates based on MV particles suffer from the drawback that they still contain a significant amount of impurities. Though the amounts might be acceptable for vaccines, OV therapy due to a high dose and the route of administration demand much higher purities and yet high yields.

To this end, the present invention provides for a method suitable for purifying MV particles, a system for carrying out said method as well as compositions that are suitable for being used in therapy and prophylaxis obtained thereby. Moreover, various uses of the compositions in therapy are disclosed.

Accordingly, in a first aspect of the present invention, there is provided a method for purifying MV particles from a sample, the method comprising in sequential order the following steps: (i) loading a sample containing MV particles and one or more impurities onto a stationary phase material for carrying out flow-through chromatography to bind at least a fraction of the one or more impurities contained in the sample and to produce a flow-through comprising at least a fraction of the MV particles contained in the sample; and (ii) carrying out filtration, preferably ultrafiltration, and obtaining a retentate having an increased MV titer relative to the MV titer comprised in the flow-through.

According to the inventive concept disclosed herein, a concentration step is carried out after a chromatography step, wherein the chromatography step is operated specifically in flow-through mode. Since samples such as cell culture supernatants originating from virus infected host cell usually contain virus particles in large dilution - which is all the more true when producing recombinant virus particles - it has been generally thought that for an economic process one of the very first steps of downstream processing has to be a volume reduction step (Nestola et al., *supra*). This sequence of steps combined with chromatography to be carried out specifically in flow-through mode as defined in the first aspect of the invention thus represents a complete departure from previous practice for purifying MV particles.

A big advantage of operating the chromatography in flow-through mode is that it avoids the drawback that large viruses such as MV can often not diffuse into the pores of commercially stationary phase materials formed into a packed bed or as monoliths (e.g., for a bind-elute modus of operation). Purification therefore will lead to low recoveries, when the chromatography step is carried out in conventional bind-elute mode. Further elucidation of this observation lead to the hypothesis that binding of MV particles to the stationary phase material increases back pressure to an unacceptable extent. MV must be treated as >1 μm particles that are extremely shear sensitive, in order to maximize recoveries and retain infectivity. Without being bound by theory, it is thought that the large size of MV particles, which, when bound to a stationary phase material contained in a column, strongly increase the backpressure, even when the sample has not been concentrated before.

Due to viscosity increase, this situation would be further complicated, when the sample was concentrated as a very first step of downstream processing, i.e. before chromatography. In fact, known purification protocols of MV particles, to the best of the inventor's knowledge, do not comprise a packed bed chromatography step at all. This is a severe draw-back, in particular in the context of providing highly pure and concentrated compositions for OV therapy, since chromatography is a potent purification technique, generally allowing high recoveries and high purity.

These facts and advantages of the strategy of the present invention may be further explained by the following general formula illustrating the importance of viscosity consideration in fluid mechanics and its influence on the shear stress:

$$\tau = \eta \times \dot{\gamma}$$

wherein $\tau$ is the shear stress, $\dot{\gamma}$ is the shear rate, and $\eta$ is the apparent viscosity (constant only for Newtonian fluids). In fluid mechanics, for a Newtonian fluid, the shear stress, $\tau$, on a unit area moving parallel to itself, is generally found to be proportional to the rate of change of velocity with distance perpendicular to the unit area (see Bird et al., Transport Phenomena, Wiley, 1960).

In a column, the back pressure of the column and the shear stress are thus only correlated by the viscosity of the fluid. For example, during elution in a bind-elute mode, viscosity (and in turn the shear stress) will thus increase, as concentration increases (particularly when using monolithic columns, as packed bed settings will likely not increase that high concentrations).

In comparison to the method disclosed in WO 2017/109211, higher capacities are achieved by the flow-through chromatography step comprised in the method of the invention than in a monolithic column configuration operated in bind-elute mode. It was observed that the more virus was bound to the monolith column, the stronger the pressure increased. In some cases, the pressure increased to such extent that a "bypass" was formed in the column leading to (1) breakthrough of viruses (the theoretical capacity of the column cannot be used) and (2) widening of elution peaks, i.e. dilution of the product. In contrast, when the chromatography step is operated in flow-through mode, impurities selectively bind to the stationary phase material, while the MV particles run there through. This mode thus allows influencing viscosity and thus shear stress in a highly favorable manner during the whole chromatography step. These conditions in turn are favorable for both the purity and the infectivity of the MV particles.

Another advantage is that the various steps comprised by the method of the present invention can be carried out aseptically under GMP conditions throughout the process resulting in a purified and concentrated virus preparation that does not require sterile filtration. In the production of pharmaceutical compositions, such as vaccine compositions, for the administration to subjects, sterility and safety of the composition must be ensured. Sterility of such compositions is typically achieved by means of sterile filtration, i.e., though a 0.2 $\mu$m filter membrane, prior to administration. For compositions comprising relatively large viruses (i.e., approximately 100 nm or larger), sterile filtration may result in a substantial loss of virus due to it being retained on the membrane, reducing the viral yield from the purification process. Additionally, some viruses, such as MV are sensitive to shear stress, which pose high requirements to a purification process. Since each step of the purification process must in this case be performed under gentle conditions, alternative sterilization methods are rendered useless. Hence, it is preferred that the unit operations of the method and the various embodiments of the invention are carried out aseptically.

As a further advantage, the various steps comprised by the method of the present invention of the invention allow to be integrated and carried out continuously. In a real continuous process, a product is manufactured un-interrupted in time. In theory the mass flow is constant over time. Process disturbance change the mass flow over time, but to such an extent the process is still considered fully continuous. In a quasi or pseudo continuous process, unit operations are operated in a cyclic manner. The mass flow changes/fluctuates over time. In an integrated process the outflow of one unit operation is directly fed in the next one without any hold step in between. Sometime surge tanks are put between unit operations to compensate for different flow rates. Further advantages include relatively high flow rates and thus rapid processing via flow-through chromatography.

Suitable buffers for use in the flow-through chromatography step include HEPES, Sodium phosphate, Tris, Triethanolamine, BICINE, bis-Tris, or diethanolamine, preferably HEPES, or other comparable buffer systems known to the skilled person. Depending on the functionality of a stationary phase material used in the flow-through chromatography unit, the pH value and conductivity is set so that the at least one impurity bind to the stationary phase material, whereas the MV particles do not in order to allow separation. If the stationary phase material has/have size-exclusion functionality and/or hydrophobic interaction functionality, the pH may range between pH 6.0 and 9.0, preferably between pH 7.0 and 8.0. In the context of using Capto Core 700 stationary phase material, the load, i.e. the MV particles-containing sample subjected to flow-through chromatography, comprises HEPES (or another of the above cited buffers or buffer systems based thereon), preferably in the range of 10 to 200 mM, more preferably in the range of 10 to 150 mM and most preferably in the range of 20 to 100 mM, and optionally NaCl, which is preferably present in an amount of 20 to 300 mM, more preferably in an amount of 50 to 250 mM and most preferably in an amount of 100 to 200 mM. The pH value in this case is preferably between pH 6.0 and 9.0, more preferably between pH 6.5 and 8.5 and most preferably between pH 7.0 and 8.0.

Impurities arising from virus particle manufacturing processes include process related impurities and product related impurities, in particular including, but not restricted to, host cells, cell debris, host cell proteins, or lipids, polysaccharides, and nucleic acids stemming from the host or virus particles and virus proteins, protein contaminants, either resulting from cell culture additives or from enzymes added during cultivation and processing, e.g. bovine serum albumin, if no serum-free approach is chosen, or endonucleases added for nucleic acid digestion, microcarriers used for host cell cultivation, or foreign nucleic acids neither belonging to the host cell nor the MV particles of interest. Preferably the at least one impurity is selected from the group consisting of host cell proteins, nucleic acids and in particular both host cell proteins and nucleic acids.

To obtain a retentate having an increased MV titer relative to the MV titer comprised in the flow-through, the MV particles are retained by the filter, while the liquid, preferably along with further impurities, is allowed to pass through the filter. Therefore, in the context of the present invention, step (b) defines a filtration method, wherein the product of interest is retained in the retentate while liquid, and preferably impurities, are allowed to cross to the permeate side.

An advantage of the present inventive concept is that method according to the present invention is suited for integrated manufacturing and/or quasi, semi or fully continuous processing. This is of great importance for large virus particles such as MV because the product cannot be subjected to a sterile filtration before filling. A fully continuous process manufactures the product un-interrupted in time. In theory the mass flow is constant over time. Process disturbance change the mass flow over time, but in such the process is still considered fully continuous. In a quasi or pseudo continuous process, unit operations are operated in a cyclic manner. The mass flow changes/fluctuates over time. In an integrated process the outflow of one unit operation is directly fed in the next one without any hold steps. Sometime surge tanks are put between unit operations, e.g. to compensate for differing flow rates occurring in two unit operations being in direct fluid communication.

Further advantages of carrying out filtration step (ii) after flow-through chromatography step (i) include the possibility to increase purity to a higher degree; and decrease filter area, as compared to a corresponding method, wherein filtration is carried out first. More specifically, it was observed that tangential flow filtration (TFF) carried out with clarified and endonuclease treated cell culture supernatant was strongly affected by rapid fouling of the membrane. This results in a lower capacity, a longer duration of the process and, hence, an inefficient process. These limitations could be completely avoided by carrying out filtration after flow-through chromatography step (i). It was further found that, in stark contrast to the method according to the first aspect of the present invention, purity could not be increased by TFF, if it was carried out with clarified and endonuclease treated cell culture supernatant. Moreover, the sequence of steps enables the concentration of MV particles in the retentate, whereas this is not achieved when carrying out TFF at the beginning of the downstream process. Last but not least, filtration step (ii) can be easily implemented with a buffer exchange step. Thereby, filtration step (ii) enables to provide MV particles in a final formulation and shorten the number of process steps.

A successful MV purification platform should further contain an upstream processing which is based on defined culture media with a virus titer and a downstream processing which is suited for integrated manufacturing, because the product cannot be subjected to a sterile filtration step.

In preferred embodiments of the present invention, there is thus provided a method according to the first aspect of the invention and the various embodiments thereof, wherein the sample is obtained by a method comprising one or more of the following steps: (a) infecting at least one host cell with a virus stock comprising at least one MV particle; (b) incubating the at least one host cell infected with the virus stock to allow virus production; (c) obtaining a cell culture supernatant containing MV particles and one or more impurities; (d) clarifying the cell culture supernatant to obtain a clarified cell culture supernatant. More specifically, the sample is preferably obtained by a method comprising at least step (c) obtaining a cell culture supernatant containing MV particles and one or more impurities. More preferably, the sample is obtained by a method comprising at least steps (c) obtaining a cell culture supernatant containing MV particles and one or more impurities and (d) clarifying the cell culture supernatant to obtain a clarified cell culture supernatant. Most preferably, the method by which the sample is obtained comprises all the steps (a) to (d) as defined above.

The at least one host cell shall be understood to refer to one host cells, preferably more than one host cells and most preferably a plurality of host cells. The at least one host cell, and preferably plurality of host cells, can be obtained by cultivating one or a population of host cells, for example originating from a cell bank, to a larger population of host cells. In preferred embodiments, the at least one host cell comprises or consists of cells selected from the group consisting of a variety of established vertebrate, preferably mammalian, or invertebrate cells lines, including Vero cells (African green monkey kidney cells), chicken embryo fibroblast cells, HEK293 cells, WI-38 cells, Madin Darby canine kidney (MDCK) cells, HeLa cells, BJAB (Human Burkitt's Lymphoma), Caco-2, CAL-148, hMSC-TERT (human bone marrow derived mesenchymal stem cell), MIA-PaCa-2, SCLC-21H, RMPI-8226, and MRC5 cells, preferably Vero or MCR-5 cells, but also designer cells for viral vaccine production, including, for example, PER.C6, CAP, AGE1.CR, AGE1.CR.pIX, EB66 cells, PBS-1, QOR/2E11, SogE, MFF-8C1 cells and the like, which are publicly available.

One of the key parameters in process design for anchorage dependent cells is the availability of a robust and scalable seed train. Using classical T-flasks puts a high work load to the operators of such a process and the usage of stirred formats such as spinner flasks and seed bioreactors in combination with microcarrier bear severe problems caused by bead-to-bead transfers. To overcome these problems, in a preferred embodiment of the present invention, a seed train that is fully based on static cultures using multi-layer flasks, preferably in different formats, is used to produce a plurality of host cells, which is then infected as defined above in step (a). In this context, the expression "fully based on static cultures" is to be understood as a culture, wherein the cells for inoculation are cells cultivated, e.g., in flasks, in a static culture in contrast to cells cultivated in a stirred system.

Due to the adherent nature of Vero cells a bioreactor usually needs to provide specific surfaces for the cells to grow on. Such surfaces can be provided by way of fiber-cell matrices or different types of microcarrier. Microcarrier have in the past been used for cell culture processes and they have been shown to be suitable for the cultivation of Vero cells even at large scales. In the context of the present invention, it is preferred that a microcarrier is employed in the production, and preferably also in the infection step (a) and/or incubation step (b), of the at least one host cell and preferably a plurality of host cells. By using a microcarrier cells readily attach thereto within 1 hour after inoculation and continue growing upon confluence. A specifically preferred microcarrier is comprises or consists of dextran beads, preferably having a particle size of 10 $\mu$m to 500 $\mu$m, preferably 20 $\mu$m to 300 $\mu$m, more preferably 30 $\mu$m to 200 $\mu$m and most preferably 50 $\mu$m to 100 $\mu$m. Such microcarriers are available under the trade name Cytodex® 1. The microcarriers are preferably contained in the bioreactor in an amount of 1 to 20 g/L, more preferably in an amount of 1.5 to 6 g/L and most preferably in an amount of 2 to 4 g/L. In a preferred embodiment, host cells are inoculated in a density of $1x10^3$ to $1x10^5$ cells/$cm^2$ microcarrier surface, preferably $2x10^3$ to $5x10^4$ cells/$cm^2$ microcarrier surface, more preferably $5x10^3$ to $2x10^4$ cells/$cm^2$ microcarrier surface and most preferably about $1x10^4$ cells/$cm^2$ microcarrier surface. Using lower numbers would be beneficial regarding seed-train steps but the number of microcarrier not covered with cells increases to a level that reduces the overall productivity of the process.

Step (a) involves infecting at least one host cell with a virus stock comprising at least one MV particle. In a preferred embodiment, the at least one host cell is infected with a Multiplicity Of Infection (MOI) of 0.0001 to 0.1, preferably 0.0002 to 0.005 and more preferably 0.0005 to 0.002 and most preferably 0.001 $\pm$ 50 %. As it is known to the skilled person, the MOI will have to be and can easily be determined for a host cell as well as a virus stock of interest. In order to preserve infectivity of the virus particles, temperature is preferably maintained at 32.5 $\pm$ 3°C, more preferably 32.5 $\pm$ 2°C and most preferably 32.5 $\pm$ 1°C.

Step (b), incubating the at least one host cell infected with the virus stock to allow virus production, typically involves cultivation of the at least one host cell under suitable conditions. For example, it / they can be grown under the same conditions, which were used for infection. In some cases, the at least one host cell undergoes autolysis. In this case, MV particles containing cell culture supernatant is directly obtained. In other cases, step (b) may comprise cell lysis, i.e. an active step, wherein the at least one host cell is lysed. This can be achieved by adding a chemical agent having cell lysis activity and/or exercising mechanical force such as high pressure on the at least one host cell.

The method according to the various embodiments of the invention, may further comprise: determining viable cell density. Monitoring total cell density is generally a reliable method for measuring cell growth. The most relevant information is obtained during the lag and growth phase before significant cell death occurs. When virus particles are produced using host cells attached to a microcarrier, it is however difficult to determine cell growth on-line. However, a lack of on-line data limits the possibility to understand and control the process in detail. It has now been surprisingly found that a probe can determine viable cell density based on permittivity measurements, even when the cells are attached to the microcarrier.

Hence, in a preferred embodiment, the production of the at least one host cell, preferably a plurality of host cells and/or step (a) and/or (b) comprises determining viable cell density and optionally total cell density. The viable and/or cell density determination is preferably based on (an) on-line technique(s). An exemplarily, and herein preferred, technique for online determination of viable cell density is based on capacitance. In an alternating electrical field, viable cells behave like small capacitors. The charge from these small capacitors is measured by a sensor and reported as permittivity (capacitance per area). A big advantage of such determination is that it allows on-line determination in real-time and gives a user a deep insight on cell culture or fermentation. Moreover, it is insensitive to microcarriers and cell debris. The total cell density determination may be based on on-line measurements including turbidity and optical density measurement at NIR (Near Infra-Red) wavelengths, or any other suitable measurement, whether allowing on-line or off-line determination of total cell density.

Using data provided by the viable cell probe, it is possible to precisely determine the ideal infection and harvest time points. A great advantage of such a principle is that only live cells are measured, so that the measurement is deteriorated neither by cellular debris nor by the microcarrier. A decrease of the signal thus correlates with a decrease in the total amount of viable cells in the system. Under the prerequisite that all process parameters are within defined specifications, the first decrease of permittivity signals in the course of infection can therefore be defined as ideal point of infection. Similar considerations can be made for harvesting the culture. If the signal falls below a certain threshold most of the cells are dead and the culture is ready for further processing. Predicting the ideal harvest time point is crucial for further downstream processing regarding highest possible yields and lowest possible contaminations due to lysed cells.

Step (c) obtaining a cell culture supernatant containing MV particles and one or more impurities may follow a procedure, wherein the cell culture supernatant is recovered. In such procedure, the microcarriers may be let to settle down, for example for at least 15 min, before withdrawing the cell culture supernatant. The cell culture supernatant may then be subjected to step (i) as defined above, or to one of the following steps (d) or (e). Optionally, the use of a harvestainer for separation of microcarrier beads may be employed. In certain embodiments, microcarrier free culture may be preferred depending on a host cell of interest.

For the purpose of the present invention, the bulk product consists of the virus particles produced in and released from a plurality of host cells (i.e. a cell population) or from a host cell. Clarification aims at removing cells and cell debris from the host cells infected with and producing a virus. Clarification usually does not include separation of impurities that have a size corresponding to the virus particles to be purified or smaller. Common methods for clarification are centrifugation and microfiltration, including tangential flow filtration (TFF), ultracentrifugation, dead end filtering and the like. In the context of clarifying a bulk product comprising MV particles centrifugation is less desired, as centrifugation processes are not easily scalable under GMP conditions.

Step (d), clarifying the cell culture supernatant to obtain a clarified cell culture supernatant, is therefore preferably accomplished by a method other than centrifugation, more preferably by means of dead end filtration, including *inter alia* depth filtration and/or membrane filtration, and most preferably by depth filtration. A single filter may be used, or a filter cascade comprising two or more filters connected in series may be employed for the purpose of the present invention. A preferred embodiment can rely on a single filter of 3 μm. In another embodiment in the context of the present invention, a filter cascade is used comprising or consisting of a first and a second filter, wherein the first filter has preferably a nominal pore size of 10 to 200 μm, more preferably 20 to 150 μm, even more preferably 30 to 120 μm and most preferably 40 to 100 μm. The second filter has preferably a nominal pore size of 0.5 to 20 μm, more preferably 0.8 to 10 μm and most preferably 1 to 5 μm. In a particular embodiment, the first filter has a nominal pore size of 40 to 100 μm and the second filter has a nominal pore size of 1 to 5 μm. In another particular embodiment, a filter cascade comprising or consisting of a first 50 μM filter and a second 3 μM filter is used. It is to be understood that the second filter comes after the first filter, i.e. is arranged downstream of the first filter, and preferably that the first and the second filter are in direct fluid communication with each other. Single filter embodiments may be preferred for practical reasons (economic concerns, yield etc.).

Any other method known to the skilled person and suitable to perform a crude separation of the host cellular material and the supernatant comprising the virus particles of interest are also comprised by the term "clarifying" or "clarification". As discussed herein, TFF suffers from certain drawbacks when performed at an early stage of the process. Hence, in preferred embodiments, clarification does not comprise TFF and/or a volume reduction step associated with an increase of viscosity.

To reduce or eliminate contaminating nucleic acids, the cell culture supernatant obtained in step (c) as defined above, or the clarified cell culture supernatant obtained in step (d) as defined above, can be treated with an agent having nucleic acid digesting activity. Accordingly, in a further embodiment of the present invention, the method additionally includes step (e) treating the cell culture supernatant obtained in step (c) as defined above, or the clarified cell culture supernatant obtained in step (d) as defined above, with an agent having nucleic acid digesting activity. The agent is preferably an endonuclease such as Benzonase. Benzonase is a genetically engineered endonuclease from *Serratia marcescens.* The enzyme is a dimer of 30 kDa subunits with two essential disulfide bonds. This endonuclease attacks and degrades all forms of DNA and RNA (single stranded, double stranded, linear and circular) and is effective over a wide range of operating conditions. The optimum pH for enzyme activity is found to be 8.0-9.2. It completely digests nucleic acids to 5'- monophosphate terminated oligonucleotides 3 to 5 bases in length. This is ideal for removal of nucleic acids for applications where complete digestion of nucleic acids is desirable. It also reduces viscosity in protein extracts and prevents cell clumping. Treatment of the (clarified) cell culture supernatant is preferably carried out by Benzonase in the presence of a magnesium source such as magnesium chloride.

After addition of the agent having nucleic acid digesting activity to the cell culture supernatant obtained in step (c) as defined above, or the clarified cell culture supernatant obtained in step (d) as defined above, it can be incubated under conditions which allow for at least 50 % removal of nucleic acids, preferably at least 60 % removal of nucleic acids, more preferably at least 70 % removal of nucleic acids, even more preferably at least 80 % removal of nucleic acids and most preferably at least 90 % removal of nucleic acids. The term "removal of nucleic acids" as used herein means that the nucleic acids are digested to 5'- monophosphate terminated oligonucleotides less than 5 bases in length. Typical conditions include incubation at 20°C to 37°C for up to 24 hours. The higher the temperature the shorter is usually the incubation time provided that the same amount of agent having nucleic acid digesting activity is used. For example, the sample can be incubated at 20 to 25 °C for about 12 to 24 hours, or at 30 to 37°C for about 0.5 to 4 h. Afterwards, the treated cell culture supernatant can be further processed, e.g. by directly loading onto the stationary phase material for carrying out flow-through chromatography.

According to a specific embodiment, the cell culture supernatant obtained in step (c) as defined above, or the clarified cell culture supernatant obtained in step (d), is incubated for 1 h at 37.0 ± 1.0 °C and preferably mixed. The required amount of Benzonase and magnesium chloride to obtain a final concentration of 50 U/mL Benzonase and 2 mM magnesium chloride (as a cofactor) are mixed together into a homogenous solution and then aseptically added to the (clarified) cell culture supernatant obtained in step (c) or (d), and the solution is preferably mixed. The (clarified) cell culture supernatant is then incubated at 37.0 ± 1.0 °C, 50 rpm for 1-2 hours. Afterwards, the (clarified) cell culture supernatant is removed from the incubator. To stop or at least slow down the endonuclease, preferably the Benzonase, EDTA may be added, preferably in an amount to obtain a final concentration of 5 mM EDTA.

If both steps (d) and (e) are encompassed by the method of the present invention, they can be operated either in consecutive order, (d) → (e), or vice versa, (e)→(d). After the sample has been obtained by a method that comprises at least step (c) as defined above, or at least steps (c) and (d) as defined above, or at least steps (c), (d) and (e) as defined above, the sample is, preferably directly, subjected to the flow-through chromatography step (i) as defined above.

The method according to the various embodiments of the invention, may further comprise: determining the concentration of contaminating nucleic acids and/or the amount of total protein and/or the amount of host cell proteins and/or virus titer, e.g. of the load and/or the flow-through and/or the retentate. In certain embodiments, the method may alternatively, or additionally, comprise determining viscosity, in particular of the load.

Methods to determine the concentration of contaminating nucleic acids such as host cell DNA are known to the person having skill in the art. A standard method for detecting small amounts of contaminating DNA in a sample is quantitative real-time PCR (qPCR or qRT PCR) (e.g. PicoGreen® assay (Life Technologies)). Another method for detecting contaminating DNA or proteins in a sample of interest are threshold DNA assays (e.g. Threshold® Immunoligand Assay (ILA) or Threshold® Total DNA Assay Molecular Devices). Said methods both show a high sensitivity and a good detection limit in the pictogram range and are readily available to the skilled person.

Likewise, methods for performing quantification of total protein, or of specific proteins contained as contaminants in a sample or a composition comprising the purified virus particles can be quantified by methods including a BCA (bicinchoninic acid) assay or a Vero cell host cell protein (HCP) ELISA assay (Cygnus Technologies, current detection limit as declared by the manufacturer: 700 pg/mL) or other enzyme and/or fluorescence based methods. Said methods are readily available to the skilled person. The various assays, which can be used for virus titer quantification include, but are not limited to, plaque assays, endpoint dilution assays, protein assays and transmission electron microscopy.

Plaque-based assays are the standard method used to determine virus concentration in terms of infectious dose. Viral plaque assays determine the number of plaque forming units (pfu) in a virus sample, which is one measure of virus quantity. Endpoint dilution assays report 50% Tissue culture Infective Dose (TCID50) as measure of infectious virus titer. The endpoint dilution assay quantifies the amount of virus required to kill 50% of infected hosts or to produce a cytopathic effect in 50% of inoculated tissue culture cells. An alternative method for determining the TCID is quantitative PCR (qPCR). The $TCID_{50}$ as used herein refers to median tissue culture infective dose as defined above. When reference is made herein to specific values of viral titers these are preferably determined by endpoint dilution assay, e.g. on Vero cells, and $TCID_{50}$ is be calculated by using the Kärber method.

In a further embodiment of the present invention, there is provided a method according to the first aspect of the invention and the various embodiments thereof, wherein the sample loaded onto the flow-through chromatography unit has a MV titer less than 5 times higher, preferably less than 4 times higher, more preferably less than 3 times higher, even more preferably less than 2 times higher and most preferably less than 1.5 times higher, than the MV titer in the cell culture supernatant obtained in step (c) as defined above or in the clarified cell culture supernatant obtained in step (d) as defined above. In some embodiments, this may mean that there occurs no concentration step between said step (c), or said step (d), and said step flow-through chromatography step (i) as defined further above, or at least no concentration step that increases MV titer 5-fold or more, preferably 4-fold or more, more preferably 3-fold or more, even more preferably 2-fold or more and most preferably 1.5-fold or more. In some embodiments, there occurs no volume reduction step between said step (c), or said step (d), and said step flow-through chromatography step (i) as defined further above, or at least no volume reduction step that decreases the volume by 5-fold or more, preferably 4-fold or more, more preferably 3-fold or more, even more preferably 2-fold or more and most preferably 1.5-fold or more. In addition to or alternatively to the preceding embodiment, the sample loaded onto the flow-through chromatography unit has a host cell protein content of at least 50 %, preferably at least 60 %, more preferably at least 70 %, even more preferably at least 80 % and most preferably at least 85 % relative to the host cell protein content in the cell culture supernatant obtained in step (c) or in the clarified cell culture supernatant obtained in step (d). In some embodiments, this may mean that the cell culture supernatant obtained in step (c) or the clarified cell culture supernatant obtained in step (d) is directly loaded onto the flow-through chromatography unit. In some embodiments, there is no host cell removal step arranged between said step (c), or said step (d), and flow-through chromatography step (i).

Advantageously, the method according to the first aspect of the present invention is not based on a concentration step at an early step of downstream processing. In fact, the best results in terms of yield (recovery) and purity are achieved if no ultrafiltration step is carried out before the flow-through chromatography in order to keep viscosity low and thus avoid a potentially harmful pressure increase during chromatography.

In accordance with this observation, a further embodiment of the present invention provides a method according to the first aspect of the invention and the various embodiments thereof, wherein the sample containing MV particles as defined in the method of the first aspect of the present invention, or the cell culture supernatant obtained in step (c) as defined above, or the clarified cell culture supernatant obtained in step (d) as defined above, or the treated cell culture supernatant obtained in step (e) as defined above, is directly loaded onto the stationary phase material for carrying out flow-through chromatography and/or wherein no concentration step and/or no buffer exchange step and/or no host cell protein removal step occurs in between. In some embodiments, no concentration step occurs between the step said step (c), or (d), or (e), and flow-through chromatography step (i). In some embodiments, no buffer exchange step occurs between the step said step (c), or (d), or (e) and flow-through chromatography step (i). In some embodiments, no host cell protein removal step occurs between the step said step (c), or (d), or (e), and flow-through chromatography step (i).

As outlined above, operating the flow-through chromatography in flow-through mode advantageously allows a high recovery of MV particles. Furthermore, impurities, preferably host cell proteins and/or nucleic acids can be significantly reduced to a low level. Hence, in a further embodiment of the present invention, there is provided a method according to the first aspect of the invention and the various embodiments thereof, wherein the MV titer in the flow-through is at least 20 %, preferably at least 30 %, more preferably at least 40 % and most preferably at least 50 %, or even at least 70 %, preferably at least 80 % and most preferably at least 90 %, of the MV titer in the sample loaded onto the flow-through chromatography unit; and/or wherein the host cell protein content in the flow-through is 60 % or less, preferably 50 % or less, more preferably 40 % or less and most preferably 30 % or less, or even 25 % or less and preferably 20 % or less, relative to the host cell protein content in the sample loaded onto the flow-through chromatography unit; and/or wherein the polynucleotide content in the flow-through is 70 % or less, preferably 60 % or less, more preferably 50 % or less and most preferably 60 % or less, relative to the polynucleotide content in the sample loaded onto the flow-through chromatography unit.

The finding that column chromatography, in particular packed bed column chromatography, is suited for the purification of MV particles was unexpected in view of the difficulties encountered when working with MV, which difficulties arises from the fact that MV is huge and fragile. In accordance with this finding, in a further embodiment of the present invention, there is provided a method according to the first aspect of the invention and the various embodiments thereof, wherein the flow-through chromatography step involves column chromatography. The stationary phase material is preferably selected from the group consisting of at least a resin, at least a matrix, at least a gel and, preferably, beads.

It is further preferred that the stationary phase material, preferably the beads, has/have at least size-exclusion functionality, hydrophobic interaction functionality or ion exchange functionality, or a combination thereof, preferably at least size-exclusion functionality, optionally combined with hydrophobic interaction functionality. Preferred beads may be core beads. These beads do not carry ligands at the outside/surface of the beads, but only in the inside. Therefore, substances not small enough to enter the core, will not be able to interact in a specific manner with the ligands. In contrast to gel filtration or size exclusion, where the separation is exclusively based on the size of the substances to be purified (which can only be directed by the flow rate), core beads in general have the advantage that higher flow rates can be used (small substances can easily enter the cores with the specific ligand, large molecules will rapidly be eluted without clogging the entry into the core for small substances). Another advantage of core beads in general (independent of the specific ligand used in the core) is the fact that much higher loading volumes can be used in comparison to size-exclusion/gelfiltration formats, where only a very small percentage of the actual column volume can be loaded onto the column as sample to be purified. Therefore, a stationary phase material in general should have an inert surface and very small pores to be suitable for a flow through approach according to the present disclosure.

With respect to the physical structure, materials with extremely large pores may be suitable for certain embodiments so that the MV can pass and bind, as it is the case for membrane adsorber, monoliths or fibers, but a process will specifically have to be configured for each new material. Notably, conventional chromatographic beads will suffer the huge disadvantage that the largest pores presently available (macroporous resins) have a pore size of around 400 nm. This will not suffice to allow entry of a MV during column purification simply as the MV or MV structure cannot diffuse into the beads.

The bind-elute mode in contrast to the flow-through mode) also requires that a bound structure, e.g., a virus, cannot only be bound tightly and specifically, but it can also be eluted later on under physiological conditions. This often represents a huge problem in bind-elute settings. In particular, the elution causes huge problems for MV, as it is a large virus having many binding sites making strong interactions with the material. Harsh elution conditions, however, will influence the yield of intact MV. Furthermore, the elution can result in pressure problems, as the increased concentration results in a higher viscosity and thus in a higher shear stress when leaving the flow rate constant.

All these factors might explain why certain membrane adsorber materials, monolithic materials and Eshmuno gels (see below in the Examples section) can likely cause problems when purifying large and pleomorphic MV in high yields and simultaneously with high purities and economically favorable high flow rates in a flow-through mode without further adaptions.

In a further embodiment of the present invention, there is provided a method according to the first aspect of the invention and the various embodiments thereof, wherein the stationary phase material, preferably the beads, comprises/comprise a ligand-activated core, and an inactive shell comprising pores. The pores comprised in the inactive shell have a molecular weight cut off smaller than the MV particles to exclude the MV particles from entering the ligand-activated core, whereas a molecule smaller than the molecular weight cut off can enter the pores and bind to the ligand-activated core. Preferably, the ligand-activated core comprises octylamine. With regard to the pores, it is preferred that the molecular weight cut off is in the range of 100 kDa to 2,000 kDa, preferably 200 kDa to 1,500 kDa, more preferably 400 kDa to 1,200 kDa and most preferably 500 kDa to 1,000 kDa.

According to a specifically preferred embodiment, the flow-through chromatography step (i) involves a column chromatography using Capto Core 700 stationary phase material. Capto Core 700 is composed of a ligand-activated core and inactive shell. The inactive shell excludes large molecules (cut-off ~ 700 kDa) from entering the core through the pores of the shell. These larger molecules are collected in the column flow-through while smaller impurities bind to the internalized ligands. The core bead technology and multimodal, octylamine ligand give Capto Core 700 dual functionality, namely size exclusion and binding properties. Notably, further cut-off ranges may be suitable for certain applications according to the present invention, wherein smaller cut-offs can be associated with a higher degree of impurities, whereas large cut-off ranges might complicate resin production.

The MV particle-containing flow-through can be directly used as a feed for filtration step (ii). Thus, in one embodiment of the present invention, there is provided a method according to the first aspect of the invention and the various embodiments thereof, wherein the flow-through is directly used as a feed for the filtration. In this case, there is no additional step arranged between flow-through chromatography step (i) and filtration step (ii). In another embodiment of the present invention, there is a further chromatographic step arranged between flow-through chromatography step (i) and filtration step (ii). In this case, the MV particles containing eluate of said further chromatographic step is directly used as a feed for the filtration. Regardless whether there is an additional step between flow-through chromatography step (i) and filtration step (ii), due to the inherent difficulties to establish efficient chromatography techniques for the purification of MV particles, the method of the invention preferably comprises the flow-through chromatography step of (i) as the only chromatography step in order to maximize yield (recovery).

In a preferred embodiment of the present invention, there is provided a method according to the first aspect of the invention and the various embodiments thereof, wherein simultaneously with or sequentially to the filtration the retentate buffer is exchanged. In other words, the retentate buffer in which the MV particles are contained is exchanged by adding a (fresh) buffer to the feed reservoir as the filtration, preferably ultrafiltration, progresses in a process called diafiltration.

In case filtration and buffer exchange occur simultaneously, this may mean that the retentate volume or mass is kept at a constant level by adding exchange buffer. In case filtration and buffer exchange occur sequentially, this may mean that the retentate volume or weight is reduced, e.g., to 10 % of the initial level, in a first step by (ultra-) filtration and, in a second step, exchange buffer is added at the same rate as permeate forms. This can be controlled by maintaining a constant retentate volume or weight. In some embodiments, the step of retentate buffer exchange comprises adding a solution comprising sorbitol, wherein the solution preferably comprises sorbitol in an amount of 1 - 20 weight-%, more preferably 2 - 15 weight-%, even more preferably 3 - 10 weight-% and most preferably 5 $\pm$ 2 weight-%. In some embodiments, the filtration and, if present, the buffer exchange is carried out using a filter comprising regenerated cellulose. Advantageously, such filters do not show significant binding to MV particles. In some embodiment, the filters have a Molecular Weight Cut-Off (MWCO) of 10 to 1,000 kDa, preferably 15 to 800 kDa, more preferably 20 to 500 kDa and most preferably 30 to 300 kDa.

Because MV particles tend to adsorb to hollow fiber module, filtration step (ii), i.e. virus concentration, and if present the buffer exchange step, preferably does not involve hollow fiber filtration. Instead, in a preferred embodiment, the filtration step (ii), and if present the buffer exchange step, involves an ultrafiltration (UF) membrane and/or an UF cassette. Suitable membranes comprise cellulose, in particular regenerated cellulose (RC) or stabilized cellulose (SC), highly cross-linked regenerated cellulose, polysulfone (PS), modified polyethersulfone (mPES), polyvinylidene fluoride (PVDF) or a combination thereof. In preferred embodiments, the UF membrane comprises RC or modified RC, more preferably a composite RC and most preferably a RC membrane casted onto a microporous polyethylene substrate. RC and modified RC have shown the best trade-off between unspecific binding to MV particles, mechanical strength, and resistance to cleaning procedures (chemical agents and temperature).

More specifically, ultrafiltration/diafiltration was operated by ÄKTA flux s (GE Healthcare, Uppsala, Sweden). 300 kDa composite regenerated cellulose (RC) cassettes (Pellicon® XL, Merck KGaA, Darmstadt, Germany), 300 kDa polysulfone hollow fiber (HF) modules (Start AXM Cartridge, GE Healthcare, Uppsala, Sweden) and 30 kDa stabilized cellulose (SC) based membranes (Hydrosart®, Sartorius, Göttingen, Germany) were tested. A membrane area of 50 $cm^2$ was tested and the membrane feed was kept constant at 110 $L/m^2$. The transmembrane pressure was kept constant throughout the process between 0.4 to 0.5 bar by adjusting a retentate pressure control valve. Membranes were pre-treated and flushed as described in the particular manufacturer's instructions before the experiments. Feed material was chromatographically pre-purified using Capto Core 700 and frozen material was thawed at 37°C. Throughout the filtration experiments samples were collected from the feed material (F), the concentrate (C) and the permeate (P) at stored at -80°C.

The MWCO of the UF membrane and/or cassettes preferably ranges between 5 and 1,000 kDa, preferably 10 to 700 kDa, more preferably 15 to 500 kDa, even more preferably 20 to 400 kDa and most preferably 30 to 300 kDa. Furthermore, in particular preferred embodiments, the UF membrane and/or cassettes are characterized by a MWCO of 300 kDa, in particular if a RC based UF membrane is used. In other preferred embodiments, the UF membrane and/or cassettes are characterized by a nominal MWCO of 30 kDa, in particular if a SC based UF membrane is used. In a yet further embodiment, filtration step (ii), and optionally the buffer exchange step, is carried out by means of at least two membranes and/or at least two cassettes, having different MWCO, e.g. one having 100 to 1,000 kDa and/or another having 10 to 100 kDa, preferably one having 150 to 700 kDa and/or another having 15 to 70 kDa, more preferably one having 200 to 500 kDa and/or another having 20 to 50 kDa and most preferably one having 300 kDa and/or another having 30 kDa.

In a further embodiment of the present invention, there is provided a method according to the first aspect of the invention and the various embodiments thereof, wherein the filtration and, if present, the retentate buffer exchange involves tangential flow filtration. The tangential flow filtration is preferably carried out at a transmembrane pressure in the range of 0.1 to 1 bar, preferably 0.2 to 0.8 bar, more preferably 0.3 to 0.6 bar and most preferably 0.4 to 0.5 bar. It is further preferred that the transmembrane pressure is maintained within said range throughout the filtration and, if present, the retentate buffer exchange.

It is to be understood that the various embodiments of the present invention can be used to purify VLPs based on MV. VLPs lack genetic information and are thus non-replicative. VLPs *per se* are thus non-infectious in the sense that they cannot replicate in a cell to give rise to new viral particles and thus to spread to further cells after a replicative cycle. Still, VLPs, after their assembly and based on the molecules exposed on their surface, can interact with a host cell, e.g. through surface receptors, or, after uptake and/or processing by an immune cell, e.g. an antigen-representing cell, epitopes or antigens comprised by a VLP can be presented or cross-presented by the immune cell to effector cells. By means of this interaction, VLPs can induce an immune response in an organism. This ability makes VLPs suitable structures for the provision of safe immunogenic or vaccine compositions. Hence, in further embodiments, the MV particles comprise, or essentially consist of, VLPs derived from MV. In case the sample to be subjected to flow-through chromatography step (i) comprises both virions and VLPs, the method may additionally comprise a further purification step preceding or following flow-through chromatography step (i), comprising: further purifying the virus particles comprised in the sample, respectively flow-through by means of at least one separation technique selected from the group consisting of filtration, centrifugation, tangential flow filtration, membrane filtration, purification with grafted media, aqueous two phase extraction, precipitation, buffer exchange, dialysis or chromatography, including size exclusion chromatography, e.g. for separating the virus particles into a fraction containing virions and another fraction containing virus-like particles (VLPs) (VLPs). VLPs possess relevant surface antigens, but cannot further be propagated in a host cell. This makes VLPs an interesting target for several applications in immunology.

In a further embodiment of the present invention, there is provided a method according to the first aspect of the invention and the various embodiments thereof, wherein the MV particles are selected from the group consisting of live, attenuated and inactivated virus particles, or a mixture thereof, and/or wherein the MV particles are recombinant and/or infectious particles, preferably infectious recombinant particles.

According to all aspects and embodiments of the present disclosure, the MV particles are preferably derived from an attenuated virus strain, preferably being selected from the group consisting of the Schwarz strain, the Zagreb strain, the AIK-C strain and the Moraten strain.

In a particular preferred embodiment, a MV particle is encoded by a nucleic acid molecule comprising all or parts of the antigenomic region of a MV, preferably including further recombinant enhancements. A suitable MV scaffold is the MV Schwarz strain pTM having a nucleic acid sequence as shown in SEQ ID NO:1. Further suitable MV scaffolds with ATUs as backbone structures are represented in SEQ ID NOs:2 and 3. The skilled person is well aware of the fact that a sequence having slightly varying deviations from the exemplary sequences presented herein, e.g., a sequence identity of at least 75%, 80%, 85%, 90%, 91%, 92%, 93%, 94%, 95%, 96%, 97%, 98%, or 99% (or even less, e.g., in the case that a sequence is codon optimized for use in a specific host cell) may still be suitable for the purpose of the present invention as long as there is no mutation at a position encoding an essential amino acid or the like, or that no stop codon is introduced, as long as the encoded sequence still encodes all relevant features of a MV-based construct and optionally for an insert of interest.

Moreover, the MV particles herein may comprise (exogenous) genetic material for expressing one or more (foreign) antigens. As such, a platform is provided that is based on the well-established MV vector in order to expose a subject to any antigen of interest. This design enables any antigen to be generated in vivo in infected cells, in particular in infected cells of a mammalian, and thus to elicit an immune response and/or provide efficient and long-lasting immunity, especially which induces life-long immunity after only a single or two administration steps. Accordingly, in preferred embodiments of the various aspects of the present invention, the MV particles comprise, or essentially consist of, recombinant and preferably infectious virus particles derived from and/or comprising a MV scaffold, the scaffold being encoded by at least one nucleic acid sequence, wherein the nucleic acid sequence comprises a first nucleic acid sequence encoding a MV scaffold and at least one second nucleic acid sequence operably linked to the first nucleic acid sequence. The at least one second nucleic acid sequence may encode an antigen, preferably at least one foreign virus antigen, in particular if the virus particles are intended to be used in a vaccine, in particular against infection with said virus(es), which naturally comprise(s) said at least one foreign virus antigen. The at least one second nucleic acid sequence may also encode a protein or a regulatory RNA other than a viral antigen, for example a micro RNA (miRNA), assisting tumor treatment, preferably in a mammalian, or more particularly in a human subject, in particular in case the MV particles are for use in OV therapy. The protein or RNA encoded by the second nucleic acid may for example mediate the interaction between a MV particle and a tumor cell and/or its uptake into a tumor cell. In further embodiments, the second nucleic acid sequence may encode a gene toxic for a tumor cell of interest, e.g. a suicide gene comprising a fusion of a cytosine deaminase, particularly yeast cytosine deaminase, and an uracil phosphoribosyltransferase, particularly yeast uracil phosphoribosyltransferase. In yet further embodiments, the second nucleic acid sequence encodes a protein/RNA that enhances antitumor cytotoxicity and immunity. Furthermore, the second non-viral nucleic acid can be configured to promote a strong anti-tumor immune response, e.g. by activating antigen presenting cells, preferably dendritic cells, for example plasmacytoid dendritic cells, by activating their ability to produce high quantities of IFN-$\alpha$ and/or to cross-present tumor antigens from infected tumor cells to tumor-specific CD8+ T lymphocytes to achieve a strong cellular immune response against the tumor cells or tissue. "Cross-presentation" or "cross-presenting" in this context means the ability of certain antigen-presenting cells to take up, process and present extracellular antigens with MHC class I molecules to CD8+ T cells (cytotoxic T cells). Cross-priming, the result of this process, describes the stimulation of the naïve cytotoxic CD8+ T cell. This process is necessary for immunity against most tumors and against viruses that do not readily infect antigen-presenting cells or impair dendritic cell normal function. It is also required for induction of cytotoxic immunity by vaccination with protein antigens, for example, tumor vaccination.

In accordance with this embodiment, the nucleic acid construct encoding a recombinant infectious virus particles comprising an infectious MV (MV) scaffold thus comprises the following gene transcription units encompassing from 5' to 3': (a) a polynucleotide encoding the N protein of a MV, (b) a polynucleotide encoding the P protein of a MV, (c) the polynucleotide encoding at least one structural protein used as antigen, for example at least one Chikungunya structural protein, suitable as antigen (d) a polynucleotide encoding the M protein of a MV, (e) a polynucleotide encoding the F protein of a MV, (f) a polynucleotide encoding the H protein of a MV, and (g) a polynucleotide encoding the L protein of a MV, said polynucleotides and nucleic acid construct being operably linked and under the control of viral replication and transcription regulatory sequences such as MV leader and trailer sequences. The expressions "N protein", "P protein", "M protein", "F protein", "H protein" and "L protein" refer respectively to the nucleoprotein (N), the phosphoprotein (P), the matrix protein (M), the fusion protein (F), the hemagglutinin protein (H) and the RNA polymerase large protein (L) of a MV. The expression "operably linked" refers to the functional link existing between the at least one antigen encoding nucleic acid sequence according to the methods of the invention such that said at least one nucleic acid sequence within the MV scaffold is efficiently transcribed and translated, in particular in cells or cell lines, especially in cells or cell lines used as cell bank according to the present invention so that an antigenic epitope can be presented after.

The nucleic acid sequence encoding at least one antigen is preferably selected from the group consisting of a nucleic acid sequence derived from a virus belonging to the family of *Flaviviridae,* including a nucleic acid sequence derived from a West-Nile virus (cf. NCBI reference sequence NC_009942.1), a tick-borne encephalitis virus (NCBI reference sequence NC_001672.1), a Japanese encephalitis virus (NCBI reference sequence NC_001437.1), a yellow fever virus (NCBI reference sequence NC_002031.1), a Zika virus (NCBI reference sequence NC_012532.1), or a Dengue virus (e.g. NCBI Dengue virus 1/strain Nauru/West Pac/1974: NC_001477.1), a Chikungunya virus, a norovirus (e.g. Norwalk virus, NCBI NC_001959.2), a virus belonging to the family of *Paramyxoviridae,* including a nucleic acid sequence derived from a human respiratory syncytical virus (RSV) (e.g. NCBI: NC_001781.1), a MV or a metapneumovirus (e.g. human: NCBI Gene ID: 2830349; avian: NCBI Gene ID: 5130032), a parvovirus (e.g. human parvovirus B19, NCBI: NC_001348.1), a coronavirus, including a nucleic acid sequence derived from a Middle East respiratory syndrome antigen (see e.g. NCBI: NC_019843.3), or a severe acute respiratory syndrome antigen (e.g. NCBI: NC_004718.3), a human enterovirus 71 (e.g. enterovirus A, NCBI: NC_001612.1), a cytomegalovirus (e.g. human herpesvirus 5, NCBI: NC_006273.2), a poliovirus (e.g. human enterovirus C serotype PV-1, NCBI: NC_002058.3, an Epstein-Barr virus (e.g. human herpesvirus 4, NCBI: NC_009334.1 or NC_007605.1, respectively), a hepatitis E virus (e.g. NCBI: NC_001434.1), a human papilloma virus , preferably a human papilloma virus 16, a human papilloma virus 5, a human papilloma virus 4, a human papilloma virus 1 or a human papilloma virus 41 (see e.g. NCBI: HPV-16: NC_001526.2; HPV-5: NC_001531.1; HPV-4: NC_001457.1; HPV-1: NC_001356.1; HPV-41: NC_001354.1), or a varicella zoster virus (e.g. human herpesvirus 3, NCBI: NC_001348.1).

Further details of suitable nucleic acid constructs encoding MV particles are for example disclosed in EP 2 712 871 A1 and EP 3 184 119 A1, which are herein incorporated by reference, in particular for the purpose of preparation and design of nucleic acid constructs comprising genetic information of MV (scaffold) and one more foreign antigens, vectors comprising said nucleic acid constructs as well as the resulting recombinant infectious (replicating) MV particles prepared from said vectors.

From a structural point of view, the MV scaffold represents the majority of material to be transcribed/translated into a virus particle. As the purification and/or production method according to the various embodiments according to the first aspect of the present invention has been specifically designed to produce and/or purify MV particles by relying on physicochemical properties of the MV scaffold comprised as structural element by the MV particles, any foreign antigen and in particular any of the above antigens operably linked to the MV scaffold can thus be provided by the present invention in high yields and high purity. Hence, also virus particles derived from a MV scaffold comprising other antigens than those disclosed herein can be purified according to the methods of the present invention. The broad applicability of the method according to the present invention are elucidated by the fact that despite different antigens can be expressed, as a common feature the same or similar scaffold structure needs to be produced and/or purified.

Further examples for a nucleic acid sequence encoding a MV particle according to the disclosure of the present invention, which can be produced and/or purified according to the methods of the present invention, comprise a nucleic acid sequence according to SEQ ID NO:4 (MV GFP or MV-GFP with green fluorescent protein encoding insert), SEQ ID NO:5 (MV-Chikungunya, MV-CHIK), SEQ ID NO:6 (MV-Dengue or MV DVAX1) or SEQ ID NO:7 (MV Zika sE (soluble E-protein)) or, or a homologous sequence having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity thereto provided that the homologous sequence after transcription and optionally translation in a cell population or host cell still results in a MV scaffold optionally operably linked to at least one antigen of at least one virus, which is infectious and/or immunogenic, but does not comprise a relevant mutation in a region of the MV genome, which would disturb its natural replication cycle, or which would revert the attenuated MV scaffold back into a non-attenuated virus form. Said sequence homology range is thus caused by the fact that a MV scaffold can, by means of recombinant technology, comprise codon optimized positions, further regulatory or antigen positions and the like. Said modifications would, however, not significantly alter the physical and chemical properties of the MV particle, which properties are responsible for production and/or purification by the method of the invention.

Moreover, the MV particles may alternatively, or additionally to the aforementioned antigens, comprise (exogenous) genetic material for expressing one or more (foreign) proteins. Suitable genetic material may include marker genes, whose expression can be easily determined, e.g. via detection of electromagnetic radiation. Therefore, the at least one second nucleic acid sequence operably linked to the first nucleic acid sequence may encode a protein having properties that allow its easy determination, e.g. via detection of electromagnetic radiation, and/or being capable of emitting electromagnetic radiation. In particularly preferred embodiments of the present invention, the at least one second nucleic acid sequence encodes for a fluorescent protein such as Green Fluorescence Protein (GFP). An exemplary nucleic acid sequence encoding for the corresponding MV particle (MV GFP) is shown in SEQ ID NO:4.

Furthermore, according to any aspect, and any embodiment thereof, of the present invention, the disclosed nucleic acid molecules can be further modified by means of molecular biology to introduce a new or a modified regulatory sequence, restriction enzyme binding/cutting site as well as various nucleic acid sequences encoding an antigenic region of interest, preferably respecting the above identified "rule of six". This rule was established for certain viruses belonging to the *Paramyxoviridae* family where the MV scaffold of the present disclosure phylogenetically is derived from / belongs to. This rule is thus derived from the fact that in order for the entire process of RNA synthesis, genome replication and encapsidation which the MV proceeds through in a host cell to be efficient at generating full-length genomic and antigenomic molecules it is necessary that the viral genome is enclosed within its protein coat, specifically the N proteins. Without this, the virus replication machinery will find problems to begin replication. Each N molecule associates with exactly 6 nucleotides, which explains the reason as to why these viruses require their genomes to be a multiple of six. It is thus evident that a variety of modifications of the MV scaffold can be undertaken with the proviso that it still results in a MV scaffold able to infect a host cell. Therefore, means like codon optimization and the like can be applied as long as no mutation introduced which would change the functional properties of a regulatory sequence or a structural protein of the MV. Codon optimization implies that the codon usage of a given nucleic acid sequence can be modified to be compatible with the codon usage of a host cell of interest to allow better transcription rates and the expression of functional amino acid sequences in a host cell of interest. Furthermore, in the case of virions comprising genetic material, it has to be ensured by sequencing that the resulting purified recombinant infectious virus particles derived from a MV scaffold do not comprise a mutation rendering the attenuated virus virulent again. Such methods of nucleic acid sequencing, including deep sequencing, for means of sequence confirmation belong to the common general knowledge of the skilled person in the field of molecular biology and virology and can be applied at any stage of the methods according to the present disclosure.

As outlined above, any recombinant virus particle comprising and/or derived from a MV scaffold, can be purified according to the methods of the present invention, as the methods are specifically optimized taking into consideration the peculiar chemical and physical properties of the huge and pleomorphic MV, which properties are mainly influenced by the MV envelope/capsid making up the majority of the surface accessible area of the recombinant virus particle comprising and/or derived from a MV scaffold. A skilled reader will further note from the present disclosure that the purification steps comprised in the method of the invention rely mainly on the huge size of the MV particles as compared to the small size of the one or more impurities, e.g. host cell proteins and/or nucleic acids. It is therefore evident that the method of the present invention is also particularly useful for the production and/or purification of other huge and sterically-demanding virus particles, in particular those having a size similar to MV, e.g., having a size in the range of 50 nm and 2 μm, preferably in the range of 80 nm and 1.5 μm, more preferably in the range of 100 nm and 1,000 nm, even more preferably in the range of 100 nm and 500 nm. The present disclosure is not restricted to the purification of *Paramyxoviridae* or MV particles but encompasses methods according to the various embodiments of the first aspect of the invention, wherein instead of MV particles other sterically-demanding virus particles, preferably having a viral envelop covering the capsid, and in particular those described above, are produced and/or purified. Specific classes of enveloped viruses containing human pathogens, which can be purified according to the present disclosure, comprise, for example, Herpesvirus, Poxvirus, Hepadnavirus, Flavivirus, Togavirus, Coronavirus, Hepatitis D virus, Orthomyxovirus, Rhabdovirus, Bunyavirus, Filovirus, or certain retroviruses.

In a particular embodiment of the methods of the present invention, the construct is prepared by cloning a polynucleotide sequence encoding one structural protein or a plurality of structural proteins of a virus other than a MV in the cDNA encoding the full-length antigenomic (+) RNA of the MV. Alternatively, a nucleic acid construct of the disclosure may be prepared using steps of synthesis of nucleic acid fragments or polymerization from a template, including by PCR. It is further disclosed that the polynucleotide encoding the at least one protein of the virus other than a MV, or each of these polynucleotides, is cloned into an ATU (Additional Transcription Unit) inserted in the cDNA of the MV. Usually, there is one ATU per construct. ATU sequences usually comprise three potential regions of inserting a nucleic acid and further comprise, for use in steps of cloning into cDNA of MV, cis-acting sequences necessary for MV-dependent expression of a recombinant transgene, such as a promoter preceding a gene of interest, in MV cDNA, the insert represented by the polynucleotide encoding the viral protein(s) inserted into a multiple cloning sites cassette. The ATU is advantageously located in the N-terminal sequence of the cDNA molecule encoding the full-length (+)RNA strand of the antigenome of the MV and is especially located between the P and M genes of this virus or between the H and L genes. It has been observed that the transcription of the viral RNA of MV follows a gradient from the 5' to the 3' end. This explains that, when inserted in the 5' end of the coding sequence of the cDNA, the ATU will enable a more efficient expression of the heterologous recombinant nucleic acid DNA sequence. The ATU sequence can, however, be located at any position of SEQ ID NO:1 provided that it does not disrupt a coding sequence or a regulatory sequence thereof.

As outlined above, the method of the various embodiments of the present invention can advantageously be used to produce and/or purify recombinant infectious virus particles for providing said particles as vaccines or the like. A huge advantage of such produced and/or purified replication-competent vector, i.e. the recombinant infectious virus particles, present in a therapeutic or prophylactic composition is that it may provide a powerful, antigen-focused immune response to confer long-term immunity by continuously expressing antigens even after immunization.

In a second aspect of the present invention, there is provided a system for producing and/or purifying MV particles. The system comprises or consists of: (i) at least one bioreactor; (ii) a clarification unit, preferably a dead-end filter unit, downstream to the bioreactor; (iii) a flow through chromatography unit downstream to the clarification unit; and (iv) a filtration unit, preferably a tangential flow filtration unit, downstream to the flow through chromatography unit.

The term "consisting of "in the context of a system for producing and/or purifying MV particles is to be understood to refer to a series of operation units consisting of (i) at least one bioreactor; (ii) a clarification unit, preferably a dead-end filter unit, downstream to the bioreactor; (iii) a flow through chromatography unit downstream to the clarification unit; and (iv) a filtration unit, preferably a tangential flow filtration unit, downstream to the flow through chromatography unit. In this case, no further purification unit(s), concentration unit(s) and/or buffer exchange unit(s), and preferably no operation unit(s) at all (besides the optional nucleic acid digestion unit), is arranged between the at least one bioreactor and the filtration unit. Other operation units may optionally be arranged upstream of the at least one bioreactor and/or downstream of the filtration unit, in particular operation units for filling and/or packaging and the like. In some embodiments, the whole system does not comprise any further purification unit, concentration unit and/or buffer exchange unit. In other words, the aforementioned series of operation units may make up a whole process or form a consecutive part of an overall bigger process. Tanks such as surge tanks are not to be understood as operation units in the context of the present invention. As such, a system consisting of operation units may comprise surge vessels between some or all of the operation units. Moreover, since nucleic acid digestion is usually carried out in a tank, a system consisting of the above operation units may optionally comprise a nucleic acid digestion unit that follows the last of the least one bioreactor or the clarification unit.

Herein, a bioreactor is an apparatus, which allows cultivation, in particular cell growth, of microorganisms, preferably a pure culture, under appropriate conditions suitable for cell growth. Usually, a sterile bioreactor is inoculated with microorganisms to be grown under aseptic conditions. To maintain a pure culture over prolonged time, a bioreactor is to be understood to be designed so that contamination with other living organisms prevented. To provide a surface on which the host cells can efficiently grow, the at least one bioreactor preferably includes microparticles such as those described in the context of the first aspect of the invention. Moreover, the bioreactor may be a filled bioreactor including at least one host cell and/or MV particles.

A clarification unit herein denotes an operation unit suitable for carrying out clarification, i.e. a step for removing large impurities from a bulk product such as a cell culture supernatant to be clarified. It shall not be understood to optionally comprise an operation unit different to the clarification unit. In the present context, the clarification unit is suitable for removing cells and cell debris from the host cells infected with and producing MV particles. Suitable means for clarification include centrifugation, ultracentrifugation, microfiltration, including tangential flow filtration, dead end filtering and the like. In preferred embodiments, the clarification unit does not rely on separation by density, e.g. does not comprise a centrifuge. As outlined in the context of the first aspect of the present invention, the clarification unit preferably comprises a dead-end filter, preferably one or more depth filters. In order to be suited for clarification the dead-end filter must allow MV particles to pass through the pores of the filter, while large impurities such as cells and cell debris are retained. Further preferred embodiments in relation to the clarification unit comprised in the second aspect of the present invention are described in the context of clarification step (d) according to an embodiment of the first aspect the invention.

The flow-through chromatography unit as defined herein at least comprises a stationary phase material that is preferably selected from the group consisting of at least a resin, at least a matrix, at least a gel and, preferably, beads. In a preferred embodiment of the second aspect of the present invention, the stationary phase material is comprised in a container, e.g. for carrying out column chromatography. In preferred embodiments, the stationary phase material, preferably the beads, has/have at least size-exclusion functionality, hydrophobic interaction functionality or ion exchange functionality, or a combination thereof, preferably at least size-exclusion functionality, optionally combined with hydrophobic interaction functionality. Further preferred embodiments in relation to the flow-through chromatography unit, and in particular in relation to the stationary phase material, comprised in embodiments of the second aspect of the present invention are described in the context of flow-through chromatography step (i) comprised in embodiments of the method according to the first aspect the invention.

For example, in preferred embodiments of the second aspect of the present invention, the stationary phase material, preferably the beads, comprises/comprise a ligand-activated core, and an inactive shell comprising pores. The pores comprised in the inactive shell have a molecular weight cut off smaller than the MV particles to exclude the MV particles from entering the ligand-activated core, whereas a molecule smaller than the molecular weight cut off can enter the pores and bind to the ligand-activated core. Preferably, the ligand-activated core comprises octylamine. With regard to the pores, it is preferred that the molecular weight cut off is in the range of 100 kDa to 2000 kDa, preferably 200 kDa to 1500 kDa, more preferably 400 kDa to 1200 kDa and most preferably 500 kDa to 1,000 kDa. According to a specifically preferred embodiment, the flow-through chromatography unit comprises Capto Core 700 stationary phase material contained in a container such as a column.

The filtration unit comprised in the system of the second aspect of the invention shall be generally understood to allow an increase of a concentration of MV particles. Hence, in a preferred embodiment, the filtration unit comprises a filter for retaining MV particles. The filter may be designed for letting liquid, preferably along with further impurities, pass through the filter. In preferred embodiments, the filtration unit comprises an ultrafiltration module (preferably a TFF module), a feed tank, a retentate line connecting an outlet on a retentate side of the filtration unit with the feed tank, a feed line connecting an inlet on the retentate side of the ultrafiltration module with the feed tank and a permeate line connected to an outlet on a permeate side of the ultrafiltration module. Further tanks may be included, for example, a permeate receiving tank connected to the permeate line and/or a tank for holding diafiltration buffer connected via a diafiltration line to the feed tank, the retentate line or an inlet on the retentate side of the ultrafiltration module. Furthermore, the filtration module may comprise one or more flow generating means such as pumps, for example, at least one flow generating means in the feed line and/or at least one flow generating means in the diafiltration line. The filtration module may further comprise one or more valves such as a check valve arranged in the retentate and/or permeate line. Furthermore, at least one pressure sensor must be present to enable a constant pressure during the methods, or in the system according to the present invention. Preferably, a pressure sensor is at least present in the feed line, the retentate line and the permeate line to ensure a constant pressure and thus process control.

Further details of preferred embodiments relating to the filtration unit result from the various embodiments described in the context of the first aspect of the invention, which shall be taken to define embodiments of the second aspect of the present invention.

In one embodiment of the second aspect of the present invention, there is provided a system according to the second aspect of the invention and the various embodiments thereof, wherein the bioreactor includes a probe for determining viable cell density and/or total cell density. The viable and/or total cell density probe is preferably based on (an) online technique(s). As outlined in the context of an embodiment according to method of the first aspect of the invention, an exemplarily, and herein preferred, technique for on-line determination of viable cell density is based on capacitance. This allows on-line determination in real-time and is insensitive to microcarriers as well as cell debris. The total cell density determination may be based on on-line measurements including turbidity and optical density measurement at NIR (Near Infra-Red) wavelengths, or any other suitable measurement, whether allowing on-line or off-line determination of total cell density.

In correspondence of the various embodiments according to the first aspect of the invention, the system according to the second aspect of the present invention does not necessarily reflect a method that is based on a concentration step at an early step of downstream processing. For the advantages associated with such feature, reference is made to the corresponding part of the first aspect of the present invention. Hence, in one embodiment of the second aspect of the present invention, there is provided a system according to the second aspect of the invention and the various embodiments thereof, wherein no concentration unit, preferably no ultrafiltration-based concentration unit and more preferably no tangential flow filtration unit, is arranged between the at least bioreactor and the flow through chromatography unit. Further embodiments can be deduced from the description made in the context of the first aspect of the present invention, which, as repeatedly emphasized herein, shall be understood to form corresponding embodiments in the context of the second aspect of the invention.

The system according to the second aspect of the invention, and the various embodiments thereof, can be advantageously used for continuous and/or integrated processing. In one embodiment of the second aspect of the present invention, there is provided a system, wherein the clarification unit, preferably the dead end filter unit, is either in direct fluid communication with the bioreactor or via a first vessel; and/or wherein the flow through chromatography unit is either in direct fluid communication with the clarification unit, preferably the dead end filter unit, or via a second vessel; and/or wherein the filtration unit, preferably the tangential flow filtration unit, is either in direct fluid communication with the flow through chromatography unit, or via a third vessel; and preferably wherein the first vessel is a storage vessel or a mixable vessel, and/or the second vessel and/or the third vessel is a storage vessel.

A main difference between the prior art methods and systems for producing and/or purifying MV particles is that it relies on flow-through chromatography. Using a flow-through chromatography unit in a method or system according to the first, respectively second aspect of the present invention overcomes problems relating to low purity in case no chromatography step at all is used, and low recovery in case the chromatography step is performed in bind-elute mode. A hypothesis for these observations has been provided above in the context of the first aspect of the present invention. Hence, a third aspect of the present invention relates to the use of flow-through chromatography as defined in the various embodiments of the first and second aspect of the present invention for the purification of MV particles.

Due to the high purity and high recovery that can be achieved by the first and second aspect of the invention, the various embodiments of these aspects are particularly useful in providing a prophylactic and/or therapeutic composition. Hence, in a fourth aspect of the present invention, there is provided a prophylactic or therapeutic composition produced by the method according to the various embodiments of the first aspect of the invention and/or the system according to the various embodiments of the second aspect of the invention, wherein infectious MV particles are comprised in the composition as an active ingredient.

Preferred prophylactic and/or therapeutic compositions include immunogenic and/or vaccine compositions, which are suitable and/or intended to be used for use in a method of eliciting an immune response, or in a method of prophylactic treatment of a subject for protecting the subject from infection with a virus. In such method, protection is achieved by exposing the subject to the MV particles comprised by the immunogenic composition or the vaccine composition. Further preferred prophylactic and/or therapeutic compositions include compositions, which are suitable and/or intended to be used for tumor therapy such as oncolytic tumor therapy based on infectious MV particles.

Hence in a fifth aspect of the present invention, there is provided a composition according to the fourth aspect of the present invention for use in a method of therapeutic and/or prophylactic treatment. In one embodiment of this aspect, a subject is exposed to the MV particles comprised by the composition for protecting the subject from infection with a virus and/or an immune response is elicited by exposing the subject to the MV particles comprised in the composition. In another embodiment of this aspect, a subject is exposed to the MV particles comprised by the composition for tumor therapy such as oncolytic tumor therapy and/or an immune response is elicited by exposing the subject to the MV particles comprised in the composition.

In a sixth aspect of the present invention, there is provided a method of prophylactic and/or therapeutic treatment, the method comprising exposing a subject to the MV particles comprised by the composition of the fourth aspect of the present invention. In one embodiment of this aspect, the method is used for protecting the subject from infection with a virus. In another embodiment of this aspect, the method is used for (oncolytic) tumor therapy. In both cases, the method may comprise exposing the subject to the MV particles comprised in the composition and/or elicit an immune response by exposing the subject to the MV particles comprised in the composition.

In a seventh aspect, the present invention relates to use of MV particles, obtained by the various embodiments of the method according to the first aspect or the various embodiments of the system according to the second aspect of the invention, for the manufacture of a medicament for therapeutic and/or prophylactic treatment, in particular as a vaccine or in OV therapy.

The invention will now be further described with reference to the following not limiting examples.

## Examples

The following acronyms are used herein above and below: Master Cell Bank (MCB), Working Cell Bank (WCB), Phosphate Buffered Saline (PBS), Bulk Drug Substance (BDS), Drug Product, In-process Sample, Water for Injection (WFI), Unpurified Harvest (UH), Benzonase treated unpurified harvest (BUH), Purified bulk (PB), Concentrated bulk (CB), Column volume (CV), Transmembrane pressure (TMP), Upstream processing (USP), Downstream processing (DSP), Room temperature (RT), Tangential flow filtration (TFF).

Examples 9 to 20 have been carried using MV-CHIK encoded by a nucleic acid having the sequence according to SEQ ID NO:5. Example 21 employed MV-GFP encoded by a nucleic acid having the sequence according to SEQ ID NO:4. These examples shall not be understood to be limited to MV-CHIK respectively MV-GFP but shall be understood to provide support for the production and/or purification of any MV particle, and in particular those specifically disclosed herein, as well as large virus particles in general that preferably have a similar size and optionally similar physico-chemical surface characteristics as MV. Moreover, the examples are intended to provide disclosure for further preferred embodiments, wherein individual features, whether disclosed alone or in combination with further features, can be isolated and combined with further features, whether disclosed in the same example or in different examples or whether occurring in the description or the claims, to define further embodiments which are covered by the present disclosure.

Furthermore, while the following examples employed a cell bank comprising specific host cells, the present invention shall not be understood to be limited to a specific type of host cells. It is clear that any host cell susceptible to infection with MV particles will be capable to produce MV particles, which will either result in autolysis or in lysis of the cell by means of a lysis step. Hence, regardless which host cells have been used to produce a supernatant can be obtained comprising MV particles and one or more impurities such as host cell proteins and/or nucleic acids. Though the precise physico-chemical properties of impurities stemming from different host cells might slightly differ, the overall purification concept still allows to readily exploit the advantages of the present invention also for these kinds of host cells. This is in part due to the fact that a substantial part of the purification scheme is based on separation by size.

### Example 1: USP Starting material

A master cell bank (MCB) of Vero 10-87 cells, lot No 1416.01 MCB, P#145 was produced. The MCB is contained in cryovials, each containing 1 mL of cell suspension at a concentration of 1.0 x $10^7$ cells/mL. The MCB should be stored in a vapor phase liquid nitrogen cryogenic tank at two different locations.

From the Vero MCB a working cell bank (WCB) with a concentration of 1.0 x $10^7$ cells/mL was produced and 120 cryovials were stored in a vapor phase liquid nitrogen cryogenic tank.

### Example 2: WCB revival

One cryovial of the WCB containing 1.0 x $10^7$ cells in a total volume of 1 mL is removed from storage in the vapor phase of liquid nitrogen and placed in a 50 mL tube containing 25 mL of 70% ethanol. The tube is transported to a cleanroom where the cryovial is removed from the ethanol and immediately thawed in hand until the cell suspension is fully thawed. For sanitization, the vial is placed back in the tube filled with 70% ethanol and transferred into a biosafety cabinet. The cryovial is poured on a layer of tissue papers and the thawed cell suspension is pipetted dropwise to a 50 mL tube filled with 10 mL VP-SFM medium. The cell suspension is subsequently centrifuged at 300 x g for 5 minutes at room temperature. The supernatant is discarded and the cell pellet is loosened by gently tapping the tube to the working area of the biosafety cabinet. The cells are then re-suspended in 10.5 mL of VP-SFM medium and 0.5 mL of the suspension are removed for cell counting. Exemplary revival parameters for the WCB are shown in Table 1 below.

Table 1: WCB Revival Parameters

| **Stage** | **Parameter** | **Operating criteria (range)** |
|---|---|---|
| WCB storage conditions | Temperature | Vapor phase liquid nitrogen |

(continued)

| Stage | Parameter | Operating criteria (range) |
|---|---|---|
| WCB thaw and recovery Stage 0 | Thaw temperature | In hands |
| | Centrifugation | 300 x g |
| | Centrifugation time | 5 minutes |
| | Centrifugation temperature | Room temperature |
| | Medium used | VP-SFM |
| | Pellet resuspension media volume | 10.5 mL |

In order to determine cell number and viability 10 μL of cell suspension is mixed with 10 μL of trypan blue (0.4% in 0.81% sodium chloride and 0.06% potassium phosphate dibasic solution) in a microcentrifuge tube and briefly vortexed. 10 μL of the mixture are withdrawn and pipetted into a counting slide. Cell count and viability is measured using an automated hemacytometer (e.g. Biorad TC20). Cell counts should be documented. Usual acceptance criteria for cell viability are ≥ 85% viability. Lower viability values are possible, yet this might guarantee optimum results. The remaining 10 mL are used for cell expansion.

**Example 3: Cell expansion**

A procedure to obtain the at least one host cell and preferably the plurality of host cells is described, exemplarily for a 3-stage cell expansion using Vero 10-87 cells, in the following and illustrated in Figure 1. Notably, the final clarification step has been performed according to different strategies, i.e., as depicted with e 3 μm polypropylene filter, but also with a filter cascade (50 μm and 3 μm).

In stage 1, the remaining 10 mL cell suspension from stage 0 are transferred to a 5-layer cell culture multi-flask filled with 140 mL VP-SFM medium and incubated for 6 days. Exemplary parameters for stage 1 are shown below in Table 2.

Table 2: Process parameters for stage 1

| Stage | Parameter | Operating criteria (range) |
|---|---|---|
| Cell expansion Stage 1 | Seeding density | From WCB Revival |
| | Culture volume | 150 mL |
| | Culture medium | VP-SFM |
| | Temperature | 36.5 ± 1°C |
| | Duration | Approximately 6 ± 1 days |
| | $CO_2$ | 5.0 % ± 1 % |
| | Humidity | 80 % ± 10 % |
| | Final cell density | ≥ 90% confluent cells |
| | Final cell viability | ≥ 90% viability |

In stage 2, upon confluence of the 5-layer flask from stage 1, the supernatant is removed, discarded and the cell monolayer is washed with PBS. TrypLE Select is added and distributed evenly over the monolayer. The flask is incubated to detach cells and then observed for cell detachment under the microscope. If necessary, the flask is tapped gently for cell detachment. If detachment is below 90%, the flask is further incubated until detachment of greater than 90% is reached. (Parameters are the same as in Stage 3 described below).

25 mLVP-SFM medium are added to the flask and the cell suspension is transferred to a sterile centrifuge tube. The cell suspension is then centrifuged as described in Table 3 below. After centrifugation, the supernatant is removed and discarded while the cell pellet is loosened by tapping and re-suspended in 25 mL VP-SFM medium by pipetting up and down. The culture is fully suspended if no cell clumps are visible. 0.5 mL from the prepared cell suspension are removed and used to determine cell number and viability as described above.

Table 3: Cell harvest parameters

| Stage | Parameter | Operating criteria (range) |
|---|---|---|
| Cell harvest Stage 1 | PBS Cell wash volume (T875) | 50 mL |
| | TrypLE select volume (T875) | 25 mL |
| | Cell detachment incubation temperature | 36.5 ± 1°C |
| | Cell detachment incubation time | 5 - 10 min until 90% detachment |
| | VP-SFM added for centrifugation | 25 mL |
| | Centrifugation (g) | 300 x g ± 5% |
| | Centrifugation time | 5 minutes |
| | Centrifugation temperature | Room temperature |
| | Pellet resuspension media (VP-SFM) volume | 25 mL |

Based on the determined viable cell count the cells are diluted with VP-SFM medium to a concentration of 4.2 x $10^4$ cells / mL in a total volume of 1500 mL and filled in a 10-layer CellSTACK. Cell expansion parameters are shown in Table 4 below:

Table 4: Cell expansion parameters

| Stage | Parameter | Operating criteria (range) |
|---|---|---|
| Cell expansion Stage 2 | Seeding density | 1.00 x $10^4$ cells/$cm^2$ |
| | Growth area (10-layer CellSTACK) | 6360 $cm^2$ |
| | Culture volume (10-layer CellSTACK) | 1500 mL |
| | Culture media | VP-SFM |
| | Incubation Temperature | 36.5 ± 1°C |
| | Duration | Approximately 4 ± 1 days |
| | $CO_2$ | 5.0% ± 2% |
| | Humidity | 80% ± 10% |
| | Final cell density | ≥90% confluent cells |
| | Final cell viability | ≥90% viability |

Before starting with stage 3 of the cell expansion, the bioreactor may be prepared according to the following exemplary procedure. All bioreactor tubings and pipes are pre-assembled and the pH, DO and cell density probes are calibrated. For a process run with 3 L working volume around 9 g (or more, i.e., 18 g) dry Cytodex I microcarriers are weight in a class container and swollen in 500 mL (or more, e.g., 800 mL for 18 g microcarrier) Ca2+ and Mg2+ free PBS for at least 3 h at room temperature. The supernatant is decanted and the microcarriers are washed for a few minutes in 400 mL fresh Ca2+ and Mg2+ free PBS. The PBS is discarded and replaced with 2000 mL fresh Ca2+ and Mg2+ free PBS. The swollen microcarrier are finally transferred into the pre-assembled bioreactor vessel, the system is fully assembled and autoclaved for 20 minutes at ≥121 °C.

When the bioreactor is sterilized and connected to the control unit, the microcarrier were let to settle down and the PBS is removed using overpressure. In order to wash the microcarrier 500 mL VP-SFM medium are pumped in the bioreactor vessel using a peristaltic pump and stirred at 40 rpm for 5 minutes. The microcarrier were again let to settle down and the medium is removed using overpressure. The bioreactor vessel is now filled to 2900 mL final working volume with VP-SFM using a peristaltic pump. Subsequently the heating, the stirring, all relevant process loops and the cell density probe are started and the system is let to stabilize overnight (Process parameters are shown in table 5). The bioreactor is now ready for inoculation with the cells.

Table 5: Process parameters - Stage 3

| Stage | Parameter | Operating criteria (range) |
|---|---|---|
| Process parameters Stage 3 | Vessel type | 3.8 L Bioreactor |
| | Surface area per 9 g Cytodex I | 39600 $cm^2$ |
| | Total culture volume | 2900 mL |
| | Revolutions per minute | 40 |
| | Culture media | VP-SFM |
| | Incubation Temperature | 36.5 ± 1°C |
| | Dissolved oxygen control by process air via sparger | >40% |
| | pH control<br>by CO2 via overlay (to decrease pH) and by sodium bicarbonate (to increase pH) | 7.2 ± 0.2 |
| | Temperature control | 36.5 ± 1°C (during cell culture)<br>32.5 ± 1°C (during virus production) |
| | Cell Density probe - Incyte | $f_{mes}$: 382 kHz $f_{high}$: 10000 kHz integration: High |
| | Agitation control for homogeneous mixing | One pitched blade impeller |
| | Process Air | Air via overlay, 0.8 mL/min |
| | CO2 | CO2 via overlay with pH control |
| | Air ($O_2$) | Air ($O_2$) via sparger with DO control |

Upon confluence of the 10-layer CellSTACK from stage 2, the supernatant is removed, discarded and the cell monolayer is washed with PBS. TrypLE Select is added and distributed evenly over the monolayer. The flask is incubated to detach cells and then optically observed for cell detachment. If necessary, the flask is tapped gently for cell detachment. 550 mLVP-SFM medium are added to the flask and the cell suspension is equally distributed to 4 sterile 175mL centrifuge tubes. The cell suspension is then centrifuged as described in Table 6 below. After centrifugation, the supernatant is removed and discarded while the cell pellet is loosened by tapping and re-suspended in 62.5 mLVP-SFM medium each tube by pipetting up and down. The culture is fully suspended if no cell clumps are visible. Cell suspensions are pooled in a sterile container resulting in a final volume of 250 mL and 0.5 mL are removed and used to perform a cell count determining viable cells and viability as disclosed herein.

Table 6: Cell harvest parameters - Stage 2

| Stage | Parameter | Operating criteria (range) |
|---|---|---|
| Cell harvest Stage 2 | PBS Cell wash volume (10L-CS) | 500 mL |
| | TrypLE select volume (10L-CS) | 250 mL |
| | Cell detachment incubation temperature | 36.5 ± 1°C |
| | Cell detachment incubation time | 5 - 10 min until detachment |
| | VP-SFM added for centrifugation | 550 mL |
| | Centrifugation (g) | 300 x g ± 5% |
| | Centrifugation time | 8 minutes |
| | Centrifugation temperature | Room temperature |
| | Pellet resuspension media (VP- SFM) volume | 250 mL |

General process parameters are not modified during inoculation procedure. The required amount of cells for inoculation of the bioreactor is filled in a transport vessel equipped with tubings for welding. The vessel is aseptically welded to the bioreactor and the cell suspension is transferred into the bioreactor using overpressure. Parameters for inoculation are shown in the table 7.

Table 7: Inoculation parameters - Stage 3

| Stage | Parameter | Operating criteria (range) |
|---|---|---|
| Inoculation of bioreactor Stage 3 | Available surface area per 9 g Cytodex I | 39600 cm2 |
| | Seeding density | 1.00 x $10^4$ cells/$cm^2$ |
| | Total cell number needed | 3.96 x $10^8$ cells |
| | Seeding culture volume | 100 - 250 mL |
| | Total volume bioreactor | 3000 - 3150 mL |

Following inoculation cells were let to attach the microcarrier for 2 hours. Then the bioreactor is sampled in order to microscopically monitor the cellular distribution on the microcarrier and to determine cell counts of cells attached to the microcarrier and in suspension (Counting procedure for cells attached to the microcarrier is shown in section 3.4.4).

The bioreactor was sampled every 24 hours as follows. Using the sample port of the bioreactor 10 mL of microcarrier suspension is withdrawn and discarded. Using syringe 2 and 3, 10 mL sample each are immediately withdrawn and the microcarrier suspensions are transferred to 50 mL tubes. From the analysis tube 1 mL suspension is transferred to one well of a 12-well plate and the attachment/growth of the cells is microscopically analyzed. 10 μL are taken from the analysis tube and the cells in suspension are counted. The cells in the cell counting tube are let settle down and the supernatant is discarded using a serological pipet. The same amount of crystal violet/citric acid solution is added, well mixed and incubated for at least 30 min. A sample of 10 μL is taken and the released nuclei is counted using a hemacytometer, or alternatively a nucleocounter. Cells are grown under process parameters indicated above for stage 3 and sampled every 24 hours.

Since the medium is depleted during cell growth phase and toxic by-products accumulated, a medium exchange is preferably performed. The stirring, as well as the DO control are stopped, the microcarrier are let to settle down for at least 15 minutes and the spent medium is removed using overpressure. The bioreactor is re-filled with fresh medium and the stirring and the DO control are started again and. Now, the host cells are ready to be infected.

**Example 4: Virus production**

Process parameters for infection phase are shown in table 8.

Table 8: Process parameters - Stage 4

| **Stage** | **Parameter** | **Operating criteria (range)** |
|---|---|---|
| Virus Production Stage 4 | Total culture volume | 3000 mL |
| | Revolutions per minute | 40 |
| | Culture media | VP-SFM |
| | Incubation Temperature | 32.5 ± 1°C |
| | Dissolved oxygen control by process air via sparger | >40% |
| | pH control<br>by CO2 via overlay (to decrease pH) and by sodium bicarbonate (to increase pH) | 7.2 ± 0.2 |
| | Process Air | Air via overlay, 0.8 mL/min |
| | $CO_2$ | CO2 via overlay with pH control |
| | Air ($O_2$) | Air ($O_2$) via sparger with DO control |

General process parameters are not modified during infection procedure. The required amount of virus for the infection with a MOI of 0,001 is filled in a transport vessel equipped with tubing for welding. The vessel is aseptically welded to the bioreactor and the seed virus is transferred into the bioreactor using overpressure.

This MOI may again vary depending on the host cell and the MVSS chosen, but can be easily determined after standard pre-testings with the respective host cell and the respective virus. MOIs between 0.0001 and 0.1 are preferable. Notably, the time point for harvest will change depending on the chosen MOI, which can be determined by the skilled person. Virus is grown under parameters listed in Table 8 and sampled every 24 hours.

**Example 5: Clarification**

In order to clarify the virus containing cell broth, the stirring and DO control are stopped and the microcarrier are let to settle down for at least 15 min. The cell broth above the microcarrier bed is withdrawn using overpressure and filtered through a 3 μM polypropylene filter (cf. Figure 1), or a filter cascade consisting of a 50 μM pre- filter and a 3 μM main filter.

The clarified material is transferred to the downstream part directly (processing on one day) or stored at room temperature overnight (processing on two days). Do not store the viral suspension longer than overnight.

The clarification method chosen here can vary depending on material to be clarified and any suitable filtration method can be applied. It is important to consider the polymorphic large surface of the MV. Therefore, suitable filter materials have to be chosen, which do not show unspecific binding of the MV based preparation, which would result in a loss of yield or functionality. As detailed above, centrifugation should be avoided due to the limited scalability thereof under GMP conditions and/or the risk of contaminations associated with this procedure.

The following examples 6 to 8 relate to downstream manufacturing for MC-CHIK. An overview is provided by way of Figure 2 showing a corresponding process scheme.

**Example 6: Endonuclease treatment**

Additionally, the protocols disclosed herein can comprise a DNAse treatment step. This treatment can be performed before or after clarifying the virus suspension depending on the host cell and the recombinant infectious virus particle to be purified. A preferred DNAse is a benzonase, but any suitable DNAse having comparable activity, specificity and purity can be chosen for this purpose, whereas the choice of a suitable DNAse can easily be made by a person skilled in the art.

The bag is incubated for 1 h at 37.0 ± 1.0 °C and 50 rpm. The required amount of Benzonase, magnesium chloride and EDTA is calculated to obtain a final concentration of 50 U/mL Benzonase, 2 mM magnesium chloride and 5 mM EDTA in the 4 L bag. Benzonase/1 M magnesium chloride stock solution /500 mM EDTA stock solution is removed from -20.0 °C storage, transported to the cleanroom on dry ice and thawed at ambient/ 37.0 °C. The required amount of magnesium chloride and Benzonase are mixed together into a homogenous solution and then aseptically added to the viral suspension and swirled gently. The final concentration in the viral suspension is 50 U/mL Benzonase and 2 mM magnesium chloride. The bag is incubated at 37.0 ± 1.0 °C, 50 rpm for 1-2 hours. The bag is removed from the incubator and the required amount of EDTA is aseptically added to the viral suspension and swirled gently. The final concentration in the viral suspension is 5 mM EDTA.

Table 9: Benzonase treatment parameters

| **Stage** | **Parameter** | **Operating criteria (range)** |
|---|---|---|
| Material for Benzonase treatment | Concentration of magnesium chloride stock solution | 1M |
| | Concentration and pH of EDTA stock solution | 500 mM, pH 8.0 ± 0.1 |
| | Benzonase | High purity grade |

(continued)

| Stage | Parameter | Operating criteria (range) |
|---|---|---|
| Benzonase treatement | Final concentration of magnesium chloride in viral suspension | 2 mM |
| | Final concentration of Benzonase in viral suspension | 50 U/mL |
| | Benzonase incubation temperature | 37.0 ± 1.0 °C |
| | Incubation shaker speed | 50 rpm |
| | Benzonase incubation time | 1 - 2 hours |
| | Final concentration of EDTA in the viral suspenstion | 5 mM |

These parameters may vary depending on the DNAse used but can easily be adapted by the person skilled in the art in the knowledge of the present disclosure.

The 4 L bag containing the Benzonase treated virus suspension is immediately transferred to purification. It should be avoided to store the Benzonase treated pool longer than over night.

**Example 7: Flow-through chromatography**

The chromatography system can be set up as described, exemplarily for a Capto Core 700 26/40 column connected to an ÄKTA Pilot, in the following. The pH electrode is calibrated according to standard procedures. Inlet tubings are connected to the ÄKTA Pilot system inlets A1, A2, B1, B2, B3 and sample inlet S1 are tie wrapped. Using a sterile connective device (SCD), a two T-piece is connected to the inlet tubing on S1 to create a bypass on this line. Using the SCD, cleaning tubing is connected to the inlet tubing on A1, A2, B1, B2, B3 and both lines of the T-piece on S1. Outlet tubing is connected to the ÄKTA Pilot system outlets F1, F3, F5, F7 and each connection is tie wrapped. Air vents are clamped off on the outlet tubing with Kocher clamps. Using the SCD a cleaning outlet tubing set is connected up to outlet tubing on F1, F3, F5 and F7 and to one 10 L waste bag. Using the SCD, the 1 M NaOH solution (>3 L) is connected to the inlet cleaning tubing. All inlets, outlets and the system are flushed with 1 M NaOH. Using the SCD the 1 M NaOH connected to the inlet cleaning tubing is replaced with 0.1 M NaOH (> 3 L). All inlets, outlets and the system are flushed with 0.1 M NaOH. Using the SCD the 0.1 M NaOH connected to the inlet cleaning tubing is replaced with WFI (> 3L). All inlets, outlets and the system are flushed with WFI. The Capto Core 700 column is connected aseptically in up flow direction to column position 2. Using the SCD the equilibration buffer (> 3 L) is connected to A1 and S1, the regeneration buffer (> 3 L) to B1, 1.0 M NaOH (> 3 L) to A2, WFI (> 3 L) to B2, 20% EtOH (> 2 L) to B3. All inlets, outlets and the system are flushed with the respective buffers. When flush is complete, the outlet tubings are emptied by opening the attached air vent. Using the SCD the waste bag on F1 is replaced with a new waste bag. Using the SCD a sterile 4 L bag is connected to F3 to collect the flow-through. Using the SCD a sterile 1 L bag is connected to F5 to collect the wash fraction. Using the SCD a sterile 1 L bag is connected to F7 to collect the regeneration fraction.

Table 10: Capto Core 700 column preparation parameters

| Stage | Parameter | Operating criteria (range) |
|---|---|---|
| Connections | Inlets - Buffer | A1, A2, B1, B2, B3 |
| | Inlets - Sample | S1 |
| | Outlets | F1, F3, F5, F7 |
| NaOH flush (1.0 M) | Buffer | 1.0 M NaOH |
| | Volume | 3L |
| | Flow rate | 300 mL/min |
| | Flow path | Complete system |

(continued)

| Stage | Parameter | Operating criteria (range) |
|---|---|---|
| NaOH flush (0.1 M) | Buffer | 0.1 M NaOH |
| | Volume | 3L |
| | Flow rate | Equilibration flowrate |
| | Flow path | Complete system |
| WFI flush | Buffer | WFI |
| | Volume | 3L |
| | Flow rate | 300 mL/min |
| | Flow path | Complete system |
| Buffer flush | Buffer | Equilibration buffer (A1, S1), elution buffer (B1), NaOH (A2), WFI (B2), EtOH (B3) |
| | Volume | 3L |
| | Flow rate | 300 mL/min |
| | Flow path | Respective connections |

Using the above example, the purification is now described. The maximum loading volume is set to 55 CV. A bag comprising the MV particles containing sample is connected to one of the bypass lines of inlet S1, using the sterile connective device (SCD). Inlet S1 is flushed with a minimum of sample material. The bypass on S1 is used to remove air from the line. No air should be introduced into inlet S1 later on. Otherwise only part of the virus material will be processed on the column. Purification is performed as outlined in Table 11 below. Virus flow through is collected in F3 with UV monitoring (start > 100 mAU, stop < 50 mAU). Maximum delta column pressure is set to 2.0 bar. After completion of the run, all outlets are emptied by opening the air vent on the outlet. The outlet air vents and bags are clamped off and the bags are then disconnected from the outlets using the SCD. Product collection during flow-through is determined by UV280 reading on the UV detector. The collection is started during sample loading when UV280 is > 100 mAU and is stopped during the wash step when the UV280 reading is < 50 mAU. Product collection parameters are summarized in Table below. The exact volume of the flow-through fraction is noted. Typically the volume of the flow-through fraction (purified bulk material) is 1.0 up to 1.1-fold of the loaded sample volume.

Table 11: Virus purification parameters

| Stage | Parameter | Operating criteria (range) |
|---|---|---|
| Final connection setup | A1 | Equilibration buffer |
| | A2 | 1 M NaOH |
| | B1 | Regeneration buffer |
| | B2 | WFI |
| | B3 | 20% EtOH |
| | S1 | Sample (Benzonase treated unpurified harvest) |
| | F1 | Waste |
| | F3 | Collect flow-through |
| | F5 | Collect wash fraction |
| | F7 | Collect regeneration fraction |

(continued)

| Stage | Parameter | Operating criteria (range) |
|---|---|---|
| Equilibration of the column | Buffer | Equilibration buffer |
| | Volume | > 500 mL/ 10 CV |
| | Flow rate/ flow velocity | 140 cm/h |
| | Flow path | A1>Column>F1 |
| | Column flow direction | Normal flow direction |
| Sample loading | Buffer | Sample |
| | Volume | 55 CV |
| | Flow velocity/ residence time | 140 cm/h / 4 min |
| | Flow path | S1> column > F3 |
| | Column flow direction | Normal flow direction |
| Wash | Buffer | Elution buffer |
| | Volume | 500 mL/ 10 CV |
| | Flow velocity | 140 cm/h |
| | Flow path | A1> column > F3 (UV280 > 100 mAU) /F5 (UV280 < 50 mAU) |
| | Column flow direction | Normal flow direction |
| Regeneration | Buffer | Regeneration buffer |
| | Volume | 500 mL/ 10 CV |
| | Flow velocity | 140 cm/h |
| | Flow path | B1>column> F7 |
| | Column flow direction | Normal flow direction |
| Clean column with NaOH | Buffer | 1 M NaOH |
| | Volume | 500 mL/ 10 CV |
| | Flow velocity/ contact time | 140 cm/h / min. 120 min contact time |
| | Flow path | A2> column > F1 |
| | Column flow direction | Normal flow direction |
| Clean column with WFI | Buffer | WFI |
| | Volume | 3L |
| | Flow velocity | 140 cm/h |
| | Flow path | B2> column > F1 |
| | Column flow direction | Normal flow direction |

The purified bulk material is then transferred to the concentration step (processing on one day) or stored at room temperature overnight (processing on two days).

The buffers used during chromatographic purification correspond to those shown in Table 12. Usually, all buffers are filtered 0.2 μm and de-aerated in a sonicating bath for 15 minutes.

Table 12: Buffer preparation

| Stage | Parameter | Operating criteria (range) |
|---|---|---|
| Equilibration | Equilibration buffer | 50 mM HEPES, 150 mM NaCl, pH 7.5 ± 0.1 |
| Wash | Equilibration buffer | 50 mM HEPES, 150 mM NaCl, pH 7.5 ± 0.1 |
| Regeneration | Regeneration buffer | 50 mM HEPES, 2 M NaCl, pH 7.5 ± 0.1 |
| Sanitization | | 1 M NaOH |

Any other suitable stationary phase material can be used for purification provided that it allows specific retention of the one or more impurities and pass the MV particles through the stationary phase material. An exemplary procedure using an ÄKTA Pilot chromatography system is set up as described above. Any suitable chromatography system can be chosen for the purpose of the present invention provided that it is compatible with the column chosen.

The main peak fraction can additionally be subjected to a further round of purification, polishing or buffer-exchange to separate recombinant infectious virus particles containing genetic material from optionally present virus-like particles.

**Example 8: Virus concentration and diafiltration**

In this exemplary embodiment, the virus concentration / diafiltration is performed with a sterile Hydrosart, or a Pellicon 2 cartridge (Ultracel®, PLCMK = 300 kDa / 30 kDa) in a TFF system. During the concentration the purified bulk material obtained from flow-through chromatography purification is fed and the retentate is recirculated to the feed container. The required membrane area is calculated to obtain a maximum feed of 110 L/$m^2$. After concentration the diafiltration is performed by adding diafiltration buffer to the feed container at the same rate as permeate is removed from the process. The entire concentration / diafiltration is performed at a constant transmembrane pressure (TMP) of 0.4-0.5 bar.

The conditioning buffer and diafiltration buffer shown in Table 13 are used in the concentration and, respectively, diafiltration step. Prior to purification, all buffers are usually filtered 0.2 μm and de-aerated in a sonicating bath for 15 minutes.

Table 13: Buffer preparation

| Stage | Parameter | Operating criteria (range) |
|---|---|---|
| Conditioning | Conditioning buffer | 50 mM HEPES, 150 mM NaCl, pH 7.5 ± 0.1 |
| Diafiltration | Diafiltration buffer | 5% Sorbitol (w/v) or PBS for Hydrosart |
| Sanitization | Sanitization buffer | 0.1 M NaOH |

According to a specific embodiment, membrane parameters are as follows: Typical TFF flow rates for Pellicon 2 membranes (C-screen): 540 -876 LMH (9.5- 14.6 L/min/$m^2$). The flux unit of L/hr/$m^2$ is usually abbreviated as LMH, which will also be used herein. Chemical stability Ultracel material: 0.1 M NaOH - contact time 30 - 60 min, pH compatibility: 2-13. Hold up volume 0.1 $m^2$ membrane: upstream: 18 mL; downstream: 10 mL

The TFF system can be set-up as described below. Flush volumes are calculated based on a system hold up volume of approx. 200 mL. Either, a sterile single-use flow path is provided, or the system has to be sterilized using 1 M NaOH. Inlet and outlet tubings (feed, permeate, retentate) are connected using a SCD. Waste bags (20 L) are connected to all outlets. All inlets, outlets and the system are flushed with 1 M NaOH (> 5 L). Using the SCD the 1 M NaOH connected to the inlet cleaning tubing is replaced with WFI (> 5 L). All inlets, outlets and the system are flushed with WFI (> 5 L). The transfer lines are flushed with conditioning buffer and diafiltration buffer (> 2 L each). One Pellicon 2 filter capsule is aseptically connected to the TFF system. The membrane is flushed with 100 L/$m^2$ WFI with a crossflow rate of 360 - 600 LMH. A feed tank containing the appropriate amount of WFI is aseptically connected and the feed flow is started. Additional WFI is added to the feed tank if required. One third of the total required WFI amount is flushed through the retentate outlet. Two third of the total required WFI amount is flushed through the permeate outlet. An integrity testing can be optionally carried out using routine procedures. The membrane is pre-conditioned with 50 L/$m^2$ conditioning buffer at a crossflow rate of 360 - 600 LMH. The feed tank is filed with the appropriate amount of conditioning buffer and the feed flow is started at the crossflow rate of 360 - 600 LMH. One third of the total required volume is flushed through the retentate outlet. Two third of the total required WFI amount is flushed through the permeate outlet. When pre-conditioning is complete, the outlet tubing is emptied by opening the attached air vent. Using the SCD all waste bags are replaced with new sterile bags. The purified bulk material is transferred from the chromatography part to the concentration / diafiltration part.

The concentration is carried out as follows. The feed tank is filled with the purified bulk material and gently stirred at 50 rpm. The feed pump is set at crossflow rate of 240 - 360 LMH (e.g., 4-6 L/min/$m^2$) for Pellicon 2 cartridges, or 360 to 600 LMH for Hydrosart in full recycle mode. The feed flow, retentate pressure and the permeate flow are adjusted to obtain a constant TMP of 0.4 - 0.5 bar. The permeate outlet is opened. The TFF is performed until the feed volume is concentrated 10 times. The feed pump is stopped and the permeate port is closed. The permeate waste bag is replaced with a new sterile bag. The concentrated bulk material remains in the feed/retentate recycle tank for the diafiltration. The volume of the concentrated bulk material is 1/10 of the purified bulk material.

After concentration the diafiltration is performed by adding diafiltration buffer to the feed container at the same rate as permeate is removed from the process. The TFF system is configured to the constant volume diafiltration mode. The feed pump is set at crossflow rate of 240 - 360 LMH, or 360 to 600 LMH for Hydrosart, in full recycle mode. Adjust the feed flow, retentate pressure and the permeate flow to obtain a constant TMP of 0.4 - 0.5 bar. The permeate outlet is opened and the permeate flow rate is measured. The diafiltration buffer is added to the feed tank at the same flow rate as the permeate is removed. In total 5 diavolumes are used. The end volume is 1/10 of the purified bulk material. The diafiltration is stopped. The MV particles are now present in a formulation buffer and can be transferred to fill.

**Example 9: Generation of cellular inoculum/Expression of Measles viruses (MVs)**

An important parameter in process design for anchorage dependent cells is the availability of a robust and scalable seed train. Using classical T-flasks puts a high work load to the operators of such a process and the usage of stirred formats such as spinner flasks and seed bioreactors in combination with microcarrier bear severe problems caused by bead-to-bead transfers. To address these disadvantages, a Vero cell seed train was developed that is fully based on static cultures using multi-layer flasks in different formats. This setup gives a high degree of flexibility and reduces the number of handling steps thereby eliminating the risk of possible process failure. Within 10 days cells for the inoculation of a 3 L bioreactor can be generated starting from a cell bank, using only two steps. Using a similar setup cell numbers can be expanded within 14 days for the inoculation of a 10 L bioreactor in 3 steps. To reduce the number of handling steps, robotics can be implemented into the process for handling of CellStacks to inoculate industrial scale bioreactors in short time frames.

In detail, expression of MVs was performed in Vero cells grown in VP-SFM medium supplemented with 4 mM L-Glutamine and 0.2 % (m/v) Kolliphor P188 under serum-free conditions. For passaging, cells were washed with PBS buffer and detached using TrypLE recombinant trypsin.

A vial of a Vero development cell bank was thawed and cell numbers were increased using 5-layer cell culture Multi-Flasks and 10-layer CellStacks. Bioreactor runs were performed in a BioFlo 320 system (Eppendorf, Germany), using a 5-L vessel (1.3 - 3.8 L working volume) equipped with standard pH, DO and temperature probes and in addition a capacitance probe for enabling online cell density measurements (Hamilton, Switzerland). Temperature was kept at 36.5 °C during growth phase and reduced to 32.5 °C after infection. The pH was set at 7.2, dissolved oxygen was kept at 40% air saturation and agitation was kept at 40 rpm. Cytodex-1 microcarrier concentration (GE Healthcare, Uppsala, Sweden) was set to 3 g/L and the working volume was kept at 3 L.

After cells reached confluence a medium exchange was performed and cells were infected with MV at a multiplicity of infection of 0.001. The genomic vector comprised a nucleic acid construct containing a nucleic acid encoding CHIKV structural protein being operably linked to a nucleic acid encoding the sequence of full-length, infectious antigenomic (+) RNA strand of MV (cf., for example SEQ ID NO:5).

In order to enhance separation of the virus containing supernatant from cellular debris, the microcarrier were allowed to settle down and the supernatant was clarified using a 3 μM Sartopure PP3 filter cartridge (Sartorius, Germany).

Though the examples were carried out using cells and virus particles characterized by a definite sequence of nucleic acids and/or amino acids, the person skilled in the art will readily appreciate that the general concept disclosed herein is fully applicable to other cells and viruses and in particular to large, pleomorphic viruses. Hence, the person skilled in the art is capable to readily select and/or design further cells and virus particles, respectively, having other sequences than those explicitly described herein, which cells and virus particles, respectively, are suited to be used in the context of the method and system of the present invention.

**Example 10: Production of MV using an STR setup**

Due to the adherent nature of Vero cells a bioreactor should provide specific surfaces for the cells to grow on such as fibre-cell matrices or different types of microcarrier. Microcarrier have in the past been used for cell culture processes and they have been shown to be suitable for the cultivation of Vero cells even at large scales. However, the exact type and concentration of microcarriers remained to be elucidated. Therefore, different types and concentrations of microcarrier were evaluated. 3 g/l Cytodex I turned out to be ideal for growing Vero cells in supplemented VP-SFM medium under serum-free conditions (data not shown).

Figure 3 shows that using the microcarrier in the method of the present invention, cells readily attach to the microcarrier within 1 hour after inoculation and continue to grow until confluence. Different inoculation cells densities were further evaluated, and $1x10^4$ cells/$cm^2$ microcarrier surface turned out to be ideal. Using lower numbers would be beneficial regarding seed-train steps but the number of microcarrier not covered with cells would then increase to a level that reduces the overall productivity of the process.

The transfer from static to stirred type culture formats did not affect cell growth. Upon attachment cells immediately started to grow until confluence resulting in a cell density of up to 1.5 x $10^6$ cells/mL 96 h post inoculation (Figure 4A). During this growth phase cells are metabolizing nutrients provided by the medium thereby producing toxic by-products such as lactate and ammonium at high levels (Figure 4B). In order to provide cells with conditions ideal for infection with and production of MV a medium renewal was performed after cell growth declined 96 h post inoculation.

After medium renewal, the temperature of the system was reduced to 32.5°C in order to prevent a decrease of viral infectivity. Different multiplicity of infections (MOI) were evaluated (data not shown). Infecting the culture with an MOI of 0.001 turned out to be best. Upon infection cells still consumed glucose provided by the fresh medium as shown in Figure 4B but cellular growth was almost stopped. During the course of infection cells start to detach from the microcarrier and finally lyse. After most cells have lysed the culture was stopped and the virus containing supernatant was clarified using a 3 μM filter. In order to assess productivity of each process run, samples were taken daily and tissue culture infectious dose 50 (TCID50) was determined.

Figure 5 shows that viral titers increase constantly during production process and reach a top level of up to 1 x $10^6$ $TCID_{50}$/mL 144 h post infection. After clarification the titer slightly increased which can be explained by a breakup of viral aggregates during the filtration process.

**Example 11: On-line monitoring of STR based production process**

When virus particles are produced using host cells attached to a microcarrier, it is difficult to determine cell growth on-line. However, a lack of on-line data limits the possibility to understand and control the process in detail. It has now been surprisingly found that a probe can determine viable cell density based on permittivity measurements, even when the cells are attached to the microcarrier. Viable cells are considered to behave like little capacitors and hence their polarization and depolarization in an alternating electrical field to correlate to the viable cell density. A great advantage of such a principle is that only live cells are measured, so that the measurement is deteriorated neither by cellular debris nor by the microcarrier.

Figure 6 shows progression of cell densities measured by off-line techniques and on-line permittivity measurements. The on-line signal immediately reacts on the inoculation of the bioreactor and continuously increases upon confluence of the cells on the microcarrier and depletion of the medium. Off-line values are in line with on-line counterparts and deviations can be explained by technical issues during off-line measurements. 96 h post inoculation medium is exchanged and the culture is infected with MV. This causes a stop in cell growth followed by a detachment and lysis of the cells.

Using data provided by the probe, it is possible to precisely determine the ideal infection and harvest time points. A decrease of the signal correlates with a decrease in the total amount of viable cells in the system. Under the prerequisite that all process parameters are within defined specifications, the first decrease of permittivity signals in the course of infection can therefore be defined as ideal point of infection. Similar considerations can be made for harvesting the culture. If the signal falls below a certain threshold most of the cells are dead and the culture is ready for further processing. Predicting the ideal harvest time point is crucial for further downstream processing regarding highest possible yields and lowest possible contaminations due to lysed cells.

**Example 12: Chromatography**

Chromatographic experiments were performed on an ÄKTA explorer 100 equipped with a P-960 sample-pump and fraction collector (Frac-950) (GE Healthcare, Uppsala, Sweden). Unicorn software 10.1 was used for control and data acquisition. Conductivity, pH, and absorbance at 280 and 260 nm were monitored simultaneously. Elution fractions were collected by the fraction collector, pooled according to chromatogram and stored at -80°C. For equilibration (mobile phase A) 50 mM HEPES, pH 7.5 was used and for elution and regeneration (mobile phase B) 50 mM HEPES, 2 M NaCl pH7.5. Optional sanitization was performed with 1 M NaOH.

Preliminary experiments were performed with membrane absorbers providing anion-exchange ligands NatriFlo HD-Q Recon (Column volume (CV): 0.8 mL, Natrix Separations, Burlington, ON, Canada) and Sartobind Q (CV: 1 mL, Sartorius Stedium Biotech, Goettingen, Germany). Two resin-based ion-exchangers Toyopearl Sulfate-650F (cation-exchanger, Tosoh Bioscience, Stuttgart, Germany) and Eshmuno Q (anion-exchanger, Merck KGaA, Darmstadt, Germany) and a flow-through chromatography medium, Capto Core 700 (GE Healthcare, Uppsala, Sweden) were tested. Ion-exchange medium was packed in Tricorn 5/100 columns (Eshmuno Q: CV: 1.7 - 1.9 mL; Toyopearl Sulfate-650F: CV: 2.0 mL) and Capto Core 700 was packed in Tricorn 5/50 columns (CV: 1.1 - 0.9 mL). All columns were packed according to the medium manufacturer's instructions and loaded with bulk harvest material thawed at 37°C. The detailed experimental performance parameters are summarized in Table 14.

Preparative purifications of MVs were conducted by flow-through chromatography using Capto Core 700 packed in Tricorn 5/50 or XK 16/20 columns (GE Healthcare, Uppsala, Sweden) according to the medium manufacturer's instruction. Bulk harvest material, optionally endonuclease treated, was directly loaded on the column. Equilibration and wash was performed each for 10 CV with 50 mM HEPES, 150 mM NaCl pH 7.5. For regeneration (10 CV) 50 mM HEPES, 2 M NaCl pH 7.5 was used. Optionally, a sanitization step was performed with 1 M NaOH for 10 CV hold. The flow velocity was adjusted to obtain a constant residence time of about 4 min throughout all purifications. The eluted product material was kept at -80°C for storage.

**Example 13: Preliminary screening experiments**

Different stationary phases were screened for purification of MV (MVs) allowing a direct loading procedure of filtered (clarified), and optionally endonuclease treated, cell culture supernatant. Two anion-exchange membrane adsorbers (NatriFlo HD-Q Recon and Sartobind Q) and three bead based resins, operated in flow through, cation- or anion-exchange mode (CaptoCore 700, Toyopearl Sulfate-650 F, Eshmuno Q) were tested.

Table 14: Summary of preliminary screening experiments

| Stationary phase | NatriFlo HD-Q Recon | Sartobind Q | Toyopearl Sulfate-650F | Eshmuno Q | Capto Core 700 |
|---|---|---|---|---|---|
| Column volume (mL) | 0.8 | 1 | 2 | 1.7 | 1.1 |
| Loading volume (CV) | 50 | 50 | 6.6 | 20 | 20 |
| Flow rate (mL/min) | 4 | 10 | 0.5-0.08 | 0.5-0.01 | 0.2 |
| Flow velocity (cm/h) | - | - | 153-25 | 155-3 | 62 |
| Equilibration | 50 CV 7.5 % B | 50 CV 7.5 % B | 10 CV 7.5 % B | 10 CV 7.5 % B | 10 CV 7.5 % B |
| Wash | 40 CV 7.5 % B | 40 CV 7.5 % B | 3.5 CV 7.5 % B | 10 CV 7.5 % B | 10 CV 7.5 % B |
| Elution | SGE: 40 CV each 7.5/12.5/25/50% B | SGE: 20 CV each 7.5/12.5/25/50/75% B | LGE: 7.5-50% B in 20 CV | LGE: 7.5-50% B in 20 CV | - |
| Regeneration | 40 CV 100% B | 20 CV 100% B | 10 CV 100% B | 5 CV 100% B | 10 CV 100% B |
| Recovery MV (%) | < 1.0% | < 1.0% | 1.2% | 17.0% | 61.1% |

SGE: Step gradient elution
LGE: Linear gradient elution

Notably, all methods except for the Capto Core 700 procedure were performed in the bind-elute mode as control and to compare performance. NatriFlo has a specific physical structure, as it is a 3-D macroporous hydrogel and thus has a specific structure. Principally, NatriFlo can be use in the bind-elute as well the flow-through mode.

The purification processes based on both membrane adsorbers and on the cation-exchange medium yielded very low infective virus recoveries (≤ 1%; Table 14). When the anion-exchange medium (Eshmuno Q) was used the active MVs were captured from the filtered cell culture supernatant and 17% of infective virus eluted during a linear salt gradient (150 - 1,000 mM NaCl). Majority of MVs (11.1%) eluted at the end of the gradient at high salt concentrations between (73.4 - 97.8 mS/cm). Whereas majority of protein impurities did not bind under these conditions and were present in the flow-through and wash fraction (43.5% of total loaded proteins). Majority of dsDNA impurities (63.0% of total loaded dsDNA impurities) eluted at intermediate salt concentrations (17.1 -73.4 mS/cm) prior to MVs together with another portion of 32.0% of total loaded proteins. During the wash step and the gradient elution the column back pressure increased and exceeded the maximum column pressure (data not shown). This might primarily be caused by high DNA concentrations. A benzonase or more generally DNAse digestion may thus result in a better viscosity of the material to be purified. Therefore, the flow rate initially used during the loading procedure (0.5 mL/min) could not be kept constant throughout the elution. The flow rate had to be reduced to the minimum of 0.01 mL/min to enable elution of active viruses. In contrast the purification operated in flow-through mode (Capto Core 700) enabled the capture of major contaminants and MVs were collected in the flow-through fraction. This process resulted in a high MVs recovery of 61.1%, in combination with a 75.1% dsDNA and 73.1% total protein impurity depletion. Although, the column back pressure increased exponentially during loading, the maximum column pressure indicated by the manufacturer was not exceeded in this case. Consequently, a consistent flow rate (0.2 mL/min) throughout the purification process was possible.

These screening experiments demonstrated that a chromatography operated in flow-through mode enables high recoveries of infectious virus. In contrast, all tested conventional bind-elute chromatography steps resulted in insufficient recoveries. Based on the above screening experiments results Capto Core 700 was selected as an exemplarily stationary phase material suited for performing chromatography in flow through mode.

**Example 14: Development and evaluation of the endonuclease treatment step**

Majority of free dsDNA impurities can optionally be removed by an endonuclease treatment step, e.g., using Benzonase® endonuclease. For the design and development of a dsDNA digestion procedure the efficiency of two Benzonase® activities (500 U/mL or 50 U/mL) were compared at 37°C. When 500 U/mL were used the DNA level could be reduced by 75.8% from 195 ng/mL to 47 ng/mL. When 50 U/mL were used the DNA level could be reduced by 67.0% from 251 ng/mL to 83 ng/mL. After selection of the appropriate endonuclease concentration different endonuclease incubation temperatures were tested (Table 15).

Table 15: Viral titer and dsDNA content during endonuclease treatment at different incubation temperatures.

| | before digestion | |
|---|---|---|
| Temperatur | dsDNA (ng/mL) | Viral titer ($log_{10}$ TCID50/mL) |
| 37°C | 2367.9 | 6.5 |
| 4°C | 188.9 | 5.5 |
| Room temperature | 2455.62 | **6.5** |

| | after digestion | |
|---|---|---|
| Temperatur | dsDNA (ng/mL) | Viral titer ($log_{10}$ TCID50/mL) |
| 37°C | 127.7 | 6.4 |
| 4°C | 183.9 | 5.5 |
| Room temperature | **224.0** | **6.5** |

| | Efficiency endonuclease treatment | |
|---|---|---|
| Temperatur | dsDNA reduction % | Recovery viral titer (%) |
| 37°C | 94.6 | 81.6 |
| 4°C | 2.7 | >99.9 |
| Room temperature | 90.9 | >99.9 |

Endonuclease treatment is most efficient at 37°C, whereas digestion is extremely slow at 4°C. However, it needs to be considered that digestion at lower temperatures sustains the viral infectivity. Furthermore, different stop reagents were tested and the effect on the virus infectivity and the dsDNA content were compared. Addition of 5 mM EDTA did not affect the virus infectivity and the amount of dsDNA could be reduced by 94% at 37°C within one hour of incubation time. An endonuclease step may be accomplished at a variety of different temperatures depending on the enzyme used and the material to be treated. A digestion at lower temperatures (room temperature, or even below) can thus also be suitable.

**Example 15: Flow-through chromatography**

A flow-through chromatography procedure was developed to purify MVs and capture majority of impurities directly from the filtered cell culture supernatant. Preliminary experiments were operated at 1 mL scale, using Tricorn 5/50 columns. The filtered cell culture supernatant was either directly loaded onto the stationary phase material (Figures 7A and 7B) or a previous endonuclease treatment step was included (Figures 7C and 7D). The MVs passed through the stationary phase material and did not bind thereto. The Western blot analysis (Figures 7B and 7D) and the infectivity measured by TCID50 (Table 16) demonstrated that majority of infective MVs eluted in the flow-through fractions, whereas the majority of protein and dsDNA impurities were captured.

Table 16: Mass balance of MVs purification by flow-through chromatography in 1 mL scale using Tricorn 5/50 columns. Loading material was (A) solely filtered or (B) filtered and endonuclease treated cell culture supernatant.

A) Flow-through chromatography with filtered cell culture supernatant

| | Volume (mL) | Viral titer ($log_{10}$ TCID50/mL) | % | Total protein ($\mu$g/mL) | % |
|---|---|---|---|---|---|
| BH = Load | 29.7 | 6.6 | 100.0 | 123 | 100.0 |
| **FT (1-9)** | **32.0** | **6.0** | **27.6** | **30** | **26.7** |
| W | 4.0 | 4.4 | 0.1 | 32 | 3.5 |
| R | 6.0 | 4.3 | 0.1 | 36 | 5.9 |
| Mass balance chromatography | | | 27.8 | | 36.1 |

| Total protein/$10^5$ inf. particles (pg/1x$10^5$ part.) | dsDNA (ng/mL) | % | dsDNA/$10^5$ inf. particles (ng/x$10^5$ part.) |
|---|---|---|---|
| 3 | 2161 | 100.0 | 59 |
| **3** | **614** | **30.6** | **66** |
| 140 | 23 | 0.1 | 100 |
| 187 | 2440 | 22.8 | 12776 |
| | | 53.6 | |

B) Flow-through chromatography with filtered and enduculase treated cell culture supernatant

| | Volume (mL) | Viral titer ($log_{10}$ TCID50/mL) | % | Total protein ($\mu$g/mL) | % |
|---|---|---|---|---|---|
| BH | 32.2 | 6.8 | 100.0 | 81 | 100.0 |
| Load | 39.7 | 6.2 | 29.6 | 100 | 152.0 |
| **FT (1-11)** | **43.7** | **5.8** | **13.9** | **17** | **27.8** |
| W | 5.0 | 3.9 | 0.0 | 12 | 2.2 |
| R | 4.5 | 4.4 | 0.1 | 9 | 1.6 |
| Mass balance chromatography | | | 14.0 | | 31.7 |

| Total protein/$10^5$ inf. particles ($\mu$g/ 1x$10^5$ part.) | dsDNA (ng/mL) | % | dsDNA/$10^5$ inf. particles (ng/x$10^5$ part.) |
|---|---|---|---|
| 1 | 2118 | 100.0 | 32 |
| 6 | 80 | 4.6 | 5 |
| **2** | **40** | **2.5** | **6** |
| 134 | 0 | 0.0 | 5 |
| 34 | 151 | 1.0 | 555 |
| | | 3.5 | |

The process including an endonuclease treatment step prior to the flow-through chromatography resulted in viruses of even higher purity compared to the purification without an endonuclease treatment step. Furthermore, the column pressure increase during the loading could thereby be minimized.

In both cases the protein level of the virus (FT) fraction could be reduced to a comparable level of 3 μg/1x10$^5$ infective particles (without endonuclease treatment) and 2 μg/ 1x10$^5$ infective particles (with endonuclease treatment), whereas the dsDNA level in the virus fraction could be decreased by 11-fold, from 66 ng/1x10$^5$ infective particles to 6 ng/1x10$^5$ infective particles, only when applying the endonuclease treatment step.

Overall, the recovery of MVs was only between 27.6 and 13.9% and no closed mass balance could be obtained. This indicates that MVs were tightly bound to the chromatography medium or to other column/system components. To investigate this phenomenon, the used column filter frits, were treated with reducing agents for 30 min at 100°C (F1: LDS-sample buffer; F2: SDS-running buffer) to dissolve strongly bound compounds. Western blot analysis of this fractions (Figure 7D samples F1 and F2) proved the presence of MVs proteins, suggesting the adsorption of MVs to the filter frit material. In this case quantification of MVs by TCID50 was not possible because of the harsh reducing agents which had to be used for the extraction. To circumvent the usage of filter based frit material the column hardware equipment was substituted from Tricorn (e.g., 7 μm porosity used herein, but also other formats can be used) to XK columns, which provide net based frits instead of filter based ones. Using a net instead of a filter, may have the advantage that the viruses cannot bind in an unspecific manner to the material. Still, suitable filters not showing unspecific binding and having an adequate pore size are also suitable.

The materials used were thus: Tricorn with a 7μm porosity - material: Ethylene propylene diene (EPDM)* Polyethylene (PE); and XK column: 10μm net porosity - Polypropylene (PP) (reinforced glass fiber), Polyamide (PA). Still, it has to be noted that porosity might be measured differently by different manufacturers.

Table 17: Mass balance of MVs purification by flow-through chromatography in 1 mL scale using a XK 16/20 column. The loading material was filtered and endonuclease treated cell culture supernatant.

| | Volume (mL) | Viral titer ($log_{10}$ TCID50/mL) | % | Total protein (μg/mL) | % |
|---|---|---|---|---|---|
| BH | 27.9 | 5.6 | 100.0 | 214 | 100.0 |
| Load | 34.4 | 5.4 | 85.9 | 173 | 99.8 |
| **FT** | **38.4** | **5.4** | **95.0** | **23** | **14.5** |
| W | 4.0 | 4.1 | 0.4 | nd | - |
| R | 6.5 | 1.7 | 0.0 | nd | - |
| Mass balance chromatography | | | 95.4 | | 14.5 |

| Total protein/10$^5$ inf. particles (μg/1x10$^5$ part.) | dsDNA (ng/mL) | % | dsDNA/10$^5$ inf. particles (ng/x10$^5$ part.) |
|---|---|---|---|
| 55 | 196 | 100.0 | 50 |
| 63 | 38 | 24.3 | 14 |
| **8** | **21** | **14.8** | **8** |
| - | nd | - | - |
| - | 5 | 0.6 | 8626 |
| | | 15.4 | |

n.d.: not detectable

Using the XK column housing enabled the elution of the entire loaded amount of MVs and a closed mass balance could be calculated (Table 17). However, 85.3% of total loaded protein and the 8.9% of total loaded dsDNA impurities could not be regenerated from the column using 2 M NaCl or even 1 M NaOH. This indicates strong hydrophobic or irreversible binding of those impurities to the stationary phase which cannot be altered by aqueous buffer conditions. Only a regeneration procedure containing 1 M NaOH and 30% Isopropanol enabled the full regeneration of residual impurities (data not shown).

### Example 16: Scale up

Based on the results gained during process development the process was scaled up to a 10.5 mL and 188.5 mL column keeping the residence time of 4 min constant. Reproducible process performance was demonstrated for four individual runs for purification of three different starting material batches (A, B and C). A typical example chromatogram at 10 mL scale and 188 mL scale and the corresponding Western blot analysis is shown in Figure 8. A detailed summary of all three runs is presented in Table 18.

Table 18: Summary of MVs purification by flow-through chromatography in three individual runs using starting material batch A, B and C.

| | Purification run | 1 | 2 | 3 | 4 |
|---|---|---|---|---|---|
| | BH batch | A | B | B | C |
| Chromatographic parameters & column characteristic | Column | XK 16/20 | XK 16/20 | XK 16/20 | XK 50/30 |
| | Column volume (mL) | 10.25 | 9.25 | 10.46 | 188.50 |
| | Loading volume (CV) | 48 | 56 | 51 | 48 |
| | Flow velocity (cm/h) | 75 | 69 | 78 | 144 |
| | Residence time (min) | 4.1 | 4 | 4 | 4 |
| Product | BH titer ($log_{10}$ TCID50/mL) | 5.4 | 5.3 | 5.1 | 5.1 |
| | Load titer ($log_{10}$ TCID50/mL) | 5.4 | 5.1 | 5.2 | 5.5 |
| | Final titer ($log_{10}$ TCID50/mL) | 5.3 | 4.8 | 5.2 | 5.4 |
| | **Recovery** (%) | **73.7** | **36.6** | **>99.9** | **>99.9** |
| Purity | BH total protein (μg/ $1x10^5$ part.) | 93 | 158 | 289 | 185 |
| | Load total protein (μg/ $1x10^5$ part.) | 86 | 224 | 174 | 75 |
| | Final total protein (μg/ $1x10^5$ part.) | 20 | 66 | 37 | 7 |
| | **Overall total protein depletion (%)** | **84.0** | **84.8** | **82.5** | **92.3** |
| | BH dsDNA (ng/$1x10^5$ part.) | 96 | 71 | 149 | 261 |
| | Load dsDNA (ng/ $1x10^5$ part.) | 29 | 55 | 40 | 72 |
| | Final dsDNA (ng/ $1x10^5$ part.) | 26 | 55 | 29 | 50 |
| | **Overall dsDNA depletion (%)** | **80.1** | **72.0** | **73.2** | **57.9** |

In summary, the process enables consistent and reproducible total protein depletion between 82.5 and 92.3% and dsDNA depletion between 57.9 and 80.1%. The residual dsDNA content in the final MVs fraction varies from 26 to 55 ng dsDNA/$1x10^5$ infective particles (Table 18). The total protein content in the MVs varies from 7 to 66 μg total protein /$1x10^5$ infective particles (Table 18). The MVs concentration stays constant during the chromatographic step and typically the total amount of loaded virus was fully recovered. Deviations of the impurity level normalized to $1x10^5$ infective particles might be ascribed to the variations in the virus titer determination. In general, the methodological error of TCID50 values which is within 0.5 $log_{10}$ TCID50/mL.

**Example 17: Ultrafiltration/Diafiltration**

After chromatographic purification and depletion of majority of impurities the process volume was reduced and the buffer composition was adjusted during an ultrafiltration/diafiltration step by tangential flow filtration. Three ultrafiltration membranes, one hollow fiber (HF) module, one membrane cassette based on composite regenerated cellulose (RC) and one membrane cassette based on stabilized cellulose based membrane (SC) were compared in terms of productivity and final product purity.

More specifically, ultrafiltration/diafiltration was operated by ÄKTA flux s (GE Healthcare, Uppsala, Sweden).

300 kDa composite regenerated cellulose (RC) cassettes (Pellicon® XL, Merck KGaA, Darmstadt, Germany), 300 kDa polysulfone hollow fibre (HF) modules (Start AXM Cartridge, GE Healthcare, Uppsala, Sweden) and 30 kDa stabilized cellulose (SC) based membranes (Hydrosart®, Sartorius, Göttingen, Germany) were tested. A membrane area of 50 $cm^2$ was tested and the membrane feed was kept constant at 110 $L/m^2$. The transmembrane pressure was kept constant throughout the process between 0.4 to 0.5 bar by adjusting a retentate pressure control valve. Membranes were pre-treated and flushed as described in the particular manufacturer's instructions before the experiments. Feed material was chromatographically pre-purified using Capto Core 700 and frozen material was thawed at 37°C. Throughout the filtration experiments samples were collected from the feed material (F), the concentrate (C) and the permeate (P) at stored at -80°C.

The ultrafiltration and diafiltration was performed at a constant, low transmembrane pressure (0.4 - 0.5 bar) to maintain conditions which do not harm the shear-sensitive viruses. Consequently, the permeate flux was decreased during the filtration. The volume of the virus material purified by flow-through chromatography was reduced about 12-fold using the HF module (Figures 9A and 9B) and about 9-fold using the RC membrane (Figures 9C and 9D).

Table 19: Comparison of ultrafiltration MVs material by HF module, RC membrane and SC membrane.

| | Membrane type | HF | RC | SC |
|---|---|---|---|---|
| | Membrane area ($cm^2$) | 50 | 50 | 50 |
| Volume | Total dead volume (membrane & system) (mL) | 15 | 33 | 32 |
| | Feed volume (mL)* | 565 | 583 | 496 |
| | Concentrate volume (mL)* | 45 | 63 | 53 |
| | Volume reduction factor* | 12.6 | 9.3 | 9.4 |
| | Throughput ($L/m^2/h$)* | 21.7 | 34.3 | 71.4 |
| Product | Titer Feed ($log_{10}$ TCID50/mL) | 5.2 | 5.3 | 5.9 |
| | Titer Concentrate ($log_{10}$ TCID50/mL) | 5.9 | 6.2 | 6.9 |
| | Concentration factor | 5.4 | 8.5 | 12.5 |
| | **Step recovery (%)** | **43.9** | **99.9** | > **99.9** |
| Purity | Feed total protein ($\mu g/1x10^5$ part.) | 33 | 23 | 25 |
| | Concentrate total protein ($\mu g/1x10^5$ part.) | 38 | 27 | 13 |
| | **Massbalance total protein (%)†** | **58.3** | **> 99.9** | **75.9** |
| | Feed dsDNA ($ng/1x10^5$ part.) | 33 | 36 | 108 |
| | Concentrate dsDNA ($ng/1x10^5$ part.) | 64 | 40 | 56 |
| | **Massbalance dsDNA (%)†** | **92.2** | **> 99.9** | **69.7** |

* Total dead volume was included for calculation
† Massbalance = concentrate + permeate

As shown in Table 19, when the cellulose based membranes (RC and SC) were used the entire loaded virus amount could be recovered again. The viral titer was concentrated 8.5 up to 12.5 times. The highest average throughput of 71.4 LMH was achieved using the SC membrane, resulting in significantly reduced process time in comparison to HF module and RC membrane. During diafiltration for five volume exchanges the viral titer was not significantly affected in any of these systems. It has to be noted that RC/SC and HF structurally represent completely different materials. HF is structured as fibres, whereas the cellulose-based RC/SC are flat sheets which may be packed in cassettes,

**Example 18: Process summary**

A summary of the above exemplified process is shown in Figure 10, which shows a process scheme according to an embodiment of the method of the present invention. Recoveries (yields) in terms of TCID50/mL as well as relative recoveries calculated from TCID50/mL are indicated for each step. Furthermore, relative amounts of impurities, specifically total protein content and dsDNA content, are indicated. As it is known in the relevant technical field, the calculation of TCID50 values can be associated with a certain error range (0.5 log). For the determination of impurities (protein, DNA etc.) rather sensitive determinations methods are available.

**Example 19: Testing**

In examples 1 to 18, reference is made to the analysis techniques, which were carried out as follows unless otherwise dictated by context.

*Sodium dodecyl sulfate-polyacrylamide gel electrophoresis (SDS-PAGE) and Western blot analysis*

For electrophoresis under reducing conditions NuPAGE Bis/Tris gels 4-12% (Invitrogen, Carlsbad, CA, USA) and MES-SDS running conditions were used in accordance to manufacturer's instructions. If required, samples were diluted in deionized water to obtain similar protein concentrations. SeeBlue® Plus2 Pre-stained Protein Standard (Invitrogen, Carlsbad, CA, USA) or Page Ruler Plus Prestained Protein Ladder (Thermo Fisher Scientific, Waltham, MA USA) was used as the protein molecular weight ladder. Protein bands were stained by Coomassie Brilliant Blue G-250 based EZBlue™ Gel Staining Reagent (Sigma Aldrich, St. Louis, MO, USA) or by silver using a silver staining procedure as described by Heukeshoven et al. [3]. After SDS-PAGE, proteins were transferred onto a 0.2 μm nitrocellulose membrane using a Trans-Blot® Turbo™ Transfer System with Trans-Blot Turbo Mini Nitrocellulose Transfer Packs (Bio-Rad Laboratories, Hercules, CA, USA). After electro blotting the membrane was blocked with 3% BSA in PBS-T (0.1% w/v Tween-20 in PBS) for 2 h at room temperature. For detection a mouse monoclonal antibody against Measles NP (3E1) (Santa Cruz Biotechnology, Dallas, TX, USA) with a dilution of 1:1000 (in PBS-T containing 1% BSA) was incubated for 2 h at room temperature. The secondary antibody, anti-mouse IgG conjugated with alkaline phosphatase (Sigma Aldrich, St. Louis, MO, USA), was diluted 1:1000 in PBS-T with 1% BSA and was incubated for 1 h at room temperature. Visualization of bands carried out by BCIP®/NBT solution (Sigma Aldrich, St. Louis, MO, USA), according to manufacturer's instructions. Wash steps between individual steps were performed three times with 1% BSA in PBS-T for 10 min each.

*Total protein concentration and dsDNA concentration*

The total protein concentration was determined by Bradford assay using Coomassie blue G-250-based protein dye reagent (Bio-Rad Laboratories, Hercules, CA, USA). The calibration curve was obtained by bovine serum albumin (BSA) standards diluted in TE-Buffer. DNA content was determined by Quant-iT™ PicoGreen® dsDNA kit (Life technologies, Waltham, MA, USA). Protein and DNA assays were performed according to the particular manufacturer's instructions in 96-well plate format. Signals were measured by Infinite F220/M200 PRO plate reader (Tecan, Männedorf, Switzerland).

*Tissue culture invective dose 50 (TCID50)*

For determination of a virus sample two 96 well plates were seeded with 5000 Vero cells/well and incubated at 37°C for 1-2 hours. In the meantime, virus dilutions were performed. Therefore, a 96 well plate containing 200μL VP-SFM medium supplemented with antibiotics each well was prepared. 50μL virus sample was applied to row 1, mixed thoroughly and serially diluted to row 11. Row 12 served as a negative control. The cells were infected by addition of 20μL virus dilution/well and incubated at 37°C over night. 100μL supplemented VP-SFM medium was added to each well and then incubated for another 6 days. Each well was observed under a microscope for cytotoxicity or syncytia formation, if one was present the well was noted positive. With the numbers of positive and negative wells per plate a TCID 50 titerwas calculated using the Spearman-Kärberformula.

*Host cell protein (HCP) concentration*

The HCP concentration was measured by a Vero Cell HCP ELISA (Cygnus Technologies, Southport, NC, USA) according to the manufacturer's instructions. Signals were measured by Infinite F220/M200 PRO plate reader (Tecan, Männedorf, Switzerland).

*Endonuclease treatment*

The sample was treated for 1 h with Benzonase purity grade II (Merck KgA, Darmstadt Germany) at a final concentration of 50 U/mL or 500 U/mL in the presence of 2 mM $MgCl_2$ at 37 °C and 50 rpm on a shaker. Digestion was stopped during preliminary experiments by (A) 5 mM EDTA, (B) 25 mM HEPES, 1.8 M $(NH_4)_2SO_4$, pH 7.5, (C) 5 mM HEPES, 150 mM NaCl, pH 7.5 or (D) $H_2O$. The standard endonuclease treatment procedure used for preparative purposes (50 U/mL for 1 h at 37°C and 50 rpm) was topped by 5 mM EDTA.

*Transmission electron microscopy (TEM)*

Copper grids (400-mesh, Agar Scientific Ltd, Stansted, UK) coated with Pioloform film and shaded with carbon were incubated with samples for 1 min. Sample fixation was carried out with 2.5% glutaraldehyde solution for 15 min and was followed by three wash steps with water. Samples were stained with 1% uranyl acetate in 100 mM cacodylate buffer, pH 7.0 for 30 seconds [4]. The negatively stained and air dried specimens were analysed in a Tecnai G2 200 kV transmission electron microscope (FEI, Eindhoven, The Netherlands), operating at 80 keV.

**Example 20: Viscosity of MV-CHIK containing loads**

Cell culture media and buffers were purchased from Thermo Fisher Scientific (Waltham, USA). Plastic ware used in cell culture was purchased from Corning (New York, USA). All chemicals were purchased from Merck KGaA (Darmstadt, Germany) or Sigma Aldrich (St. Louis, MO, USA).

Expression of MVs was performed in Vero cells grown in VP-SFM medium supplemented with 4 mM L-Glutamine and 0.2 % (m/v) Kolliphor P188 under serum-free conditions. For passaging, cells were washed with PBS buffer and detached using TrypLE recombinant trypsin.

A vial of a Vero development cell bank was thawed and cell numbers were increased using 5-layer cell culture Multi-Flasks and 10-layer CellStacks. Bioreactor runs were performed in a BioFlo 320 system (Eppendorf, Germany), using a 5-L vessel (1.3 - 3.8 L working volume) equipped with standard pH, DO and temperature probes and in addition a capacitance probe for enabling online cell density measurements (Hamilton, Switzerland). Temperature was kept at 36.5 °C during growth phase and reduced to 32.5 °C after infection. pH was set at 7.2, dissolved oxygen was kept at 40% air saturation and agitation was kept at 40 rpm. Cytodex-1 microcarrier concentration (GE Healthcare, Uppsala, Sweden) was set to 3 g/L and the working volume was kept at 3 L.

After cells reached confluence a medium exchange was performed and cells were infected with MV at a multiplicity of infection of 0.001. In order to separate the virus containing supernatant from cellular debris, the microcarrier were allowed to settle down and the supernatant was clarified using a 3 μM Sartopure PP3 filter cartridge (Sartorius, Germany).

The sample was treated for 1 h with Benzonase purity grade II (Merck KgA, Darmstadt Germany) at a final concentration of 50 U/mL in the presence of 2 mM $MgCl_2$ at room temperature and 50 rpm. Digestion was stopped by adding 5 mM EDTA.

Chromatographic experiments were performed on an ÄKTA explorer 100 equipped with a P-960 sample-pump and fraction collector (Frac-950) (GE Healthcare, Uppsala, Sweden). Unicorn software 10.1 was used for control and data acquisition. Conductivity, pH, and absorbance at 280 and 260 nm were monitored simultaneously. Preparative purifications of MVs was conducted by flow-through chromatography using Capto Core 700 packed in XK 16/20 columns (GE Healthcare, Uppsala, Sweden) according to the medium manufacturer's instruction. Bulk harvest material, was filtered and endonuclease treated and directly loaded on the column. Equilibration and wash was performed for 10 CV with 50 mM HEPES, 150 mM NaCl pH 7.5. The flow velocity during purification was adjusted to obtain a constant residence time of about 4 min throughout all purifications. The purified material was collected during the flow-through fraction and was stored at -80°C.

Ultrafiltration/diafiltration was operated by ÄKTA flux s (GE Healthcare, Uppsala, Sweden). Using a 30 kDa stabilized cellulose (SC) based membrane (Hydrosart®, Sartorius, Göttingen, Germany) at a membrane feed of 110 $L/m^2$. The transmembrane pressure was kept constant throughout the process between 0.4 to 0.5 bar by adjusting the retentate pressure control valve. Membrane were pretreated and flushed as described in the manufacturer's instructions before the experiments. Feed material was chromatographically pre-purified using Capto Core 700 and frozen material (-80°C) was thawed at 37°C. After 10-fold concentration the concentrated material was diafiltrated for 5 volume-exchanges to PBS.

Viscosity of process fluid was measured at room temperature by DV-II + Pro viscometer (Brookfield Engineering Laboratories, Middleboro, MA, USA). Shear stress and corresponding shear rate data were used for evaluation of viscosity by power-law according to Ostwald-de Waele. The viscosities measured at a shear rate of 525 1/sec were selected for comparison.

As explained in the general description, the viscosity of the process stream is a critical process parameter during downstream processing, influencing the process control and process sequence. The viscosity of the individual process streams after each unit operation was analyzed and results are summarized in Figure 11.

The results demonstrated that the viscosity of concentrated material (concentrated and diafiltrated material) is significantly higher compared to the non-concentrated process fluid (bulk harvest, filtered harvest, endonuclease harvest, purified material).

**Example 21: Purification of MV-GFP**

Next, purification of MV-GFP is described using embodiments of the method of the present invention. GFP co-expressed - here as soluble protein and not as virus like particle - with MV (scaffold) proteins can serve as excellent fluorescent marker in evaluating success of infection. Afterwards, it is an impurity that needs to be removed by the downstream process.

Cell culture media and buffers were purchased from Thermo Fisher Scientific (Waltham, USA). Plastic ware used in cell culture was purchased from Corning (New York, USA). All chemicals were purchased from Merck KGaA (Darmstadt, Germany) or Sigma Aldrich (St. Louis, MO, USA).

Cell culture supernatant was obtained in an analogous manner as described for MV above in the example section with the difference that the host cells were infected by MV-GFP encoded by a nucleic acid sequence according to SEQ ID NO:4. Progress of infection and virus production was monitored by fluorescence microscopy (Figure 13A to F).

The filtered bulk harvest material (i.e. clarified cell culture supernatant) was then treated for 1 h with Benzonase purity grade II (Merck KgA, Darmstadt Germany) at a final concentration of 50 U/mL in the presence of 2 mM $MgCl_2$ at room temperature and 50 rpm. Digestion was stopped by adding 5 mM EDTA. with an endonuclease and filtered.

The Benzonase treated cell culture supernatant was then directly loaded onto a Capto Core 700 column and chromatographic purification carried out using a procedure that was substantially the same as exemplified above for MV. More specifically, chromatographic experiments were performed on an ÄKTA explorer 100 equipped with a P-960 sample-pump and fraction collector (Frac-950) (GE Healthcare, Uppsala, Sweden). Unicorn software 10.1 was used for control and data acquisition. Conductivity, pH, and absorbance at 280 and 260 nm were monitored simultaneously. Preparative purifications of MVs was conducted by flow-through chromatography using a Capto Core 700 packed in XK 16/20 column (GE Healthcare, Uppsala, Sweden) with a column volume of 9.25 mL. The column was packed according to the medium manufacturer's instruction. Bulk harvest material, was filtered and endonuclease treated and directly loaded on the column. Equilibration and wash was performed for 10 CV with 50 mM HEPES, 150 mM NaCl pH 7.5. The flow velocity during purification was adjusted to obtain a constant residence time of about 4 min. The purified material was collected during the flow-through fraction and was stored at -80°C.

For electrophoresis under reducing conditions NuPAGE Bis/Tris gels 4-12% (Invitrogen, Carlsbad, CA, USA) and MES-SDS running conditions were used in accordance to manufacturer's instructions. If required, samples were diluted in deionized water to obtain similar protein concentrations. SeeBlue® Plus2 Pre-stained Protein Standard (Invitrogen, Carlsbad, CA, USA) or Page Ruler Plus Pre-stained Protein Ladder (Thermo Fisher Scientific, Waltham, MA USA) was used as the protein molecular weight ladder. Protein bands were stained by Coomassie Brilliant Blue G-250 based EZBlue™ Gel Staining Reagent (Sigma Aldrich, St. Louis, MO, USA) or by silver using a silver staining procedure as described by Heukeshoven et al. [3]. After SDS-PAGE, proteins were transferred onto a 0.2 μm nitrocellulose membrane using a Trans-Blot® Turbo™ Transfer System with Trans-Blot Turbo Mini Nitrocellulose Transfer Packs (Bio-Rad Laboratories, Hercules, CA, USA). After electro blotting the membrane was blocked with 3% BSA in PBS-T (0.1% w/v Tween-20 in PBS) for 2 h at room temperature. For detection a mouse monoclonal antibody against Measles NP (3E1) (Santa Cruz Biotechnology, Dallas, TX, USA) with a dilution of 1:1000 (in PBS-T containing 1% BSA) was incubated for 2 h at room temperature. The secondary antibody, anti-mouse IgG conjugated with alkaline phosphatase (Sigma Aldrich, St. Louis, MO, USA), was diluted 1:1000 in PBS-T with 1% BSA and was incubated for 1 h at room temperature. Visualization of bands carried out by BCIP®/NBT solution (Sigma Aldrich, St. Louis, MO, USA), according to manufacturer's instructions. Wash steps between individual steps were performed three times with 1% BSA in PBS-T for 10 min each.

The total protein concentration was determined by Bradford assay using Coomassie blue G-250-based protein dye reagent (Bio-Rad Laboratories, Hercules, CA, USA). The calibration curve was obtained by bovine serum albumin (BSA) standards diluted in TE-Buffer. DNA content was determined by Quant-iT™ PicoGreen® dsDNA kit (Life technologies, Waltham, MA, USA). Protein and DNA assays were performed according to the particular manufacturer's instructions in 96-well plate format. Signals were measured by Infinite F220/M200 PRO plate reader (Tecan, Männedorf, Switzerland).

For the determination of a virus sample two 96 well plates were seeded with 5000 Vero cells /well and incubated at 37°C for 1-2 hours. In the meantime, virus dilutions were performed. Therefore, a 96 well plate containing 200μL VP-SFM medium supplemented with antibiotics each well was prepared. 50μL virus sample was applied to row 1, mixed thoroughly and serially diluted to row 11. Row 12 served as a negative control. The cells were infected by addition of 20μL virus dilution/well and incubated at 37°C over night. 100μL supplemented VP-SFM medium was added to each well and then incubated for another 6 days. Each well was observed under a microscope for cytotoxicity or syncytia formation, if one was present the well was noted positive. With the numbers of positive and negative wells per plate a TCID 50 titerwas calculated using the Spearman-Karber formula.

Process performance is summarized in Table 20. Here, the results obtained for MV-GFP (purification run 5) is compared to the results obtained for MV-CHIK (purification runs 1 to 4).

Table 20: Summary of MVs purification by flow-through chromatography in different runs using starting material batch A, B, C and D.

| | Purification run | 1 | 2 | 3 | 4 | 5 |
|---|---|---|---|---|---|---|
| | BH batch | A | B | B | C | D - GFP |
| Chromatographic parameters & column characteristic | Column | XK 16/20 | XK 16/20 | XK 16/20 | XK 50/30 | XK 16/20 |
| | Column volume (mL) | 10.25 | 9.25 | 10.46 | 188.50 | 9.25 |
| | Loading volume (CV) | 48 | 56 | 51 | 48 | 55 |
| | Flow velocity (cm/h) | 75 | 69 | 78 | 144 | 69 |
| | Residence time (min) | 4.1 | 4 | 4 | 4 | 4 |
| Product | BH titer ($log_{10}$ TCID50/mL) | 5.4 | 5.3 | 5.1 | 5.1 | 6.2 |
| | Load titer ($log_{10}$ TCID50/mL) | 5.4 | 5.1 | 5.2 | 5.5 | 7.0 |
| | Final titer ($log_{10}$ TCID50/mL) | 5.3 | 4.8 | 5.2 | 5.4 | 6.1 |
| | **Recovery (%)** | **73.7** | **36.6** | **>99.9** | **>99.9** | **83.6** |
| Purity | BH total protein (µg/1x10$^5$ part.) | 93 | 158 | 289 | 185 | 8 |
| | Load total protein (µg/1x10$^5$ part.) | 86 | 224 | 174 | 75 | 2 |
| | Final total protein (µg/1x10$^5$ part.) | 20 | 66 | 37 | 7 | 2 |
| | **Overall total protein depletion (%)** | **84.0** | **84.8** | **82.5** | **92.3** | **77.9** |
| | BH dsDNA (ng/1x10$^5$ part.) | 96 | 71 | 149 | 261 | na* |
| | Load dsDNA (ng/1x10$^5$ part.) | 29 | 55 | 40 | 72 | na* |
| | Final dsDNA (ng/1x10$^5$ part.) | 26 | 55 | 29 | 50 | na* |
| | **Overall dsDNA depletion (%)** | **80.1** | **72.0** | **73.2** | **57.9** | **na*** |

* The dsDNA content of a MV-GFP cannot be measured by Picogreen-Assay (fluorescent assay).

The chromatogram and the Western blot analysis and mass balance of the purification is shown in Figure 12 and Table 21.

Table 21: Mass balance of MVs purification by flow-through chromatography in 10 mL scale using a XK 16/20 column. The loading material was filtered and endonuclease treated cell culture supernatant containing MV-GFP.

| | Volume (mL) | Viral titer ($log_{10}$ TCID50/mL) | % | Total protein (µg/mL) | % |
|---|---|---|---|---|---|
| BH | 502.7 | 6.2 | 100.0 | 148 | 100.0 |
| Load | 508.8 | 7.0 | 553.8 | 150 | 102.2 |
| **FT** | **535.3** | **6.1** | **83.6** | **31** | **22.1** |
| W | 66.3 | 5.2 | 1.2 | 27 | 2.4 |
| R | 92.5 | 4.4 | 0.2 | 31 | 3.8 |
| Mass balance chromatography | | | 85.0 | | 28.2 |

n.d.: not detectable

Table 11: (continued)

| | Total protein/$10^5$ inf. particles (µg/1x$10^5$ part.) | GFP (µg/mL) | % | GFP/$10^5$ inf. particles (µg/x$10^5$ part.) |
|---|---|---|---|---|
| BH | 8 | 51.3 | 100.0 | 2.9 |
| Load | 2 | 57.1 | 112.6 | 0.6 |
| **FT** | **2** | **2.0** | **4.2** | **0.1** |
| W | 16 | 0.9 | 0.2 | 0.6 |
| R | 130 | 0.9 | 0.3 | 3.8 |
| Mass balance chromatography | | | 4.7 | |

n.d.: not detectable

The results demonstrated that the GFP tightly bounds to the column. The GFP sticks to the column which was visible by eye, due to the yellow color of GFP. The MV-containing flow-through can then be subjected to (ultra)filtration and/or buffer exchange es described above exemplarily for MV-CHIK.

**Example 22: Immunization experiments**

To evaluate the immunogenicity of the purified material, i.e. infectious virus particles derived from a MV scaffold (MV-Xp), in comparison to the crude, unpurified material (MV-Xup) two animal studies can be conducted:

1. Challenge study - lethal challenge after two immunizations

2. T cell response after one immunization

The animal model of choice would be a transgenic mouse carrying the human MV entry receptor CD46. In addition, these mice are deficient in the type 1 interferon receptor ($CD46^{tg}$/ IFNAR $^{-/-}$). In previous studies the immunogenicity of various MV/Schwarz based construct was demonstrated (MV-CHIK, MV-DVAX1, etc.). For MV-CHIK we showed that doses as low as $1x10^3TCID_{50}$ fully protect animals against a lethal dose of CHIKV. Thus, a lower dose would allow the comparison between two formulations in terms of potency. A result of this type of study would be:
Formulation A (purified, MV-Xp) protects x out of 10 mice
Formulation B (unpurified, MV-Xup) protects y out of 10 mice
For the challenge study we propose the following study set up:
$CD46^{tg}$/ IFNAR $^{-/-}$mice will receive two immunizations. The lethal challenge with the respective pathogen will show % protection against death. In addition, antibody levels as determined by ELISA can be quantified and compared.

Table 22:

| Group | No of Mice | Treatment | Dose (MV-X) | Vaccination Schedule | Challenge |
|---|---|---|---|---|---|
| 1 | 10 | MV-Xp Formulation A | $1x10^2$ | Day 0, 28 | Day 56 |
| 2 | 10 | MV-Xup Formulation B | $1x10^2$ | Day 0, 28 | Day 56 |
| 4 | 5 | MV-Schw | - | Day 0, 28 | Day 56 |

T cell study - IFNy producing cells after one immunization

Mice will be immunized with a low dose of MV-X (Formulation A (purified) or B (unpurified)) or a control MV/Schwarz. One week after immunization the mice will be sacrificed and spleenocytes will be harvested. The cells will be challenged in vitro with pathogen specific peptides and the number of interferon gamma (IFNγ) producing T cells will be determined by ELISPOT.

Table 23:

| Group | No of Mice | Treatment | Dose | Vaccination Schedule | Spleenocyte harvest |
|---|---|---|---|---|---|
| 1 | 5 | Purified MV-Xp | $1x10^3$ | Day 0 | Day 7 |
| 2 | 5 | Unpurified MV-Xup | $1x10^3$ | Day 0 | Day 7 |
| 4 | 5 | MV/Schw | - | Day 0 | Day 7 |

**Example 23: Toxicity studies in macaques**

To evaluate the safety and potential toxicity of the immunogenic and vaccine compositions as produced according to the present invention, the following experiment can be performed under good laboratory practice (GLP) conditions as pre-experiment potentially followed by Phase 1 clinical trials. One group of five male and five female purpose-bred cynomolgus macaques is treated on days 1, 22 and 36 by intramuscular route of the test immunogenic or vaccine composition. The animals are sero-negative to measles. Furthermore, animals have to be sero-negative for the antigen comprised by the MV scaffold and presented in the recombinant infectious virus particles. For MV-CHIK (e.g., SEQ ID NO:5) obtained by the method according to the present invention, treatment is performed at a dose of $1.925 \times 10^6$ $TCID_{50}$/day of injection. Two other groups of two males and two females receive the composition at doses of $1.925 \times 10^4$ or $1.925 \times 10^5$ $TCID_{50}$/day of injection. A further control group of three males and three females receives vehicle only (sterile saline). A summary of treatment groups is presented in Table 24 below. The person skilled in the art will readily be able to adapt said scheme to any recombinant infectious virus particle as immunogenic and vaccine composition purified according to the methods of the present invention.

Table 24: Summary of treatment groups for cynomolgus macaque toxicity studies

| | Males (M)/Females (F) | Dose ($TCID_{50}$/day of injection) | Volume administered (mL) |
|---|---|---|---|
| Group 1 | 3M/3F | 0 | 2.5 |
| Group 2 | 2M/2F | $1.925 X 10^4$ | 0.025 |
| Group 3 | 2M/2F | $1.925 X 10^5$ | 0.25 |
| Group 4 | 5M/5F | $1.925 X 10^6$ | 2.5 |
| $TCID_{50}$ = 50% tissue culture infective dose | | | |

At the end of the treatment period (day 37), the animals are sacrificed, except for the last two animals of each sex in Group 4, which are observed for a 13-day treatment-free period (and sacrificed on day 50). Blood samples are taken for the determination of serum levels of antibodies against the vaccine antigen, for measles serology and for haematology and biochemistry. Other assessments known to the skilled person can comprise body weight, functional observation battery, rectal temperature, ECG and ophthalmology examinations. On completion of the treatment period or treatment-free period, the animals are sacrificed and a full macroscopic *post-mortem* examination is performed. Designated organs can be weighed and selected tissue specimens can be preserved. A microscopic examination can be performed on designated tissues from Group 1 and Group 4 animals sacrificed on completion of the treatment period.

The following results are expected. No unscheduled deaths occurred during the study. There were no test item-related clinical signs during the treatment and treatment-free periods. In particular, no local reactions were reported. There were no test item-related findings at functional observation battery. There were no effects on the rectal temperature or body weight throughout the study. Qualitative and quantitative parameters at ECG examination were unaffected throughout the study. No test item-related ophthalmological findings were observed at the end of treatment or the treatment-free period. No remarkable changes were noted in haematological parameters at the end of the treatment period, while slightly increased lymphocyte counts were recorded in males and females at the end of the treatment-free period. After each round of vaccination and then in detail at the end of the treatment period injection site inflammatory lesions (e.g. increases in inflammatory mononuclear and/or granulocytic cell infiltrates or interstitial oedema).

## Claims

1. A method for producing and/or purifying measles virus (MV) particles from a sample, the method comprising in sequential order the following steps:

    (i) loading a sample containing MV particles and one or more impurities onto a stationary phase material for carrying out flow-through chromatography to bind at least a fraction of the one or more impurities contained in the sample and to produce a flow-through comprising at least a fraction of the MV particles contained in the sample; and
    (ii) carrying out filtration, preferably ultrafiltration, and obtaining a retentate having an increased MV titer relative to the MV titer comprised in the flow-through.

2. The method for producing and/or purifying MV particles according to claim 1, wherein the sample is obtained by a method comprising one or more of the following steps, preferably at least step (c), more preferably at least steps (c) and (d) and most preferably all of the following steps:

    (a) infecting at least one host cell with a virus stock comprising at least one MV particle;
    (b) incubating the at least one host cell infected with the virus stock to allow virus production;
    (c) obtaining a cell culture supernatant containing MV particles and one or more impurities;
    (d) clarifying the cell culture supernatant to obtain a clarified cell culture supernatant.

3. The method for producing and/or purifying MV particles according to claim 1 or 2, wherein the sample loaded onto the flow-through chromatography unit has a MV titer less than 5 times higher, preferably less than 4 times higher, more preferably less than 3 times higher and most preferably less than 2 times higher, than the MV titer in the cell culture supernatant obtained in step (c) or in the clarified cell culture supernatant obtained in step (d), and/or wherein the sample loaded onto the flow-through chromatography unit has a host cell protein content of at least 50 %, preferably at least 60 %, more preferably at least 70 % and most preferably at least 80 % relative to the host cell protein content in the cell culture supernatant obtained in step (c) or in the clarified cell culture supernatant obtained in step (d).

4. The method for producing and/or purifying MV particles according to any one of the preceding claims,
wherein the sample containing MV particles as defined in claim 1, or the cell culture supernatant obtained in step (c) as defined in claim 2, or the clarified cell culture supernatant obtained in step (d) as defined in claim 2, is directly loaded onto the stationary phase material for carrying out flow-through chromatography, and/or
wherein no concentration step and/or no buffer exchange step and/or no host cell protein removal step occurs in between.

5. The method for producing and/or purifying MV particles according to any one of the preceding claims,
wherein the MV titer in the flow-through is at least 20 %, preferably at least 30 %, more preferably at least 40 % and most preferably at least 50 %, or even at least 70 %, preferably at least 80 % and most preferably at least 90 %, of the MV titer in the sample loaded onto the stationary phase material for carrying out flow-through chromatography; and/or
wherein the host cell protein content in the flow-through is 60 % or less, preferably 50 % or less, more preferably 40 % or less and most preferably 30 % or less, or even 25 % or less and preferably 20 % or less, relative to the host cell protein content in the sample loaded onto the stationary phase material for carrying out flow-through chromatography; and/or
wherein the polynucleotide content in the flow-through is 70 % or less, preferably 60 % or less, more preferably 50 % or less and most preferably 60 % or less, relative to the polynucleotide content in the sample loaded onto the stationary phase material for carrying out flow-through chromatography.

**6.** The method for producing and/or purifying MV particles according to any one of the preceding claims,
wherein the flow-through chromatography step involves column chromatography;
wherein the stationary phase material is preferably selected from the group consisting of at least a resin, at least a matrix, at least a gel and, preferably, beads; and
wherein the stationary phase material, preferably the beads, has/have preferably size-exclusion functionality, hydrophobic interaction functionality or ion exchange functionality, or a combination thereof, preferably size-exclusion functionality.

**7.** The method for producing and/or purifying MV particles according to any one of the preceding claims,
wherein the stationary phase material, preferably the beads, comprises/comprise a ligand-activated core, and an inactive shell comprising pores,
wherein the ligand-activated core preferably comprises octylamine,
wherein the pores have a molecular weight cut off smaller than the MV particles to exclude the MV particles from entering the ligand-activated core,
wherein a molecule smaller than the molecular weight cut off can enter the pores and bind to the ligand-activated core, and
wherein the molecular weight cut off is preferably in the range of 100 kDa to 2000 kDa, preferably 200 kDa to 1500 kDa, more preferably 400 kDa to 1200 kDa and most preferably 500 kDa to 1,000 kDa.

**8.** The method for producing and/or purifying MV particles according to any one of the preceding claims,
wherein the flow-through is directly used as a feed for the filtration.

**9.** The method for producing and/or purifying MV particles according to any one of the preceding claims,
wherein simultaneously with or sequentially to the filtration the retentate buffer is exchanged.

**10.** The method for producing and/or purifying MV particles according to any one of the preceding claims,
wherein the filtration and, if present, the retentate buffer exchange involves tangential flow filtration,
wherein the tangential flow filtration is preferably carried out at a transmembrane pressure in the range of 0.1 to 1 bar, preferably 0.2 to 0.8 bar, more preferably 0.3 to 0.6 bar and most preferably 0.4 to 0.5 bar, and
wherein the transmembrane pressure is preferably maintained within the range throughout the filtration and, if present, the retentate buffer exchange.

**11.** The method for producing and/or purifying MV particles according to any one of the preceding claims,
wherein the MV particles are selected from the group consisting of live, attenuated and inactivated virus particles, or a mixture thereof; and/or
wherein the MV particles are recombinant and/or infectious particles, preferably infectious recombinant particles.

**12.** A system for producing and/or purifying MV particles, the system comprising:

(i) at least one bioreactor;
(ii) a clarification unit, preferably a dead end filter unit, downstream to the bioreactor;
(iii) a flow through chromatography unit downstream to the clarification unit; and
(iv) a filtration unit, preferably a tangential flow filtration unit, downstream to the flow through chromatography unit.

**13.** The system for producing and/or purifying MV particles according to claim 12,
wherein the bioreactor includes a probe for determining viable cell density based on permittivity,
wherein the probe preferably allows for online determination of the viable cell density.

**14.** The system for producing and/or purifying MV particles according to claim 12 or 13,
wherein no concentration unit, preferably no tangential flow filtration unit, is arranged between the at least bioreactor and the flow through chromatography unit.

**15.** The system for producing and/or purifying MV particles according to any one of claims 12 to 14,
wherein the clarification unit, preferably the dead-end filter unit, is either in direct fluid communication with the bioreactor or via a first vessel; and/or
wherein the flow through chromatography unit is either in direct fluid communication with the clarification unit, preferably the dead-end filter unit, or via a second vessel; and/or
wherein the filtration unit, preferably the tangential flow filtration unit, is either in direct fluid communication with the flow through chromatography unit, or via a third vessel; and preferably
wherein the first vessel is a storage vessel or a mixable vessel, and/or the second vessel and/or the third vessel is a storage vessel.

Figure 1

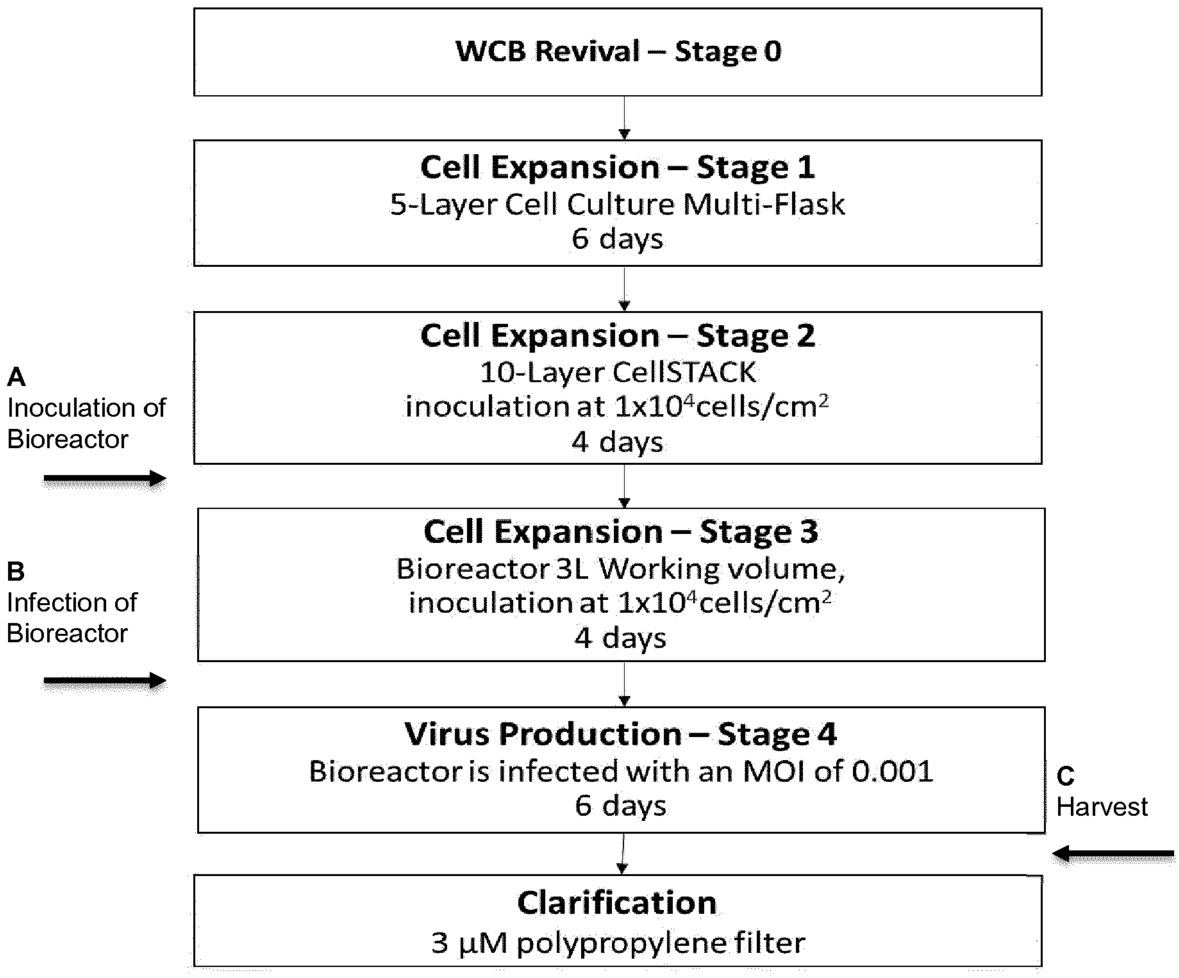
WCB Revival – Stage 0
Cell Expansion – Stage 1
5-Layer Cell Culture Multi-Flask
6 days
Cell Expansion – Stage 2
10-Layer CellSTACK
inoculation at 1x10^4 cells/cm^2
4 days
A
Inoculation of Bioreactor
Cell Expansion – Stage 3
Bioreactor 3L Working volume,
inoculation at 1x10^4 cells/cm^2
4 days
B
Infection of Bioreactor
Virus Production – Stage 4
Bioreactor is infected with an MOI of 0.001
6 days
C
Harvest
Clarification
3 µM polypropylene filter

Figure 2

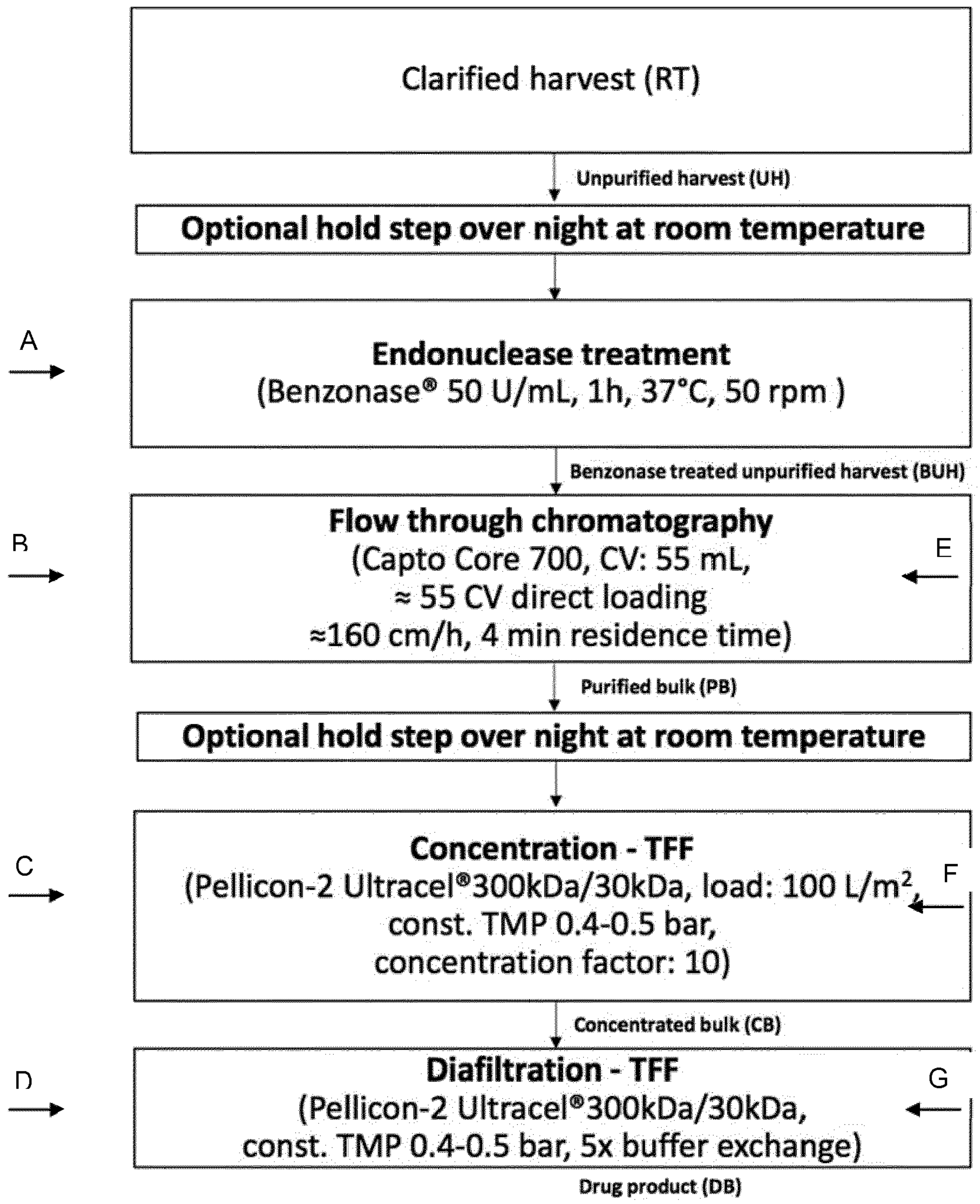
Clarified harvest (RT)
Unpurified harvest (UH)
Optional hold step over night at room temperature
A
Endonuclease treatment
(Benzonase® 50 U/mL, 1h, 37°C, 50 rpm )
Benzonase treated unpurified harvest (BUH)
B
Flow through chromatography
(Capto Core 700, CV: 55 mL,
≈ 55 CV direct loading
≈160 cm/h, 4 min residence time)
E
Purified bulk (PB)
Optional hold step over night at room temperature
C
Concentration - TFF
(Pellicon-2 Ultracel®300kDa/30kDa, load: 100 L/m²,
const. TMP 0.4-0.5 bar,
concentration factor: 10)
F
Concentrated bulk (CB)
D
Diafiltration - TFF
(Pellicon-2 Ultracel®300kDa/30kDa,
const. TMP 0.4-0.5 bar, 5x buffer exchange)
G
Drug product (DB)

Figure 3

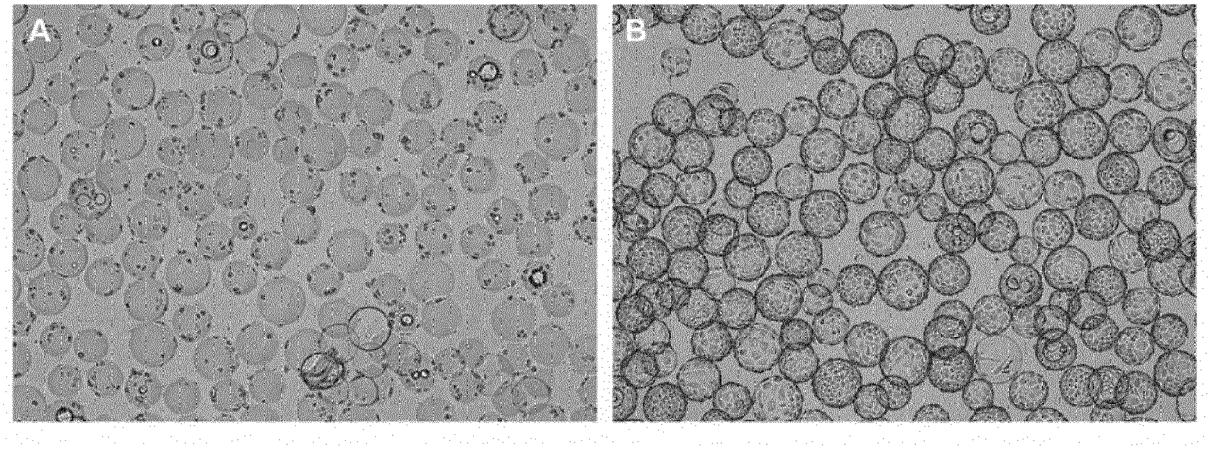


Figure 4

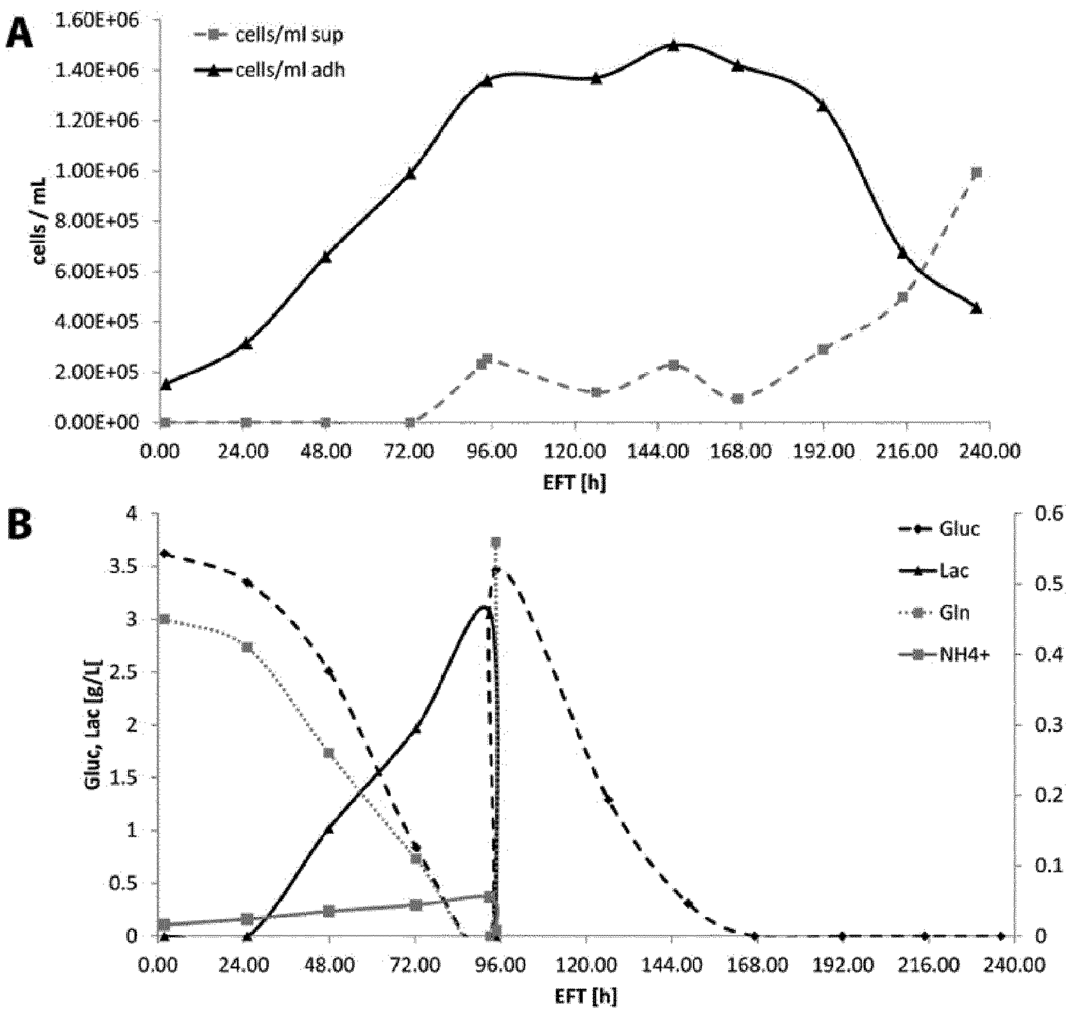

Figure 5

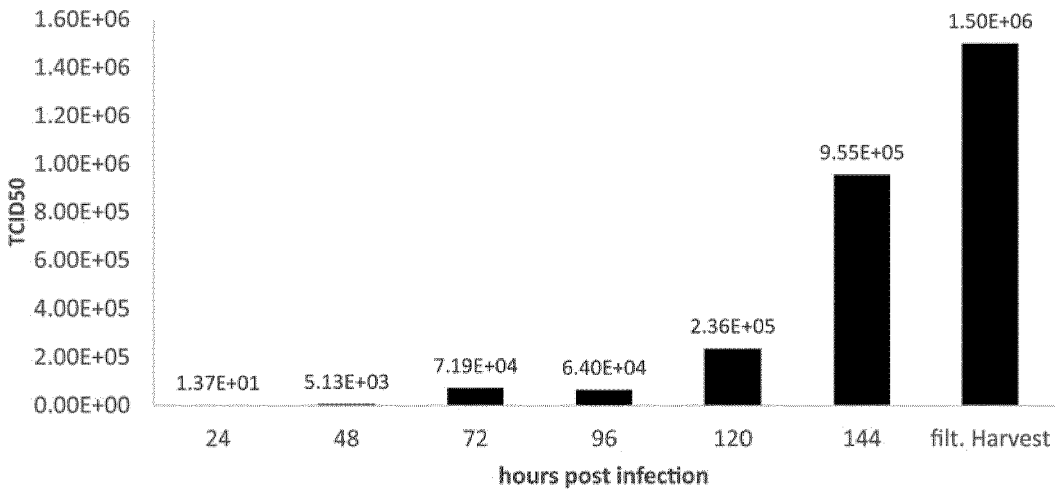


Figure 6

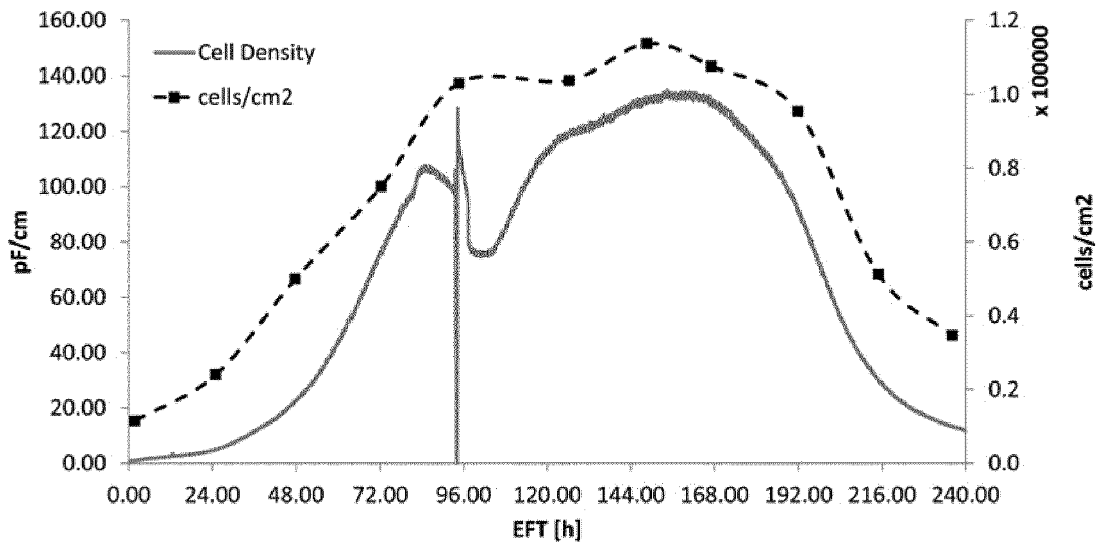

Figure 7

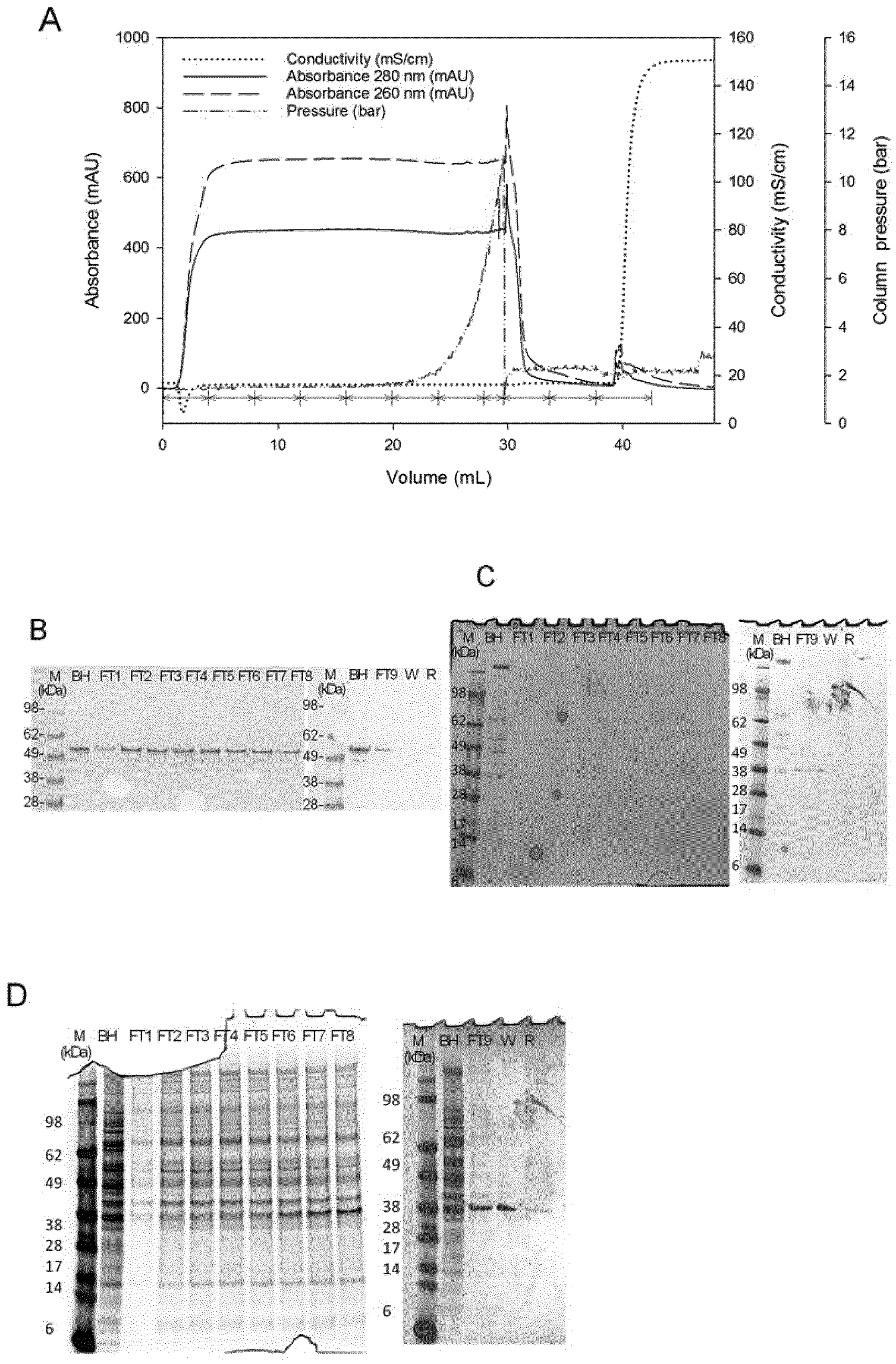
A
Conductivity (mS/cm)
Absorbance 280 nm (mAU)
Absorbance 260 nm (mAU)
Pressure (bar)
Absorbance (mAU)
Conductivity (mS/cm)
Column pressure (bar)
Volume (mL)
B
M (kDa) BH FT1 FT2 FT3 FT4 FT5 FT6 FT7 FT8 M (kDa) BH FT9 W R
98- 62- 49- 38- 28-
C
M (kDa) BH FT1 FT2 FT3 FT4 FT5 FT6 FT7 FT8
98 62 49 38 28 17 14 6
M (kDa) BH FT9 W R
D
M (kDa) BH FT1 FT2 FT3 FT4 FT5 FT6 FT7 FT8
98 62 49 38 28 17 14 6
M (kDa) BH FT9 W R

E

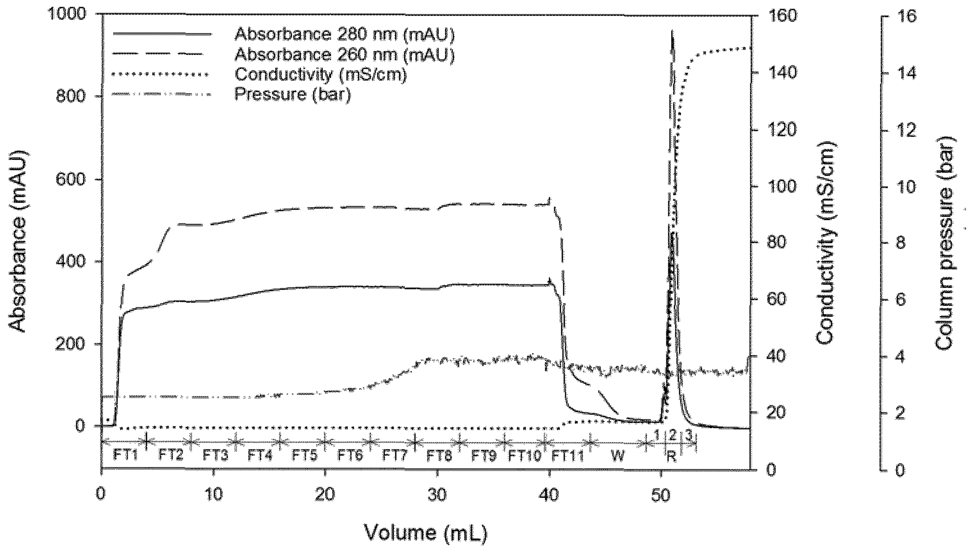
Absorbance 280 nm (mAU)
Absorbance 260 nm (mAU)
Conductivity (mS/cm)
Pressure (bar)
Absorbance (mAU)
Conductivity (mS/cm)
Column pressure (bar)
Volume (mL)
FT1 FT2 FT3 FT4 FT5 FT6 FT7 FT8 FT9 FT10 FT11 W R
1 2 3

F

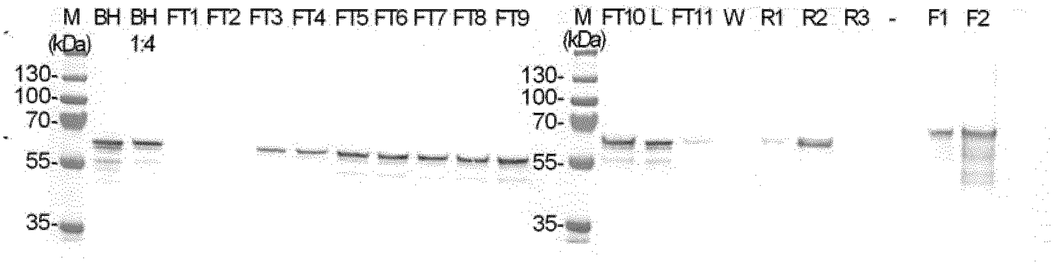
M (kDa) BH BH 1:4 FT1 FT2 FT3 FT4 FT5 FT6 FT7 FT8 FT9
M (kDa) FT10 L FT11 W R1 R2 R3 - F1 F2
130- 100- 70- 55- 35-

G

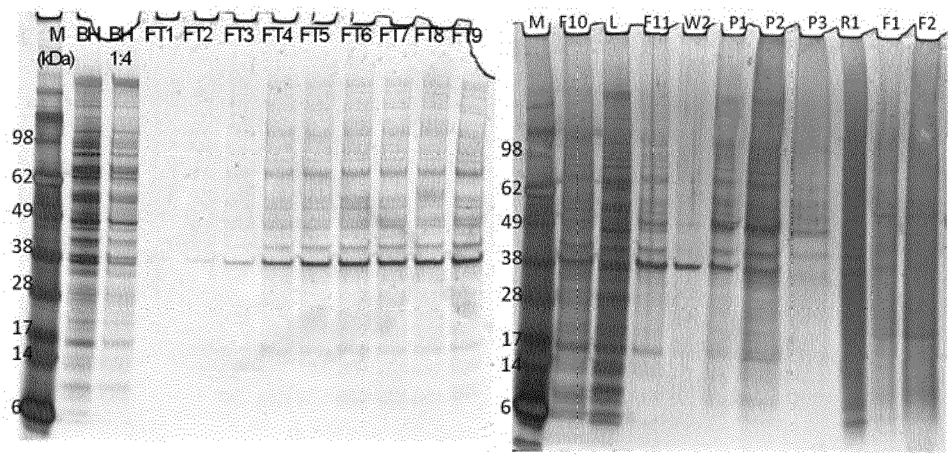
M (kDa) BH BH 1:4 FT1 FT2 FT3 FT4 FT5 FT6 FT7 FT8 FT9
M F10 L F11 W2 P1 P2 P3 R1 F1 F2
98 62 49 38 28 17 14 6

Figure 8

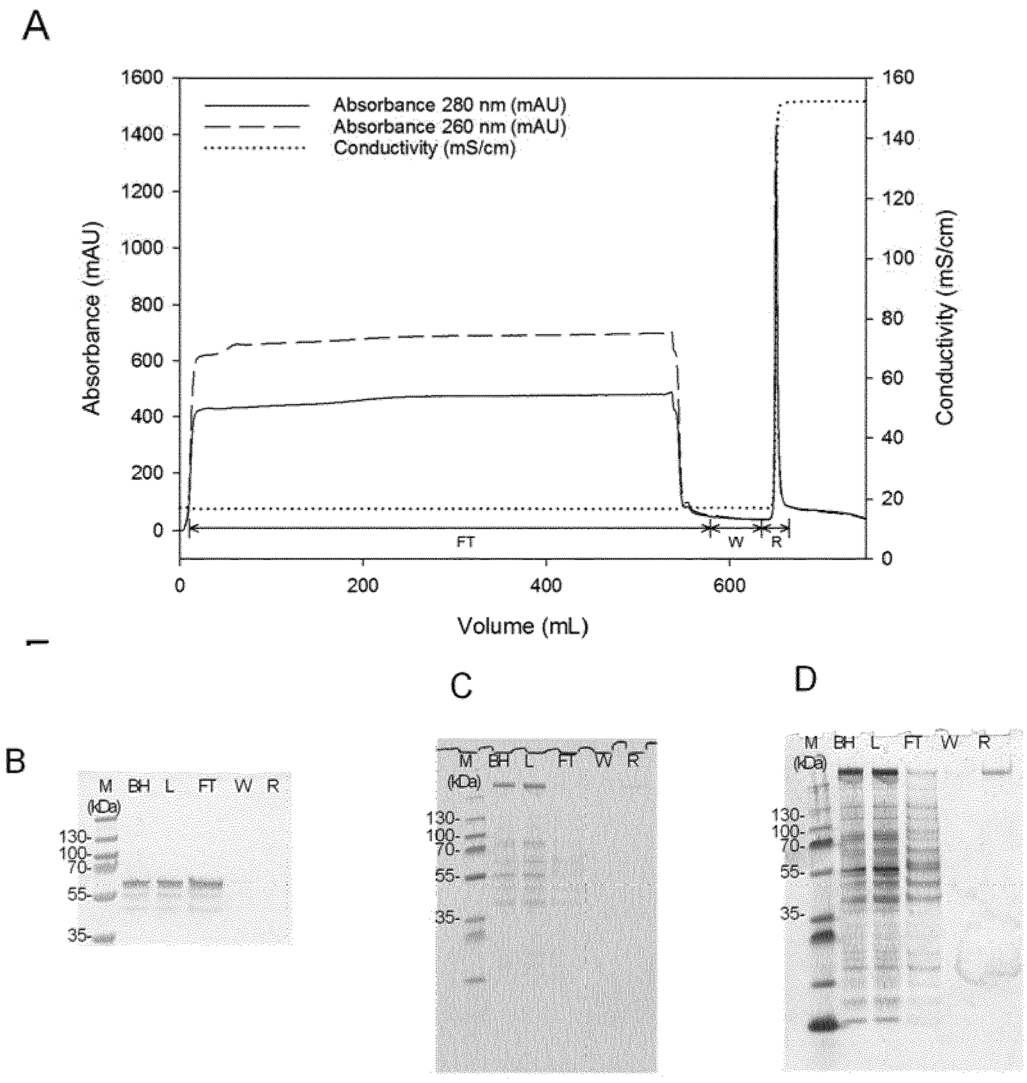
A
Absorbance 280 nm (mAU)
Absorbance 260 nm (mAU)
Conductivity (mS/cm)
Absorbance (mAU)
Conductivity (mS/cm)
Volume (mL)
FT
W
R
0
200
400
600
800
1000
1200
1400
1600
20
40
60
80
100
120
140
160
B
C
D
M
BH
L
FT
W
R
(kDa)
130-
100-
70-
55-
35-

E

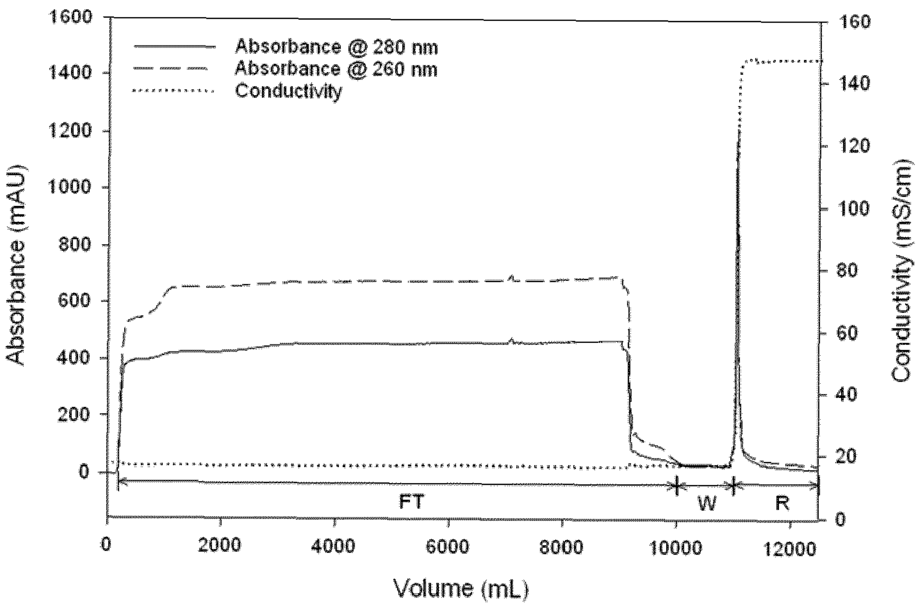
Absorbance @ 280 nm
Absorbance @ 260 nm
Conductivity
Absorbance (mAU)
Conductivity (mS/cm)
Volume (mL)
FT
W
R
0
200
400
600
800
1000
1200
1400
1600
20
40
60
80
100
120
140
160
2000
4000
6000
8000
10000
12000

F

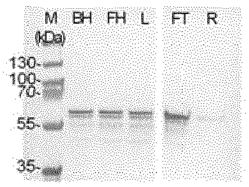
M
(kDa)
BH
FH
L
FT
R
130-
100-
70-
55-
35-

G

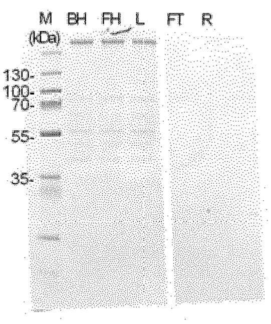
M
(kDa)
BH
FH
L
FT
R
130-
100-
70-
55-
35-

H

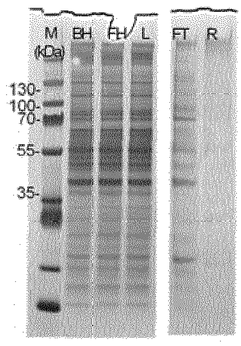
M
(kDa)
BH
FH
L
FT
R
130-
100-
70-
55-
35-

Figure 9

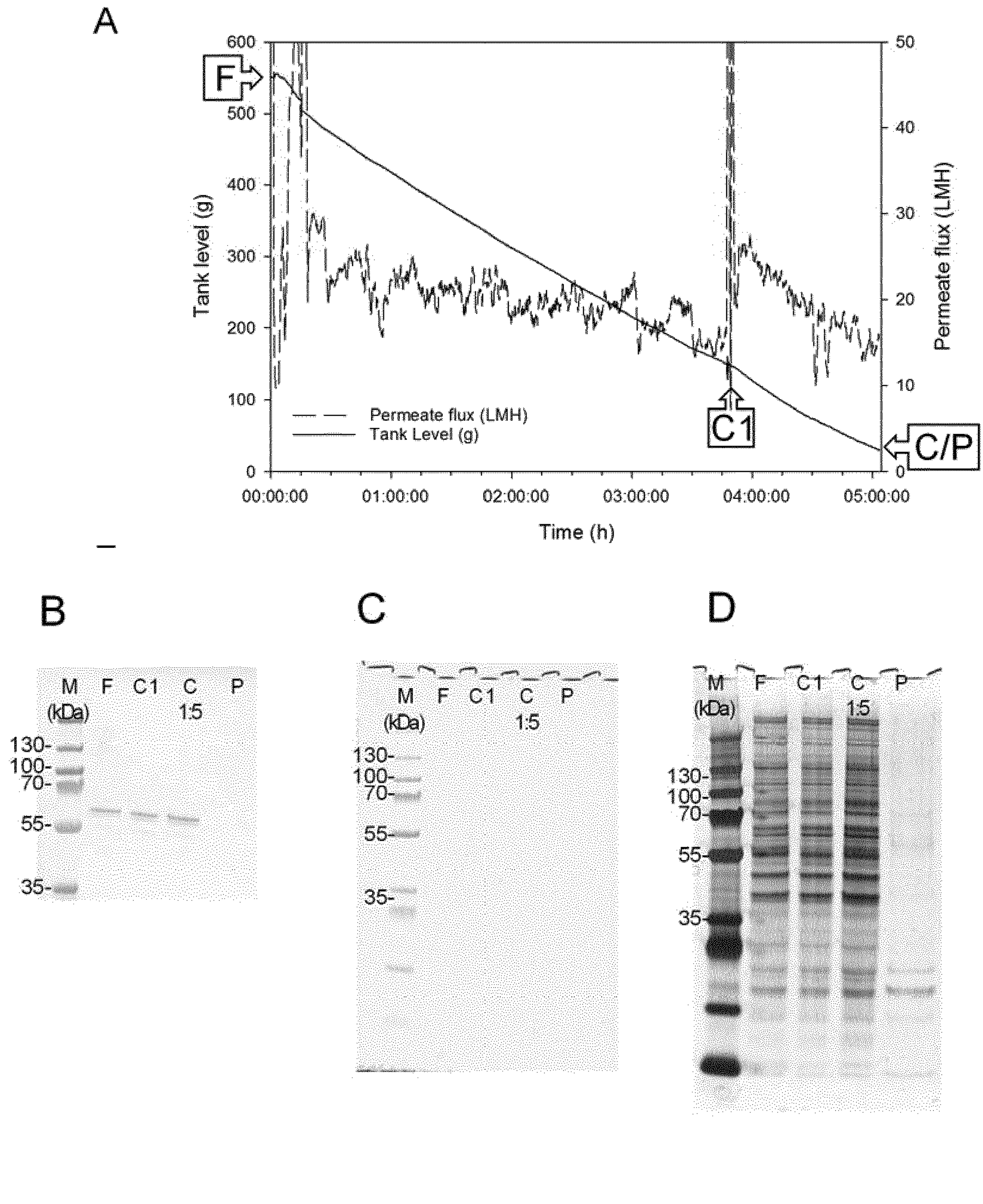
A
Tank level (g)
Permeate flux (LMH)
Time (h)
F
C1
C/P
Permeate flux (LMH)
Tank Level (g)
00:00:00
01:00:00
02:00:00
03:00:00
04:00:00
05:00:00
B
C
D
M
(kDa)
F
C1
C
1:5
P
130-
100-
70-
55-
35-

E

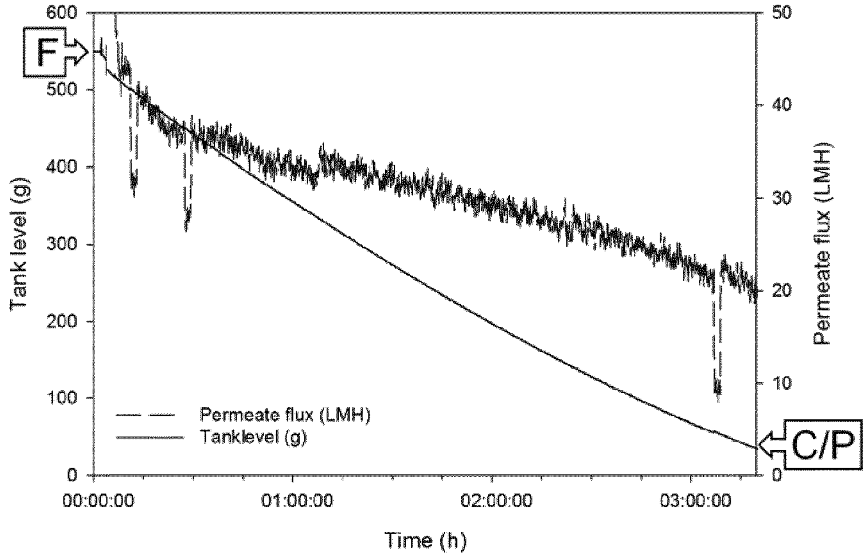
F
C/P
Tank level (g)
Permeate flux (LMH)
Time (h)
Permeate flux (LMH)
Tanklevel (g)
00:00:00
01:00:00
02:00:00
03:00:00

F G H

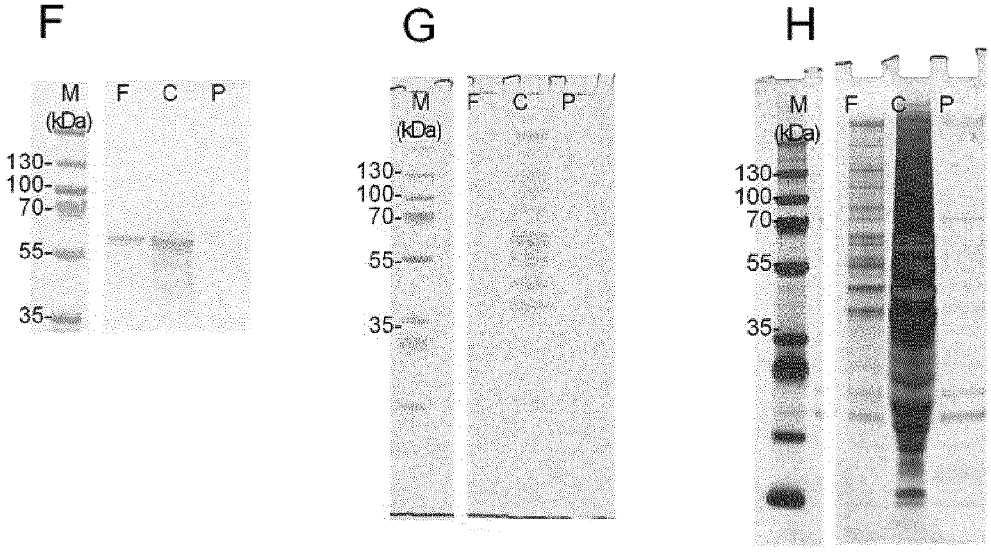
M (kDa) F C P
130-
100-
70-
55-
35-

I

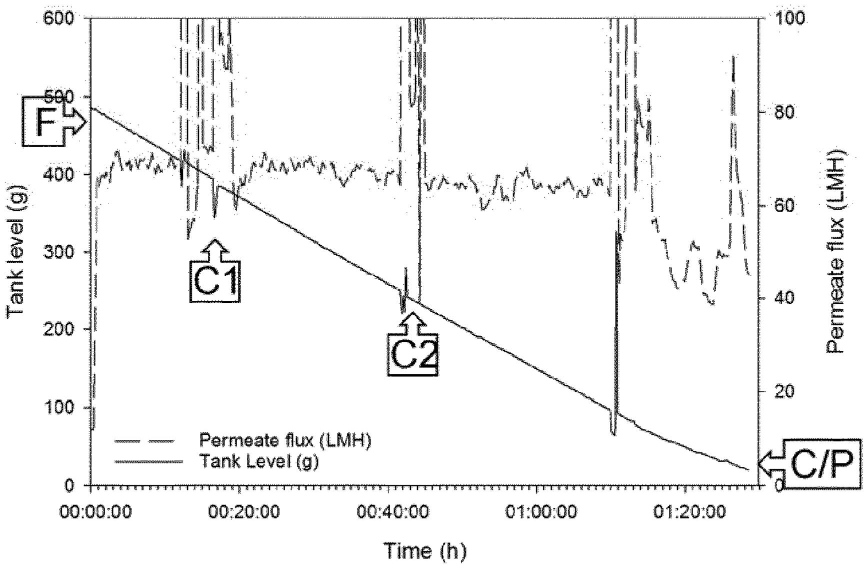
F
C1
C2
C/P
Tank level (g)
Permeate flux (LMH)
Time (h)
Permeate flux (LMH)
Tank Level (g)
00:00:00
00:20:00
00:40:00
01:00:00
01:20:00

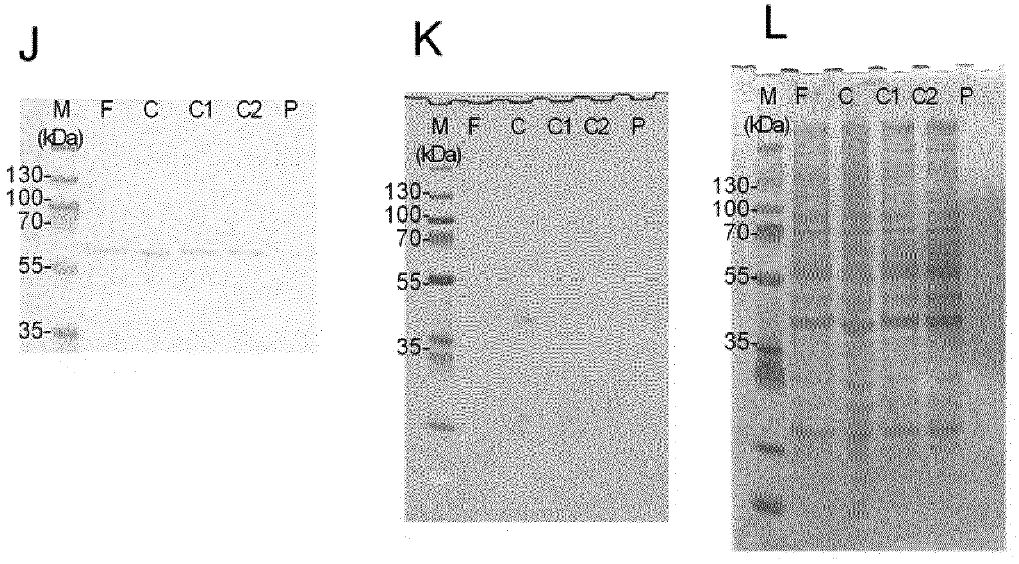
J
M F C C1 C2 P
(kDa)
130-
100-
70-
55-
35-
K
M F C C1 C2 P
(kDa)
130-
100-
70-
55-
35-
L
M F C C1 C2 P
(kDa)
130-
100-
70-
55-
35-

Figure 10

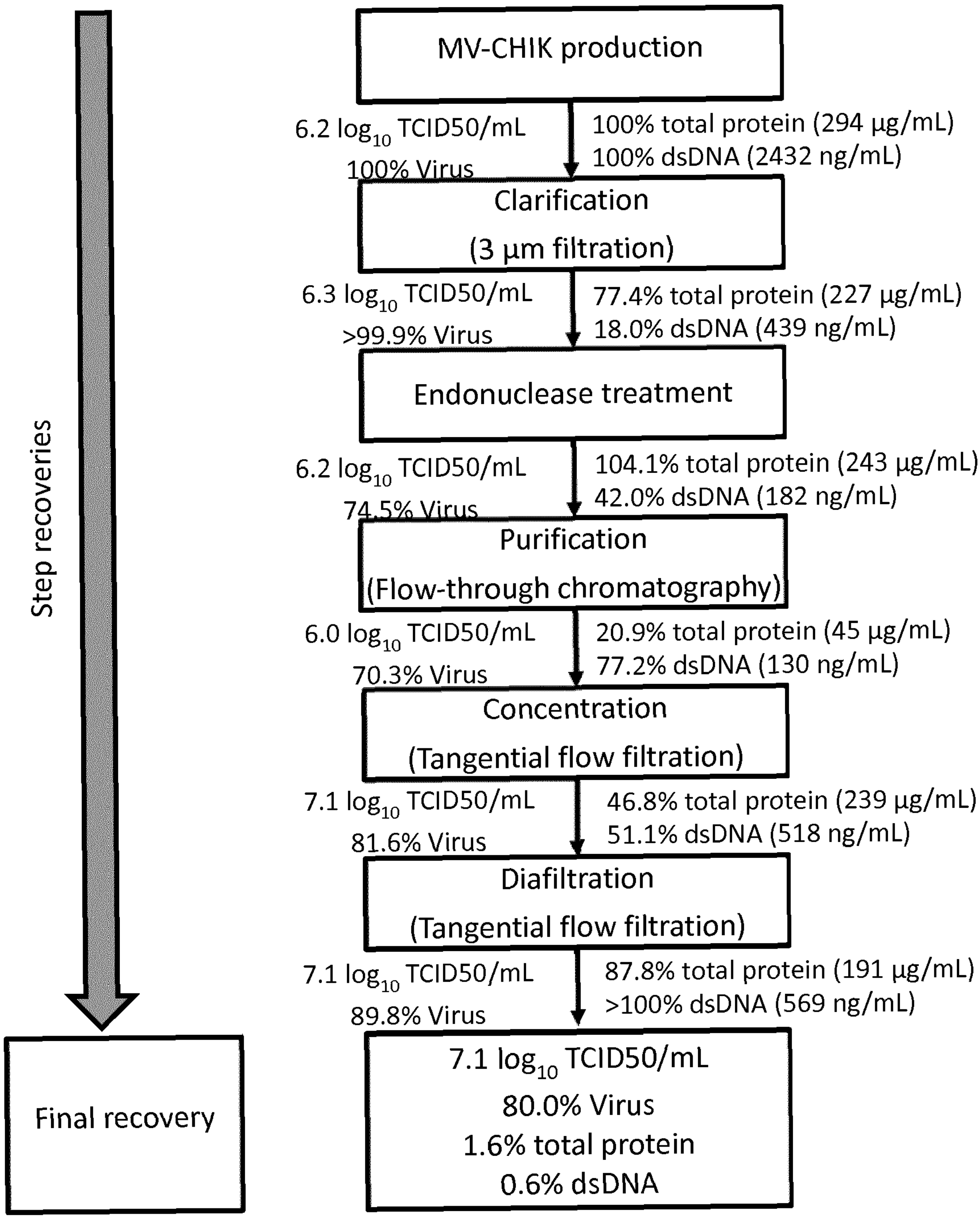
Step recoveries
MV-CHIK production
6.2 $\log_{10}$ TCID50/mL
100% Virus
100% total protein (294 µg/mL)
100% dsDNA (2432 ng/mL)
Clarification
(3 µm filtration)
6.3 $\log_{10}$ TCID50/mL
>99.9% Virus
77.4% total protein (227 µg/mL)
18.0% dsDNA (439 ng/mL)
Endonuclease treatment
6.2 $\log_{10}$ TCID50/mL
74.5% Virus
104.1% total protein (243 µg/mL)
42.0% dsDNA (182 ng/mL)
Purification
(Flow-through chromatography)
6.0 $\log_{10}$ TCID50/mL
70.3% Virus
20.9% total protein (45 µg/mL)
77.2% dsDNA (130 ng/mL)
Concentration
(Tangential flow filtration)
7.1 $\log_{10}$ TCID50/mL
81.6% Virus
46.8% total protein (239 µg/mL)
51.1% dsDNA (518 ng/mL)
Diafiltration
(Tangential flow filtration)
7.1 $\log_{10}$ TCID50/mL
89.8% Virus
87.8% total protein (191 µg/mL)
>100% dsDNA (569 ng/mL)
Final recovery
7.1 $\log_{10}$ TCID50/mL
80.0% Virus
1.6% total protein
0.6% dsDNA

Figure 11

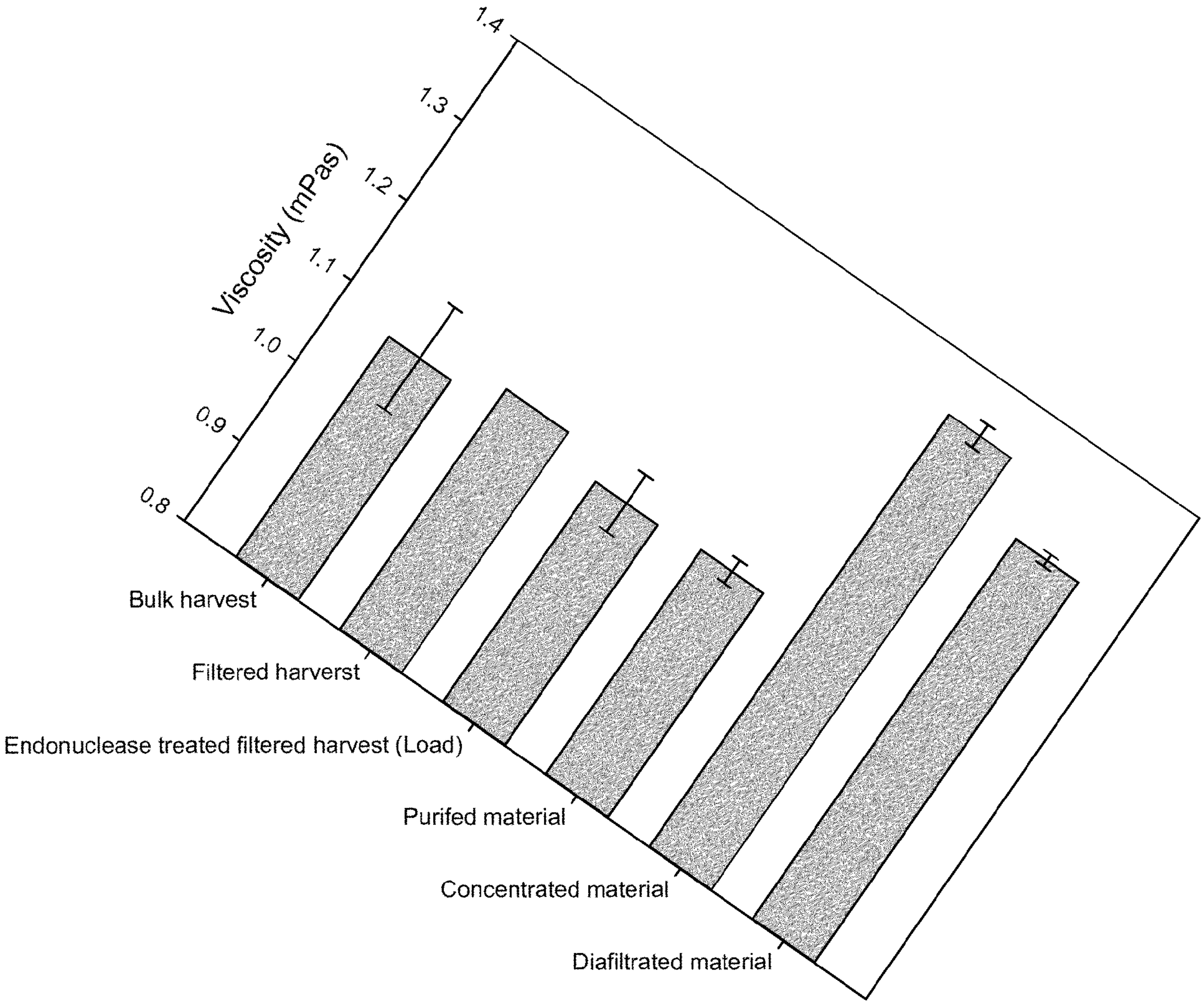
Viscosity (mPas)
0.8
0.9
1.0
1.1
1.2
1.3
1.4
Bulk harvest
Filtered harverst
Endonuclease treated filtered harvest (Load)
Purifed material
Concentrated material
Diafiltrated material

Figure 12

A

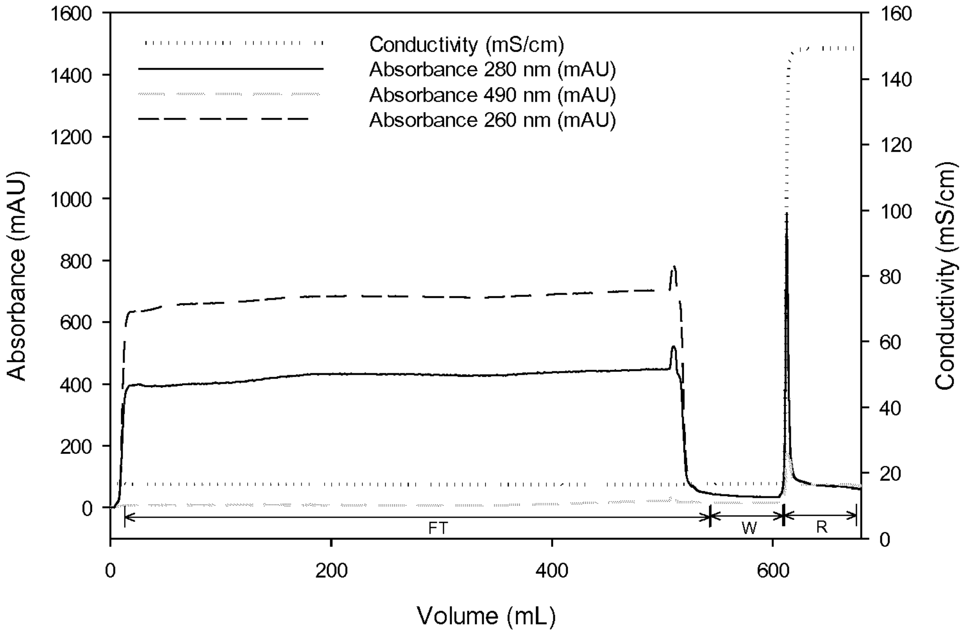
Conductivity (mS/cm)
Absorbance 280 nm (mAU)
Absorbance 490 nm (mAU)
Absorbance 260 nm (mAU)
Absorbance (mAU)
Conductivity (mS/cm)
Volume (mL)
FT
W
R
0
200
400
600
800
1000
1200
1400
1600
20
40
60
80
100
120
140
160

B

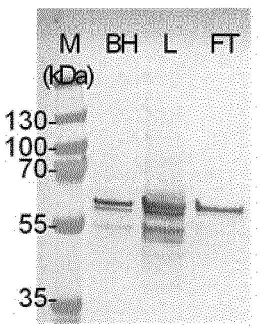
M
(kDa)
BH
L
FT
130-
100-
70-
55-
35-

Figure 13

Fig. 13A

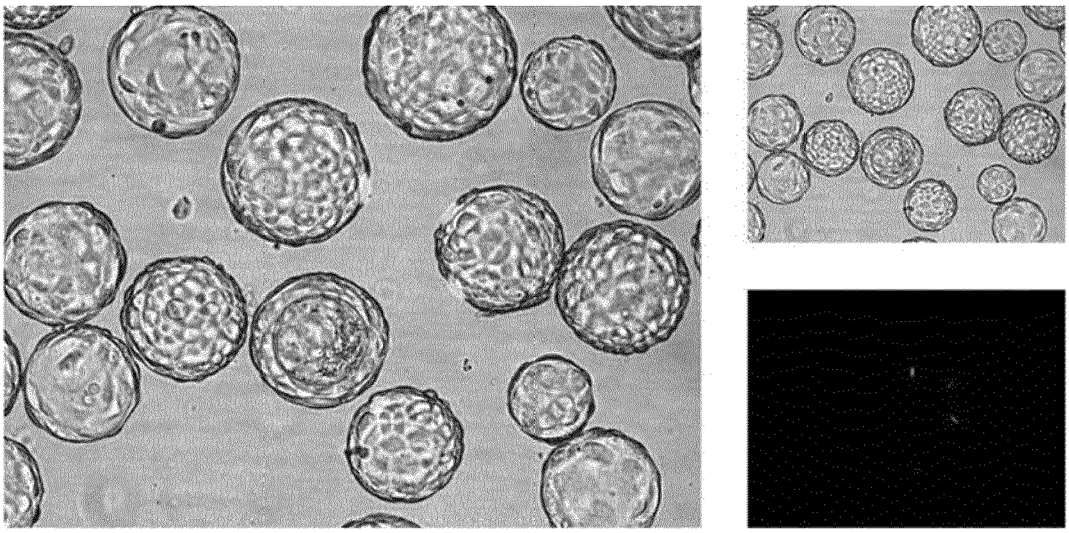

Fig. 13B

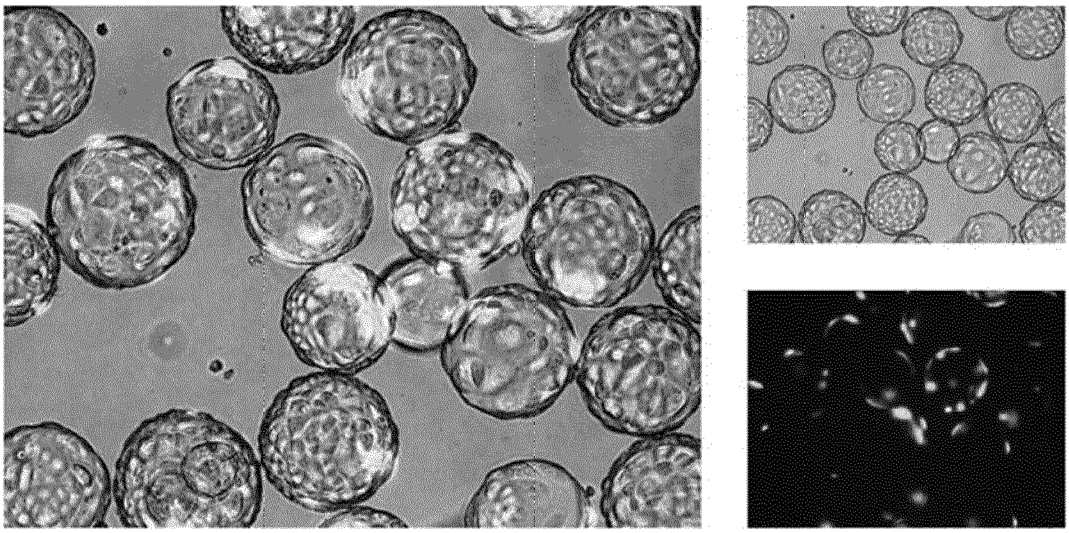

Fig. 13C

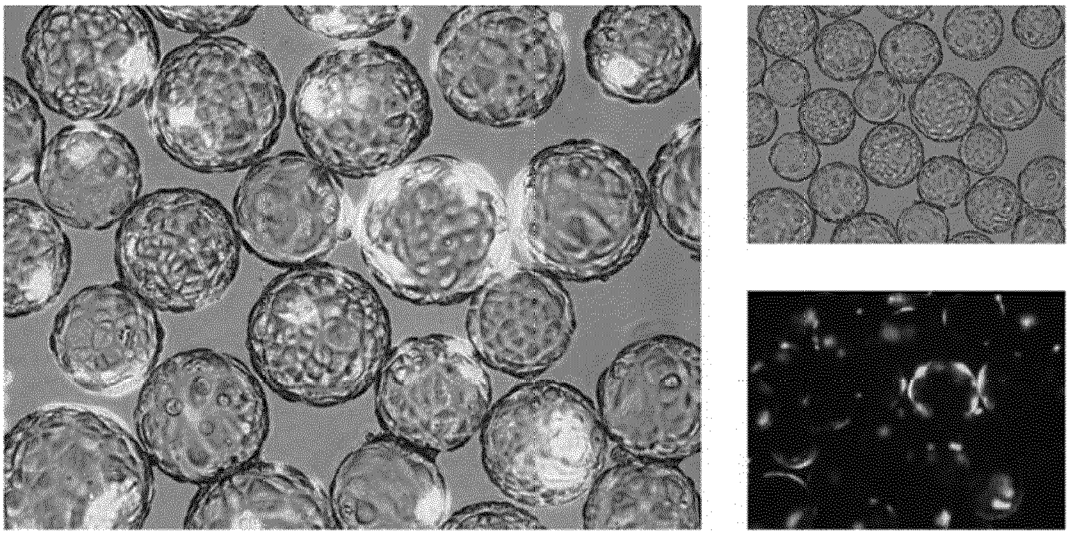

Fig. 13D

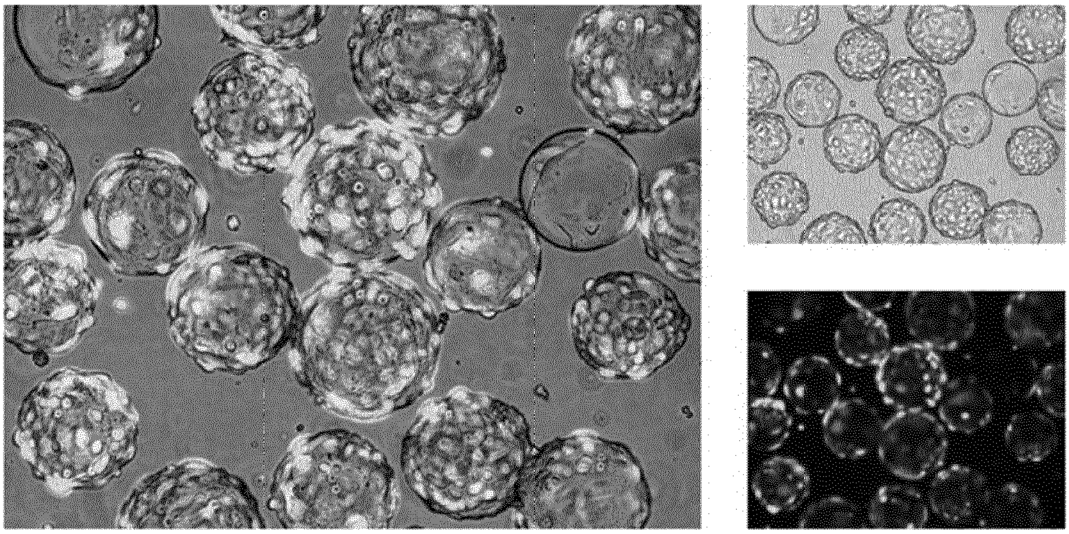

Fig. 13E

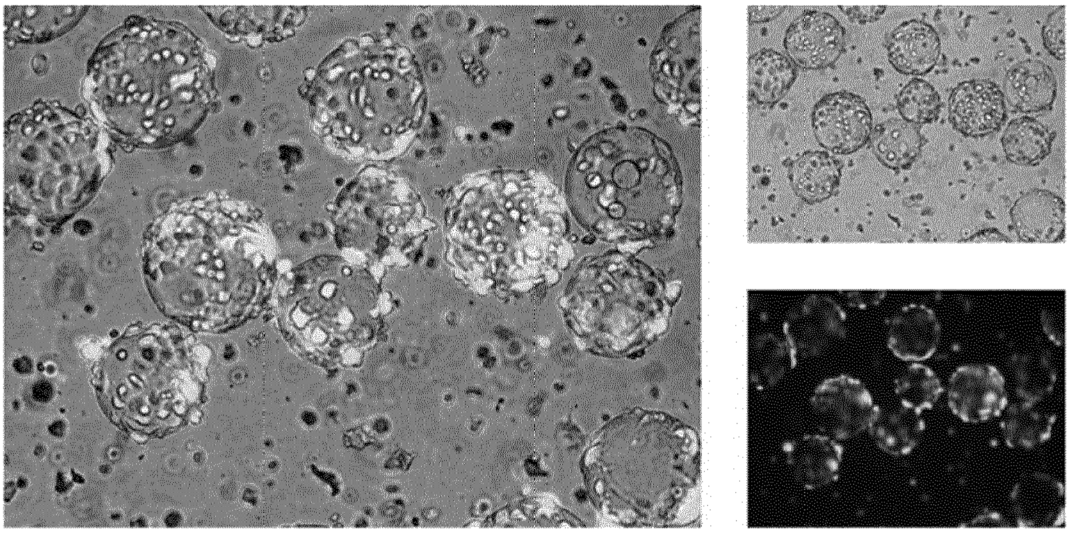

Fig. 13F

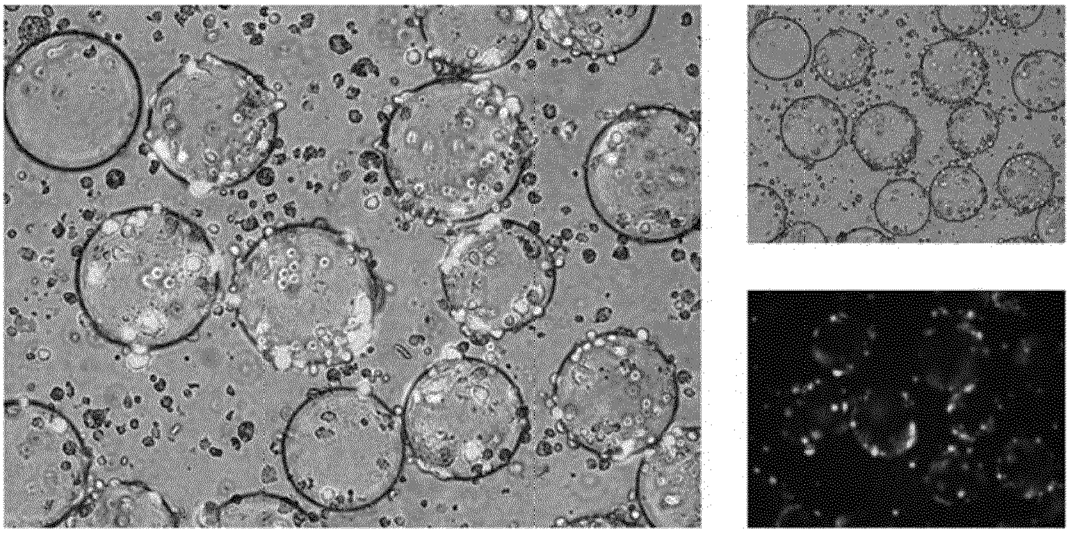

Europäisches Patentamt
European Patent Office
Office européen des brevets

# EUROPEAN SEARCH REPORT

Application Number
EP 18 17 8037

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2016/156613 A1 (VALNEVA SE [FR]) 6 October 2016 (2016-10-06) * p. 1 li. 17-25, p. 2 li. 4, p. 11 li. 11-13, p. 11 li. 18-20, p. 2 li. 8-9, p. 12 li. 1, p. 2 li. 15-17, p. 2 li. 17-18, p. 8 li. 4, p. 8 li. 16-17, p. 9 li. 8-17, p. 8 li. 29-30, p. 10 li. 13-14, p. 11 li. 24-28; claims 1, 6, 21; figure 1; examples 1-2 * ----- | 1-12,14, 15<br>13 | INV.<br>C12N7/02<br>C12N15/86 |
| X | FR 3 014 901 A1 (GENETHON [FR]) 19 June 2015 (2015-06-19) * p. 3 li. 8-11, p. 13 li. 13-21, p. 15 li. 3-6, p. 3 li. 18, p. 5 li. 8-9, p. 5 li. 29-32, p. 6 li. 12-13, p. 6 li. 31-32, p. 7 li. 25-p. 8 li. 9, p. 9 li. 20-23, p. 9 li. 32-34, p. 11 li. 10-11; claim 17 * ----- | 1-11 | |
| X | WO 2015/059714 A1 (BHARAT BIOTECH INT LTD [IN]) 30 April 2015 (2015-04-30) * p. 4 li. 26-27, p. 5 li. 1-4, p. 6 li. last line-p. 7 li. 5; example 3 * ----- | 12,14,15 | TECHNICAL FIELDS SEARCHED (IPC)<br>C12N |
| | -/-- | | |

The present search report has been drawn up for all claims

1

| Place of search | Date of completion of the search | Examiner |
|---|---|---|
| Munich | 31 August 2018 | Landré, Julien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 17 8037

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SVEN ANSORGE ET AL: "On-line monitoring of infected Sf-9 insect cell cultures by scanning permittivity measurements and comparison with off-line biovolume measurements", CYTOTECHNOLOGY, KLUWER ACADEMIC PUBLISHERS, DO, vol. 55, no. 2-3, 11 October 2007 (2007-10-11), pages 115-124, XP019550377, ISSN: 1573-0778, DOI: 10.1007/S10616-007-9093-0 * abstract * * p. 117 col. 1 li. 40-41, p. 118 col. 1 li. 21-22, p. 115 col. 2 li. 2-6, p. 119 col. 2 li. 1-4, p. 121 col. 1 li. 35-37, p. 123 col. 1 li. 4-7 * ----- | 13 | |
| A | LEE MICKY FU XIANG ET AL: "Negative chromatography of hepatitis B virus-like particle: Comparative study of different adsorbent designs", JOURNAL OF CHROMATOGRAPHY A, ELSEVIER, AMSTERDAM, NL, vol. 1445, 24 March 2016 (2016-03-24), pages 1-9, XP029509370, ISSN: 0021-9673, DOI: 10.1016/J.CHROMA.2016.03.066 * the whole document * ----- | 1-15 | TECHNICAL FIELDS SEARCHED (IPC) |
| A | EP 3 184 119 A1 (THEMIS BIOSCIENCE GMBH [AT]) 28 June 2017 (2017-06-28) * the whole document * ----- | 1-15 | |

The present search report has been drawn up for all claims

1

| Place of search | Date of completion of the search | Examiner |
|---|---|---|
| Munich | 31 August 2018 | Landré, Julien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 18 17 8037

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2018

| Patent document cited in search report | Publication date | Patent family member(s) | Publication date |
|---|---|---|---|
| WO 2016156613 A1 | 06-10-2016 | AU 2016239877 A1 | 26-10-2017 |
| | | CA 2980812 A1 | 06-10-2016 |
| | | CN 107567496 A | 09-01-2018 |
| | | EP 3277802 A1 | 07-02-2018 |
| | | JP 2018511347 A | 26-04-2018 |
| | | KR 20170138462 A | 15-12-2017 |
| | | US 2018119110 A1 | 03-05-2018 |
| | | WO 2016156613 A1 | 06-10-2016 |
| FR 3014901 A1 | 19-06-2015 | CN 105980571 A | 28-09-2016 |
| | | EP 3083970 A1 | 26-10-2016 |
| | | FR 3014901 A1 | 19-06-2015 |
| | | JP 2017503486 A | 02-02-2017 |
| | | SG 11201605805R A | 29-09-2016 |
| | | US 2017002332 A1 | 05-01-2017 |
| | | WO 2015092287 A1 | 25-06-2015 |
| WO 2015059714 A1 | 30-04-2015 | AU 2014338520 A1 | 31-03-2016 |
| | | CN 105744952 A | 06-07-2016 |
| | | PH 12016500500 A1 | 13-06-2016 |
| | | RU 2016114285 A | 19-10-2017 |
| | | WO 2015059714 A1 | 30-04-2015 |
| EP 3184119 A1 | 28-06-2017 | EP 3184118 A1 | 28-06-2017 |
| | | EP 3184119 A1 | 28-06-2017 |
| | | SG 11201804496U A | 30-07-2018 |
| | | WO 2017109211 A1 | 29-06-2017 |
| | | WO 2017109222 A1 | 29-06-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017109211 A
- EP 2712871 A1
- EP 3184119 A1


### Non-patent literature cited in the description


- **FUKUHARA et al.** *Cancer Sci.,* October 2016, vol. 107 (10), 1373-1379
- *Mol Ther Methods Clin Dev.,* 2016, vol. 3, 16018
- **NESTOLA et al.** *Biotechnology and Bioengineering,* May 2015, vol. 112 (5
- **SMITH, T.F. ; WATERMAN, M.S.** Identification of common molecular subsequences. *Journal of Molecular Biology,* 1981, vol. 147 (1), 195-197, http://www.ebi.ac.uk/Tools/psa
- **JUO ; PEI-SHOW.** Concise Dictionary of Biomedicine and Molecular Biology. CRC Press, 2002
- The Dictionary of Cell and Molecular Biology. Academic Press, 1999
- Oxford Dictionary Of Biochemistry And Molecular Biology, Revised. Oxford University Press, 2000
- **BIRD et al.** Transport Phenomena. Wiley, 1960

SEQUENCE LISTING

<110> Themis Bioscience GmbH

<120> Integrated manufacturing and chromatographic system for virus production

<130> TM 5534-01EP

<160> 7

<170> PatentIn version 3.5

<210> 1
<211> 18994
<212> DNA
<213> Artificial Sequence

<220>
<223> MV Schwarz pTM

<400> 1
tcatgaacgc ggccgctaat acgactcact atagggccaa ctttgtttgg tctgatgagt 60

ccgtgaggac gaaacccgga gtcccgggtc accaaacaaa gttgggtaag gatagttcaa 120

tcaatgatca tcttctagtg cacttaggat tcaagatcct attatcaggg acaagagcag 180

gattagggat atccgagatg gccacacttt taaggagctt agcattgttc aaaagaaaca 240

aggacaaacc acccattaca tcaggatccg gtggagccat cagaggaatc aaacacatta 300

ttatagtacc aatccctgga gattcctcaa ttaccactcg atccagactt ctggaccggt 360

tggtgaggtt aattggaaac ccggatgtga gcgggcccaa actaacaggg gcactaatag 420

gtatattatc cttatttgtg gagtctccag gtcaattgat tcagaggatc accgatgacc 480

ctgacgttag cataaggctg ttagaggttg tccagagtga ccagtcacaa tctggcctta 540

ccttcgcatc aagaggtacc aacatggagg atgaggcgga ccaatacttt tcacatgatg 600

atccaattag tagtgatcaa tccaggttcg gatggttcgg gaacaaggaa atctcagata 660

ttgaagtgca agaccctgag ggattcaaca tgattctggg taccatccta gcccaaattt 720

gggtcttgct cgcaaaggcg gttacggccc cagacacggc agctgattcg gagctaagaa 780

ggtggataaa gtacacccaa caaagaaggg tagttggtga atttagattg gagagaaaat 840

ggttggatgt ggtgaggaac aggattgccg aggacctctc cttacgccga ttcatggtcg 900

ctctaatcct ggatatcaag agaacacccg gaaacaaacc caggattgct gaaatgatat 960

gtgacattga tacatatatc gtagaggcag gattagccag ttttatcctg actattaagt 1020

ttgggataga aactatgtat cctgctcttg gactgcatga atttgctggt gagttatcca 1080

cacttgagtc cttgatgaac ctttaccagc aaatggggga aactgcaccc tacatggtaa 1140

tcctggagaa ctcaattcag aacaagttca gtgcaggatc ataccctctg ctctggagct 1200

atgccatggg agtaggagtg gaacttgaaa actccatggg aggtttgaac tttggccgat 1260

cttactttga tccagcatat tttagattag ggcaagagat ggtaaggagg tcagctggaa 1320
aggtcagttc cacattggca tctgaactcg gtatcactgc cgaggatgca aggcttgttt 1380
cagagattgc aatgcatact actgaggaca agatcagtag agcggttgga cccagacaag 1440
cccaagtatc atttctacac ggtgatcaaa gtgagaatga gctaccgaga ttgggggca 1500
aggaagatag gagggtcaaa cagagtcgag gagaagccag ggagagctac agagaaaccg 1560
ggcccagcag agcaagtgat gcgagagctg cccatcttcc aaccggcaca cccctagaca 1620
ttgacactgc aacggagtcc agccaagatc cgcaggacag tcgaaggtca gctgacgccc 1680
tgcttaggct gcaagccatg gcaggaatct cggaagaaca aggctcagac acggacaccc 1740
ctatagtgta caatgacaga aatcttctag actaggtgcg agaggccgag ggccagaaca 1800
acatccgcct accatccatc attgttataa aaaacttagg aaccaggtcc acacagccgc 1860
cagcccatca accatccact cccacgattg gagccaatgg cagaagagca ggcacgccat 1920
gtcaaaaacg gactggaatg catccgggct ctcaaggccg agcccatcgg ctcactggcc 1980
atcgaggaag ctatggcagc atggtcagaa atatcagaca acccaggaca ggagcgagcc 2040
acctgcaggg aagagaaggc aggcagttcg ggtctcagca aaccatgcct ctcagcaatt 2100
ggatcaactg aaggcggtgc acctcgcatc cgcggtcagg gacctggaga gagcgatgac 2160
gacgctgaaa ctttgggaat ccccccaaga aatctccagg catcaagcac tgggttacag 2220
tgttattacg tttatgatca cagcggtgaa gcggttaagg gaatccaaga tgctgactct 2280
atcatggttc aatcaggcct tgatggtgat agcaccctct caggaggaga caatgaatct 2340
gaaaacagcg atgtggatat tggcgaacct gataccgagg gatatgctat cactgaccgg 2400
ggatctgctc ccatctctat ggggttcagg gcttctgatg ttgaaactgc agaaggaggg 2460
gagatccacg agctcctgag actccaatcc agaggcaaca actttccgaa gcttgggaaa 2520
actctcaatg ttcctccgcc cccggacccc ggtagggcca gcacttccgg gacacccatt 2580
aaaaagggca cagacgcgag attagcctca tttggaacgg agatcgcgtc tttattgaca 2640
ggtggtgcaa cccaatgtgc tcgaaagtca ccctcggaac catcagggcc aggtgcacct 2700
gcggggaatg tccccgagtg tgtgagcaat gccgcactga tacaggagtg gacacccgaa 2760
tctggtacca caatctcccc gagatcccag aataatgaag aagggggaga ctattatgat 2820
gatgagctgt tctctgatgt ccaagatatt aaaacagcct tggccaaaat acacgaggat 2880
aatcagaaga taatctccaa gctagaatca ctgctgttat tgaagggaga agttgagtca 2940
attaagaagc agatcaacag gcaaaatatc agcatatcca ccctggaagg acacctctca 3000
agcatcatga tcgccattcc tggacttggg aaggatccca acgaccccac tgcagatgtc 3060
gaaatcaatc ccgacttgaa acccatcata ggcagagatt caggccgagc actggccgaa 3120

gttctcaaga aacccgttgc cagccgacaa ctccaaggaa tgacaaatgg acggaccagt 3180
tccagaggac agctgctgaa ggaatttcag ctaaagccga tcgggaaaaa gatgagctca 3240
gccgtcgggt ttgttcctga caccggccct gcatcacgca gtgtaatccg ctccattata 3300
aaatccagcc ggctagagga ggatcggaag cgttacctga tgactctcct tgatgatatc 3360
aaaggagcca atgatcttgc caagttccac cagatgctga tgaagataat aatgaagtag 3420
ctacagctca acttacctgc caaccccatg ccagtcgacc caactagtac aacctaaatc 3480
cattataaaa aacttaggag caaagtgatt gcctcccaag gtccacaatg acagagacct 3540
acgacttcga caagtcggca tgggacatca aagggtcgat cgctccgata caacccacca 3600
cctacagtga tggcaggctg gtgccccagg tcagagtcat agatcctggt ctaggcgaca 3660
ggaaggatga atgctttatg tacatgtttc tgctgggggt tgttgaggac agcgattccc 3720
tagggcctcc aatcgggcga gcatttgggt tcctgccctt aggtgttggc agatccacag 3780
caaagcccga aaaactcctc aaagaggcca ctgagcttga catagttgtt agacgtacag 3840
cagggctcaa tgaaaaactg gtgttctaca acaacacccc actaactctc ctcacacctt 3900
ggagaaaggt cctaacaaca gggagtgtct tcaacgcaaa ccaagtgtgc aatgcggtta 3960
atctgatacc gctcgatacc ccgcagaggt tccgtgttgt ttatatgagc atcacccgtc 4020
tttcggataa cgggtattac accgttccta gaagaatgct ggaattcaga tcggtcaatg 4080
cagtggcctt caacctgctg gtgaccctta ggattgacaa ggcgataggc cctgggaaga 4140
tcatcgacaa tacagagcaa cttcctgagg caacatttat ggtccacatc gggaacttca 4200
ggagaaagaa gagtgaagtc tactctgccg attattgcaa aatgaaaatc gaaaagatgg 4260
gcctggtttt tgcacttggt gggatagggg gcaccagtct tcacattaga agcacaggca 4320
aaatgagcaa gactctccat gcacaactcg ggttcaagaa gaccttatgt tacccgctga 4380
tggatatcaa tgaagacctt aatcgattac tctggaggag cagatgcaag atagtaagaa 4440
tccaggcagt tttgcagcca tcagttcctc aagaattccg catttacgac gacgtgatca 4500
taaatgatga ccaaggacta ttcaaagttc tgtagaccgt agtgcccagc aatgcccgaa 4560
aacgaccccc ctcacaatga cagccagaag gcccggacaa aaaagccccc tccgaaagac 4620
tccacggacc aagcgagagg ccagccagca gccgacggca agcgcgaaca ccaggcggcc 4680
ccagcacaga acagccctga cacaaggcca ccaccagcca ccccaatctg catcctcctc 4740
gtgggacccc cgaggaccaa cccccaaggc tgcccccgat ccaaaccacc aaccgcatcc 4800
ccaccacccc cgggaaagaa acccccagca attggaaggc ccctccccct cttcctcaac 4860
acaagaactc cacaaccgaa ccgcacaagc gaccgaggtg acccaaccgc aggcatccga 4920
ctccctagac agatcctctc tccccggcaa actaaacaaa acttagggcc aaggaacata 4980
cacacccaac agaacccaga ccccggccca cggcgccgcg cccccaaccc ccgacaacca 5040

gagggagccc ccaaccaatc ccgccggctc ccccggtgcc cacaggcagg gacaccaacc 5100
cccgaacaga cccagcaccc aaccatcgac aatccaagac gggggggccc ccccaaaaaa 5160
aggcccccag gggccgacag ccagcaccgc gaggaagccc acccacccca cacacgacca 5220
cggcaaccaa accagaaccc agaccaccct gggccaccag ctcccagact cggccatcac 5280
cccgcagaaa ggaaaggcca caacccgcgc accccagccc cgatccggcg gggagccacc 5340
caacccgaac cagcacccaa gagcgatccc cgaaggaccc ccgaaccgca aaggacatca 5400
gtatcccaca gcctctccaa gtcccccggt ctcctcctct tctcgaaggg accaaaagat 5460
caatccacca cacccgacga cactcaactc cccacccta aaggagacac cgggaatccc 5520
agaatcaaga ctcatccaat gtccatcatg ggtctcaagg tgaacgtctc tgccatattc 5580
atggcagtac tgttaactct ccaaacaccc accggtcaaa tccattgggg caatctctct 5640
aagatagggg tggtaggaat aggaagtgca agctacaaag ttatgactcg ttccagccat 5700
caatcattag tcataaaatt aatgcccaat ataactctcc tcaataactg cacgagggta 5760
gagattgcag aatacaggag actactgaga acagttttgg aaccaattag agatgcactt 5820
aatgcaatga cccagaatat aagaccggtt cagagtgtag cttcaagtag gagacacaag 5880
agatttgcgg gagtagtcct ggcaggtgcg gccctaggcg ttgccacagc tgctcagata 5940
acagccggca ttgcacttca ccagtccatg ctgaactctc aagccatcga caatctgaga 6000
gcgagcctgg aaactactaa tcaggcaatt gagacaatca gacaagcagg gcaggagatg 6060
atattggctg ttcagggtgt ccaagactac atcaataatg agctgatacc gtctatgaac 6120
caactatctt gtgatttaat cggccagaag ctcgggctca aattgctcag atactataca 6180
gaaatcctgt cattatttgg ccccagttta cgggacccca tatctgcgga gatatctatc 6240
caggctttga gctatgcgct tggaggagac atcaataagg tgttagaaaa gctcggatac 6300
agtggaggtg atttactggg catcttagag agcggaggaa taaaggcccg gataactcac 6360
gtcgacacag agtcctactt cattgtcctc agtatagcct atccgacgct gtccgagatt 6420
aagggggtga ttgtccaccg gctagagggg gtctcgtaca acataggctc tcaagagtgg 6480
tataccactg tgcccaagta tgttgcaacc caagggtacc ttatctcgaa ttttgatgag 6540
tcatcgtgta ctttcatgcc agaggggact gtgtgcagcc aaaatgcctt gtacccgatg 6600
agtcctctgc tccaagaatg cctccggggg tacaccaagt cctgtgctcg tacactcgta 6660
tccgggtctt ttgggaaccg gttcatttta tcacaaggga acctaatagc caattgtgca 6720
tcaatccttt gcaagtgtta cacaacagga acgatcatta atcaagaccc tgacaagatc 6780
ctaacataca ttgctgccga tcactgcccg gtagtcgagg tgaacggcgt gaccatccaa 6840
gtcgggagca ggaggtatcc agacgctgtg tacttgcaca gaattgacct cggtcctccc 6900

atatcattgg agaggttgga cgtagggaca aatctgggga atgcaattgc taagttggag 6960
gatgccaagg aattgttgga gtcatcggac cagatattga ggagtatgaa aggtttatcg 7020
agcactagca tagtctacat cctgattgca gtgtgtcttg gagggttgat agggatcccc 7080
gctttaatat gttgctgcag gggcgttgt aacaaaaagg gagaacaagt tggtatgtca 7140
agaccaggcc taaagcctga tcttacggga acatcaaaat cctatgtaag gtcgctctga 7200
tcctctacaa ctcttgaaac acaaatgtcc cacaagtctc ctcttcgtca tcaagcaacc 7260
accgcaccca gcatcaagcc cacctgaaat tatctccggc ttccctctgg ccgaacaata 7320
tcggtagtta atcaaaactt agggtgcaag atcatccaca atgtcaccac aacgagaccg 7380
gataaatgcc ttctacaaag ataacccca tcccaaggga agtaggatag tcattaacag 7440
agaacatctt atgattgata gaccttatgt tttgctggct gttctgtttg tcatgtttct 7500
gagcttgatc gggttgctag ccattgcagg cattagactt catcgggcag ccatctacac 7560
cgcagagatc cataaaagcc tcagcaccaa tctagatgta actaactcaa tcgagcatca 7620
ggtcaaggac gtgctgacac cactcttcaa aatcatcggt gatgaagtgg gcctgaggac 7680
acctcagaga ttcactgacc tagtgaaatt aatctctgac aagattaaat tccttaatcc 7740
ggatagggag tacgacttca gagatctcac ttggtgtatc aacccgccag agagaatcaa 7800
attggattat gatcaatact gtgcagatgt ggctgctgaa gagctcatga atgcattggt 7860
gaactcaact ctactggaga ccagaacaac caatcagttc ctagctgtct caaagggaaa 7920
ctgctcaggg cccactacaa tcagaggtca attctcaaac atgtcgctgt ccctgttaga 7980
cttgtattta ggtcgaggtt acaatgtgtc atctatagtc actatgacat cccagggaat 8040
gtatgggga acttacctag tggaaaagcc taatctgagc agcaaaaggt cagagttgtc 8100
acaactgagc atgtaccgag tgtttgaagt aggtgttatc agaaatccgg gtttgggggc 8160
tccggtgttc catatgacaa actatcttga gcaaccagtc agtaatgatc tcagcaactg 8220
tatggtggct ttggggggagc tcaaactcgc agccctttgt cacggggaag attctatcac 8280
aattccctat cagggatcag ggaaaggtgt cagcttccag ctcgtcaagc taggtgtctg 8340
gaaatcccca accgacatgc aatcctgggt ccccttatca acggatgatc cagtgataga 8400
caggctttac ctctcatctc acagaggtgt tatcgctgac aatcaagcaa aatgggctgt 8460
cccgacaaca cgaacagatg acaagttgcg aatggagaca tgcttccaac aggcgtgtaa 8520
gggtaaaatc caagcactct gcgagaatcc cgagtgggca ccattgaagg ataacaggat 8580
tccttcatac ggggtcttgt ctgttgatct gagtctgaca gttgagctta aaatcaaaat 8640
tgcttcggga ttcgggccat tgatcacaca cggttcaggg atggacctat acaaatccaa 8700
ccacaacaat gtgtattggc tgactatccc gccaatgaag aacctagcct taggtgtaat 8760
caacacattg gagtggatac cgagattcaa ggttagtccc tacctcttca ctgtcccaat 8820

taaggaagca ggcgaagact gccatgcccc aacataccta cctgcggagg tggatggtga 8880
tgtcaaactc agttccaatc tggtgattct acctggtcaa gatctccaat atgttttggc 8940
aacctacgat acttccaggg ttgaacatgc tgtggtttat tacgtttaca gcccaagccg 9000
ctcattttct tacttttatc cttttaggtt gcctataaag gggtcccca tcgaattaca 9060
agtggaatgc ttcacatggg accaaaaact ctggtgccgt cacttctgtg tgcttgcgga 9120
ctcagaatct ggtggacata tcactcactc tgggatggtg ggcatgggag tcagctgcac 9180
agtcacccgg gaagatggaa ccaatcgcag atagggctgc tagtgaacca atcacatgat 9240
gtcacccaga catcaggcat acccactagt gtgaaataga catcagaatt aagaaaaacg 9300
tagggtccaa gtggttcccc gttatggact cgctatctgt caaccagatc ttataccctg 9360
aagttcacct agatagcccg atagttacca ataagatagt agccatcctg gagtatgctc 9420
gagtccctca cgcttacagc ctggaggacc ctacactgtg tcagaacatc aagcaccgcc 9480
taaaaaacgg attttccaac caaatgatta taaacaatgt ggaagttggg aatgtcatca 9540
agtccaagct taggagttat ccggcccact ctcatattcc atatccaaat tgtaatcagg 9600
atttatttaa catagaagac aaagagtcaa cgaggaagat ccgtgaactc ctcaaaaagg 9660
ggaattcgct gtactccaaa gtcagtgata aggttttcca atgcttaagg gacactaact 9720
cacggcttgg cctaggctcc gaattgaggg aggacatcaa ggagaaagtt attaacttgg 9780
gagtttacat gcacagctcc cagtggtttg agccctttct gttttggttt acagtcaaga 9840
ctgagatgag gtcagtgatt aaatcacaaa cccatacttg ccataggagg agacacacac 9900
ctgtattctt cactggtagt tcagttgagt tgctaatctc tcgtgacctt gttgctataa 9960
tcagtaaaga gtctcaacat gtatattacc tgacatttga actggttttg atgtattgtg 10020
atgtcataga ggggaggtta atgacagaga ccgctatgac tattgatgct aggtatacag 10080
agcttctagg aagagtcaga tacatgtgga aactgataga tggtttcttc cctgcactcg 10140
ggaatccaac ttatcaaatt gtagccatgc tggagcctct ttcacttgct tacctgcagc 10200
tgagggatat aacagtagaa ctcagaggtg ctttccttaa ccactgcttt actgaaatac 10260
atgatgttct tgaccaaaac gggttttctg atgaaggtac ttatcatgag ttaactgaag 10320
ctctagatta cattttcata actgatgaca tacatctgac aggggagatt ttctcatttt 10380
tcagaagttt cggccacccc agacttgaag cagtaacggc tgctgaaaat gttaggaaat 10440
acatgaatca gcctaaagtc attgtgtatg agactctgat gaaaggtcat gccatatttt 10500
gtggaatcat aatcaacggc tatcgtgaca ggcacggagg cagttggcca ccgctgaccc 10560
tcccccctgca tgctgcagac acaatccgga atgctcaagc ttcaggtgaa gggttaacac 10620
atgagcagtg cgttgataac tggaaatctt ttgctggagt gaaatttggc tgctttatgc 10680

ctcttagcct ggatagtgat ctgacaatgt acctaaagga caaggcactt gctgctctcc 10740

aaagggaatg ggattcagtt tacccgaaag agttcctgcg ttacgaccct cccaagggaa 10800

ccgggtcacg gaggcttgta gatgttttcc ttaatgattc gagctttgac ccatatgatg 10860

tgataatgta tgttgtaagt ggagcttacc tccatgaccc tgagttcaac ctgtcttaca 10920

gcctgaaaga aaaggagatc aaggaaacag gtagactttt tgctaaaatg acttacaaaa 10980

tgagggcatg ccaagtgatt gctgaaaatc taatctcaaa cgggattggc aaatatttta 11040

aggacaatgg gatggccaag gatgagcacg atttgactaa ggcactccac actctagctg 11100

tctcaggagt ccccaaagat ctcaaagaaa gtcacagggg ggggccagtc ttaaaaacct 11160

actcccgaag cccagtccac acaagtacca ggaacgtgag agcagcaaaa gggtttatag 11220

ggttccctca agtaattcgg caggaccaag acactgatca tccggagaat atggaagctt 11280

acgagacagt cagtgcattt atcacgactg atctcaagaa gtactgcctt aattggagat 11340

atgagaccat cagcttgttt gcacagaggc taaatgagat ttacggattg ccctcatttt 11400

tccagtggct gcataagagg cttgagacct ctgtcctgta tgtaagtgac cctcattgcc 11460

cccccgacct tgacgcccat atcccgttat ataaagtccc caatgatcaa atcttcatta 11520

agtaccctat gggaggtata gaagggtatt gtcagaagct gtggaccatc agcaccattc 11580

cctatctata cctggctgct tatgagagcg gagtaaggat tgcttcgtta gtgcaagggg 11640

acaatcagac catagccgta acaaaaaggg tacccagcac atggccctac aaccttaaga 11700

aacgggaagc tgctagagta actagagatt actttgtaat tcttaggcaa aggctacatg 11760

atattggcca tcacctcaag gcaaatgaga caattgtttc atcacatttt tttgtctatt 11820

caaaaggaat atattatgat gggctacttg tgtcccaatc actcaagagc atcgcaagat 11880

gtgtattctg gtcagagact atagttgatg aaacaagggc agcatgcagt aatattgcta 11940

caacaatggc taaaagcatc gagagaggtt atgaccgtta ccttgcatat tccctgaacg 12000

tcctaaaagt gatacagcaa attctgatct ctcttggctt cacaatcaat tcaaccatga 12060

cccgggatgt agtcataccc ctcctcacaa acaacgacct cttaataagg atggcactgt 12120

tgcccgctcc tattgggggg atgaattatc tgaatatgag caggctgttt gtcagaaaca 12180

tcggtgatcc agtaacatca tcaattgctg atctcaagag aatgattctc gcctcactaa 12240

tgcctgaaga gaccctccat caagtaatga cacaacaacc gggggactct tcattcctag 12300

actgggctag cgacccttac tcagcaaatc ttgtatgtgt ccagagcatc actagactcc 12360

tcaagaacat aactgcaagg tttgtcctga tccatagtcc aaacccaatg ttaaaaggat 12420

tattccatga tgacagtaaa gaagaggacg agggactggc ggcattcctc atggacaggc 12480

atattatagt acctagggca gctcatgaaa tcctggatca tagtgtcaca ggggcaagag 12540

agtctattgc aggcatgctg gataccacaa aaggcttgat tcgagccagc atgaggaagg 12600

gggggttaac ctctcgagtg ataaccagat tgtccaatta tgactatgaa caattcagag 12660
cagggatggt gctattgaca ggaagaaaga gaaatgtcct cattgacaaa gagtcatgtt 12720
cagtgcagct ggcgagagct ctaagaagcc atatgtgggc gaggctagct cgaggacggc 12780
ctatttacgg ccttgaggtc cctgatgtac tagaatctat gcgaggccac cttattcggc 12840
gtcatgagac atgtgtcatc tgcgagtgtg gatcagtcaa ctacggatgg ttttttgtcc 12900
cctcgggttg ccaactggat gatattgaca aggaaacatc atccttgaga gtcccatata 12960
ttggttctac cactgatgag agaacagaca tgaagcttgc cttcgtaaga gccccaagtc 13020
gatccttgcg atctgctgtt agaatagcaa cagtgtactc atgggcttac ggtgatgatg 13080
atagctcttg gaacgaagcc tggttgttgg ctaggcaaag ggccaatgtg agcctggagg 13140
agctaagggt gatcactccc atctcaactt cgactaattt agcgcatagg ttgagggatc 13200
gtagcactca agtgaaatac tcaggtacat cccttgtccg agtggcgagg tataccacaa 13260
tctccaacga caatctctca tttgtcatat cagataagaa ggttgatact aactttatat 13320
accaacaagg aatgcttcta gggttgggtg ttttagaaac attgtttcga ctcgagaaag 13380
ataccggatc atctaacacg gtattacatc ttcacgtcga aacagattgt tgcgtgatcc 13440
cgatgataga tcatcccagg atacccagct cccgcaagct agagctgagg gcagagctat 13500
gtaccaaccc attgatatat gataatgcac ctttaattga cagagatgca acaaggctat 13560
acacccagag ccataggagg caccttgtgg aatttgttac atggtccaca ccccaactat 13620
atcacatttt agctaagtcc acagcactat ctatgattga cctggtaaca aaatttgaga 13680
aggaccatat gaatgaaatt tcagctctca taggggatga cgatatcaat agtttcataa 13740
ctgagtttct gctcatagag ccaagattat tcactatcta cttgggccag tgtgcggcca 13800
tcaattgggc atttgatgta cattatcata gaccatcagg gaaatatcag atgggtgagc 13860
tgttgtcatc gttcctttct agaatgagca aaggagtgtt taaggtgctt gtcaatgctc 13920
taagccaccc aaagatctac aagaaattct ggcattgtgg tattatagag cctatccatg 13980
gtccttcact tgatgctcaa aacttgcaca caactgtgtg caacatggtt tacacatgct 14040
atatgaccta cctcgacctg ttgttgaatg aagagttaga agagttcaca tttctcttgt 14100
gtgaaagcga cgaggatgta gtaccggaca gattcgacaa catccaggca aaacacttat 14160
gtgttctggc agatttgtac tgtcaaccag ggacctgccc accaattcga ggtctaagac 14220
cggtagagaa atgtgcagtt ctaaccgacc atatcaaggc agaggctatg ttatctccag 14280
caggatcttc gtggaacata aatccaatta ttgtagacca ttactcatgc tctctgactt 14340
atctccggcg aggatcgatc aaacagataa gattgagagt tgatccagga ttcattttcg 14400
acgccctcgc tgaggtaaat gtcagtcagc caaagatcgg cagcaacaac atctcaaata 14460

tgagcatcaa ggctttcaga cccccacacg atgatgttgc aaaattgctc aaagatatca 14520
acacaagcaa gcacaatctt cccatttcag ggggcaatct cgccaattat gaaatccatg 14580
ctttccgcag aatcgggttg aactcatctg cttgctacaa agctgttgag atatcaacat 14640
taattaggag atgccttgag ccaggggagg acggcttgtt cttgggtgag ggatcgggtt 14700
ctatgttgat cacttataaa gagatactta aactaaacaa gtgcttctat aatagtgggg 14760
tttccgccaa ttctagatct ggtcaaaggg aattagcacc ctatccctcc gaagttggcc 14820
ttgtcgaaca cagaatggga gtaggtaata ttgtcaaagt gctctttaac gggaggcccg 14880
aagtcacgtg ggtaggcagt gtagattgct tcaatttcat agttagtaat atccctacct 14940
ctagtgtggg gtttatccat tcagatatag agaccttgcc tgacaaagat actatagaga 15000
agctagagga attggcagcc atcttatcga tggctctgct cctgggcaaa ataggatcaa 15060
tactggtgat taagcttatg cctttcagcg gggattttgt tcagggattt ataagttatg 15120
tagggtctca ttatagagaa gtgaaccttg tataccctag atacagcaac ttcatctcta 15180
ctgaatctta tttggttatg acagatctca aggctaaccg gctaatgaat cctgaaaaga 15240
ttaagcagca gataattgaa tcatctgtga ggacttcacc tggacttata ggtcacatcc 15300
tatccattaa gcaactaagc tgcatacaag caattgtggg agacgcagtt agtagaggtg 15360
atatcaatcc tactctgaaa aaacttacac ctatagagca ggtgctgatc aattgcgggt 15420
tggcaattaa cggacctaag ctgtgcaaag aattgatcca ccatgatgtt gcctcagggc 15480
aagatggatt gcttaattct atactcatcc tctacaggga gttggcaaga ttcaaagaca 15540
accaaagaag tcaacaaggg atgttccacg cttaccccgt attggtaagt agcaggcaac 15600
gagaacttat atctaggatc acccgcaaat tctgggggca cattcttctt tactccggga 15660
acaaaaagtt gataaataag tttatccaga atctcaagtc cggctatctg atactagact 15720
tacaccagaa tatcttcgtt aagaatctat ccaagtcaga gaaacagatt attatgacgg 15780
ggggtttgaa acgtgagtgg gtttttaagg taacagtcaa ggagaccaaa gaatggtata 15840
agttagtcgg atacagtgcc ctgattaagg actaattggt tgaactccgg aaccctaatc 15900
ctgccctagg tggttaggca ttatttgcaa tatattaaag aaaactttga aaatacgaag 15960
tttctattcc cagctttgtc tggtggccgg catggtccca gcctcctcgc tggcgccggc 16020
tgggcaacat tccgagggga ccgtcccctc ggtaatggcg aatgggacgc ggccgatccg 16080
gctgctaaca aagcccgaaa ggaagctgag ttggctgctg ccaccgctga gcaataacta 16140
gcataacccc ttggggcctc taaacgggtc ttgaggggtt ttttgctgaa aggaggaact 16200
atatccggat gcggccgcgg gccctatggt acccagcttt tgttcccttt agtgagggtt 16260
aattccgagc ttggcgtaat catggtcata gctgtttcct gtgtgaaatt gttatccgct 16320
cacaattcca cacaacatag gagccggaag cataaagtgt aaagcctggg gtgcctaatg 16380

agtgaggtaa ctcacattaa ttgcgttgcg ctcactgccc gctttccagt cgggaaacct 16440
gtcgtgccag ctgcattaat gaatcggcca acgcgcgggg agaggcggtt tgcgtattgg 16500
gcgctcttcc gcttcctcgc tcactgactc gctgcgctcg gtcgttcggc tgcggcgagc 16560
ggtatcagct cactcaaagg cggtaatacg gttatccaca gaatcagggg ataacgcagg 16620
aaagaacatg tgagcaaaag gccagcaaaa ggccaggaac cgtaaaaagg ccgcgttgct 16680
ggcgtttttc cataggctcg gcccccctga cgagcatcac aaaaatcgac gctcaagtca 16740
gaggtggcga aacccgacag gactataaag ataccaggcg ttccccctg gaagctccct 16800
cgtgcgctct cctgttccga ccctgccgct taccggatac ctgtccgcct ttctcccttc 16860
gggaagcgtg gcgctttctc aatgctcacg ctgtaggtat ctcagttcgg tgtaggtcgt 16920
tcgctccaag ctgggctgtg tgcacgaacc ccccgttcag cccgaccgct gcgccttatc 16980
cggtaactat cgtcttgagt ccaacccggt aagacacgac ttatcgccac tggcagcagc 17040
cactggtaac aggattagca gagcgaggta tgtaggcggt gctacagagt tcttgaagtg 17100
gtggcctaac tacggctaca ctagaaggac agtatttggt atctgcgctc tgctgaagcc 17160
agttaccttc ggaaaaagag ttggtagctc ttgatccggc aaacaaacca ccgctggtag 17220
cggtggtttt tttgtttgca agcagcagat tacgcgcaga aaaaaaggat ctcaagaaga 17280
tcctttgatc ttttctacgg ggtctgacgc tcagtggaac gaaaactcac gttaagggat 17340
tttggtcatg agattatcaa aaaggatctt cacctagatc cttttaaatt aaaaatgaag 17400
ttttaaatca atctaaagta tatatgagta aacttggtct gacagttacc aatgcttaat 17460
cagtgaggca cctatctcag cgatctgtct atttcgttca tccatagttg cctgactgcc 17520
cgtcgtgtag ataactacga tacgggaggg cttaccatct ggccccagtg ctgcaatgat 17580
accgcgagac ccacgctcac cggctccaga tttatcagca ataaaccagc cagccggaag 17640
ggccgagcgc agaagtggtc ctgcaacttt atccgcctcc atccagtcta ttaattgttg 17700
ccgggaagct agagtaagta gttcgccagt taatagtttg cgcaacgttg ttgccattgc 17760
tacaggcatc gtggtgtcac gctcgtcgtt tggtatggct tcattcagct ccggttccca 17820
acgatcaagg cgagttacat gatcccccat gttgtgaaaa aaagcggtta gctccttcgg 17880
tcctccgatc gttgtcagaa gtaagttggc cgcagtgtta tcactcatgc ttatggcagc 17940
actgcataat tctcttactg tcatgccatc cgtaagatgc ttttctgtga ctggtgagta 18000
ctcaaccaag tcattctgag aatagtgtat gcggcgaccg agttgctctt gcccggcgtc 18060
aatacgggat aataccgcgc cacatagcag aactttaaaa gtgctcatca ttggaaaacg 18120
ttcttcgggg cgaaaactct caaggatctt accgctgttg agatccagtt cgatgtaacc 18180
cactcgtgca cccaactgat cttcagcatc ttttactttc accagcgttt ctgggtgagc 18240

aaaaacagga aggcaaaatg ccgcaaaaaa gggaataagg gcgacacgga aatgttgaat 18300

actcatactc ttcctttttc aatattattg aagcatttat cagggttatt gtctcatgag 18360

cggatacata tttgaatgta tttagaaaaa taaacaaata ggggttccgc gcacatttcc 18420

ccgaaaagtg ccacctgaaa ttgtaaacgt taatattttg ttaaaattcg cgttaaattt 18480

ttgttaaatc agctcatttt ttaaccaata ggccgaaatc ggcaaaatcc cttataaatc 18540

aaaagaatag accgagatag ggttgagtgt tgttccagtt tggaacaaga gtccactatt 18600

aaagaacgtg gactccaacg tcaaagggcg aaaaaccgtc tatcagggcg atggcccact 18660

acgtgaacca tcaccctaat caagtttttt ggggtcgagg tgccgtaaag cactaaatcg 18720

gaaccctaaa gggagccccc gatttagagc ttgacgggga aagccggcga acgtggcgag 18780

aaaggaaggg aagaaagcga aaggagcggg cgctagggcg ctggcaagtg tagcggtcac 18840

gctgcgcgta accaccacac ccgccgcgct taatgcgccg ctacagggcg cgtcccattc 18900

gccattcagg ctgcgcaact gttgggaagg gcgatcggtg cgggcctctt cgctattacg 18960

ccagccaccg cggtgatcct tataaagcta gatg 18994

<210> 2
<211> 19075
<212> DNA
<213> Artificial Sequence

<220>
<223> pTM 2ATU MV Schwarz short

<400> 2
gcggccgcta atacgactca ctatagggcc aactttgttt ggtctgatga gtccgtgagg 60

acgaaacccg gagtcccggg tcaccaaaca aagttgggta aggatagttc aatcaatgat 120

catcttctag tgcacttagg attcaagatc ctattatcag ggacaagagc aggattaggg 180

atatccgaga tggccacact tttaaggagc ttagcattgt tcaaaagaaa caaggacaaa 240

ccacccatta catcaggatc cggtggagcc atcagaggaa tcaaacacat tattatagta 300

ccaatccctg gagattcctc aattaccact cgatccagac ttctggaccg gttggtgagg 360

ttaattggaa acccggatgt gagcgggccc aaactaacag gggcactaat aggtatatta 420

tccttatttg tggagtctcc aggtcaattg attcagagga tcaccgatga ccctgacgtt 480

agcataaggc tgttagaggt tgtccagagt gaccagtcac aatctggcct taccttcgca 540

tcaagaggta ccaacatgga ggatgaggcg gaccaatact tttcacatga tgatccaatt 600

agtagtgatc aatccaggtt cggatggttc gggaacaagg aaatctcaga tattgaagtg 660

caagaccctg agggattcaa catgattctg ggtaccatcc tagcccaaat ttgggtcttg 720

ctcgcaaagg cggttacggc cccagacacg gcagctgatt cggagctaag aaggtggata 780

aagtacaccc aacaaagaag ggtagttggt gaatttagat tggagagaaa atggttggat 840

gtggtgagga acaggattgc cgaggacctc tccttacgcc gattcatggt cgctctaatc 900
ctggatatca agagaacacc cggaaacaaa cccaggattg ctgaaatgat atgtgacatt 960
gatacatata tcgtagaggc aggattagcc agttttatcc tgactattaa gtttgggata 1020
gaaactatgt atcctgctct tggactgcat gaatttgctg gtgagttatc cacacttgag 1080
tccttgatga acctttacca gcaaatgggg gaaactgcac cctacatggt aatcctggag 1140
aactcaattc agaacaagtt cagtgcagga tcataccctc tgctctggag ctatgccatg 1200
ggagtaggag tggaacttga aaactccatg ggaggtttga actttggccg atcttacttt 1260
gatccagcat attttagatt agggcaagag atggtaagga ggtcagctgg aaaggtcagt 1320
tccacattgg catctgaact cggtatcact gccgaggatg caaggcttgt ttcagagatt 1380
gcaatgcata ctactgagga caagatcagt agagcggttg gacccagaca agcccaagta 1440
tcatttctac acggtgatca aagtgagaat gagctaccga gattgggggg caaggaagat 1500
aggagggtca aacagagtcg aggagaagcc agggagagct acagagaaac cgggcccagc 1560
agagcaagtg atgcgagagc tgcccatctt ccaaccggca caccccctaga cattgacact 1620
gcaacggagt ccagccaaga tccgcaggac agtcgaaggt cagctgacgc cctgcttagg 1680
ctgcaagcca tggcaggaat ctcggaagaa caaggctcag acacggacac ccctatagtg 1740
tacaatgaca gaaatcttct agactaggtg cgagaggccg agggccagaa caacatccgc 1800
ctaccatcca tcattgttat aaaaaactta ggaaccaggt ccacacagcc gccagcccat 1860
caaccatcca ctcccacgat tggagccaat ggcagaagag caggcacgcc atgtcaaaaa 1920
cggactggaa tgcatccggg ctctcaaggc cgagcccatc ggctcactgg ccatcgagga 1980
agctatggca gcatggtcag aaatatcaga caacccagga caggagcgag ccacctgcag 2040
ggaagagaag gcaggcagtt cgggtctcag caaaccatgc ctctcagcaa ttggatcaac 2100
tgaaggcggt gcacctcgca tccgcggtca gggacctgga gagagcgatg acgacgctga 2160
aactttggga atccccccaa gaaatctcca ggcatcaagc actgggttac agtgttatta 2220
cgtttatgat cacagcggtg aagcggttaa gggaatccaa gatgctgact ctatcatggt 2280
tcaatcaggc cttgatggtg atagcaccct ctcaggagga gacaatgaat ctgaaaacag 2340
cgatgtggat attggcgaac ctgataccga gggatatgct atcactgacc ggggatctgc 2400
tcccatctct atggggttca gggcttctga tgttgaaact gcagaaggag gggagatcca 2460
cgagctcctg agactccaat ccagaggcaa caactttccg aagcttggga aaactctcaa 2520
tgttcctccg cccccggacc ccggtagggc cagcacttcc gggacaccca ttaaaaaggg 2580
cacagacgcg agattagcct catttggaac ggagatcgcg tctttattga caggtggtgc 2640
aacccaatgt gctcgaaagt caccctcgga accatcaggg ccaggtgcac ctgcggggaa 2700

tgtccccgag tgtgtgagca atgccgcact gatacaggag tggacacccg aatctggtac 2760
cacaatctcc ccgagatccc agaataatga agaaggggga gactattatg atgatgagct 2820
gttctctgat gtccaagata ttaaaacagc cttggccaaa atacacgagg ataatcagaa 2880
gataatctcc aagctagaat cactgctgtt attgaaggga gaagttgagt caattaagaa 2940
gcagatcaac aggcaaaata tcagcatatc caccctggaa ggacacctct caagcatcat 3000
gatcgccatt cctggacttg ggaaggatcc caacgacccc actgcagatg tcgaaatcaa 3060
tcccgacttg aaacccatca taggcagaga ttcaggccga gcactggccg aagttctcaa 3120
gaaacccgtt gccagccgac aactccaagg aatgacaaat ggacggacca gttccagagg 3180
acagctgctg aaggaatttc agctaaagcc gatcgggaaa aagatgagct cagccgtcgg 3240
gtttgttcct gacaccggcc ctgcatcacg cagtgtaatc cgctccatta taaaatccag 3300
ccggctagag gaggatcgga agcgttacct gatgactctc cttgatgata tcaaaggagc 3360
caatgatctt gccaagttcc accagatgct gatgaagata ataatgaagt agctacagct 3420
caacttacct gccaacccca tgccagtcga cccaactagc ctaccctcca tcattgttat 3480
aaaaaactta ggaaccaggt ccacacagcc gccagcccat caacgcgtac ggcgcgcagc 3540
gcttagacgt ctcgcgatcg atactagtac aacctaaatc cattataaaa aacttaggag 3600
caaagtgatt gcctcccaag gtccacaatg acagagacct acgacttcga caagtcggca 3660
tgggacatca aagggtcgat cgctccgata caacccacca cctacagtga tggcaggctg 3720
gtgccccagg tcagagtcat agatcctggt ctaggcgaca ggaaggatga atgctttatg 3780
tacatgtttc tgctgggggt tgttgaggac agcgattccc tagggcctcc aatcgggcga 3840
gcatttgggt tcctgccctt aggtgttggc agatccacag caaagcccga aaaactcctc 3900
aaagaggcca ctgagcttga catagttgtt agacgtacag cagggctcaa tgaaaaactg 3960
gtgttctaca acaacacccc actaactctc ctcacacctt ggagaaaggt cctaacaaca 4020
gggagtgtct tcaacgcaaa ccaagtgtgc aatgcggtta atctgatacc gctcgatacc 4080
ccgcagaggt tccgtgttgt ttatatgagc atcacccgtc tttcggataa cgggtattac 4140
accgttccta gaagaatgct ggaattcaga tcggtcaatg cagtggcctt caacctgctg 4200
gtgaccctta ggattgacaa ggcgataggc cctgggaaga tcatcgacaa tacagagcaa 4260
cttcctgagg caacatttat ggtccacatc gggaacttca ggagaaagaa gagtgaagtc 4320
tactctgccg attattgcaa aatgaaaatc gaaaagatgg gcctggtttt tgcacttggt 4380
gggatagggg gcaccagtct tcacattaga agcacaggca aaatgagcaa gactctccat 4440
gcacaactcg ggttcaagaa gaccttatgt tacccgctga tggatatcaa tgaagacctt 4500
aatcgattac tctggaggag cagatgcaag atagtaagaa tccaggcagt tttgcagcca 4560
tcagttcctc aagaattccg catttacgac gacgtgatca taaatgatga ccaaggacta 4620

ttcaaagttc tgtagaccgt agtgcccagc aatgcccgaa aacgaccccc ctcacaatga 4680

cagccagaag gcccggacaa aaaagccccc tccgaaagac tccacggacc aagcgagagg 4740

ccagccagca gccgacggca agcgcgaaca ccaggcggcc ccagcacaga acagccctga 4800

cacaaggcca ccaccagcca ccccaatctg catcctcctc gtgggacccc cgaggaccaa 4860

cccccaaggc tgcccccgat ccaaaccacc aaccgcatcc ccaccacccc cgggaaagaa 4920

acccccagca attggaaggc ccctccccct cttcctcaac acaagaactc cacaaccgaa 4980

ccgcacaagc gaccgaggtg acccaaccgc aggcatccga ctccctagac agatcctctc 5040

tccccggcaa actaaacaaa acttagggcc aaggaacata cacacccaac agaacccaga 5100

ccccggccca cggcgccgcg cccccaaccc ccgacaacca gagggagccc ccaaccaatc 5160

ccgccggctc cccccggtgcc cacaggcagg gacaccaacc cccgaacaga cccagcaccc 5220

aaccatcgac aatccaagac gggggggccc ccccaaaaaa aggcccccag gggccgacag 5280

ccagcaccgc gaggaagccc acccacccca cacacgacca cggcaaccaa accagaaccc 5340

agaccaccct gggccaccag ctcccagact cggccatcac cccgcagaaa ggaaaggcca 5400

caacccgcgc accccagccc cgatccggcg gggagccacc caacccgaac cagcacccaa 5460

gagcgatccc cgaaggaccc ccgaaccgca aaggacatca gtatcccaca gcctctccaa 5520

gtcccccggt ctcctcctct tctcgaaggg accaaaagat caatccacca cacccgacga 5580

cactcaactc cccaccccta aaggagacac cgggaatccc agaatcaaga ctcatccaat 5640

gtccatcatg ggtctcaagg tgaacgtctc tgccatattc atggcagtac tgttaactct 5700

ccaaacaccc accggtcaaa tccattgggg caatctctct aagatagggg tggtaggaat 5760

aggaagtgca agctacaaag ttatgactcg ttccagccat caatcattag tcataaaatt 5820

aatgcccaat ataactctcc tcaataactg cacgagggta gagattgcag aatacaggag 5880

actactgaga acagttttgg aaccaattag agatgcactt aatgcaatga cccagaatat 5940

aagaccggtt cagagtgtag cttcaagtag gagacacaag agatttgcgg gagtagtcct 6000

ggcaggtgcg gccctaggcg ttgccacagc tgctcagata acagccggca ttgcacttca 6060

ccagtccatg ctgaactctc aagccatcga caatctgaga gcgagcctgg aaactactaa 6120

tcaggcaatt gagacaatca gacaagcagg gcaggagatg atattggctg ttcagggtgt 6180

ccaagactac atcaataatg agctgatacc gtctatgaac caactatctt gtgatttaat 6240

cggccagaag ctcgggctca aattgctcag atactataca gaaatcctgt cattatttgg 6300

ccccagttta cgggacccca tatctgcgga gatatctatc caggctttga gctatgcgct 6360

tggaggagac atcaataagg tgttagaaaa gctcggatac agtggaggtg atttactggg 6420

catcttagag agcggaggaa taaaggcccg gataactcac gtcgacacag agtcctactt 6480

cattgtcctc agtatagcct atccgacgct gtccgagatt aagggggtga ttgtccaccg 6540
gctagagggg gtctcgtaca acataggctc tcaagagtgg tataccactg tgcccaagta 6600
tgttgcaacc caagggtacc ttatctcgaa ttttgatgag tcatcgtgta ctttcatgcc 6660
agaggggact gtgtgcagcc aaaatgcctt gtacccgatg agtcctctgc tccaagaatg 6720
cctccggggg tacaccaagt cctgtgctcg tacactcgta tccgggtctt ttgggaaccg 6780
gttcatttta tcacaaggga acctaatagc caattgtgca tcaatccttt gcaagtgtta 6840
cacaacagga acgatcatta atcaagaccc tgacaagatc ctaacataca ttgctgccga 6900
tcactgcccg gtagtcgagg tgaacggcgt gaccatccaa gtcgggagca ggaggtatcc 6960
agacgctgtg tacttgcaca gaattgacct cggtcctccc atatcattgg agaggttgga 7020
cgtagggaca aatctgggga atgcaattgc taagttggag gatgccaagg aattgttgga 7080
gtcatcggac cagatattga ggagtatgaa aggtttatcg agcactagca tagtctacat 7140
cctgattgca gtgtgtcttg gagggttgat agggatcccc gctttaatat gttgctgcag 7200
ggggcgttgt aacaaaaagg gagaacaagt tggtatgtca agaccaggcc taaagcctga 7260
tcttacggga acatcaaaat cctatgtaag gtcgctctga tcctctacaa ctcttgaaac 7320
acaaatgtcc cacaagtctc ctcttcgtca tcaagcaacc accgcaccca gcatcaagcc 7380
cacctgaaat tatctccggc ttccctctgg ccgaacaata tcggtagtta atcaaaactt 7440
agggtgcaag atcatccaca atgtcaccac aacgagaccg gataaatgcc ttctacaaag 7500
ataacccca tcccaaggga agtaggatag tcattaacag agaacatctt atgattgata 7560
gaccttatgt tttgctggct gttctgtttg tcatgtttct gagcttgatc gggttgctag 7620
ccattgcagg cattagactt catcgggcag ccatctacac cgcagagatc cataaaagcc 7680
tcagcaccaa tctagatgta actaactcaa tcgagcatca ggtcaaggac gtgctgacac 7740
cactcttcaa aatcatcggt gatgaagtgg gcctgaggac acctcagaga ttcactgacc 7800
tagtgaaatt aatctctgac aagattaaat tccttaatcc ggatagggag tacgacttca 7860
gagatctcac ttggtgtatc aacccgccag agagaatcaa attggattat gatcaatact 7920
gtgcagatgt ggctgctgaa gagctcatga atgcattggt gaactcaact ctactggaga 7980
ccagaacaac caatcagttc ctagctgtct caaagggaaa ctgctcaggg cccactacaa 8040
tcagaggtca attctcaaac atgtcgctgt ccctgttaga cttgtattta ggtcgaggtt 8100
acaatgtgtc atctatagtc actatgacat cccagggaat gtatgggga acttacctag 8160
tggaaaagcc taatctgagc agcaaaaggt cagagttgtc acaactgagc atgtaccgag 8220
tgtttgaagt aggtgttatc agaaatccgg gtttgggggc tccggtgttc catatgacaa 8280
actatcttga gcaaccagtc agtaatgatc tcagcaactg tatggtggct ttggggggagc 8340
tcaaactcgc agccctttgt cacggggaag attctatcac aattccctat cagggatcag 8400

ggaaaggtgt cagcttccag ctcgtcaagc taggtgtctg gaaatcccca accgacatgc 8460
aatcctgggt ccccttatca acggatgatc cagtgataga caggctttac ctctcatctc 8520
acagaggtgt tatcgctgac aatcaagcaa aatgggctgt cccgacaaca cgaacagatg 8580
acaagttgcg aatggagaca tgcttccaac aggcgtgtaa gggtaaaatc caagcactct 8640
gcgagaatcc cgagtgggca ccattgaagg ataacaggat tccttcatac ggggtcttgt 8700
ctgttgatct gagtctgaca gttgagctta aaatcaaaat tgcttcggga ttcgggccat 8760
tgatcacaca cggttcaggg atggacctat acaaatccaa ccacaacaat gtgtattggc 8820
tgactatccc gccaatgaag aacctagcct taggtgtaat caacacattg gagtggatac 8880
cgagattcaa ggttagtccc tacctcttca ctgtcccaat taaggaagca ggcgaagact 8940
gccatgcccc aacataccta cctgcggagg tggatggtga tgtcaaactc agttccaatc 9000
tggtgattct acctggtcaa gatctccaat atgttttggc aacctacgat acttccaggg 9060
ttgaacatgc tgtggtttat tacgtttaca gcccaagccg ctcattttct tacttttatc 9120
cttttaggtt gcctataaag gggtccccca tcgaattaca agtggaatgc ttcacatggg 9180
accaaaaact ctggtgccgt cacttctgtg tgcttgcgga ctcagaatct ggtggacata 9240
tcactcactc tgggatggtg ggcatgggag tcagctgcac agtcacccgg gaagatggaa 9300
ccaatcgcag atagggctgc tagtgaacca atcacatgat gtcacccaga catcaggcat 9360
acccactagt gtgaaataga catcagaatt aagaaaaacg tagggtccaa gtggttcccc 9420
gttatggact cgctatctgt caaccagatc ttataccctg aagttcacct agatagcccg 9480
atagttacca ataagatagt agccatcctg gagtatgctc gagtccctca cgcttacagc 9540
ctggaggacc ctacactgtg tcagaacatc aagcaccgcc taaaaaacgg attttccaac 9600
caaatgatta taaacaatgt ggaagttggg aatgtcatca agtccaagct taggagttat 9660
ccggcccact ctcatattcc atatccaaat tgtaatcagg atttatttaa catagaagac 9720
aaagagtcaa cgaggaagat ccgtgaactc ctcaaaaagg ggaattcgct gtactccaaa 9780
gtcagtgata aggttttcca atgcttaagg gacactaact cacggcttgg cctaggctcc 9840
gaattgaggg aggacatcaa ggagaaagtt attaacttgg gagtttacat gcacagctcc 9900
cagtggtttg agccctttct gttttggttt acagtcaaga ctgagatgag gtcagtgatt 9960
aaatcacaaa cccatacttg ccataggagg agacacacac ctgtattctt cactggtagt 10020
tcagttgagt tgctaatctc tcgtgacctt gttgctataa tcagtaaaga gtctcaacat 10080
gtatattacc tgacatttga actggttttg atgtattgtg atgtcataga ggggaggtta 10140
atgacagaga ccgctatgac tattgatgct aggtatacag agcttctagg aagagtcaga 10200
tacatgtgga aactgataga tggtttcttc cctgcactcg ggaatccaac ttatcaaatt 10260

gtagccatgc tggagcctct ttcacttgct tacctgcagc tgagggatat aacagtagaa 10320
ctcagaggtg ctttccttaa ccactgcttt actgaaatac atgatgttct tgaccaaaac 10380
gggttttctg atgaaggtac ttatcatgag ttaactgaag ctctagatta cattttcata 10440
actgatgaca tacatctgac aggggagatt ttctcatttt tcagaagttt cggccacccc 10500
agacttgaag cagtaacggc tgctgaaaat gttaggaaat acatgaatca gcctaaagtc 10560
attgtgtatg agactctgat gaaaggtcat gccatatttt gtggaatcat aatcaacggc 10620
tatcgtgaca ggcacggagg cagttggcca ccgctgaccc tcccccctgca tgctgcagac 10680
acaatccgga atgctcaagc ttcaggtgaa gggttaacac atgagcagtg cgttgataac 10740
tggaaatctt ttgctggagt gaaatttggc tgctttatgc ctcttagcct ggatagtgat 10800
ctgacaatgt acctaaagga caaggcactt gctgctctcc aaagggaatg ggattcagtt 10860
tacccgaaag agttcctgcg ttacgaccct cccaagggaa ccgggtcacg gaggcttgta 10920
gatgttttcc ttaatgattc gagctttgac ccatatgatg tgataatgta tgttgtaagt 10980
ggagcttacc tccatgaccc tgagttcaac ctgtcttaca gcctgaaaga aaaggagatc 11040
aaggaaacag gtagactttt tgctaaaatg acttacaaaa tgagggcatg ccaagtgatt 11100
gctgaaaatc taatctcaaa cgggattggc aaatatttta aggacaatgg gatggccaag 11160
gatgagcacg atttgactaa ggcactccac actctagctg tctcaggagt ccccaaagat 11220
ctcaaagaaa gtcacagggg ggggccagtc ttaaaaacct actcccgaag cccagtccac 11280
acaagtacca ggaacgtgag agcagcaaaa gggtttatag ggttccctca agtaattcgg 11340
caggaccaag acactgatca tccggagaat atggaagctt acgagacagt cagtgcattt 11400
atcacgactg atctcaagaa gtactgcctt aattggagat atgagaccat cagcttgttt 11460
gcacagaggc taaatgagat ttacggattg ccctcatttt tccagtggct gcataagagg 11520
cttgagacct ctgtcctgta tgtaagtgac cctcattgcc cccccgacct tgacgcccat 11580
atcccgttat ataaagtccc caatgatcaa atcttcatta agtaccctat gggaggtata 11640
gaagggtatt gtcagaagct gtggaccatc agcaccattc cctatctata cctggctgct 11700
tatgagagcg gagtaaggat tgcttcgtta gtgcaagggg acaatcagac catagccgta 11760
acaaaaaggg tacccagcac atggccctac aaccttaaga aacgggaagc tgctagagta 11820
actagagatt actttgtaat tcttaggcaa aggctacatg atattggcca tcacctcaag 11880
gcaaatgaga caattgtttc atcacatttt tttgtctatt caaaaggaat atattatgat 11940
gggctacttg tgtcccaatc actcaagagc atcgcaagat gtgtattctg gtcagagact 12000
atagttgatg aaacaagggc agcatgcagt aatattgcta caacaatggc taaaagcatc 12060
gagagaggtt atgaccgtta ccttgcatat tccctgaacg tcctaaaagt gatacagcaa 12120
attctgatct ctcttggctt cacaatcaat tcaaccatga cccgggatgt agtcataccc 12180

ctcctcacaa acaacgacct cttaataagg atggcactgt tgcccgctcc tattgggggg 12240
atgaattatc tgaatatgag caggctgttt gtcagaaaca tcggtgatcc agtaacatca 12300
tcaattgctg atctcaagag aatgattctc gcctcactaa tgcctgaaga gaccctccat 12360
caagtaatga cacaacaacc gggggactct tcattcctag actgggctag cgacccttac 12420
tcagcaaatc ttgtatgtgt ccagagcatc actagactcc tcaagaacat aactgcaagg 12480
tttgtcctga tccatagtcc aaacccaatg ttaaaaggat tattccatga tgacagtaaa 12540
gaagaggacg agggactggc ggcattcctc atggacaggc atattatagt acctagggca 12600
gctcatgaaa tcctggatca tagtgtcaca ggggcaagag agtctattgc aggcatgctg 12660
gataccacaa aaggcttgat tcgagccagc atgaggaagg ggggggttaac ctctcgagtg 12720
ataaccagat tgtccaatta tgactatgaa caattcagag cagggatggt gctattgaca 12780
ggaagaaaga gaaatgtcct cattgacaaa gagtcatgtt cagtgcagct ggcgagagct 12840
ctaagaagcc atatgtgggc gaggctagct cgaggacggc ctatttacgg ccttgaggtc 12900
cctgatgtac tagaatctat gcgaggccac cttattcggc gtcatgagac atgtgtcatc 12960
tgcgagtgtg gatcagtcaa ctacggatgg tttttttgtcc cctcgggttg ccaactggat 13020
gatattgaca aggaaacatc atccttgaga gtcccatata ttggttctac cactgatgag 13080
agaacagaca tgaagcttgc cttcgtaaga gccccaagtc gatccttgcg atctgctgtt 13140
agaatagcaa cagtgtactc atgggcttac ggtgatgatg atagctcttg gaacgaagcc 13200
tggttgttgg ctaggcaaag ggccaatgtg agcctggagg agctaagggt gatcactccc 13260
atctcaactt cgactaattt agcgcatagg ttgagggatc gtagcactca agtgaaatac 13320
tcaggtacat cccttgtccg agtggcgagg tataccacaa tctccaacga caatctctca 13380
tttgtcatat cagataagaa ggttgatact aactttatat accaacaagg aatgcttcta 13440
gggttgggtg ttttagaaac attgtttcga ctcgagaaag ataccggatc atctaacacg 13500
gtattacatc ttcacgtcga aacagattgt tgcgtgatcc cgatgataga tcatcccagg 13560
atacccagct cccgcaagct agagctgagg gcagagctat gtaccaaccc attgatatat 13620
gataatgcac ctttaattga cagagatgca acaaggctat acacccagag ccataggagg 13680
caccttgtgg aatttgttac atggtccaca ccccaactat atcacatttt agctaagtcc 13740
acagcactat ctatgattga cctggtaaca aaatttgaga aggaccatat gaatgaaatt 13800
tcagctctca taggggatga cgatatcaat agtttcataa ctgagtttct gctcatagag 13860
ccaagattat tcactatcta cttgggccag tgtgcggcca tcaattgggc atttgatgta 13920
cattatcata gaccatcagg gaaatatcag atgggtgagc tgttgtcatc gttcctttct 13980
agaatgagca aaggagtgtt taaggtgctt gtcaatgctc taagccaccc aaagatctac 14040

aagaaattct ggcattgtgg tattatagag cctatccatg gtccttcact tgatgctcaa 14100
aacttgcaca caactgtgtg caacatggtt tacacatgct atatgaccta cctcgacctg 14160
ttgttgaatg aagagttaga agagttcaca tttctcttgt gtgaaagcga cgaggatgta 14220
gtaccggaca gattcgacaa catccaggca aaacacttat gtgttctggc agatttgtac 14280
tgtcaaccag ggacctgccc accaattcga ggtctaagac cggtagagaa atgtgcagtt 14340
ctaaccgacc atatcaaggc agaggctatg ttatctccag caggatcttc gtggaacata 14400
aatccaatta ttgtagacca ttactcatgc tctctgactt atctccggcg aggatcgatc 14460
aaacagataa gattgagagt tgatccagga ttcattttcg acgccctcgc tgaggtaaat 14520
gtcagtcagc caaagatcgg cagcaacaac atctcaaata tgagcatcaa ggctttcaga 14580
cccccacacg atgatgttgc aaaattgctc aaagatatca acacaagcaa gcacaatctt 14640
cccatttcag gggcaatct cgccaattat gaaatccatg ctttccgcag aatcgggttg 14700
aactcatctg cttgctacaa agctgttgag atatcaacat taattaggag atgccttgag 14760
ccaggggagg acggcttgtt cttgggtgag ggatcgggtt ctatgttgat cacttataaa 14820
gagatactta aactaaacaa gtgcttctat aatagtgggg tttccgccaa ttctagatct 14880
ggtcaaaggg aattagcacc ctatccctcc gaagttggcc ttgtcgaaca cagaatggga 14940
gtaggtaata ttgtcaaagt gctctttaac gggaggcccg aagtcacgtg ggtaggcagt 15000
gtagattgct tcaatttcat agttagtaat atccctacct ctagtgtggg gtttatccat 15060
tcagatatag agaccttgcc tgacaaagat actatagaga agctagagga attggcagcc 15120
atcttatcga tggctctgct cctgggcaaa ataggatcaa tactggtgat taagcttatg 15180
cctttcagcg ggattttgt tcagggattt ataagttatg tagggtctca ttatagagaa 15240
gtgaaccttg tataccctag atacagcaac ttcatctcta ctgaatctta tttggttatg 15300
acagatctca aggctaaccg gctaatgaat cctgaaaaga ttaagcagca gataattgaa 15360
tcatctgtga ggacttcacc tggacttata ggtcacatcc tatccattaa gcaactaagc 15420
tgcatacaag caattgtggg agacgcagtt agtagaggtg atatcaatcc tactctgaaa 15480
aaacttacac ctatagagca ggtgctgatc aattgcgggt tggcaattaa cggacctaag 15540
ctgtgcaaag aattgatcca ccatgatgtt gcctcagggc aagatggatt gcttaattct 15600
atactcatcc tctacaggga gttggcaaga ttcaaagaca accaaagaag tcaacaaggg 15660
atgttccacg cttaccccgt attggtaagt agcaggcaac gagaacttat atctaggatc 15720
acccgcaaat tctgggggca cattcttctt tactccggga acaaaaagtt gataaataag 15780
tttatccaga atctcaagtc cggctatctg atactagact tacaccagaa tatcttcgtt 15840
aagaatctat ccaagtcaga gaaacagatt attatgacgg ggggtttgaa acgtgagtgg 15900
gtttttaagg taacagtcaa ggagaccaaa gaatggtata agttagtcgg atacagtgcc 15960

ctgattaagg actaattggt tgaactccgg aaccctaatc ctgccctagg tggttaggca 16020

ttatttgcaa tatattaaag aaaactttga aaatacgaag tttctattcc cagctttgtc 16080

tggtggccgg catggtccca gcctcctcgc tggcgccggc tgggcaacat tccgagggga 16140

ccgtcccctc ggtaatggcg aatgggacgc ggccgatccg gctgctaaca aagcccgaaa 16200

ggaagctgag ttggctgctg ccaccgctga gcaataacta gcataacccc ttggggcctc 16260

taaacgggtc ttgaggggtt ttttgctgaa aggaggaact atatccggat gcggccgcgg 16320

gccctatggt acccagcttt tgttcccttt agtgagggtt aattccgagc ttggcgtaat 16380

catggtcata gctgtttcct gtgtgaaatt gttatccgct cacaattcca cacaacatag 16440

gagccggaag cataaagtgt aaagcctggg gtgcctaatg agtgaggtaa ctcacattaa 16500

ttgcgttgcg ctcactgccc gctttccagt cgggaaacct gtcgtgccag ctgcattaat 16560

gaatcggcca acgcgcgggg agaggcggtt tgcgtattgg gcgctcttcc gcttcctcgc 16620

tcactgactc gctgcgctcg gtcgttcggc tgcggcgagc ggtatcagct cactcaaagg 16680

cggtaatacg gttatccaca gaatcagggg ataacgcagg aaagaacatg tgagcaaaag 16740

gccagcaaaa ggccaggaac cgtaaaaagg ccgcgttgct ggcgtttttc cataggctcg 16800

gcccccctga cgagcatcac aaaaatcgac gctcaagtca gaggtggcga aacccgacag 16860

gactataaag ataccaggcg tttccccctg gaagctccct cgtgcgctct cctgttccga 16920

ccctgccgct taccggatac ctgtccgcct ttctcccttc gggaagcgtg gcgctttctc 16980

aatgctcacg ctgtaggtat ctcagttcgg tgtaggtcgt tcgctccaag ctgggctgtg 17040

tgcacgaacc ccccgttcag cccgaccgct gcgccttatc cggtaactat cgtcttgagt 17100

ccaacccggt aagacacgac ttatcgccac tggcagcagc cactggtaac aggattagca 17160

gagcgaggta tgtaggcggt gctacagagt tcttgaagtg gtggcctaac tacggctaca 17220

ctagaaggac agtatttggt atctgcgctc tgctgaagcc agttaccttc ggaaaaagag 17280

ttggtagctc ttgatccggc aaacaaacca ccgctggtag cggtggtttt tttgtttgca 17340

agcagcagat tacgcgcaga aaaaaaggat ctcaagaaga tcctttgatc ttttctacgg 17400

ggtctgacgc tcagtggaac gaaaactcac gttaagggat tttggtcatg agattatcaa 17460

aaaggatctt cacctagatc cttttaaatt aaaaatgaag ttttaaatca atctaaagta 17520

tatatgagta aacttggtct gacagttacc aatgcttaat cagtgaggca cctatctcag 17580

cgatctgtct atttcgttca tccatagttg cctgactgcc cgtcgtgtag ataactacga 17640

tacgggaggg cttaccatct ggccccagtg ctgcaatgat accgcgagac ccacgctcac 17700

cggctccaga tttatcagca ataaaccagc cagccggaag ggccgagcgc agaagtggtc 17760

ctgcaacttt atccgcctcc atccagtcta ttaattgttg ccgggaagct agagtaagta 17820

gttcgccagt taatagtttg cgcaacgttg ttgccattgc tacaggcatc gtggtgtcac 17880

gctcgtcgtt tggtatggct tcattcagct ccggttccca acgatcaagg cgagttacat 17940

gatcccccat gttgtgaaaa aaagcggtta gctccttcgg tcctccgatc gttgtcagaa 18000

gtaagttggc cgcagtgtta tcactcatgc ttatggcagc actgcataat tctcttactg 18060

tcatgccatc cgtaagatgc ttttctgtga ctggtgagta ctcaaccaag tcattctgag 18120

aatagtgtat gcggcgaccg agttgctctt gcccggcgtc aatacgggat aataccgcgc 18180

cacatagcag aactttaaaa gtgctcatca ttggaaaacg ttcttcgggg cgaaaactct 18240

caaggatctt accgctgttg agatccagtt cgatgtaacc cactcgtgca cccaactgat 18300

cttcagcatc ttttactttc accagcgttt ctgggtgagc aaaaacagga aggcaaaatg 18360

ccgcaaaaaa gggaataagg gcgacacgga aatgttgaat actcatactc ttcctttttc 18420

aatattattg aagcatttat cagggttatt gtctcatgag cggatacata tttgaatgta 18480

tttagaaaaa taaacaaata ggggttccgc gcacatttcc ccgaaaagtg ccacctgaaa 18540

ttgtaaacgt taatattttg ttaaaattcg cgttaaattt ttgttaaatc agctcatttt 18600

ttaaccaata ggccgaaatc ggcaaaatcc cttataaatc aaaagaatag accgagatag 18660

ggttgagtgt tgttccagtt tggaacaaga gtccactatt aaagaacgtg gactccaacg 18720

tcaaagggcg aaaaaccgtc tatcagggcg atggcccact acgtgaacca tcaccctaat 18780

caagtttttt ggggtcgagg tgccgtaaag cactaaatcg gaaccctaaa gggagccccc 18840

gatttagagc ttgacgggga aagccggcga acgtggcgag aaaggaaggg aagaaagcga 18900

aaggagcggg cgctagggcg ctggcaagtg tagcggtcac gctgcgcgta accaccacac 18960

ccgccgcgct taatgcgccg ctacagggcg cgtcccattc gccattcagg ctgcgcaact 19020

gttgggaagg gcgatcggtg cgggcctctt cgctattacg ccagccaccg cggtg 19075

<210> 3
<211> 19107
<212> DNA
<213> Artificial Sequence

<220>
<223> pTM 2ATU MV Schwarz long

<400> 3
tccaaccgcg cgcgcggccg ctaatacgac tcactatagg gccaactttg tttggtctga 60

tgagtccgtg aggacgaaac ccggagtccc gggtcaccaa acaaagttgg gtaaggatag 120

ttcaatcaat gatcatcttc tagtgcactt aggattcaag atcctattat cagggacaag 180

agcaggatta gggatatccg agatggccac acttttaagg agcttagcat tgttcaaaag 240

aaacaaggac aaaccaccca ttacatcagg atccggtgga gccatcagag gaatcaaaca 300

cattattata gtaccaatcc ctggagattc ctcaattacc actcgatcca gacttctgga 360

ccggttggtg aggttaattg gaaacccgga tgtgagcggg cccaaactaa caggggcact 420
aataggtata ttatccttat ttgtggagtc tccaggtcaa ttgattcaga ggatcaccga 480
tgaccctgac gttagcataa ggctgttaga ggttgtccag agtgaccagt cacaatctgg 540
ccttaccttc gcatcaagag gtaccaacat ggaggatgag gcggaccaat acttttcaca 600
tgatgatcca attagtagtg atcaatccag gttcggatgg ttcgggaaca aggaaatctc 660
agatattgaa gtgcaagacc ctgagggatt caacatgatt ctgggtacca tcctagccca 720
aatttgggtc ttgctcgcaa aggcggttac ggccccagac acggcagctg attcggagct 780
aagaaggtgg ataaagtaca cccaacaaag aagggtagtt ggtgaattta gattggagag 840
aaaatggttg gatgtggtga ggaacaggat tgccgaggac ctctccttac gccgattcat 900
ggtcgctcta atcctggata tcaagagaac acccggaaac aaacccagga ttgctgaaat 960
gatatgtgac attgatacat atatcgtaga ggcaggatta gccagtttta tcctgactat 1020
taagtttggg atagaaacta tgtatcctgc tcttggactg catgaatttg ctggtgagtt 1080
atccacactt gagtccttga tgaaccttta ccagcaaatg gggaaactg caccctacat 1140
ggtaatcctg gagaactcaa ttcagaacaa gttcagtgca ggatcatacc ctctgctctg 1200
gagctatgcc atgggagtag gagtggaact tgaaaactcc atgggaggtt tgaactttgg 1260
ccgatcttac tttgatccag catattttag attagggcaa gagatggtaa ggaggtcagc 1320
tggaaaggtc agttccacat tggcatctga actcggtatc actgccgagg atgcaaggct 1380
tgtttcagag attgcaatgc atactactga ggacaagatc agtagagcgg ttggacccag 1440
acaagcccaa gtatcatttc tacacggtga tcaaagtgag aatgagctac cgagattggg 1500
gggcaaggaa gataggaggg tcaaacagag tcgaggagaa gccagggaga gctacagaga 1560
aaccgggccc agcagagcaa gtgatgcgag agctgcccat cttccaaccg gcacacccct 1620
agacattgac actgcaacgg agtccagcca agatccgcag gacagtcgaa ggtcagctga 1680
cgccctgctt aggctgcaag ccatggcagg aatctcggaa gaacaaggct cagacacgga 1740
cacccctata gtgtacaatg acagaaatct tctagactag gtgcgagagg ccgagggcca 1800
gaacaacatc cgcctaccat ccatcattgt tataaaaaac ttaggaacca ggtccacaca 1860
gccgccagcc catcaaccat ccactcccac gattggagcc aatggcagaa gagcaggcac 1920
gccatgtcaa aaacggactg gaatgcatcc gggctctcaa ggccgagccc atcggctcac 1980
tggccatcga ggaagctatg gcagcatggt cagaaatatc agacaaccca ggacaggagc 2040
gagccacctg cagggaagag aaggcaggca gttcgggtct cagcaaacca tgcctctcag 2100
caattggatc aactgaaggc ggtgcacctc gcatccgcgg tcagggacct ggagagagcg 2160
atgacgacgc tgaaactttg ggaatccccc caagaaatct ccaggcatca agcactgggt 2220

tacagtgtta ttacgtttat gatcacagcg gtgaagcggt taagggaatc caagatgctg 2280
actctatcat ggttcaatca ggccttgatg gtgatagcac cctctcagga ggagacaatg 2340
aatctgaaaa cagcgatgtg gatattggcg aacctgatac cgagggatat gctatcactg 2400
accggggatc tgctcccatc tctatggggt tcagggcttc tgatgttgaa actgcagaag 2460
gaggggagat ccacgagctc ctgagactcc aatccagagg caacaacttt ccgaagcttg 2520
ggaaaactct caatgttcct ccgcccccgg accccggtag ggccagcact tccgggacac 2580
ccattaaaaa gggcacagac gcgagattag cctcatttgg aacggagatc gcgtctttat 2640
tgacaggtgg tgcaacccaa tgtgctcgaa agtcaccctc ggaaccatca gggccaggtg 2700
cacctgcggg gaatgtcccc gagtgtgtga gcaatgccgc actgatacag gagtggacac 2760
ccgaatctgg taccacaatc tccccgagat cccagaataa tgaagaaggg ggagactatt 2820
atgatgatga gctgttctct gatgtccaag atattaaaac agccttggcc aaaatacacg 2880
aggataatca gaagataatc tccaagctag aatcactgct gttattgaag ggagaagttg 2940
agtcaattaa gaagcagatc aacaggcaaa atatcagcat atccaccctg gaaggacacc 3000
tctcaagcat catgatcgcc attcctggac ttgggaagga tcccaacgac cccactgcag 3060
atgtcgaaat caatcccgac ttgaaaccca tcataggcag agattcaggc cgagcactgg 3120
ccgaagttct caagaaaccc gttgccagcc gacaactcca aggaatgaca aatggacgga 3180
ccagttccag aggacagctg ctgaaggaat ttcagctaaa gccgatcggg aaaaagatga 3240
gctcagccgt cgggtttgtt cctgacaccg gccctgcatc acgcagtgta atccgctcca 3300
ttataaaatc cagccggcta gaggaggatc ggaagcgtta cctgatgact ctccttgatg 3360
atatcaaagg agccaatgat cttgccaagt tccaccagat gctgatgaag ataataatga 3420
agtagctaca gctcaactta cctgccaacc ccatgccagt cgacccaact agcctaccct 3480
ccatcattgt tataaaaaac ttaggaacca ggtccacaca gccgccagcc catcaacgcg 3540
tacggcgcgc agcgcttaga cgtctcgcga tcgatactag tacaacctaa atccattata 3600
aaaaacttag gagcaaagtg attgcctccc aaggtccaca atgacagaga cctacgactt 3660
cgacaagtcg gcatgggaca tcaaagggtc gatcgctccg atacaaccca ccacctacag 3720
tgatggcagg ctggtgcccc aggtcagagt catagatcct ggtctaggcg acaggaagga 3780
tgaatgcttt atgtacatgt ttctgctggg ggttgttgag gacagcgatt ccctagggcc 3840
tccaatcggg cgagcatttg ggttcctgcc cttaggtgtt ggcagatcca cagcaaagcc 3900
cgaaaaactc ctcaaagagg ccactgagct tgacatagtt gttagacgta cagcagggct 3960
caatgaaaaa ctggtgttct acaacaacac ccccactaact ctcctcacac cttggagaaa 4020
ggtcctaaca acagggagtg tcttcaacgc aaaccaagtg tgcaatgcgg ttaatctgat 4080
accgctcgat accccgcaga ggttccgtgt tgtttatatg agcatcaccc gtctttcgga 4140

taacgggtat tacaccgttc ctagaagaat gctggaattc agatcggtca atgcagtggc 4200

cttcaacctg ctggtgaccc ttaggattga caaggcgata ggccctggga agatcatcga 4260

caatacagag caacttcctg aggcaacatt tatggtccac atcgggaact tcaggagaaa 4320

gaagagtgaa gtctactctg ccgattattg caaaatgaaa atcgaaaaga tgggcctggt 4380

ttttgcactt ggtgggatag gggcaccag tcttcacatt agaagcacag gcaaaatgag 4440

caagactctc catgcacaac tcgggttcaa gaagacctta tgttacccgc tgatggatat 4500

caatgaagac cttaatcgat tactctggag gagcagatgc aagatagtaa gaatccaggc 4560

agttttgcag ccatcagttc ctcaagaatt ccgcatttac gacgacgtga tcataaatga 4620

tgaccaagga ctattcaaag ttctgtagac cgtagtgccc agcaatgccc gaaaacgacc 4680

cccctcacaa tgacagccag aaggcccgga caaaaaagcc ccctccgaaa gactccacgg 4740

accaagcgag aggccagcca gcagccgacg gcaagcgcga acaccaggcg gccccagcac 4800

agaacagccc tgacacaagg ccaccaccag ccaccccaat ctgcatcctc ctcgtgggac 4860

ccccgaggac caacccccaa ggctgccccc gatccaaacc accaaccgca tccccaccac 4920

ccccgggaaa gaaaccccca gcaattggaa ggcccctccc cctcttcctc aacacaagaa 4980

ctccacaacc gaaccgcaca agcgaccgag gtgacccaac cgcaggcatc cgactcccta 5040

gacagatcct ctctccccgg caaactaaac aaaacttagg gccaaggaac atacacaccc 5100

aacagaaccc agaccccggc ccacggcgcc gcgcccccaa cccccgacaa ccagagggag 5160

cccccaacca atcccgccgg ctcccccggt gcccacaggc agggacacca acccccgaac 5220

agacccagca cccaaccatc gacaatccaa gacgggggg cccccccaaa aaaaggcccc 5280

caggggccga cagccagcac cgcgaggaag cccacccacc ccacacacga ccacggcaac 5340

caaaccagaa cccagaccac cctgggccac cagctcccag actcggccat caccccgcag 5400

aaaggaaagg ccacaacccg cgcaccccag ccccgatccg gcggggagcc acccaacccg 5460

aaccagcacc caagagcgat ccccgaagga cccccgaacc gcaaaggaca tcagtatccc 5520

acagcctctc caagtccccc ggtctcctcc tcttctcgaa gggaccaaaa gatcaatcca 5580

ccacacccga cgacactcaa ctccccaccc ctaaaggaga caccgggaat cccagaatca 5640

agactcatcc aatgtccatc atgggtctca aggtgaacgt ctctgccata ttcatggcag 5700

tactgttaac tctccaaaca cccaccggtc aaatccattg gggcaatctc tctaagatag 5760

gggtggtagg aataggaagt gcaagctaca aagttatgac tcgttccagc catcaatcat 5820

tagtcataaa attaatgccc aatataactc tcctcaataa ctgcacgagg gtagagattg 5880

cagaatacag gagactactg agaacagttt tggaaccaat tagagatgca cttaatgcaa 5940

tgacccagaa tataagaccg gttcagagtg tagcttcaag taggagacac aagagatttg 6000

cgggagtagt cctggcaggt gcggccctag gcgttgccac agctgctcag ataacagccg 6060
gcattgcact tcaccagtcc atgctgaact ctcaagccat cgacaatctg agagcgagcc 6120
tggaaactac taatcaggca attgagacaa tcagacaagc agggcaggag atgatattgg 6180
ctgttcaggg tgtccaagac tacatcaata atgagctgat accgtctatg aaccaactat 6240
cttgtgattt aatcggccag aagctcgggc tcaaattgct cagatactat acagaaatcc 6300
tgtcattatt tggccccagt ttacgggacc ccatatctgc ggagatatct atccaggctt 6360
tgagctatgc gcttggagga gacatcaata aggtgttaga aaagctcgga tacagtggag 6420
gtgatttact gggcatctta gagagcggag gaataaaggc ccggataact cacgtcgaca 6480
cagagtccta cttcattgtc ctcagtatag cctatccgac gctgtccgag attaaggggg 6540
tgattgtcca ccggctagag ggggtctcgt acaacatagg ctctcaagag tggtatacca 6600
ctgtgcccaa gtatgttgca acccaagggt accttatctc gaattttgat gagtcatcgt 6660
gtactttcat gccagagggg actgtgtgca gccaaaatgc cttgtacccg atgagtcctc 6720
tgctccaaga atgcctccgg gggtacacca agtcctgtgc tcgtacactc gtatccgggt 6780
cttttgggaa ccggttcatt ttatcacaag ggaacctaat agccaattgt gcatcaatcc 6840
tttgcaagtg ttacacaaca ggaacgatca ttaatcaaga ccctgacaag atcctaacat 6900
acattgctgc cgatcactgc ccggtagtcg aggtgaacgg cgtgaccatc caagtcggga 6960
gcaggaggta tccagacgct gtgtacttgc acagaattga cctcggtcct cccatatcat 7020
tggagaggtt ggacgtaggg acaaatctgg ggaatgcaat tgctaagttg gaggatgcca 7080
aggaattgtt ggagtcatcg gaccagatat tgaggagtat gaaaggttta tcgagcacta 7140
gcatagtcta catcctgatt gcagtgtgtc ttggagggtt gatagggatc cccgctttaa 7200
tatgttgctg caggggggcgt tgtaacaaaa agggagaaca agttggtatg tcaagaccag 7260
gcctaaagcc tgatcttacg ggaacatcaa aatcctatgt aaggtcgctc tgatcctcta 7320
caactcttga aacacaaatg tcccacaagt ctcctcttcg tcatcaagca accaccgcac 7380
ccagcatcaa gcccacctga aattatctcc ggcttccctc tggccgaaca atatcggtag 7440
ttaatcaaaa cttagggtgc aagatcatcc acaatgtcac cacaacgaga ccggataaat 7500
gccttctaca aagataaccc ccatcccaag ggaagtagga tagtcattaa cagagaacat 7560
cttatgattg atagacctta tgttttgctg gctgttctgt ttgtcatgtt tctgagcttg 7620
atcgggttgc tagccattgc aggcattaga cttcatcggg cagccatcta caccgcagag 7680
atccataaaa gcctcagcac caatctagat gtaactaact caatcgagca tcaggtcaag 7740
gacgtgctga caccactctt caaaatcatc ggtgatgaag tgggcctgag gacacctcag 7800
agattcactg acctagtgaa attaatctct gacaagatta aattccttaa tccggatagg 7860
gagtacgact tcagagatct cacttggtgt atcaacccgc cagagagaat caaattggat 7920

tatgatcaat actgtgcaga tgtggctgct gaagagctca tgaatgcatt ggtgaactca 7980
actctactgg agaccagaac aaccaatcag ttcctagctg tctcaaaggg aaactgctca 8040
gggcccacta caatcagagg tcaattctca aacatgtcgc tgtccctgtt agacttgtat 8100
ttaggtcgag gttacaatgt gtcatctata gtcactatga catcccaggg aatgtatggg 8160
ggaacttacc tagtggaaaa gcctaatctg agcagcaaaa ggtcagagtt gtcacaactg 8220
agcatgtacc gagtgtttga agtaggtgtt atcagaaatc cgggtttggg ggctccggtg 8280
ttccatatga caaactatct tgagcaacca gtcagtaatg atctcagcaa ctgtatggtg 8340
gctttggggg agctcaaact cgcagccctt tgtcacgggg aagattctat cacaattccc 8400
tatcagggat cagggaaagg tgtcagcttc cagctcgtca agctaggtgt ctggaaatcc 8460
ccaaccgaca tgcaatcctg ggtcccctta tcaacggatg atccagtgat agacaggctt 8520
tacctctcat ctcacagagg tgttatcgct gacaatcaag caaaatgggc tgtcccgaca 8580
acacgaacag atgacaagtt gcgaatggag acatgcttcc aacaggcgtg taagggtaaa 8640
atccaagcac tctgcgagaa tcccgagtgg gcaccattga aggataacag gattccttca 8700
tacggggtct tgtctgttga tctgagtctg acagttgagc ttaaaatcaa aattgcttcg 8760
ggattcgggc cattgatcac acacggttca gggatggacc tatacaaatc caaccacaac 8820
aatgtgtatt ggctgactat cccgccaatg aagaacctag ccttaggtgt aatcaacaca 8880
ttggagtgga taccgagatt caaggttagt ccctacctct tcactgtccc aattaaggaa 8940
gcaggcgaag actgccatgc cccaacatac ctacctgcgg aggtggatgg tgatgtcaaa 9000
ctcagttcca atctggtgat tctacctggt caagatctcc aatatgtttt ggcaacctac 9060
gatacttcca gggttgaaca tgctgtggtt tattacgttt acagcccaag ccgctcattt 9120
tcttactttt atcctttttag gttgcctata aagggggtcc ccatcgaatt acaagtggaa 9180
tgcttcacat gggaccaaaa actctggtgc cgtcacttct gtgtgcttgc ggactcagaa 9240
tctggtggac atatcactca ctctgggatg gtgggcatgg gagtcagctg cacagtcacc 9300
cgggaagatg gaaccaatcg cagatagggc tgctagtgaa ccaatcacat gatgtcaccc 9360
agacatcagg catacccact agtgtgaaat agacatcaga attaagaaaa acgtagggtc 9420
caagtggttc cccgttatgg actcgctatc tgtcaaccag atcttatacc ctgaagttca 9480
cctagatagc ccgatagtta ccaataagat agtagccatc ctggagtatg ctcgagtccc 9540
tcacgcttac agcctggagg accctacact gtgtcagaac atcaagcacc gcctaaaaaa 9600
cggattttcc aaccaaatga ttataaacaa tgtggaagtt gggaatgtca tcaagtccaa 9660
gcttaggagt tatccggccc actctcatat tccatatcca aattgtaatc aggatttatt 9720
taacatagaa gacaaagagt caacgaggaa gatccgtgaa ctcctcaaaa aggggaattc 9780

gctgtactcc aaagtcagtg ataaggtttt ccaatgctta agggacacta actcacggct 9840

tggcctaggc tccgaattga gggaggacat caaggagaaa gttattaact tgggagttta 9900

catgcacagc tcccagtggt ttgagccctt tctgttttgg tttacagtca agactgagat 9960

gaggtcagtg attaaatcac aaacccatac ttgccatagg aggagacaca cacctgtatt 10020

cttcactggt agttcagttg agttgctaat ctctcgtgac cttgttgcta taatcagtaa 10080

agagtctcaa catgtatatt acctgacatt tgaactggtt ttgatgtatt gtgatgtcat 10140

agaggggagg ttaatgacag agaccgctat gactattgat gctaggtata cagagcttct 10200

aggaagagtc agatacatgt ggaaactgat agatggtttc ttccctgcac tcgggaatcc 10260

aacttatcaa attgtagcca tgctggagcc tctttcactt gcttacctgc agctgaggga 10320

tataacagta gaactcagag gtgctttcct taaccactgc tttactgaaa tacatgatgt 10380

tcttgaccaa aacgggtttt ctgatgaagg tacttatcat gagttaactg aagctctaga 10440

ttacattttc ataactgatg acatacatct gacaggggag attttctcat tttcagaag 10500

tttcggccac cccagacttg aagcagtaac ggctgctgaa aatgttagga aatacatgaa 10560

tcagcctaaa gtcattgtgt atgagactct gatgaaaggt catgccatat tttgtggaat 10620

cataatcaac ggctatcgtg acaggcacgg aggcagttgg ccaccgctga ccctccccct 10680

gcatgctgca gacacaatcc ggaatgctca agcttcaggt gaagggttaa cacatgagca 10740

gtgcgttgat aactggaaat cttttgctgg agtgaaattt ggctgcttta tgcctcttag 10800

cctggatagt gatctgacaa tgtacctaaa ggacaaggca cttgctgctc tccaaaggga 10860

atgggattca gtttacccga aagagttcct gcgttacgac cctcccaagg gaaccgggtc 10920

acggaggctt gtagatgttt tccttaatga ttcgagcttt gacccatatg atgtgataat 10980

gtatgttgta agtggagctt acctccatga ccctgagttc aacctgtctt acagcctgaa 11040

agaaaaggag atcaaggaaa caggtagact tttgctaaa atgacttaca aaatgagggc 11100

atgccaagtg attgctgaaa atctaatctc aaacgggatt ggcaaatatt ttaaggacaa 11160

tgggatggcc aaggatgagc acgatttgac taaggcactc cacactctag ctgtctcagg 11220

agtccccaaa gatctcaaag aaagtcacag gggggggcca gtcttaaaaa cctactcccg 11280

aagcccagtc cacacaagta ccaggaacgt gagagcagca aaagggttta tagggttccc 11340

tcaagtaatt cggcaggacc aagacactga tcatccggag aatatggaag cttacgagac 11400

agtcagtgca tttatcacga ctgatctcaa gaagtactgc cttaattgga gatatgagac 11460

catcagcttg tttgcacaga ggctaaatga gatttacgga ttgccctcat tttccagtg 11520

gctgcataag aggcttgaga cctctgtcct gtatgtaagt gaccctcatt gcccccccga 11580

ccttgacgcc catatcccgt tatataaagt ccccaatgat caaatcttca ttaagtaccc 11640

tatgggaggt atagaagggt attgtcagaa gctgtggacc atcagcacca ttccctatct 11700

atacctggct gcttatgaga gcggagtaag gattgcttcg ttagtgcaag gggacaatca 11760

gaccatagcc gtaacaaaaa gggtacccag cacatggccc tacaacctta agaaacggga 11820

agctgctaga gtaactagag attactttgt aattcttagg caaaggctac atgatattgg 11880

ccatcacctc aaggcaaatg agacaattgt ttcatcacat ttttttgtct attcaaaagg 11940

aatatattat gatgggctac ttgtgtccca atcactcaag agcatcgcaa gatgtgtatt 12000

ctggtcagag actatagttg atgaaacaag ggcagcatgc agtaatattg ctacaacaat 12060

ggctaaaagc atcgagagag gttatgaccg ttaccttgca tattccctga acgtcctaaa 12120

agtgatacag caaattctga tctctcttgg cttcacaatc aattcaacca tgacccggga 12180

tgtagtcata cccctcctca caaacaacga cctcttaata aggatggcac tgttgcccgc 12240

tcctattggg gggatgaatt atctgaatat gagcaggctg tttgtcagaa acatcggtga 12300

tccagtaaca tcatcaattg ctgatctcaa gagaatgatt ctcgcctcac taatgcctga 12360

agagaccctc catcaagtaa tgacacaaca accgggggac tcttcattcc tagactgggc 12420

tagcgaccct tactcagcaa atcttgtatg tgtccagagc atcactagac tcctcaagaa 12480

cataactgca aggtttgtcc tgatccatag tccaaaccca atgttaaaag gattattcca 12540

tgatgacagt aaagaagagg acgagggact ggcggcattc ctcatggaca ggcatattat 12600

agtacctagg gcagctcatg aaatcctgga tcatagtgtc acaggggcaa gagagtctat 12660

tgcaggcatg ctggatacca caaaaggctt gattcgagcc agcatgagga aggggggtt 12720

aacctctcga gtgataacca gattgtccaa ttatgactat gaacaattca gagcagggat 12780

ggtgctattg acaggaagaa agagaaatgt cctcattgac aaagagtcat gttcagtgca 12840

gctggcgaga gctctaagaa gccatatgtg ggcgaggcta gctcgaggac ggcctattta 12900

cggccttgag gtccctgatg tactagaatc tatgcgaggc caccttattc ggcgtcatga 12960

gacatgtgtc atctgcgagt gtggatcagt caactacgga tggtttttg tcccctcggg 13020

ttgccaactg gatgatattg acaaggaaac atcatccttg agagtcccat atattggttc 13080

taccactgat gagagaacag acatgaagct tgccttcgta agagccccaa gtcgatcctt 13140

gcgatctgct gttagaatag caacagtgta ctcatgggct tacggtgatg atgatagctc 13200

ttggaacgaa gcctggttgt tggctaggca aagggccaat gtgagcctgg aggagctaag 13260

ggtgatcact cccatctcaa cttcgactaa tttagcgcat aggttgaggg atcgtagcac 13320

tcaagtgaaa tactcaggta catcccttgt ccgagtggcg aggtatacca caatctccaa 13380

cgacaatctc tcatttgtca tatcagataa gaaggttgat actaacttta tataccaaca 13440

aggaatgctt ctagggttgg gtgttttaga aacattgttt cgactcgaga aagataccgg 13500

atcatctaac acggtattac atcttcacgt cgaaacagat tgttgcgtga tcccgatgat 13560

agatcatccc aggataccca gctcccgcaa gctagagctg agggcagagc tatgtaccaa 13620
cccattgata tatgataatg cacctttaat tgacagagat gcaacaaggc tatacaccca 13680
gagccatagg aggcaccttg tggaatttgt tacatggtcc acaccccaac tatatcacat 13740
tttagctaag tccacagcac tatctatgat tgacctggta acaaaatttg agaaggacca 13800
tatgaatgaa atttcagctc tcatagggga tgacgatatc aatagtttca taactgagtt 13860
tctgctcata gagccaagat tattcactat ctacttgggc cagtgtgcgg ccatcaattg 13920
ggcatttgat gtacattatc atagaccatc agggaaatat cagatgggtg agctgttgtc 13980
atcgttcctt tctagaatga gcaaaggagt gtttaaggtg cttgtcaatg ctctaagcca 14040
cccaaagatc tacaagaaat tctggcattg tggtattata gagcctatcc atggtccttc 14100
acttgatgct caaaacttgc acacaactgt gtgcaacatg gtttacacat gctatatgac 14160
ctacctcgac ctgttgttga atgaagagtt agaagagttc acatttctct tgtgtgaaag 14220
cgacgaggat gtagtaccgg acagattcga caacatccag gcaaaacact tatgtgttct 14280
ggcagatttg tactgtcaac cagggacctg cccaccaatt cgaggtctaa gaccggtaga 14340
gaaatgtgca gttctaaccg accatatcaa ggcagaggct atgttatctc cagcaggatc 14400
ttcgtggaac ataaatccaa ttattgtaga ccattactca tgctctctga cttatctccg 14460
gcgaggatcg atcaaacaga taagattgag agttgatcca ggattcattt tcgacgccct 14520
cgctgaggta aatgtcagtc agccaaagat cggcagcaac aacatctcaa atatgagcat 14580
caaggctttc agacccccac acgatgatgt tgcaaaattg ctcaaagata tcaacacaag 14640
caagcacaat cttcccattt caggggggcaa tctcgccaat tatgaaatcc atgctttccg 14700
cagaatcggg ttgaactcat ctgcttgcta caaagctgtt gagatatcaa cattaattag 14760
gagatgcctt gagccagggg aggacggctt gttcttgggt gagggatcgg gttctatgtt 14820
gatcacttat aaagagatac ttaaactaaa caagtgcttc tataatagtg gggtttccgc 14880
caattctaga tctggtcaaa gggaattagc accctatccc tccgaagttg gccttgtcga 14940
acacagaatg ggagtaggta atattgtcaa agtgctcttt aacgggaggc ccgaagtcac 15000
gtgggtaggc agtgtagatt gcttcaattt catagttagt aatatcccta cctctagtgt 15060
ggggtttatc cattcagata tagagacctt gcctgacaaa gatactatag agaagctaga 15120
ggaattggca gccatcttat cgatggctct gctcctgggc aaaataggat caatactggt 15180
gattaagctt atgcctttca gcggggattt tgttcaggga tttataagtt atgtagggtc 15240
tcattataga gaagtgaacc ttgtataccc tagatacagc aacttcatct ctactgaatc 15300
ttatttggtt atgacagatc tcaaggctaa ccggctaatg aatcctgaaa agattaagca 15360
gcagataatt gaatcatctg tgaggacttc acctggactt ataggtcaca tcctatccat 15420
taagcaacta agctgcatac aagcaattgt gggagacgca gttagtagag gtgatatcaa 15480

tcctactctg aaaaaactta cacctataga gcaggtgctg atcaattgcg ggttggcaat 15540
taacggacct aagctgtgca aagaattgat ccaccatgat gttgcctcag ggcaagatgg 15600
attgcttaat tctatactca tcctctacag ggagttggca agattcaaag acaaccaaag 15660
aagtcaacaa gggatgttcc acgcttaccc cgtattggta agtagcaggc aacgagaact 15720
tatatctagg atcacccgca aattctgggg gcacattctt ctttactccg ggaacaaaaa 15780
gttgataaat aagtttatcc agaatctcaa gtccggctat ctgatactag acttacacca 15840
gaatatcttc gttaagaatc tatccaagtc agagaaacag attattatga cgggggggttt 15900
gaaacgtgag tgggttttta aggtaacagt caaggagacc aaagaatggt ataagttagt 15960
cggatacagt gccctgatta aggactaatt ggttgaactc cggaacccta atcctgccct 16020
aggtggttag gcattatttg caatatatta aagaaaactt tgaaaatacg aagtttctat 16080
tcccagcttt gtctggtggc cggcatggtc ccagcctcct cgctggcgcc ggctgggcaa 16140
cattccgagg ggaccgtccc ctcggtaatg gcgaatggga cgcggccgat ccggctgcta 16200
acaaagcccg aaaggaagct gagttggctg ctgccaccgc tgagcaataa ctagcataac 16260
cccttggggc ctctaaacgg gtcttgaggg gttttttgct gaaaggagga actatatccg 16320
gatgcggccg cgggccctat ggtacccagc ttttgttccc tttagtgagg gttaattccg 16380
agcttggcgt aatcatggtc atagctgttt cctgtgtgaa attgttatcc gctcacaatt 16440
ccacacaaca taggagccgg aagcataaag tgtaaagcct ggggtgccta atgagtgagg 16500
taactcacat taattgcgtt gcgctcactg cccgctttcc agtcgggaaa cctgtcgtgc 16560
cagctgcatt aatgaatcgg ccaacgcgcg gggagaggcg gtttgcgtat tgggcgctct 16620
tccgcttcct cgctcactga ctcgctgcgc tcggtcgttc ggctgcggcg agcggtatca 16680
gctcactcaa aggcggtaat acggttatcc acagaatcag gggataacgc aggaaagaac 16740
atgtgagcaa aaggccagca aaaggccagg aaccgtaaaa aggccgcgtt gctggcgttt 16800
ttccataggc tcggcccccc tgacgagcat cacaaaaatc gacgctcaag tcagaggtgg 16860
cgaaacccga caggactata aagataccag gcgtttcccc ctggaagctc cctcgtgcgc 16920
tctcctgttc cgaccctgcc gcttaccgga tacctgtccg cctttctccc ttcgggaagc 16980
gtggcgcttt ctcaatgctc acgctgtagg tatctcagtt cggtgtaggt cgttcgctcc 17040
aagctgggct gtgtgcacga accccccgtt cagcccgacc gctgcgcctt atccggtaac 17100
tatcgtcttg agtccaaccc ggtaagacac gacttatcgc cactggcagc agccactggt 17160
aacaggatta gcagagcgag gtatgtaggc ggtgctacag agttcttgaa gtggtggcct 17220
aactacggct acactagaag gacagtattt ggtatctgcg ctctgctgaa gccagttacc 17280
ttcggaaaaa gagttggtag ctcttgatcc ggcaaacaaa ccaccgctgg tagcggtggt 17340

tttttgttt gcaagcagca gattacgcgc agaaaaaaag gatctcaaga agatcctttg 17400

atcttttcta cggggtctga cgctcagtgg aacgaaaact cacgttaagg gattttggtc 17460

atgagattat caaaaaggat cttcacctag atccttttaa attaaaaatg aagttttaaa 17520

tcaatctaaa gtatatatga gtaaacttgg tctgacagtt accaatgctt aatcagtgag 17580

gcacctatct cagcgatctg tctatttcgt tcatccatag ttgcctgact gccccgtcgtg 17640

tagataacta cgatacggga gggcttacca tctggcccca gtgctgcaat gataccgcga 17700

gacccacgct caccggctcc agatttatca gcaataaacc agccagccgg aagggccgag 17760

cgcagaagtg gtcctgcaac tttatccgcc tccatccagt ctattaattg ttgccgggaa 17820

gctagagtaa gtagttcgcc agttaatagt ttgcgcaacg ttgttgccat tgctacaggc 17880

atcgtggtgt cacgctcgtc gtttggtatg gcttcattca gctccggttc ccaacgatca 17940

aggcgagtta catgatcccc catgttgtga aaaaaagcgg ttagctcctt cggtcctccg 18000

atcgttgtca gaagtaagtt ggccgcagtg ttatcactca tgcttatggc agcactgcat 18060

aattctctta ctgtcatgcc atccgtaaga tgcttttctg tgactggtga gtactcaacc 18120

aagtcattct gagaatagtg tatgcggcga ccgagttgct cttgcccggc gtcaatacgg 18180

gataataccg cgccacatag cagaacttta aaagtgctca tcattggaaa acgttcttcg 18240

gggcgaaaac tctcaaggat cttaccgctg ttgagatcca gttcgatgta acccactcgt 18300

gcacccaact gatcttcagc atcttttact ttcaccagcg tttctgggtg agcaaaaaca 18360

ggaaggcaaa atgccgcaaa aaagggaata agggcgacac ggaaatgttg aatactcata 18420

ctcttccttt ttcaatatta ttgaagcatt tatcagggtt attgtctcat gagcggatac 18480

atatttgaat gtatttagaa aaataaacaa ataggggttc cgcgcacatt tccccgaaaa 18540

gtgccacctg aaattgtaaa cgttaatatt ttgttaaaat tcgcgttaaa tttttgttaa 18600

atcagctcat tttttaacca ataggccgaa atcggcaaaa tcccttataa atcaaaagaa 18660

tagaccgaga tagggttgag tgttgttcca gtttggaaca agagtccact attaaagaac 18720

gtggactcca acgtcaaagg gcgaaaaacc gtctatcagg gcgatggccc actacgtgaa 18780

ccatcaccct aatcaagttt tttggggtcg aggtgccgta aagcactaaa tcggaaccct 18840

aaagggagcc cccgatttag agcttgacgg ggaaagccgg cgaacgtggc gagaaaggaa 18900

gggaagaaag cgaaaggagc gggcgctagg gcgctggcaa gtgtagcggt cacgctgcgc 18960

gtaaccacca cacccgccgc gcttaatgcg ccgctacagg gcgcgtccca ttcgccattc 19020

aggctgcgca actgttggga agggcgatcg gtgcgggcct cttcgctatt acgccagcca 19080

ccgcggtgat ccttataaag ctagatg 19107

<210> 4
<211> 19822

<212> DNA
<213> Artificial Sequence

<220>
<223> MV GFP

<400> 4
tcatgaacgc ggccgctaat acgactcact atagggccaa ctttgtttgg tctgatgagt 60

ccgtgaggac gaaacccgga gtcccgggtc accaaacaaa gttgggtaag gatagttcaa 120

tcaatgatca tcttctagtg cacttaggat tcaagatcct attatcaggg acaagagcag 180

gattagggat atccgagatg gccacacttt taaggagctt agcattgttc aaaagaaaca 240

aggacaaacc acccattaca tcaggatccg gtggagccat cagaggaatc aaacacatta 300

ttatagtacc aatccctgga gattcctcaa ttaccactcg atccagactt ctggaccggt 360

tggtgaggtt aattggaaac ccggatgtga gcgggcccaa actaacaggg gcactaatag 420

gtatattatc cttatttgtg gagtctccag gtcaattgat tcagaggatc accgatgacc 480

ctgacgttag cataaggctg ttagaggttg tccagagtga ccagtcacaa tctggcctta 540

ccttcgcatc aagaggtacc aacatggagg atgaggcgga ccaatacttt tcacatgatg 600

atccaattag tagtgatcaa tccaggttcg gatggttcgg gaacaaggaa atctcagata 660

ttgaagtgca agaccctgag ggattcaaca tgattctggg taccatccta gcccaaattt 720

gggtcttgct cgcaaaggcg gttacggccc cagacacggc agctgattcg gagctaagaa 780

ggtggataaa gtacacccaa caaagaaggg tagttggtga atttagattg gagagaaaat 840

ggttggatgt ggtgaggaac aggattgccg aggacctctc cttacgccga ttcatggtcg 900

ctctaatcct ggatatcaag agaacacccg gaaacaaacc caggattgct gaaatgatat 960

gtgacattga tacatatatc gtagaggcag gattagccag ttttatcctg actattaagt 1020

ttgggataga aactatgtat cctgctcttg gactgcatga atttgctggt gagttatcca 1080

cacttgagtc cttgatgaac ctttaccagc aaatggggga aactgcaccc tacatggtaa 1140

tcctggagaa ctcaattcag aacaagttca gtgcaggatc ataccctctg ctctggagct 1200

atgccatggg agtaggagtg gaacttgaaa actccatggg aggtttgaac tttggccgat 1260

cttactttga tccagcatat tttagattag ggcaagagat ggtaaggagg tcagctggaa 1320

aggtcagttc cacattggca tctgaactcg gtatcactgc cgaggatgca aggcttgttt 1380

cagagattgc aatgcatact actgaggaca agatcagtag agcggttgga cccagacaag 1440

cccaagtatc atttctacac ggtgatcaaa gtgagaatga gctaccgaga ttgggggggca 1500

aggaagatag gagggtcaaa cagagtcgag gagaagccag ggagagctac agagaaaccg 1560

ggcccagcag agcaagtgat gcgagagctg cccatcttcc aaccggcaca cccctagaca 1620

ttgacactgc aacggagtcc agccaagatc cgcaggacag tcgaaggtca gctgacgccc 1680

tgcttaggct gcaagccatg gcaggaatct cggaagaaca aggctcagac acggacaccc 1740
ctatagtgta caatgacaga aatcttctag actaggtgcg agaggccgag ggccagaaca 1800
acatccgcct accatccatc attgttataa aaaacttagg aaccaggtcc acacagccgc 1860
cagcccatca accatccact cccacgattg gagccaatgg cagaagagca ggcacgccat 1920
gtcaaaaacg gactggaatg catccgggct ctcaaggccg agcccatcgg ctcactggcc 1980
atcgaggaag ctatggcagc atggtcagaa atatcagaca acccaggaca ggagcgagcc 2040
acctgcaggg aagagaaggc aggcagttcg ggtctcagca aaccatgcct ctcagcaatt 2100
ggatcaactg aaggcggtgc acctcgcatc cgcggtcagg gacctggaga gagcgatgac 2160
gacgctgaaa ctttgggaat ccccccaaga aatctccagg catcaagcac tgggttacag 2220
tgttattacg tttatgatca cagcggtgaa gcggttaagg gaatccaaga tgctgactct 2280
atcatggttc aatcaggcct tgatggtgat agcaccctct caggaggaga caatgaatct 2340
gaaaacagcg atgtggatat tggcgaacct gataccgagg gatatgctat cactgaccgg 2400
ggatctgctc ccatctctat ggggttcagg gcttctgatg ttgaaactgc agaaggaggg 2460
gagatccacg agctcctgag actccaatcc agaggcaaca actttccgaa gcttgggaaa 2520
actctcaatg ttcctccgcc cccggacccc ggtagggcca gcacttccgg gacacccatt 2580
aaaaagggca cagacgcgag attagcctca tttggaacgg agatcgcgtc tttattgaca 2640
ggtggtgcaa cccaatgtgc tcgaaagtca ccctcggaac catcagggcc aggtgcacct 2700
gcggggaatg tccccgagtg tgtgagcaat gccgcactga tacaggagtg gacacccgaa 2760
tctggtacca caatctcccc gagatcccag aataatgaag aagggggaga ctattatgat 2820
gatgagctgt tctctgatgt ccaagatatt aaaacagcct tggccaaaat acacgaggat 2880
aatcagaaga taatctccaa gctagaatca ctgctgttat tgaagggaga agttgagtca 2940
attaagaagc agatcaacag gcaaaatatc agcatatcca ccctggaagg acacctctca 3000
agcatcatga tcgccattcc tggacttggg aaggatccca acgaccccac tgcagatgtc 3060
gaaatcaatc ccgacttgaa acccatcata ggcagagatt caggccgagc actggccgaa 3120
gttctcaaga aacccgttgc cagccgacaa ctccaaggaa tgacaaatgg acggaccagt 3180
tccagaggac agctgctgaa ggaatttcag ctaaagccga tcgggaaaaa gatgagctca 3240
gccgtcgggt ttgttcctga caccggccct gcatcacgca gtgtaatccg ctccattata 3300
aaatccagcc ggctagagga ggatcggaag cgttacctga tgactctcct tgatgatatc 3360
aaaggagcca atgatcttgc caagttccac cagatgctga tgaagataat aatgaagtag 3420
ctacagctca acttacctgc caaccccatg ccagtcgacc caactagcct accctccatc 3480
attgttataa aaaacttagg aaccaggtcc acacagccgc cagcccatca acgcgtacga 3540
tggtgagcaa gggcgaggag ctgttcaccg gggtggtgcc catcctggtc gagctggacg 3600

gcgacgtaaa cggccacaag ttcagcgtgt ccggcgaggg cgagggcgat gccacctacg 3660
gcaagctgac cctgaagttc atctgcacca ccggcaagct gcccgtgccc tggcccaccc 3720
tcgtgaccac cctgacctac ggcgtgcagt gcttcagccg ctaccccgac cacatgaagc 3780
agcacgactt cttcaagtcc gccatgcccg aaggctacgt ccaggagcgc accatcttct 3840
tcaaggacga cggcaactac aagacccgcg ccgaggtgaa gttcgagggc gacaccctgg 3900
tgaaccgcat cgagctgaag ggcatcgact tcaaggagga cggcaacatc ctggggcaca 3960
agctggagta caactacaac agccacaacg tctatatcat ggccgacaag cagaagaacg 4020
gcatcaaggt gaacttcaag atccgccaca acatcgagga cggcagcgtg cagctcgccg 4080
accactacca gcagaacacc cccatcggcg acggccccgt gctgctgccc gacaaccact 4140
acctgagcac ccagtccgcc ctgagcaaag accccaacga gaagcgcgat cacatggtcc 4200
tgctggagtt cgtgaccgcc gccgggatca ctctcggcat ggacgagctg tacaagtagg 4260
cgcgcagcgc ttagacgtct cgcgatcgat actagtacaa cctaaatcca ttataaaaaa 4320
cttaggagca aagtgattgc ctcccaaggt ccacaatgac agagacctac gacttcgaca 4380
agtcggcatg ggacatcaaa gggtcgatcg ctccgataca acccaccacc tacagtgatg 4440
gcaggctggt gccccaggtc agagtcatag atcctggtct aggcgacagg aaggatgaat 4500
gctttatgta catgtttctg ctgggggttg ttgaggacag cgattcccta gggcctccaa 4560
tcgggcgagc atttgggttc ctgcccttag gtgttggcag atccacagca aagcccgaaa 4620
aactcctcaa agaggccact gagcttgaca tagttgttag acgtacagca gggctcaatg 4680
aaaaactggt gttctacaac aacaccccac taactctcct cacaccttgg agaaaggtcc 4740
taacaacagg gagtgtcttc aacgcaaacc aagtgtgcaa tgcggttaat ctgataccgc 4800
tcgatacccc gcagaggttc cgtgttgttt atatgagcat cacccgtctt tcggataacg 4860
ggtattacac cgttcctaga agaatgctgg aattcagatc ggtcaatgca gtggccttca 4920
acctgctggt gacccttagg attgacaagg cgataggccc tgggaagatc atcgacaata 4980
cagagcaact tcctgaggca acatttatgg tccacatcgg gaacttcagg agaaagaaga 5040
gtgaagtcta ctctgccgat tattgcaaaa tgaaaatcga aaagatgggc ctggtttttg 5100
cacttggtgg gataggggc accagtcttc acattagaag cacaggcaaa atgagcaaga 5160
ctctccatgc acaactcggg ttcaagaaga ccttatgtta cccgctgatg gatatcaatg 5220
aagaccttaa tcgattactc tggaggagca gatgcaagat agtaagaatc caggcagttt 5280
tgcagccatc agttcctcaa gaattccgca tttacgacga cgtgatcata aatgatgacc 5340
aaggactatt caaagttctg tagaccgtag tgcccagcaa tgcccgaaaa cgaccccct 5400
cacaatgaca gccagaaggc ccggacaaaa aagcccctc cgaaagactc cacggaccaa 5460

gcgagaggcc agccagcagc cgacggcaag cgcgaacacc aggcggcccc agcacagaac 5520
agccctgaca caaggccacc accagccacc ccaatctgca tcctcctcgt gggacccccg 5580
aggaccaacc cccaaggctg cccccgatcc aaaccaccaa ccgcatcccc accacccccg 5640
ggaaagaaac ccccagcaat tggaaggccc ctccccctct tcctcaacac aagaactcca 5700
caaccgaacc gcacaagcga ccgaggtgac ccaaccgcag gcatccgact ccctagacag 5760
atcctctctc cccggcaaac taaacaaaac ttagggccaa ggaacataca cacccaacag 5820
aacccagacc ccggcccacg gcgccgcgcc cccaaccccc gacaaccaga gggagccccc 5880
aaccaatccc gccggctccc ccggtgccca caggcaggga caccaacccc cgaacagacc 5940
cagcacccaa ccatcgacaa tccaagacgg gggggccccc ccaaaaaaag gcccccaggg 6000
gccgacagcc agcaccgcga ggaagcccac ccaccccaca cacgaccacg gcaaccaaac 6060
cagaacccag accaccctgg gccaccagct cccagactcg gccatcaccc cgcagaaagg 6120
aaaggccaca acccgcgcac cccagccccg atccggcggg gagccaccca acccgaacca 6180
gcacccaaga gcgatccccg aaggaccccc gaaccgcaaa ggacatcagt atcccacagc 6240
ctctccaagt cccccggtct cctcctcttc tcgaagggac caaaagatca atccaccaca 6300
cccgacgaca ctcaactccc caccccctaaa ggagacaccg ggaatcccag aatcaagact 6360
catccaatgt ccatcatggg tctcaaggtg aacgtctctg ccatattcat ggcagtactg 6420
ttaactctcc aaacacccac cggtcaaatc cattggggca atctctctaa gatagggggtg 6480
gtaggaatag gaagtgcaag ctacaaagtt atgactcgtt ccagccatca atcattagtc 6540
ataaaattaa tgcccaatat aactctcctc aataactgca cgagggtaga gattgcagaa 6600
tacaggagac tactgagaac agttttggaa ccaattagag atgcacttaa tgcaatgacc 6660
cagaatataa gaccggttca gagtgtagct tcaagtagga gacacaagag atttgcggga 6720
gtagtcctgg caggtgcggc cctaggcgtt gccacagctg ctcagataac agccggcatt 6780
gcacttcacc agtccatgct gaactctcaa gccatcgaca atctgagagc gagcctggaa 6840
actactaatc aggcaattga gacaatcaga caagcagggc aggagatgat attggctgtt 6900
cagggtgtcc aagactacat caataatgag ctgataccgt ctatgaacca actatcttgt 6960
gatttaatcg gccagaagct cgggctcaaa ttgctcagat actatacaga aatcctgtca 7020
ttatttggcc ccagtttacg ggaccccata tctgcggaga tatctatcca ggctttgagc 7080
tatgcgcttg gaggagacat caataaggtg ttagaaaagc tcggatacag tggaggtgat 7140
ttactgggca tcttagagag cggaggaata aaggcccgga taactcacgt cgacacagag 7200
tcctacttca ttgtcctcag tatagcctat ccgacgctgt ccgagattaa gggggtgatt 7260
gtccaccggc tagagggggt ctcgtacaac ataggctctc aagagtggta taccactgtg 7320
cccaagtatg ttgcaaccca agggtacctt atctcgaatt ttgatgagtc atcgtgtact 7380

ttcatgccag aggggactgt gtgcagccaa aatgccttgt acccgatgag tcctctgctc 7440

caagaatgcc tccgggggta caccaagtcc tgtgctcgta cactcgtatc cgggtctttt 7500

gggaaccggt tcattttatc acaagggaac ctaatagcca attgtgcatc aatcctttgc 7560

aagtgttaca caacaggaac gatcattaat caagaccctg acaagatcct aacatacatt 7620

gctgccgatc actgcccggt agtcgaggtg aacggcgtga ccatccaagt cgggagcagg 7680

aggtatccag acgctgtgta cttgcacaga attgacctcg gtcctcccat atcattggag 7740

aggttggacg tagggacaaa tctggggaat gcaattgcta agttggagga tgccaaggaa 7800

ttgttggagt catcggacca gatattgagg agtatgaaag gtttatcgag cactagcata 7860

gtctacatcc tgattgcagt gtgtcttgga gggttgatag ggatccccgc tttaatatgt 7920

tgctgcaggg ggcgttgtaa caaaaaggga gaacaagttg gtatgtcaag accaggccta 7980

aagcctgatc ttacgggaac atcaaaatcc tatgtaaggt cgctctgatc ctctacaact 8040

cttgaaacac aaatgtccca caagtctcct cttcgtcatc aagcaaccac cgcacccagc 8100

atcaagccca cctgaaatta tctccggctt ccctctggcc gaacaatatc ggtagttaat 8160

caaaacttag ggtgcaagat catccacaat gtcaccacaa cgagaccgga taaatgcctt 8220

ctacaaagat aacccccatc ccaagggaag taggatagtc attaacagag aacatcttat 8280

gattgataga ccttatgttt tgctggctgt tctgtttgtc atgtttctga gcttgatcgg 8340

gttgctagcc attgcaggca ttagacttca tcgggcagcc atctacaccg cagagatcca 8400

taaaagcctc agcaccaatc tagatgtaac taactcaatc gagcatcagg tcaaggacgt 8460

gctgacacca ctcttcaaaa tcatcggtga tgaagtgggc ctgaggacac ctcagagatt 8520

cactgaccta gtgaaattaa tctctgacaa gattaaattc cttaatccgg atagggagta 8580

cgacttcaga gatctcactt ggtgtatcaa cccgccagag agaatcaaat tggattatga 8640

tcaatactgt gcagatgtgg ctgctgaaga gctcatgaat gcattggtga actcaactct 8700

actggagacc agaacaacca atcagttcct agctgtctca aagggaaact gctcagggcc 8760

cactacaatc agaggtcaat tctcaaacat gtcgctgtcc ctgttagact tgtatttagg 8820

tcgaggttac aatgtgtcat ctatagtcac tatgacatcc cagggaatgt atgggggaac 8880

ttacctagtg gaaaagccta atctgagcag caaaaggtca gagttgtcac aactgagcat 8940

gtaccgagtg tttgaagtag gtgttatcag aaatccgggt ttgggggctc cggtgttcca 9000

tatgacaaac tatcttgagc aaccagtcag taatgatctc agcaactgta tggtggcttt 9060

gggggagctc aaactcgcag ccctttgtca cggggaagat tctatcacaa ttccctatca 9120

gggatcaggg aaaggtgtca gcttccagct cgtcaagcta ggtgtctgga aatccccaac 9180

cgacatgcaa tcctgggtcc ccttatcaac ggatgatcca gtgatagaca ggctttacct 9240

ctcatctcac agaggtgtta tcgctgacaa tcaagcaaaa tgggctgtcc cgacaacacg 9300
aacagatgac aagttgcgaa tggagacatg cttccaacag gcgtgtaagg gtaaaatcca 9360
agcactctgc gagaatcccg agtgggcacc attgaaggat aacaggattc cttcatacgg 9420
ggtcttgtct gttgatctga gtctgacagt tgagcttaaa atcaaaattg cttcgggatt 9480
cgggccattg atcacacacg gttcagggat ggacctatac aaatccaacc acaacaatgt 9540
gtattggctg actatcccgc caatgaagaa cctagcctta ggtgtaatca acacattgga 9600
gtggataccg agattcaagg ttagtcccta cctcttcact gtcccaatta aggaagcagg 9660
cgaagactgc catgccccaa catacctacc tgcggaggtg gatggtgatg tcaaactcag 9720
ttccaatctg gtgattctac ctggtcaaga tctccaatat gttttggcaa cctacgatac 9780
ttccagggtt gaacatgctg tggtttatta cgtttacagc ccaagccgct cattttctta 9840
cttttatcct tttaggttgc ctataaaggg ggtccccatc gaattacaag tggaatgctt 9900
cacatgggac caaaaactct ggtgccgtca cttctgtgtg cttgcggact cagaatctgg 9960
tggacatatc actcactctg ggatggtggg catgggagtc agctgcacag tcacccggga 10020
agatggaacc aatcgcagat agggctgcta gtgaaccaat cacatgatgt cacccagaca 10080
tcaggcatac ccactagtgt gaaatagaca tcagaattaa gaaaaacgta gggtccaagt 10140
ggttccccgt tatggactcg ctatctgtca accagatctt ataccctgaa gttcacctag 10200
atagcccgat agttaccaat aagatagtag ccatcctgga gtatgctcga gtccctcacg 10260
cttacagcct ggaggaccct acactgtgtc agaacatcaa gcaccgccta aaaaacggat 10320
tttccaacca aatgattata aacaatgtgg aagttgggaa tgtcatcaag tccaagctta 10380
ggagttatcc ggcccactct catattccat atccaaattg taatcaggat ttatttaaca 10440
tagaagacaa agagtcaacg aggaagatcc gtgaactcct caaaaagggg aattcgctgt 10500
actccaaagt cagtgataag gttttccaat gcttaaggga cactaactca cggcttggcc 10560
taggctccga attgagggag gacatcaagg agaaagttat taacttggga gtttacatgc 10620
acagctccca gtggtttgag ccctttctgt tttggtttac agtcaagact gagatgaggt 10680
cagtgattaa atcacaaacc catacttgcc ataggaggag acacacacct gtattcttca 10740
ctggtagttc agttgagttg ctaatctctc gtgaccttgt tgctataatc agtaaagagt 10800
ctcaacatgt atattacctg acatttgaac tggttttgat gtattgtgat gtcatagagg 10860
ggaggttaat gacagagacc gctatgacta ttgatgctag gtatacagag cttctaggaa 10920
gagtcagata catgtggaaa ctgatagatg gtttcttccc tgcactcggg aatccaactt 10980
atcaaattgt agccatgctg gagcctcttt cacttgctta cctgcagctg agggatataa 11040
cagtagaact cagaggtgct ttccttaacc actgctttac tgaaatacat gatgttcttg 11100
accaaaacgg gttttctgat gaaggtactt atcatgagtt aactgaagct ctagattaca 11160

ttttcataac tgatgacata catctgacag gggagatttt ctcatttttc agaagtttcg 11220

gccaccccag acttgaagca gtaacggctg ctgaaaatgt taggaaatac atgaatcagc 11280

ctaaagtcat tgtgtatgag actctgatga aaggtcatgc catattttgt ggaatcataa 11340

tcaacggcta tcgtgacagg cacggaggca gttggccacc gctgaccctc cccctgcatg 11400

ctgcagacac aatccggaat gctcaagctt caggtgaagg gttaacacat gagcagtgcg 11460

ttgataactg gaaatctttt gctggagtga aatttggctg ctttatgcct cttagcctgg 11520

atagtgatct gacaatgtac ctaaaggaca aggcacttgc tgctctccaa agggaatggg 11580

attcagttta cccgaaagag ttcctgcgtt acgaccctcc caagggaacc gggtcacgga 11640

ggcttgtaga tgttttcctt aatgattcga gctttgaccc atatgatgtg ataatgtatg 11700

ttgtaagtgg agcttacctc catgaccctg agttcaacct gtcttacagc ctgaaagaaa 11760

aggagatcaa ggaaacaggt agactttttg ctaaaatgac ttacaaaatg agggcatgcc 11820

aagtgattgc tgaaaatcta atctcaaacg ggattggcaa atattttaag gacaatggga 11880

tggccaagga tgagcacgat ttgactaagg cactccacac tctagctgtc tcaggagtcc 11940

ccaaagatct caaagaaagt cacagggggg ggccagtctt aaaaacctac tcccgaagcc 12000

cagtccacac aagtaccagg aacgtgagag cagcaaaagg gtttataggg ttccctcaag 12060

taattcggca ggaccaagac actgatcatc cggagaatat ggaagcttac gagacagtca 12120

gtgcatttat cacgactgat ctcaagaagt actgccttaa ttggagatat gagaccatca 12180

gcttgtttgc acagaggcta aatgagattt acggattgcc ctcatttttc cagtggctgc 12240

ataagaggct tgagacctct gtcctgtatg taagtgaccc tcattgcccc cccgaccttg 12300

acgcccatat cccgttatat aaagtcccca atgatcaaat cttcattaag taccctatgg 12360

gaggtataga agggtattgt cagaagctgt ggaccatcag caccattccc tatctatacc 12420

tggctgctta tgagagcgga gtaaggattg cttcgttagt gcaaggggac aatcagacca 12480

tagccgtaac aaaaagggta cccagcacat ggccctacaa ccttaagaaa cgggaagctg 12540

ctagagtaac tagagattac tttgtaattc ttaggcaaag gctacatgat attggccatc 12600

acctcaaggc aaatgagaca attgtttcat cacatttttt tgtctattca aaaggaatat 12660

attatgatgg gctacttgtg tcccaatcac tcaagagcat cgcaagatgt gtattctggt 12720

cagagactat agttgatgaa acaagggcag catgcagtaa tattgctaca acaatggcta 12780

aaagcatcga gagaggttat gaccgttacc ttgcatattc cctgaacgtc ctaaaagtga 12840

tacagcaaat tctgatctct cttggcttca caatcaattc aaccatgacc cgggatgtag 12900

tcatacccct cctcacaaac aacgacctct taataaggat ggcactgttg cccgctccta 12960

ttggggggat gaattatctg aatatgagca ggctgtttgt cagaaacatc ggtgatccag 13020

taacatcatc aattgctgat ctcaagagaa tgattctcgc ctcactaatg cctgaagaga 13080
ccctccatca agtaatgaca caacaaccgg gggactcttc attcctagac tgggctagcg 13140
acccttactc agcaaatctt gtatgtgtcc agagcatcac tagactcctc aagaacataa 13200
ctgcaaggtt tgtcctgatc catagtccaa acccaatgtt aaaaggatta ttccatgatg 13260
acagtaaaga agaggacgag ggactggcgg cattcctcat ggacaggcat attatagtac 13320
ctagggcagc tcatgaaatc ctggatcata gtgtcacagg ggcaagagag tctattgcag 13380
gcatgctgga taccacaaaa ggcttgattc gagccagcat gaggaagggg gggttaacct 13440
ctcgagtgat aaccagattg tccaattatg actatgaaca attcagagca gggatggtgc 13500
tattgacagg aagaaagaga aatgtcctca ttgacaaaga gtcatgttca gtgcagctgg 13560
cgagagctct aagaagccat atgtgggcga ggctagctcg aggacggcct atttacggcc 13620
ttgaggtccc tgatgtacta gaatctatgc gaggccacct tattcggcgt catgagacat 13680
gtgtcatctg cgagtgtgga tcagtcaact acggatggtt ttttgtcccc tcgggttgcc 13740
aactggatga tattgacaag gaaacatcat ccttgagagt cccatatatt ggttctacca 13800
ctgatgagag aacagacatg aagcttgcct tcgtaagagc cccaagtcga tccttgcgat 13860
ctgctgttag aatagcaaca gtgtactcat gggcttacgg tgatgatgat agctcttgga 13920
acgaagcctg gttgttggct aggcaaaggg ccaatgtgag cctggaggag ctaagggtga 13980
tcactcccat ctcaacttcg actaatttag cgcataggtt gagggatcgt agcactcaag 14040
tgaaatactc aggtacatcc cttgtccgag tggcgaggta taccacaatc tccaacgaca 14100
atctctcatt tgtcatatca gataagaagg ttgatactaa ctttatatac caacaaggaa 14160
tgcttctagg gttgggtgtt ttagaaacat tgtttcgact cgagaaagat accggatcat 14220
ctaacacggt attacatctt cacgtcgaaa cagattgttg cgtgatcccg atgatagatc 14280
atcccaggat acccagctcc cgcaagctag agctgagggc agagctatgt accaacccat 14340
tgatatatga taatgcacct ttaattgaca gagatgcaac aaggctatac acccagagcc 14400
ataggaggca ccttgtggaa tttgttacat ggtccacacc ccaactatat cacattttag 14460
ctaagtccac agcactatct atgattgacc tggtaacaaa atttgagaag gaccatatga 14520
atgaaatttc agctctcata ggggatgacg atatcaatag tttcataact gagtttctgc 14580
tcatagagcc aagattattc actatctact tgggccagtg tgcggccatc aattgggcat 14640
ttgatgtaca ttatcataga ccatcaggga aatatcagat gggtgagctg ttgtcatcgt 14700
tcctttctag aatgagcaaa ggagtgttta aggtgcttgt caatgctcta agccacccaa 14760
agatctacaa gaaattctgg cattgtggta ttatagagcc tatccatggt ccttcacttg 14820
atgctcaaaa cttgcacaca actgtgtgca acatggttta cacatgctat atgacctacc 14880
tcgacctgtt gttgaatgaa gagttagaag agttcacatt tctcttgtgt gaaagcgacg 14940

aggatgtagt accggacaga ttcgacaaca tccaggcaaa acacttatgt gttctggcag 15000
atttgtactg tcaaccaggg acctgcccac caattcgagg tctaagaccg gtagagaaat 15060
gtgcagttct aaccgaccat atcaaggcag aggctatgtt atctccagca ggatcttcgt 15120
ggaacataaa tccaattatt gtagaccatt actcatgctc tctgacttat ctccggcgag 15180
gatcgatcaa acagataaga ttgagagttg atccaggatt cattttcgac gccctcgctg 15240
aggtaaatgt cagtcagcca aagatcggca gcaacaacat ctcaaatatg agcatcaagg 15300
ctttcagacc cccacacgat gatgttgcaa aattgctcaa agatatcaac acaagcaagc 15360
acaatcttcc catttcaggg ggcaatctcg ccaattatga aatccatgct ttccgcagaa 15420
tcgggttgaa ctcatctgct tgctacaaag ctgttgagat atcaacatta attaggagat 15480
gccttgagcc aggggaggac ggcttgttct tgggtgaggg atcgggttct atgttgatca 15540
cttataaaga gatacttaaa ctaaacaagt gcttctataa tagtggggtt tccgccaatt 15600
ctagatctgg tcaaagggaa ttagcaccct atccctccga agttggcctt gtcgaacaca 15660
gaatgggagt aggtaatatt gtcaaagtgc tctttaacgg gaggcccgaa gtcacgtggg 15720
taggcagtgt agattgcttc aatttcatag ttagtaatat ccctacctct agtgtggggt 15780
ttatccattc agatatagag accttgcctg acaaagatac tatagagaag ctagaggaat 15840
tggcagccat cttatcgatg gctctgctcc tgggcaaaat aggatcaata ctggtgatta 15900
agcttatgcc tttcagcggg gattttgttc agggatttat aagttatgta gggtctcatt 15960
atagagaagt gaaccttgta taccctagat acagcaactt catctctact gaatcttatt 16020
tggttatgac agatctcaag gctaaccggc taatgaatcc tgaaaagatt aagcagcaga 16080
taattgaatc atctgtgagg acttcacctg gacttatagg tcacatccta tccattaagc 16140
aactaagctg catacaagca attgtgggag acgcagttag tagaggtgat atcaatccta 16200
ctctgaaaaa acttacacct atagagcagg tgctgatcaa ttgcgggttg gcaattaacg 16260
gacctaagct gtgcaaagaa ttgatccacc atgatgttgc ctcagggcaa gatggattgc 16320
ttaattctat actcatcctc tacagggagt tggcaagatt caaagacaac caaagaagtc 16380
aacaagggat gttccacgct taccccgtat tggtaagtag caggcaacga gaacttatat 16440
ctaggatcac ccgcaaattc tgggggcaca ttcttcttta ctccgggaac aaaaagttga 16500
taaataagtt tatccagaat ctcaagtccg gctatctgat actagactta caccagaata 16560
tcttcgttaa gaatctatcc aagtcagaga aacagattat tatgacgggg ggtttgaaac 16620
gtgagtgggt ttttaaggta acagtcaagg agaccaaaga atggtataag ttagtcggat 16680
acagtgccct gattaaggac taattggttg aactccggaa ccctaatcct gccctaggtg 16740
gttaggcatt atttgcaata tattaaagaa aactttgaaa atacgaagtt tctattccca 16800

gctttgtctg gtggccggca tggtcccagc ctcctcgctg gcgccggctg ggcaacattc 16860

cgaggggacc gtcccctcgg taatggcgaa tgggacgcgg ccgatccggc tgctaacaaa 16920

gcccgaaagg aagctgagtt ggctgctgcc accgctgagc aataactagc ataacccctt 16980

ggggcctcta aacgggtctt gaggggtttt ttgctgaaag gaggaactat atccggatgc 17040

ggccgcgggc cctatggtac ccagcttttg ttccctttag tgagggttaa ttccgagctt 17100

ggcgtaatca tggtcatagc tgtttcctgt gtgaaattgt tatccgctca caattccaca 17160

caacatagga gccggaagca taaagtgtaa agcctggggt gcctaatgag tgaggtaact 17220

cacattaatt gcgttgcgct cactgcccgc tttccagtcg ggaaacctgt cgtgccagct 17280

gcattaatga atcggccaac gcgcggggag aggcggtttg cgtattgggc gctcttccgc 17340

ttcctcgctc actgactcgc tgcgctcggt cgttcggctg cggcgagcgg tatcagctca 17400

ctcaaaggcg gtaatacggt tatccacaga atcaggggat aacgcaggaa agaacatgtg 17460

agcaaaaggc cagcaaaagg ccaggaaccg taaaaaggcc gcgttgctgg cgtttttcca 17520

taggctcggc cccccctgacg agcatcacaa aaatcgacgc tcaagtcaga ggtggcgaaa 17580

cccgacagga ctataaagat accaggcgtt tccccctgga agctccctcg tgcgctctcc 17640

tgttccgacc ctgccgctta ccggatacct gtccgccttt ctcccttcgg gaagcgtggc 17700

gctttctcaa tgctcacgct gtaggtatct cagttcggtg taggtcgttc gctccaagct 17760

gggctgtgtg cacgaacccc ccgttcagcc cgaccgctgc gccttatccg gtaactatcg 17820

tcttgagtcc aacccggtaa gacacgactt atcgccactg gcagcagcca ctggtaacag 17880

gattagcaga gcgaggtatg taggcggtgc tacagagttc ttgaagtggt ggcctaacta 17940

cggctacact agaaggacag tatttggtat ctgcgctctg ctgaagccag ttaccttcgg 18000

aaaaagagtt ggtagctctt gatccggcaa acaaaccacc gctggtagcg gtggtttttt 18060

tgtttgcaag cagcagatta cgcgcagaaa aaaaggatct caagaagatc ctttgatctt 18120

ttctacgggg tctgacgctc agtggaacga aaactcacgt taagggattt tggtcatgag 18180

attatcaaaa aggatcttca cctagatcct tttaaattaa aaatgaagtt ttaaatcaat 18240

ctaaagtata tatgagtaaa cttggtctga cagttaccaa tgcttaatca gtgaggcacc 18300

tatctcagcg atctgtctat ttcgttcatc catagttgcc tgactgcccg tcgtgtagat 18360

aactacgata cgggagggct taccatctgg ccccagtgct gcaatgatac cgcgagaccc 18420

acgctcaccg gctccagatt tatcagcaat aaaccagcca gccggaaggg ccgagcgcag 18480

aagtggtcct gcaactttat ccgcctccat ccagtctatt aattgttgcc gggaagctag 18540

agtaagtagt tcgccagtta atagtttgcg caacgttgtt gccattgcta caggcatcgt 18600

ggtgtcacgc tcgtcgtttg gtatggcttc attcagctcc ggttcccaac gatcaaggcg 18660

agttacatga tcccccatgt tgtgaaaaaa agcggttagc tccttcggtc ctccgatcgt 18720

tgtcagaagt aagttggccg cagtgttatc actcatgctt atggcagcac tgcataattc 18780

tcttactgtc atgccatccg taagatgctt ttctgtgact ggtgagtact caaccaagtc 18840

attctgagaa tagtgtatgc ggcgaccgag ttgctcttgc ccggcgtcaa tacgggataa 18900

taccgcgcca catagcagaa ctttaaaagt gctcatcatt ggaaaacgtt cttcggggcg 18960

aaaactctca aggatcttac cgctgttgag atccagttcg atgtaaccca ctcgtgcacc 19020

caactgatct tcagcatctt ttactttcac cagcgtttct gggtgagcaa aaacaggaag 19080

gcaaaatgcc gcaaaaaagg gaataagggc gacacggaaa tgttgaatac tcatactctt 19140

cctttttcaa tattattgaa gcatttatca gggttattgt ctcatgagcg gatacatatt 19200

tgaatgtatt tagaaaaata aacaaatagg ggttccgcgc acatttcccc gaaaagtgcc 19260

acctgaaatt gtaaacgtta atattttgtt aaaattcgcg ttaaattttt gttaaatcag 19320

ctcatttttt aaccaatagg ccgaaatcgg caaaatccct tataaatcaa aagaatagac 19380

cgagataggg ttgagtgttg ttccagtttg gaacaagagt ccactattaa agaacgtgga 19440

ctccaacgtc aaagggcgaa aaaccgtcta tcagggcgat ggcccactac gtgaaccatc 19500

accctaatca agttttttgg ggtcgaggtg ccgtaaagca ctaaatcgga accctaaagg 19560

gagcccccga tttagagctt gacggggaaa gccggcgaac gtggcgagaa aggaagggaa 19620

gaaagcgaaa ggagcgggcg ctagggcgct ggcaagtgta gcggtcacgc tgcgcgtaac 19680

caccacaccc gccgcgctta atgcgccgct acagggcgcg tcccattcgc cattcaggct 19740

gcgcaactgt tgggaagggc gatcggtgcg ggcctcttcg ctattacgcc agccaccgcg 19800

gtgatcctta taaagctaga tg 19822

<210> 5
<211> 22857
<212> DNA
<213> Artificial Sequence

<220>
<223> pTM 2ATU MV CHIK

<400> 5
tccaaccgcg cgcgcggccg ctaatacgac tcactatagg gccaactttg tttggtctga 60

tgagtccgtg aggacgaaac ccggagtccc gggtcaccaa acaaagttgg gtaaggatag 120

ttcaatcaat gatcatcttc tagtgcactt aggattcaag atcctattat cagggacaag 180

agcaggatta gggatatccg agatggccac acttttaagg agcttagcat tgttcaaaag 240

aaacaaggac aaaccaccca ttacatcagg atccggtgga gccatcagag gaatcaaaca 300

cattattata gtaccaatcc ctggagattc ctcaattacc actcgatcca gacttctgga 360

ccggttggtg aggttaattg gaaacccgga tgtgagcggg cccaaactaa caggggcact 420

aataggtata ttatccttat ttgtggagtc tccaggtcaa ttgattcaga ggatcaccga 480
tgaccctgac gttagcataa ggctgttaga ggttgtccag agtgaccagt cacaatctgg 540
ccttaccttc gcatcaagag gtaccaacat ggaggatgag gcggaccaat acttttcaca 600
tgatgatcca attagtagtg atcaatccag gttcggatgg ttcgggaaca aggaaatctc 660
agatattgaa gtgcaagacc ctgagggatt caacatgatt ctgggtacca tcctagccca 720
aatttgggtc ttgctcgcaa aggcggttac ggccccagac acggcagctg attcggagct 780
aagaaggtgg ataaagtaca cccaacaaag aagggtagtt ggtgaattta gattggagag 840
aaaatggttg gatgtggtga ggaacaggat tgccgaggac ctctccttac gccgattcat 900
ggtcgctcta atcctggata tcaagagaac acccggaaac aaacccagga ttgctgaaat 960
gatatgtgac attgatacat atatcgtaga ggcaggatta gccagtttta tcctgactat 1020
taagtttggg atagaaacta tgtatcctgc tcttggactg catgaatttg ctggtgagtt 1080
atccacactt gagtccttga tgaaccttta ccagcaaatg gggaaactg caccctacat 1140
ggtaatcctg gagaactcaa ttcagaacaa gttcagtgca ggatcatacc ctctgctctg 1200
gagctatgcc atgggagtag gagtggaact tgaaaactcc atgggaggtt tgaactttgg 1260
ccgatcttac tttgatccag catattttag attagggcaa gagatggtaa ggaggtcagc 1320
tggaaaggtc agttccacat tggcatctga actcggtatc actgccgagg atgcaaggct 1380
tgtttcagag attgcaatgc atactactga ggacaagatc agtagagcgg ttggacccag 1440
acaagcccaa gtatcatttc tacacggtga tcaaagtgag aatgagctac cgagattggg 1500
gggcaaggaa gataggaggg tcaaacagag tcgaggagaa gccagggaga gctacagaga 1560
aaccgggccc agcagagcaa gtgatgcgag agctgcccat cttccaaccg gcacacccct 1620
agacattgac actgcaacgg agtccagcca agatccgcag gacagtcgaa ggtcagctga 1680
cgccctgctt aggctgcaag ccatggcagg aatctcggaa gaacaaggct cagacacgga 1740
cacccctata gtgtacaatg acagaaatct tctagactag gtgcgagagg ccgagggcca 1800
gaacaacatc cgcctaccat ccatcattgt tataaaaaac ttaggaacca ggtccacaca 1860
gccgccagcc catcaaccat ccactcccac gattggagcc aatggcagaa gagcaggcac 1920
gccatgtcaa aaacggactg gaatgcatcc gggctctcaa ggccgagccc atcggctcac 1980
tggccatcga ggaagctatg gcagcatggt cagaaatatc agacaaccca ggacaggagc 2040
gagccacctg cagggaagag aaggcaggca gttcgggtct cagcaaacca tgcctctcag 2100
caattggatc aactgaaggc ggtgcacctc gcatccgcgg tcagggacct ggagagagcg 2160
atgacgacgc tgaaactttg ggaatccccc caagaaatct ccaggcatca agcactgggt 2220
tacagtgtta ttacgtttat gatcacagcg gtgaagcggt taagggaatc caagatgctg 2280
actctatcat ggttcaatca ggccttgatg gtgatagcac cctctcagga ggagacaatg 2340

aatctgaaaa cagcgatgtg gatattggcg aacctgatac cgagggatat gctatcactg 2400
accggggatc tgctcccatc tctatggggt tcagggcttc tgatgttgaa actgcagaag 2460
gaggggagat ccacgagctc ctgagactcc aatccagagg caacaacttt ccgaagcttg 2520
ggaaaactct caatgttcct ccgcccccgg accccggtag ggccagcact tccgggacac 2580
ccattaaaaa gggcacagac gcgagattag cctcatttgg aacggagatc gcgtctttat 2640
tgacaggtgg tgcaacccaa tgtgctcgaa agtcaccctc ggaaccatca gggccaggtg 2700
cacctgcggg gaatgtcccc gagtgtgtga gcaatgccgc actgatacag gagtggacac 2760
ccgaatctgg taccacaatc tccccgagat cccagaataa tgaagaaggg ggagactatt 2820
atgatgatga gctgttctct gatgtccaag atattaaaac agccttggcc aaaatacacg 2880
aggataatca gaagataatc tccaagctag aatcactgct gttattgaag ggagaagttg 2940
agtcaattaa gaagcagatc aacaggcaaa atatcagcat atccaccctg gaaggacacc 3000
tctcaagcat catgatcgcc attcctggac ttgggaagga tcccaacgac cccactgcag 3060
atgtcgaaat caatcccgac ttgaaaccca tcataggcag agattcaggc cgagcactgg 3120
ccgaagttct caagaaaccc gttgccagcc gacaactcca aggaatgaca aatggacgga 3180
ccagttccag aggacagctg ctgaaggaat ttcagctaaa gccgatcggg aaaaagatga 3240
gctcagccgt cgggtttgtt cctgacaccg gccctgcatc acgcagtgta atccgctcca 3300
ttataaaatc cagccggcta gaggaggatc ggaagcgtta cctgatgact ctccttgatg 3360
atatcaaagg agccaatgat cttgccaagt tccaccagat gctgatgaag ataataatga 3420
agtagctaca gctcaactta cctgccaacc ccatgccagt cgacccaact agcctaccct 3480
ccatcattgt tataaaaaac ttaggaacca ggtccacaca gccgccagcc catcaacgcg 3540
tacgatggag ttcatcccaa cccaaacttt ttacaatagg aggtaccagc ctcgaccctg 3600
gactccgcgc cctactatcc aagtcatcag gcccagaccg cgccctcaga ggcaagctgg 3660
gcaacttgcc cagctgatct cagcagttaa taaactgaca atgagagcgg taccccaaca 3720
gaagccacgc aggaatcgga agaataagaa gcaaaagcaa aaacaacagg cgccacaaaa 3780
caacacaaat caaaagaagc agccacctaa aaagaaaccg gctcaaaaga aaaagaagcc 3840
gggccgcaga gagaggatgt gcatgaaaat cgaaaatgat tgtattttcg aagtcaagca 3900
cgaaggtaag gtaacaggtt acgcgtgcct ggtgggggac aaagtaatga aaccagcaca 3960
cgtcaagggc accatcgata acgcggacct ggccaaactg gcctttaagc ggtcatctaa 4020
gtatgacctt gaatgcgctc agatacccgt gcacatgaag tccgacgctt cgaagttcac 4080
ccatgagaaa ccggaggggt actacaactg gcaccacgga gcagtacagt actcaggagg 4140
ccggttcacc atccctacag gtgctggcaa accaggggac agcggcagac cgatcttcga 4200

caacaaggga cgcgtggtgg ccatagtctt aggaggagct aatgaaggag cccgtacagc 4260
cctctcggtg gtgacctgga ataaagacat tgtcactaaa atcacccccg aggggggccga 4320
agagtggagt cttgccatcc cagttatgtg cctgttggca aacaccacgt tcccatgctc 4380
ccagccacct tgcacgccct gctgctacga gaaggaaccg gaggaaaccc tacgcatgct 4440
tgaggacaac gtcatgagac ctgggtacta tcagctgcta caagcatcct taacatgttc 4500
tccccaccgc cagcgacgca gcaccaagga caacttcaat gtctataaag ccacaagacc 4560
atacttagct cactgtcccg actgtggaga agggcactcg tgccatagtc ccgtagcact 4620
agaacgcatc agaaatgaag cgacagacgg gacgctgaaa atccaggtct ccttgcaaat 4680
cggaataaag acggatgaca gccacgattg gaccaagctg cgttatatgg acaaccacat 4740
gccagcagac gcagagaggg cggggctatt tgtaagaaca tcagcaccgt gtacgattac 4800
tggaacaatg ggacacttca tcctggcccg atgtcctaaa ggcgaaactc tgacggtggg 4860
attcactgac agtaggaaga ttagtcactc atgtacgcac ccatttcacc acgaccctcc 4920
tgtgataggt cgggagaaat tccattcccg accgcagcac ggtaaagagc taccttgcag 4980
cacctacgtg cagagcaccg ccgcaactac cgaggagata gaggtacaca tgcccccaga 5040
cacccctgat cgcacattaa tgtcacaaca gtccggcaac gtaaagatca cagtcaatgg 5100
ccagacggtg cggtacaagt gtaattgcgg tggctcaaat gaaggactaa caactacaga 5160
caaagtgatt aataactgca aggttgatca atgtcatgcc gcggtcacca atcacaaaaa 5220
gtggcagtat aactcccctc tggtcccgcg taatgctgaa cttggggacc ggaaaggaaa 5280
aattcacatc ccgtttccgc tggcaaatgt aacatgcagg gtgcctaaag caaggaaccc 5340
caccgtgacg tatggcaaaa accaagtcat catgctactg tatcctgacc acccaacact 5400
cctgtcctac cggaatatgg gagaagaacc aaactatcaa gaagagtggg tgatgcataa 5460
gaaggaagtc gtgctaaccg tgccgactga agggctcgag gtcacgtggg gcaacaacga 5520
gccgtataag tattggccgc agttatctac aaacggtaca gcccatggcc acccgcacga 5580
gataattctg tattattatg agctgtaccc cactatgact gtagtagttg tgtcagtggc 5640
cacgttcata ctcctgtcga tggtgggtat ggcagcgggg atgtgcatgt gtgcacgacg 5700
cagatgcatc acaccgtatg aactgacacc aggagctacc gtcccattcc tgcttagcct 5760
aatatgctgc atcagaacag ctaaagcggc cacataccaa gaggctgcga tatacctgtg 5820
gaacgagcag caacctttgt tttggctaca agcccttatt ccgctggcag ccctgattgt 5880
tctatgcaac tgtctgagac tcttaccatg ctgctgtaaa acgttggctt ttttagccgt 5940
aatgagcgtc ggtgcccaca ctgtgagcgc ttacgaacac gtaacagtga tcccgaacac 6000
ggtgggagta ccgtataaga ctctagtcaa tagacctggc tacagcccca tggtattgga 6060
gatggaacta ctgtcagtca ctttggagcc aacactatcg cttgattaca tcacgtgcga 6120

gtacaaaacc gtcatcccgt ctccttacgt gaagtgctgc ggtacagcag agtgcaagga 6180
caaaaaccta cctgactaca gctgtaaggt cttcaccggc gtctacccat ttatgtgggg 6240
cggcgcctac tgcttctgcg acgctgaaaa cacgcagttg agcgaagcac acgtggagaa 6300
gtccgaatca tgcaaaacag aatttgcatc agcatacagg gctcataccg catctgcatc 6360
agctaagctc cgcgtccttt accaaggaaa taacatcact gtaactgcct atgcaaacgg 6420
cgaccatgcc gtcacagtta aggacgccaa attcattgtg gggccaatgt cttcagcctg 6480
gacacctttc gacaacaaaa ttgtggtgta caaaggtgac gtctataaca tggactaccc 6540
gcccttcggc gcaggaagac caggacaatt tggcgatatc caaagtcgca cacctgagag 6600
taaagacgtc tatgctaata cacaactggt actgcagaga ccggctgtgg gtacggtaca 6660
cgtgccatac tctcaggcac catctggctt taagtattgg ctaaaagaac gcggggcgtc 6720
gctgcagcac acagcaccat ttggctgcca aatagcaaca aacccggtaa gagcggtgaa 6780
ctgcgccgta gggaacatgc ccatctccat cgacataccg gaagcggcct tcactagggt 6840
cgtcgacgcg ccctctttaa cggacatgtc gtgcgaggta ccagcctgca cccattcctc 6900
agactttggg ggcgtcgcca ttattaaata tgcagccagc aagaaaggca agtgtgcggt 6960
gcattcgatg actaacgccg tcactattcg ggaagctgag atagaagttg aagggaattc 7020
tcagctgcaa atctctttct cgacggcctt agccagcgcc gaattccgcg tacaagtctg 7080
ttctacacaa gtacactgtg cagccgagtg ccaccccccg aaggaccaca tagtcaacta 7140
cccggcgtca cataccaccc tcggggtcca ggacatctcc gctacggcga tgtcatgggt 7200
gcagaagatc acgggaggtg tgggactggt tgttgctgtt gccgcactga ttctaatcgt 7260
ggtgctatgc gtgtcgttca gcaggcacta ataggcgcgc agcgcttaga cgtctcgcga 7320
tcgatactag tacaacctaa atccattata aaaaacttag gagcaaagtg attgcctccc 7380
aaggtccaca atgacagaga cctacgactt cgacaagtcg gcatgggaca tcaaagggtc 7440
gatcgctccg atacaaccca ccacctacag tgatggcagg ctggtgcccc aggtcagagt 7500
catagatcct ggtctaggcg acaggaagga tgaatgcttt atgtacatgt ttctgctggg 7560
ggttgttgag gacagcgatt ccctagggcc tccaatcggg cgagcatttg ggttcctgcc 7620
cttaggtgtt ggcagatcca cagcaaagcc cgaaaaactc ctcaaagagg ccactgagct 7680
tgacatagtt gttagacgta cagcagggct caatgaaaaa ctggtgttct acaacaacac 7740
cccactaact ctcctcacac cttggagaaa ggtcctaaca acagggagtg tcttcaacgc 7800
aaaccaagtg tgcaatgcgg ttaatctgat accgctcgat accccgcaga ggttccgtgt 7860
tgtttatatg agcatcaccc gtctttcgga taacgggtat tacaccgttc ctagaagaat 7920
gctggaattc agatcggtca atgcagtggc cttcaacctg ctggtgaccc ttaggattga 7980

caaggcgata ggccctggga agatcatcga caatacagag caacttcctg aggcaacatt 8040
tatggtccac atcgggaact tcaggagaaa gaagagtgaa gtctactctg ccgattattg 8100
caaaatgaaa atcgaaaaga tgggcctggt ttttgcactt ggtgggatag ggggcaccag 8160
tcttcacatt agaagcacag gcaaaatgag caagactctc catgcacaac tcgggttcaa 8220
gaagacctta tgttacccgc tgatggatat caatgaagac cttaatcgat tactctggag 8280
gagcagatgc aagatagtaa gaatccaggc agttttgcag ccatcagttc ctcaagaatt 8340
ccgcatttac gacgacgtga tcataaatga tgaccaagga ctattcaaag ttctgtagac 8400
cgtagtgccc agcaatgccc gaaaacgacc cccctcacaa tgacagccag aaggcccgga 8460
caaaaaagcc ccctccgaaa gactccacgg accaagcgag aggccagcca gcagccgacg 8520
gcaagcgcga acaccaggcg gccccagcac agaacagccc tgacacaagg ccaccaccag 8580
ccaccccaat ctgcatcctc ctcgtgggac ccccgaggac caacccccaa ggctgccccc 8640
gatccaaacc accaaccgca tccccaccac ccccgggaaa gaaaccccca gcaattggaa 8700
ggcccctccc cctcttcctc aacacaagaa ctccacaacc gaaccgcaca agcgaccgag 8760
gtgacccaac cgcaggcatc cgactcccta gacagatcct ctctccccgg caaactaaac 8820
aaaacttagg gccaaggaac atacacaccc aacagaaccc agaccccggc ccacggcgcc 8880
gcgcccccaa cccccgacaa ccagagggag cccccaacca atcccgccgg ctcccccggt 8940
gcccacaggc agggacacca acccccgaac agacccagca cccaaccatc gacaatccaa 9000
gacggggggg ccccccaaa aaaaggcccc caggggccga cagccagcac cgcgaggaag 9060
cccacccacc ccacacacga ccacggcaac caaaccagaa cccagaccac cctgggccac 9120
cagctcccag actcggccat caccccgcag aaaggaaagg ccacaacccg cgcaccccag 9180
ccccgatccg gcggggagcc acccaacccg aaccagcacc caagagcgat ccccgaagga 9240
cccccgaacc gcaaaggaca tcagtatccc acagcctctc caagtccccc ggtctcctcc 9300
tcttctcgaa gggaccaaaa gatcaatcca ccacacccga cgacactcaa ctccccaccc 9360
ctaaaggaga caccgggaat cccagaatca agactcatcc aatgtccatc atgggtctca 9420
aggtgaacgt ctctgccata ttcatggcag tactgttaac tctccaaaca cccaccggtc 9480
aaatccattg ggcaatctc tctaagatag ggtggtagg aataggaagt gcaagctaca 9540
aagttatgac tcgttccagc catcaatcat tagtcataaa attaatgccc aatataactc 9600
tcctcaataa ctgcacgagg gtagagattg cagaatacag gagactactg agaacagttt 9660
tggaaccaat tagagatgca cttaatgcaa tgacccagaa tataagaccg gttcagagtg 9720
tagcttcaag taggagacac aagagatttg cgggagtagt cctggcaggt gcggccctag 9780
gcgttgccac agctgctcag ataacagccg gcattgcact tcaccagtcc atgctgaact 9840
ctcaagccat cgacaatctg agagcgagcc tggaaactac taatcaggca attgagacaa 9900

tcagacaagc agggcaggag atgatattgg ctgttcaggg tgtccaagac tacatcaata 9960
atgagctgat accgtctatg aaccaactat cttgtgattt aatcggccag aagctcgggc 10020
tcaaattgct cagatactat acagaaatcc tgtcattatt tggccccagt ttacgggacc 10080
ccatatctgc ggagatatct atccaggctt tgagctatgc gcttggagga gacatcaata 10140
aggtgttaga aaagctcgga tacagtggag gtgatttact gggcatctta gagagcggag 10200
gaataaaggc ccggataact cacgtcgaca cagagtccta cttcattgtc ctcagtatag 10260
cctatccgac gctgtccgag attaaggggg tgattgtcca ccggctagag ggggtctcgt 10320
acaacatagg ctctcaagag tggtatacca ctgtgcccaa gtatgttgca acccaagggt 10380
accttatctc gaattttgat gagtcatcgt gtactttcat gccagagggg actgtgtgca 10440
gccaaaatgc cttgtacccg atgagtcctc tgctccaaga atgcctccgg gggtacacca 10500
agtcctgtgc tcgtacactc gtatccgggt cttttgggaa ccggttcatt ttatcacaag 10560
ggaacctaat agccaattgt gcatcaatcc tttgcaagtg ttacacaaca ggaacgatca 10620
ttaatcaaga ccctgacaag atcctaacat acattgctgc cgatcactgc ccggtagtcg 10680
aggtgaacgg cgtgaccatc caagtcggga gcaggaggta tccagacgct gtgtacttgc 10740
acagaattga cctcggtcct cccatatcat tggagaggtt ggacgtaggg acaaatctgg 10800
ggaatgcaat tgctaagttg gaggatgcca aggaattgtt ggagtcatcg gaccagatat 10860
tgaggagtat gaaaggttta tcgagcacta gcatagtcta catcctgatt gcagtgtgtc 10920
ttggagggtt gatagggatc cccgctttaa tatgttgctg caggggggcgt tgtaacaaaa 10980
agggagaaca agttggtatg tcaagaccag gcctaaagcc tgatcttacg ggaacatcaa 11040
aatcctatgt aaggtcgctc tgatcctcta caactcttga aacacaaatg tcccacaagt 11100
ctcctcttcg tcatcaagca accaccgcac ccagcatcaa gcccacctga aattatctcc 11160
ggcttccctc tggccgaaca atatcggtag ttaatcaaaa cttagggtgc aagatcatcc 11220
acaatgtcac cacaacgaga ccggataaat gccttctaca aagataaccc ccatcccaag 11280
ggaagtagga tagtcattaa cagagaacat cttatgattg atagacctta tgttttgctg 11340
gctgttctgt ttgtcatgtt tctgagcttg atcgggttgc tagccattgc aggcattaga 11400
cttcatcggg cagccatcta caccgcagag atccataaaa gcctcagcac caatctagat 11460
gtaactaact caatcgagca tcaggtcaag gacgtgctga caccactctt caaaatcatc 11520
ggtgatgaag tgggcctgag gacacctcag agattcactg acctagtgaa attaatctct 11580
gacaagatta aattccttaa tccggatagg gagtacgact tcagagatct cacttggtgt 11640
atcaacccgc cagagagaat caaattggat tatgatcaat actgtgcaga tgtggctgct 11700
gaagagctca tgaatgcatt ggtgaactca actctactgg agaccagaac aaccaatcag 11760

ttcctagctg tctcaaaggg aaactgctca gggcccacta caatcagagg tcaattctca 11820

aacatgtcgc tgtccctgtt agacttgtat ttaggtcgag gttacaatgt gtcatctata 11880

gtcactatga catcccaggg aatgtatggg ggaacttacc tagtggaaaa gcctaatctg 11940

agcagcaaaa ggtcagagtt gtcacaactg agcatgtacc gagtgtttga agtaggtgtt 12000

atcagaaatc cgggtttggg ggctccggtg ttccatatga caaactatct tgagcaacca 12060

gtcagtaatg atctcagcaa ctgtatggtg gctttggggg agctcaaact cgcagccctt 12120

tgtcacgggg aagattctat cacaattccc tatcagggat cagggaaagg tgtcagcttc 12180

cagctcgtca agctaggtgt ctggaaatcc ccaaccgaca tgcaatcctg ggtcccctta 12240

tcaacggatg atccagtgat agacaggctt tacctctcat ctcacagagg tgttatcgct 12300

gacaatcaag caaaatgggc tgtcccgaca acacgaacag atgacaagtt gcgaatggag 12360

acatgcttcc aacaggcgtg taagggtaaa atccaagcac tctgcgagaa tcccgagtgg 12420

gcaccattga aggataacag gattccttca tacggggtct tgtctgttga tctgagtctg 12480

acagttgagc ttaaaatcaa aattgcttcg ggattcgggc cattgatcac acacggttca 12540

gggatggacc tatacaaatc caaccacaac aatgtgtatt ggctgactat cccgccaatg 12600

aagaacctag ccttaggtgt aatcaacaca ttggagtgga taccgagatt caaggttagt 12660

ccctacctct tcactgtccc aattaaggaa gcaggcgaag actgccatgc ccccaacatac 12720

ctacctgcgg aggtggatgg tgatgtcaaa ctcagttcca atctggtgat tctacctggt 12780

caagatctcc aatatgtttt ggcaacctac gatacttcca gggttgaaca tgctgtggtt 12840

tattacgttt acagcccaag ccgctcattt tcttactttt atccttttag gttgcctata 12900

aaggggggtcc ccatcgaatt acaagtggaa tgcttcacat gggaccaaaa actctggtgc 12960

cgtcacttct gtgtgcttgc ggactcagaa tctggtggac atatcactca ctctgggatg 13020

gtgggcatgg gagtcagctg cacagtcacc cgggaagatg gaaccaatcg cagatagggc 13080

tgctagtgaa ccaatcacat gatgtcaccc agacatcagg catacccact agtgtgaaat 13140

agacatcaga attaagaaaa acgtagggtc caagtggttc cccgttatgg actcgctatc 13200

tgtcaaccag atcttatacc ctgaagttca cctagatagc ccgatagtta ccaataagat 13260

agtagccatc ctggagtatg ctcgagtccc tcacgcttac agcctggagg accctacact 13320

gtgtcagaac atcaagcacc gcctaaaaaa cggattttcc aaccaaatga ttataaacaa 13380

tgtggaagtt gggaatgtca tcaagtccaa gcttaggagt tatccggccc actctcatat 13440

tccatatcca aattgtaatc aggatttatt taacatagaa gacaaagagt caacgaggaa 13500

gatccgtgaa ctcctcaaaa aggggaattc gctgtactcc aaagtcagtg ataaggtttt 13560

ccaatgctta agggacacta actcacggct tggcctaggc tccgaattga gggaggacat 13620

caaggagaaa gttattaact tgggagttta catgcacagc tcccagtggt ttgagccctt 13680

tctgttttgg tttacagtca agactgagat gaggtcagtg attaaatcac aaacccatac 13740
ttgccatagg aggagacaca cacctgtatt cttcactggt agttcagttg agttgctaat 13800
ctctcgtgac cttgttgcta taatcagtaa agagtctcaa catgtatatt acctgacatt 13860
tgaactggtt ttgatgtatt gtgatgtcat agaggggagg ttaatgacag agaccgctat 13920
gactattgat gctaggtata cagagcttct aggaagagtc agatacatgt ggaaactgat 13980
agatggtttc ttccctgcac tcgggaatcc aacttatcaa attgtagcca tgctggagcc 14040
tctttcactt gcttacctgc agctgaggga tataacagta gaactcagag gtgctttcct 14100
taaccactgc tttactgaaa tacatgatgt tcttgaccaa aacgggtttt ctgatgaagg 14160
tacttatcat gagttaactg aagctctaga ttacattttc ataactgatg acatacatct 14220
gacaggggag attttctcat ttttcagaag tttcggccac cccagacttg aagcagtaac 14280
ggctgctgaa aatgttagga aatacatgaa tcagcctaaa gtcattgtgt atgagactct 14340
gatgaaaggt catgccatat tttgtggaat cataatcaac ggctatcgtg acaggcacgg 14400
aggcagttgg ccaccgctga ccctccccct gcatgctgca gacacaatcc ggaatgctca 14460
agcttcaggt gaagggttaa cacatgagca gtgcgttgat aactggaaat cttttgctgg 14520
agtgaaattt ggctgcttta tgcctcttag cctggatagt gatctgacaa tgtacctaaa 14580
ggacaaggca cttgctgctc tccaaaggga atgggattca gtttacccga aagagttcct 14640
gcgttacgac cctcccaagg gaaccgggtc acggaggctt gtagatgttt tccttaatga 14700
ttcgagcttt gacccatatg atgtgataat gtatgttgta agtggagctt acctccatga 14760
ccctgagttc aacctgtctt acagcctgaa agaaaaggag atcaaggaaa caggtagact 14820
ttttgctaaa atgacttaca aaatgagggc atgccaagtg attgctgaaa atctaatctc 14880
aaacgggatt ggcaaatatt ttaaggacaa tgggatggcc aaggatgagc acgatttgac 14940
taaggcactc cacactctag ctgtctcagg agtccccaaa gatctcaaag aaagtcacag 15000
ggggggccca gtcttaaaaa cctactcccg aagcccagtc cacacaagta ccaggaacgt 15060
gagagcagca aaagggttta tagggttccc tcaagtaatt cggcaggacc aagacactga 15120
tcatccggag aatatggaag cttacgagac agtcagtgca tttatcacga ctgatctcaa 15180
gaagtactgc cttaattgga gatatgagac catcagcttg tttgcacaga ggctaaatga 15240
gatttacgga ttgccctcat ttttccagtg gctgcataag aggcttgaga cctctgtcct 15300
gtatgtaagt gaccctcatt gcccccccga ccttgacgcc catatcccgt tatataaagt 15360
ccccaatgat caaatcttca ttaagtaccc tatgggaggt atagaagggt attgtcagaa 15420
gctgtggacc atcagcacca ttccctatct atacctggct gcttatgaga gcggagtaag 15480
gattgcttcg ttagtgcaag ggacaatca gaccatagcc gtaacaaaaa gggtacccag 15540

cacatggccc tacaacctta agaaacggga agctgctaga gtaactagag attactttgt 15600
aattcttagg caaaggctac atgatattgg ccatcacctc aaggcaaatg agacaattgt 15660
ttcatcacat ttttttgtct attcaaaagg aatatattat gatgggctac ttgtgtccca 15720
atcactcaag agcatcgcaa gatgtgtatt ctggtcagag actatagttg atgaaacaag 15780
ggcagcatgc agtaatattg ctacaacaat ggctaaaagc atcgagagag gttatgaccg 15840
ttaccttgca tattccctga acgtcctaaa agtgatacag caaattctga tctctcttgg 15900
cttcacaatc aattcaacca tgacccggga tgtagtcata cccctcctca caaacaacga 15960
cctcttaata aggatggcac tgttgcccgc tcctattggg gggatgaatt atctgaatat 16020
gagcaggctg tttgtcagaa acatcggtga tccagtaaca tcatcaattg ctgatctcaa 16080
gagaatgatt ctcgcctcac taatgcctga agagaccctc catcaagtaa tgacacaaca 16140
accgggggac tcttcattcc tagactgggc tagcgaccct tactcagcaa atcttgtatg 16200
tgtccagagc atcactagac tcctcaagaa cataactgca aggtttgtcc tgatccatag 16260
tccaaaccca atgttaaaag gattattcca tgatgacagt aaagaagagg acgagggact 16320
ggcggcattc ctcatggaca ggcatattat agtacctagg gcagctcatg aaatcctgga 16380
tcatagtgtc acaggggcaa gagagtctat tgcaggcatg ctggatacca caaaaggctt 16440
gattcgagcc agcatgagga aggggggtt aacctctcga gtgataacca gattgtccaa 16500
ttatgactat gaacaattca gagcagggat ggtgctattg acaggaagaa agagaaatgt 16560
cctcattgac aaagagtcat gttcagtgca gctggcgaga gctctaagaa gccatatgtg 16620
ggcgaggcta gctcgaggac ggcctattta cggccttgag gtccctgatg tactagaatc 16680
tatgcgaggc caccttattc ggcgtcatga gacatgtgtc atctgcgagt gtggatcagt 16740
caactacgga tggtttttg tcccctcggg ttgccaactg gatgatattg acaaggaaac 16800
atcatccttg agagtcccat atattggttc taccactgat gagagaacag acatgaagct 16860
tgccttcgta agagccccaa gtcgatcctt gcgatctgct gttagaatag caacagtgta 16920
ctcatgggct tacggtgatg atgatagctc ttggaacgaa gcctggttgt tggctaggca 16980
aagggccaat gtgagcctgg aggagctaag ggtgatcact cccatctcaa cttcgactaa 17040
tttagcgcat aggttgaggg atcgtagcac tcaagtgaaa tactcaggta catcccttgt 17100
ccgagtggcg aggtatacca caatctccaa cgacaatctc tcatttgtca tatcagataa 17160
gaaggttgat actaacttta tataccaaca aggaatgctt ctagggttgg gtgttttaga 17220
aacattgttt cgactcgaga aagataccgg atcatctaac acggtattac atcttcacgt 17280
cgaaacagat tgttgcgtga tcccgatgat agatcatccc aggataccca gctcccgcaa 17340
gctagagctg agggcagagc tatgtaccaa cccattgata tatgataatg cacctttaat 17400
tgacagagat gcaacaaggc tatacaccca gagccatagg aggcaccttg tggaatttgt 17460

tacatggtcc acaccccaac tatatcacat tttagctaag tccacagcac tatctatgat 17520
tgacctggta acaaaatttg agaaggacca tatgaatgaa atttcagctc tcatagggga 17580
tgacgatatc aatagtttca taactgagtt tctgctcata gagccaagat tattcactat 17640
ctacttgggc cagtgtgcgg ccatcaattg ggcatttgat gtacattatc atagaccatc 17700
agggaaatat cagatgggtg agctgttgtc atcgttcctt tctagaatga gcaaaggagt 17760
gtttaaggtg cttgtcaatg ctctaagcca cccaaagatc tacaagaaat tctggcattg 17820
tggtattata gagcctatcc atggtccttc acttgatgct caaaacttgc acacaactgt 17880
gtgcaacatg gtttacacat gctatatgac ctacctcgac ctgttgttga atgaagagtt 17940
agaagagttc acatttctct tgtgtgaaag cgacgaggat gtagtaccgg acagattcga 18000
caacatccag gcaaaacact tatgtgttct ggcagatttg tactgtcaac cagggacctg 18060
cccaccaatt cgaggtctaa gaccggtaga gaaatgtgca gttctaaccg accatatcaa 18120
ggcagaggct atgttatctc cagcaggatc ttcgtggaac ataaatccaa ttattgtaga 18180
ccattactca tgctctctga cttatctccg gcgaggatcg atcaaacaga taagattgag 18240
agttgatcca ggattcattt tcgacgccct cgctgaggta aatgtcagtc agccaaagat 18300
cggcagcaac aacatctcaa atatgagcat caaggctttc agacccccac acgatgatgt 18360
tgcaaaattg ctcaaagata tcaacacaag caagcacaat cttcccattt caggggcaa 18420
tctcgccaat tatgaaatcc atgctttccg cagaatcggg ttgaactcat ctgcttgcta 18480
caaagctgtt gagatatcaa cattaattag gagatgcctt gagccagggg aggacggctt 18540
gttcttgggt gagggatcgg gttctatgtt gatcacttat aaagagatac ttaaactaaa 18600
caagtgcttc tataatagtg ggtttccgc caattctaga tctggtcaaa gggaattagc 18660
accctatccc tccgaagttg gccttgtcga acacagaatg ggagtaggta atattgtcaa 18720
agtgctcttt aacgggaggc ccgaagtcac gtgggtaggc agtgtagatt gcttcaattt 18780
catagttagt aatatcccta cctctagtgt ggggtttatc cattcagata tagagacctt 18840
gcctgacaaa gatactatag agaagctaga ggaattggca gccatcttat cgatggctct 18900
gctcctgggc aaaataggat caatactggt gattaagctt atgcctttca gcggggattt 18960
tgttcaggga tttataagtt atgtagggtc tcattataga gaagtgaacc ttgtataccc 19020
tagatacagc aacttcatct ctactgaatc ttatttggtt atgacagatc tcaaggctaa 19080
ccggctaatg aatcctgaaa agattaagca gcagataatt gaatcatctg tgaggacttc 19140
acctggactt ataggtcaca tcctatccat taagcaacta agctgcatac aagcaattgt 19200
gggagacgca gttagtagag gtgatatcaa tcctactctg aaaaaactta cacctataga 19260
gcaggtgctg atcaattgcg ggttggcaat taacggacct aagctgtgca aagaattgat 19320

ccaccatgat gttgcctcag ggcaagatgg attgcttaat tctatactca tcctctacag 19380

ggagttggca agattcaaag acaaccaaag aagtcaacaa gggatgttcc acgcttaccc 19440

cgtattggta agtagcaggc aacgagaact tatatctagg atcacccgca aattctgggg 19500

gcacattctt ctttactccg ggaacaaaaa gttgataaat aagtttatcc agaatctcaa 19560

gtccggctat ctgatactag acttacacca gaatatcttc gttaagaatc tatccaagtc 19620

agagaaacag attattatga cggggggttt gaaacgtgag tgggttttta aggtaacagt 19680

caaggagacc aaagaatggt ataagttagt cggatacagt gccctgatta aggactaatt 19740

ggttgaactc cggaacccta atcctgccct aggtggttag gcattatttg caatatatta 19800

aagaaaactt tgaaaatacg aagtttctat tcccagcttt gtctggtggc cggcatggtc 19860

ccagcctcct cgctggcgcc ggctgggcaa cattccgagg ggaccgtccc ctcggtaatg 19920

gcgaatggga cgcggccgat ccggctgcta acaaagcccg aaaggaagct gagttggctg 19980

ctgccaccgc tgagcaataa ctagcataac cccttggggc ctctaaacgg gtcttgaggg 20040

gttttttgct gaaaggagga actatatccg gatgcggccg cgggccctat ggtacccagc 20100

ttttgttccc tttagtgagg gttaattccg agcttggcgt aatcatggtc atagctgttt 20160

cctgtgtgaa attgttatcc gctcacaatt ccacacaaca taggagccgg aagcataaag 20220

tgtaaagcct ggggtgccta atgagtgagg taactcacat taattgcgtt gcgctcactg 20280

cccgctttcc agtcgggaaa cctgtcgtgc cagctgcatt aatgaatcgg ccaacgcgcg 20340

gggagaggcg gtttgcgtat tgggcgctct tccgcttcct cgctcactga ctcgctgcgc 20400

tcggtcgttc ggctgcggcg agcggtatca gctcactcaa aggcggtaat acggttatcc 20460

acagaatcag gggataacgc aggaaagaac atgtgagcaa aaggccagca aaaggccagg 20520

aaccgtaaaa aggccgcgtt gctggcgttt ttccataggc tcggcccccc tgacgagcat 20580

cacaaaaatc gacgctcaag tcagaggtgg cgaaacccga caggactata aagataccag 20640

gcgttccccc ctggaagctc cctcgtgcgc tctcctgttc cgaccctgcc gcttaccgga 20700

tacctgtccg cctttctccc ttcgggaagc gtggcgcttt ctcaatgctc acgctgtagg 20760

tatctcagtt cggtgtaggt cgttcgctcc aagctgggct gtgtgcacga accccccgtt 20820

cagcccgacc gctgcgcctt atccggtaac tatcgtcttg agtccaaccc ggtaagacac 20880

gacttatcgc cactggcagc agccactggt aacaggatta gcagagcgag gtatgtaggc 20940

ggtgctacag agttcttgaa gtggtggcct aactacggct acactagaag gacagtattt 21000

ggtatctgcg ctctgctgaa gccagttacc ttcggaaaaa gagttggtag ctcttgatcc 21060

ggcaaacaaa ccaccgctgg tagcggtggt ttttttgttt gcaagcagca gattacgcgc 21120

agaaaaaaag gatctcaaga agatcctttg atcttttcta cggggtctga cgctcagtgg 21180

aacgaaaact cacgttaagg gattttggtc atgagattat caaaaaggat cttcacctag 21240

atccttttaa attaaaaatg aagttttaaa tcaatctaaa gtatatatga gtaaacttgg 21300

tctgacagtt accaatgctt aatcagtgag gcacctatct cagcgatctg tctatttcgt 21360

tcatccatag ttgcctgact ccccgtcgtg tagataacta cgatacggga gggcttacca 21420

tctggcccca gtgctgcaat gataccgcga gacccacgct caccggctcc agatttatca 21480

gcaataaacc agccagccgg aagggccgag cgcagaagtg gtcctgcaac tttatccgcc 21540

tccatccagt ctattaattg ttgccgggaa gctagagtaa gtagttcgcc agttaatagt 21600

ttgcgcaacg ttgttgccat tgctacaggc atcgtggtgt cacgctcgtc gtttggtatg 21660

gcttcattca gctccggttc ccaacgatca aggcgagtta catgatcccc catgttgtga 21720

aaaaaagcgg ttagctcctt cggtcctccg atcgttgtca gaagtaagtt ggccgcagtg 21780

ttatcactca tgcttatggc agcactgcat aattctctta ctgtcatgcc atccgtaaga 21840

tgcttttctg tgactggtga gtactcaacc aagtcattct gagaatagtg tatgcggcga 21900

ccgagttgct cttgcccggc gtcaatacgg gataataccg cgccacatag cagaacttta 21960

aaagtgctca tcattggaaa acgttcttcg gggcgaaaac tctcaaggat cttaccgctg 22020

ttgagatcca gttcgatgta acccactcgt gcacccaact gatcttcagc atcttttact 22080

ttcaccagcg tttctgggtg agcaaaaaca ggaaggcaaa atgccgcaaa aaagggaata 22140

agggcgacac ggaaatgttg aatactcata ctcttccttt ttcaatatta ttgaagcatt 22200

tatcagggtt attgtctcat gagcggatac atatttgaat gtatttagaa aaataaacaa 22260

ataggggttc cgcgcacatt tccccgaaaa gtgccacctg aaattgtaaa cgttaatatt 22320

ttgttaaaat tcgcgttaaa tttttgttaa atcagctcat tttttaacca ataggccgaa 22380

atcggcaaaa tcccttataa atcaaaagaa tagaccgaga tagggttgag tgttgttcca 22440

gtttggaaca agagtccact attaaagaac gtggactcca acgtcaaagg gcgaaaaacc 22500

gtctatcagg gcgatggccc actacgtgaa ccatcaccct aatcaagttt tttggggtcg 22560

aggtgccgta aagcactaaa tcggaaccct aaagggagcc cccgatttag agcttgacgg 22620

ggaaagccgg cgaacgtggc gagaaaggaa gggaagaaag cgaaaggagc gggcgctagg 22680

gcgctggcaa gtgtagcggt cacgctgcgc gtaaccacca cacccgccgc gcttaatgcg 22740

ccgctacagg gcgcgtccca ttcgccattc aggctgcgca actgttggga agggcgatcg 22800

gtgcgggcct cttcgctatt acgccagcca ccgcggtgat ccttataaag ctagatg 22857

<210> 6
<211> 20661
<212> DNA
<213> Artificial Sequence

<220>
<223> pTM 2ATU MV DVAX1 long

<400> 6

```
tccaaccgcg cgcgcggccg ctaatacgac tcactatagg gccaactttg tttggtctga      60
tgagtccgtg aggacgaaac ccggagtccc gggtcaccaa acaaagttgg gtaaggatag     120
ttcaatcaat gatcatcttc tagtgcactt aggattcaag atcctattat cagggacaag     180
agcaggatta gggatatccg agatggccac acttttaagg agcttagcat tgttcaaaag     240
aaacaaggac aaaccaccca ttacatcagg atccggtgga gccatcagag gaatcaaaca     300
cattattata gtaccaatcc ctggagattc ctcaattacc actcgatcca gacttctgga     360
ccggttggtg aggttaattg gaaacccgga tgtgagcggg cccaaactaa caggggcact     420
aataggtata ttatccttat ttgtggagtc tccaggtcaa ttgattcaga ggatcaccga     480
tgaccctgac gttagcataa ggctgttaga ggttgtccag agtgaccagt cacaatctgg     540
ccttaccttc gcatcaagag gtaccaacat ggaggatgag gcggaccaat acttttcaca     600
tgatgatcca attagtagtg atcaatccag gttcggatgg ttcgggaaca aggaaatctc     660
agatattgaa gtgcaagacc ctgagggatt caacatgatt ctgggtacca tcctagccca     720
aatttgggtc ttgctcgcaa aggcggttac ggccccagac acggcagctg attcggagct     780
aagaaggtgg ataaagtaca cccaacaaag aagggtagtt ggtgaattta gattggagag     840
aaaatggttg gatgtggtga ggaacaggat tgccgaggac ctctccttac gccgattcat     900
ggtcgctcta atcctggata tcaagagaac acccggaaac aaacccagga ttgctgaaat     960
gatatgtgac attgatacat atatcgtaga ggcaggatta gccagtttta tcctgactat    1020
taagtttggg atagaaacta tgtatcctgc tcttggactg catgaatttg ctggtgagtt    1080
atccacactt gagtccttga tgaaccttta ccagcaaatg gggaaactg caccctacat     1140
ggtaatcctg gagaactcaa ttcagaacaa gttcagtgca ggatcatacc ctctgctctg    1200
gagctatgcc atgggagtag gagtggaact tgaaaactcc atgggaggtt tgaactttgg    1260
ccgatcttac tttgatccag catattttag attagggcaa gagatggtaa ggaggtcagc    1320
tggaaaggtc agttccacat tggcatctga actcggtatc actgccgagg atgcaaggct    1380
tgtttcagag attgcaatgc atactactga ggacaagatc agtagagcgg ttggacccag    1440
acaagcccaa gtatcatttc tacacggtga tcaaagtgag aatgagctac cgagattggg    1500
gggcaaggaa gataggaggg tcaaacagag tcgaggagaa gccagggaga gctacagaga    1560
aaccgggccc agcagagcaa gtgatgcgag agctgcccat cttccaaccg gcacacccct    1620
agacattgac actgcaacgg agtccagcca agatccgcag gacagtcgaa ggtcagctga    1680
cgccctgctt aggctgcaag ccatggcagg aatctcggaa gaacaaggct cagacacgga    1740
cacccctata gtgtacaatg acagaaatct tctagactag gtgcgagagg ccgagggcca    1800
gaacaacatc cgcctaccat ccatcattgt tataaaaaac ttaggaacca ggtccacaca    1860
```

gccgccagcc catcaaccat ccactcccac gattggagcc aatggcagaa gagcaggcac 1920
gccatgtcaa aaacggactg gaatgcatcc gggctctcaa ggccgagccc atcggctcac 1980
tggccatcga ggaagctatg gcagcatggt cagaaatatc agacaaccca ggacaggagc 2040
gagccacctg cagggaagag aaggcaggca gttcgggtct cagcaaacca tgcctctcag 2100
caattggatc aactgaaggc ggtgcacctc gcatccgcgg tcagggacct ggagagagcg 2160
atgacgacgc tgaaactttg ggaatccccc caagaaatct ccaggcatca agcactgggt 2220
tacagtgtta ttacgtttat gatcacagcg gtgaagcggt taagggaatc caagatgctg 2280
actctatcat ggttcaatca ggccttgatg gtgatagcac cctctcagga ggagacaatg 2340
aatctgaaaa cagcgatgtg gatattggcg aacctgatac cgagggatat gctatcactg 2400
accggggatc tgctcccatc tctatggggt tcagggcttc tgatgttgaa actgcagaag 2460
gaggggagat ccacgagctc ctgagactcc aatccagagg caacaacttt ccgaagcttg 2520
ggaaaactct caatgttcct ccgcccccgg accccggtag ggccagcact tccgggacac 2580
ccattaaaaa gggcacagac gcgagattag cctcatttgg aacggagatc gcgtctttat 2640
tgacaggtgg tgcaacccaa tgtgctcgaa agtcaccctc ggaaccatca gggccaggtg 2700
cacctgcggg gaatgtcccc gagtgtgtga gcaatgccgc actgatacag gagtggacac 2760
ccgaatctgg taccacaatc tccccgagat cccagaataa tgaagaaggg ggagactatt 2820
atgatgatga gctgttctct gatgtccaag atattaaaac agccttggcc aaaatacacg 2880
aggataatca gaagataatc tccaagctag aatcactgct gttattgaag ggagaagttg 2940
agtcaattaa gaagcagatc aacaggcaaa atatcagcat atccaccctg gaaggacacc 3000
tctcaagcat catgatcgcc attcctggac ttgggaagga tcccaacgac cccactgcag 3060
atgtcgaaat caatcccgac ttgaaaccca tcataggcag agattcaggc cgagcactgg 3120
ccgaagttct caagaaaccc gttgccagcc gacaactcca aggaatgaca aatggacgga 3180
ccagttccag aggacagctg ctgaaggaat ttcagctaaa gccgatcggg aaaaagatga 3240
gctcagccgt cgggtttgtt cctgacaccg gccctgcatc acgcagtgta atccgctcca 3300
ttataaaatc cagccggcta gaggaggatc ggaagcgtta cctgatgact ctccttgatg 3360
atatcaaagg agccaatgat cttgccaagt tccaccagat gctgatgaag ataataatga 3420
agtagctaca gctcaactta cctgccaacc ccatgccagt cgacccaact agcctaccct 3480
ccatcattgt tataaaaaac ttaggaacca ggtccacaca gccgccagcc catcaacgcg 3540
tacgatgggc atcatattca ttcttcttat gctggttaca ccgtctatgg cggagaaact 3600
tcgaatcaaa ggtatgagct atacgatgtg cagcggcaag ttcaagatcg agaaggaaat 3660
ggctgaaacc cagcacggta caactgtggt caaagtcaaa tatgaggggg ctggcgctcc 3720

ctgtaaagta cccattgaga ttagggacgt caataaagag aaggtggtag gtcgcatcat 3780
ctccagtaca cctttggccg agaacacgaa ctccgtcaca aacatagagt tggaaccccc 3840
gttcggagac tcatacattg tgatcggggt gggcaactct gcactcacac tgcattggtt 3900
caagaaggga agcagtatcg gccgcaggga taagagagac aaactgaaat tgaaaggtat 3960
gtcctatgcc atgtgcacga atactttcgt tctcaagaaa gaagtatctg agactcagca 4020
cggaaccatc ctgatcaaag tcgagtacaa aggagaagac gtgccctgta agatcccatt 4080
cagtaccgag gatggacagg gcaaggccca taacggcagg ctgataaccg ccaaccctgt 4140
ggttacaaag aaggaagagc cagtcaatat cgaagctgag ccaccgttcg gggagagcaa 4200
catagtaatt ggcatagggg ataatgcttt gaagatcaac tggtacaaga aaggaagctc 4260
cattggccga agagataagc gcgacaaact ccagctgaaa ggaatgagct actccatgtg 4320
tactgggaag ttcaagattg tcaaggaaat cgccgaaact cagcatggca ctattgtgat 4380
ccgcgtgcag tatgaaggcg atggtagccc ctgcaagata ccatttgaaa tcaccgattt 4440
ggagaaacgg cacgtcctgg gtcggctcat taccgtgaac ccaatcgtga ccgagaagga 4500
cagtccagtt aatatcgagg ccgagcctcc tttcggcgac agttacatca ttgtaggggt 4560
ggaaccaggg caactgaagc tgaactggtt caagaaaggc agcagtatag gacggcggga 4620
taaacgggac aaactcacac tgaaaggcat gtcatacgtt atgtgcaccg gctcattcaa 4680
actggagaag gaagttgcag agacacagca tgggaccgtg ctcgtgcagg tcaaatacga 4740
gggcaccgac gctccttgca agattccgtt cagtacacag gacgagaaag gcgtgactca 4800
gaacggcaga ttgattacag cgaaccctat cgtgactgac aaggagaagc cagttaacat 4860
cgagactgag ccgcctttcg gagaatcata cattatcgtg ggagccggcg agaaggcact 4920
gaaactcagc tggttcaaga agggcagctc aatcggtcgg agagacaagc ggtctgtcgc 4980
cctcgcaccg cacgtgggcc tgggtctgga aacgaggacc gagacgtgga tgagttccga 5040
aggcgcatgg aagcaaatcc agaaagtgga gacgtgggcc ctcaggcatc cgtaatgagc 5100
gcgcagcgct tagacgtctc gcgatcgata ctagtacaac ctaaatccat tataaaaaac 5160
ttaggagcaa agtgattgcc tcccaaggtc cacaatgaca gagacctacg acttcgacaa 5220
gtcggcatgg gacatcaaag ggtcgatcgc tccgatacaa cccaccacct acagtgatgg 5280
caggctggtg ccccaggtca gagtcataga tcctggtcta ggcgacagga aggatgaatg 5340
ctttatgtac atgtttctgc tgggggttgt tgaggacagc gattccctag ggcctccaat 5400
cgggcgagca tttgggttcc tgcccttagg tgttggcaga tccacagcaa agcccgaaaa 5460
actcctcaaa gaggccactg agcttgacat agttgttaga cgtacagcag ggctcaatga 5520
aaaactggtg ttctacaaca acaccccact aactctcctc acaccttgga gaaaggtcct 5580
aacaacaggg agtgtcttca acgcaaacca agtgtgcaat gcggttaatc tgataccgct 5640

cgataccccg cagaggttcc gtgttgttta tatgagcatc acccgtcttt cggataacgg 5700
gtattacacc gttcctagaa gaatgctgga attcagatcg gtcaatgcag tggccttcaa 5760
cctgctggtg acccttagga ttgacaaggc gataggccct gggaagatca tcgacaatac 5820
agagcaactt cctgaggcaa catttatggt ccacatcggg aacttcagga gaaagaagag 5880
tgaagtctac tctgccgatt attgcaaaat gaaaatcgaa aagatgggcc tggtttttgc 5940
acttggtggg ataggggca ccagtcttca cattagaagc acaggcaaaa tgagcaagac 6000
tctccatgca caactcgggt tcaagaagac cttatgttac ccgctgatgg atatcaatga 6060
agaccttaat cgattactct ggaggagcag atgcaagata gtaagaatcc aggcagtttt 6120
gcagccatca gttcctcaag aattccgcat ttacgacgac gtgatcataa atgatgacca 6180
aggactattc aaagttctgt agaccgtagt gcccagcaat gcccgaaaac gacccccctc 6240
acaatgacag ccagaaggcc cggacaaaaa agccccctcc gaaagactcc acggaccaag 6300
cgagaggcca gccagcagcc gacggcaagc gcgaacacca ggcggcccca gcacagaaca 6360
gccctgacac aaggccacca ccagccaccc caatctgcat cctcctcgtg ggacccccga 6420
ggaccaaccc ccaaggctgc ccccgatcca aaccaccaac cgcatcccca ccacccccgg 6480
gaaagaaacc cccagcaatt ggaaggcccc tccccctctt cctcaacaca agaactccac 6540
aaccgaaccg cacaagcgac cgaggtgacc caaccgcagg catccgactc cctagacaga 6600
tcctctctcc ccggcaaact aaacaaaact tagggccaag gaacatacac acccaacaga 6660
acccagaccc cggcccacgg cgccgcgccc ccaacccccg acaaccagag ggagccccca 6720
accaatcccg ccggctcccc cggtgcccac aggcagggac accaaccccc gaacagaccc 6780
agcacccaac catcgacaat ccaagacggg ggggcccccc caaaaaaagg cccccagggg 6840
ccgacagcca gcaccgcgag gaagcccacc caccccacac acgaccacgg caaccaaacc 6900
agaacccaga ccaccctggg ccaccagctc ccagactcgg ccatcacccc gcagaaagga 6960
aaggccacaa cccgcgcacc ccagccccga tccggcgggg agccacccaa cccgaaccag 7020
cacccaagag cgatccccga aggacccccg aaccgcaaag gacatcagta tcccacagcc 7080
tctccaagtc ccccggtctc ctcctcttct cgaagggacc aaaagatcaa tccaccacac 7140
ccgacgacac tcaactcccc accccctaaag gagacaccgg gaatcccaga atcaagactc 7200
atccaatgtc catcatgggt ctcaaggtga acgtctctgc catattcatg gcagtactgt 7260
taactctcca aacacccacc ggtcaaatcc attggggcaa tctctctaag atagggggtgg 7320
taggaatagg aagtgcaagc tacaaagtta tgactcgttc cagccatcaa tcattagtca 7380
taaaattaat gcccaatata actctcctca ataactgcac gagggtagag attgcagaat 7440
acaggagact actgagaaca gttttggaac caattagaga tgcacttaat gcaatgaccc 7500

agaatataag accggttcag agtgtagctt caagtaggag acacaagaga tttgcgggag 7560
tagtcctggc aggtgcggcc ctaggcgttg ccacagctgc tcagataaca gccggcattg 7620
cacttcacca gtccatgctg aactctcaag ccatcgacaa tctgagagcg agcctggaaa 7680
ctactaatca ggcaattgag acaatcagac aagcagggca ggagatgata ttggctgttc 7740
agggtgtcca agactacatc aataatgagc tgataccgtc tatgaaccaa ctatcttgtg 7800
atttaatcgg ccagaagctc gggctcaaat tgctcagata ctatacagaa atcctgtcat 7860
tatttggccc cagtttacgg gaccccatat ctgcggagat atctatccag gctttgagct 7920
atgcgcttgg aggagacatc aataaggtgt tagaaaagct cggatacagt ggaggtgatt 7980
tactgggcat cttagagagc ggaggaataa aggcccggat aactcacgtc gacacagagt 8040
cctacttcat tgtcctcagt atagcctatc cgacgctgtc cgagattaag ggggtgattg 8100
tccaccggct agaggggtc tcgtacaaca taggctctca agagtggtat accactgtgc 8160
ccaagtatgt tgcaacccaa gggtacctta tctcgaattt tgatgagtca tcgtgtactt 8220
tcatgccaga ggggactgtg tgcagccaaa atgccttgta cccgatgagt cctctgctcc 8280
aagaatgcct ccgggggtac accaagtcct gtgctcgtac actcgtatcc gggtcttttg 8340
ggaaccggtt cattttatca caagggaacc taatagccaa ttgtgcatca atcctttgca 8400
agtgttacac aacaggaacg atcattaatc aagaccctga caagatccta acatacattg 8460
ctgccgatca ctgcccggta gtcgaggtga acggcgtgac catccaagtc gggagcagga 8520
ggtatccaga cgctgtgtac ttgcacagaa ttgacctcgg tcctcccata tcattggaga 8580
ggttggacgt agggacaaat ctggggaatg caattgctaa gttggaggat gccaaggaat 8640
tgttggagtc atcggaccag atattgagga gtatgaaagg tttatcgagc actagcatag 8700
tctacatcct gattgcagtg tgtcttggag ggttgatagg gatccccgct ttaatatgtt 8760
gctgcagggg gcgttgtaac aaaaagggag aacaagttgg tatgtcaaga ccaggcctaa 8820
agcctgatct tacgggaaca tcaaaatcct atgtaaggtc gctctgatcc tctacaactc 8880
ttgaaacaca aatgtcccac aagtctcctc ttcgtcatca agcaaccacc gcacccagca 8940
tcaagcccac ctgaaattat ctccggcttc cctctggccg aacaatatcg gtagttaatc 9000
aaaacttagg gtgcaagatc atccacaatg tcaccacaac gagaccggat aaatgccttc 9060
tacaaagata acccccatcc caagggaagt aggatagtca ttaacagaga acatcttatg 9120
attgatagac cttatgtttt gctggctgtt ctgtttgtca tgtttctgag cttgatcggg 9180
ttgctagcca ttgcaggcat tagacttcat cgggcagcca tctacaccgc agagatccat 9240
aaaagcctca gcaccaatct agatgtaact aactcaatcg agcatcaggt caaggacgtg 9300
ctgacaccac tcttcaaaat catcggtgat gaagtgggcc tgaggacacc tcagagattc 9360
actgacctag tgaaattaat ctctgacaag attaaattcc ttaatccgga tagggagtac 9420

gacttcagag atctcacttg gtgtatcaac ccgccagaga gaatcaaatt ggattatgat 9480

caatactgtg cagatgtggc tgctgaagag ctcatgaatg cattggtgaa ctcaactcta 9540

ctggagacca gaacaaccaa tcagttccta gctgtctcaa agggaaactg ctcagggccc 9600

actacaatca gaggtcaatt ctcaaacatg tcgctgtccc tgttagactt gtatttaggt 9660

cgaggttaca atgtgtcatc tatagtcact atgacatccc agggaatgta tgggggaact 9720

tacctagtgg aaaagcctaa tctgagcagc aaaaggtcag agttgtcaca actgagcatg 9780

taccgagtgt ttgaagtagg tgttatcaga aatccgggtt tgggggctcc ggtgttccat 9840

atgacaaact atcttgagca accagtcagt aatgatctca gcaactgtat ggtggctttg 9900

ggggagctca aactcgcagc cctttgtcac ggggaagatt ctatcacaat tccctatcag 9960

ggatcaggga aaggtgtcag cttccagctc gtcaagctag gtgtctggaa atccccaacc 10020

gacatgcaat cctgggtccc cttatcaacg gatgatccag tgatagacag gctttacctc 10080

tcatctcaca gaggtgttat cgctgacaat caagcaaaat gggctgtccc gacaacacga 10140

acagatgaca agttgcgaat ggagacatgc ttccaacagg cgtgtaaggg taaaatccaa 10200

gcactctgcg agaatcccga gtgggcacca ttgaaggata acaggattcc ttcatacggg 10260

gtcttgtctg ttgatctgag tctgacagtt gagcttaaaa tcaaaattgc ttcgggattc 10320

gggccattga tcacacacgg ttcagggatg gacctataca aatccaacca caacaatgtg 10380

tattggctga ctatcccgcc aatgaagaac ctagccttag gtgtaatcaa cacattggag 10440

tggataccga gattcaaggt tagtccctac ctcttcactg tcccaattaa ggaagcaggc 10500

gaagactgcc atgccccaac atacctacct gcggaggtgg atggtgatgt caaactcagt 10560

tccaatctgg tgattctacc tggtcaagat ctccaatatg ttttggcaac ctacgatact 10620

tccagggttg aacatgctgt ggtttattac gtttacagcc caagccgctc attttcttac 10680

ttttatcctt ttaggttgcc tataaagggg gtccccatcg aattacaagt ggaatgcttc 10740

acatgggacc aaaaactctg gtgccgtcac ttctgtgtgc ttgcggactc agaatctggt 10800

ggacatatca ctcactctgg gatggtgggc atgggagtca gctgcacagt cacccgggaa 10860

gatggaacca atcgcagata gggctgctag tgaaccaatc acatgatgtc acccagacat 10920

caggcatacc cactagtgtg aaatagacat cagaattaag aaaaacgtag ggtccaagtg 10980

gttccccgtt atggactcgc tatctgtcaa ccagatctta taccctgaag ttcacctaga 11040

tagcccgata gttaccaata agatagtagc catcctggag tatgctcgag tccctcacgc 11100

ttacagcctg gaggacccta cactgtgtca gaacatcaag caccgcctaa aaaacggatt 11160

ttccaaccaa atgattataa acaatgtgga agttgggaat gtcatcaagt ccaagcttag 11220

gagttatccg gcccactctc atattccata tccaaattgt aatcaggatt tatttaacat 11280

agaagacaaa gagtcaacga ggaagatccg tgaactcctc aaaaagggga attcgctgta 11340
ctccaaagtc agtgataagg ttttccaatg cttaagggac actaactcac ggcttggcct 11400
aggctccgaa ttgagggagg acatcaagga gaaagttatt aacttgggag tttacatgca 11460
cagctcccag tggtttgagc cctttctgtt ttggtttaca gtcaagactg agatgaggtc 11520
agtgattaaa tcacaaaccc atacttgcca taggaggaga cacacacctg tattcttcac 11580
tggtagttca gttgagttgc taatctctcg tgaccttgtt gctataatca gtaaagagtc 11640
tcaacatgta tattacctga catttgaact ggttttgatg tattgtgatg tcatagaggg 11700
gaggttaatg acagagaccg ctatgactat tgatgctagg tatacagagc ttctaggaag 11760
agtcagatac atgtggaaac tgatagatgg tttcttccct gcactcggga atccaactta 11820
tcaaattgta gccatgctgg agcctctttc acttgcttac ctgcagctga gggatataac 11880
agtagaactc agaggtgctt tccttaacca ctgctttact gaaatacatg atgttcttga 11940
ccaaaacggg ttttctgatg aaggtactta tcatgagtta actgaagctc tagattacat 12000
tttcataact gatgacatac atctgacagg ggagattttc tcatttttca gaagtttcgg 12060
ccaccccaga cttgaagcag taacggctgc tgaaaatgtt aggaaataca tgaatcagcc 12120
taaagtcatt gtgtatgaga ctctgatgaa aggtcatgcc atattttgtg gaatcataat 12180
caacggctat cgtgacaggc acggaggcag ttggccaccg ctgaccctcc ccctgcatgc 12240
tgcagacaca atccggaatg ctcaagcttc aggtgaaggg ttaacacatg agcagtgcgt 12300
tgataactgg aaatcttttg ctggagtgaa atttggctgc tttatgcctc ttagcctgga 12360
tagtgatctg acaatgtacc taaaggacaa ggcacttgct gctctccaaa gggaatggga 12420
ttcagtttac ccgaaagagt tcctgcgtta cgaccctccc aagggaaccg ggtcacggag 12480
gcttgtagat gttttcctta atgattcgag ctttgaccca tatgatgtga taatgtatgt 12540
tgtaagtgga gcttacctcc atgaccctga gttcaacctg tcttacagcc tgaaagaaaa 12600
ggagatcaag gaaacaggta gactttttgc taaaatgact tacaaaatga gggcatgcca 12660
agtgattgct gaaaatctaa tctcaaacgg gattggcaaa tattttaagg acaatgggat 12720
ggccaaggat gagcacgatt tgactaaggc actccacact ctagctgtct caggagtccc 12780
caaagatctc aaagaaagtc acaggggggg gccagtctta aaaacctact cccgaagccc 12840
agtccacaca agtaccagga acgtgagagc agcaaaaggg tttatagggt tccctcaagt 12900
aattcggcag gaccaagaca ctgatcatcc ggagaatatg gaagcttacg agacagtcag 12960
tgcatttatc acgactgatc tcaagaagta ctgccttaat tggagatatg agaccatcag 13020
cttgtttgca cagaggctaa atgagattta cggattgccc tcatttttcc agtggctgca 13080
taagaggctt gagacctctg tcctgtatgt aagtgaccct cattgccccc ccgaccttga 13140
cgcccatatc ccgttatata aagtccccaa tgatcaaatc ttcattaagt accctatggg 13200

aggtatagaa gggtattgtc agaagctgtg gaccatcagc accattccct atctatacct 13260
ggctgcttat gagagcggag taaggattgc ttcgttagtg caaggggaca atcagaccat 13320
agccgtaaca aaaagggtac ccagcacatg gccctacaac cttaagaaac gggaagctgc 13380
tagagtaact agagattact ttgtaattct taggcaaagg ctacatgata ttggccatca 13440
cctcaaggca aatgagacaa ttgtttcatc acattttttt gtctattcaa aaggaatata 13500
ttatgatggg ctacttgtgt cccaatcact caagagcatc gcaagatgtg tattctggtc 13560
agagactata gttgatgaaa caagggcagc atgcagtaat attgctacaa caatggctaa 13620
aagcatcgag agaggttatg accgttacct tgcatattcc ctgaacgtcc taaaagtgat 13680
acagcaaatt ctgatctctc ttggcttcac aatcaattca accatgaccc gggatgtagt 13740
catacccctc ctcacaaaca acgacctctt aataaggatg gcactgttgc ccgctcctat 13800
tgggggggatg aattatctga atatgagcag gctgtttgtc agaaacatcg gtgatccagt 13860
aacatcatca attgctgatc tcaagagaat gattctcgcc tcactaatgc ctgaagagac 13920
cctccatcaa gtaatgacac aacaaccggg ggactcttca ttcctagact gggctagcga 13980
cccttactca gcaaatcttg tatgtgtcca gagcatcact agactcctca agaacataac 14040
tgcaaggttt gtcctgatcc atagtccaaa cccaatgtta aaaggattat tccatgatga 14100
cagtaaagaa gaggacgagg gactggcggc attcctcatg gacaggcata ttatagtacc 14160
tagggcagct catgaaatcc tggatcatag tgtcacaggg gcaagagagt ctattgcagg 14220
catgctggat accacaaaag gcttgattcg agccagcatg aggaaggggg ggttaacctc 14280
tcgagtgata accagattgt ccaattatga ctatgaacaa ttcagagcag ggatggtgct 14340
attgacagga agaaagagaa atgtcctcat tgacaaagag tcatgttcag tgcagctggc 14400
gagagctcta agaagccata tgtgggcgag gctagctcga ggacggccta tttacggcct 14460
tgaggtccct gatgtactag aatctatgcg aggccacctt attcggcgtc atgagacatg 14520
tgtcatctgc gagtgtggat cagtcaacta cggatggttt tttgtcccct cgggttgcca 14580
actggatgat attgacaagg aaacatcatc cttgagagtc ccatatattg gttctaccac 14640
tgatgagaga acagacatga agcttgcctt cgtaagagcc ccaagtcgat ccttgcgatc 14700
tgctgttaga atagcaacag tgtactcatg ggcttacggt gatgatgata gctcttggaa 14760
cgaagcctgg ttgttggcta ggcaaagggc caatgtgagc ctggaggagc taagggtgat 14820
cactcccatc tcaacttcga ctaatttagc gcataggttg agggatcgta gcactcaagt 14880
gaaatactca ggtacatccc ttgtccgagt ggcgaggtat accacaatct ccaacgacaa 14940
tctctcattt gtcatatcag ataagaaggt tgatactaac tttatatacc aacaaggaat 15000
gcttctaggg ttgggtgttt tagaaacatt gtttcgactc gagaaagata ccggatcatc 15060

taacacggta ttacatcttc acgtcgaaac agattgttgc gtgatcccga tgatagatca 15120
tcccaggata cccagctccc gcaagctaga gctgagggca gagctatgta ccaacccatt 15180
gatatatgat aatgcacctt taattgacag agatgcaaca aggctataca cccagagcca 15240
taggaggcac cttgtggaat ttgttacatg gtccacaccc caactatatc acattttagc 15300
taagtccaca gcactatcta tgattgacct ggtaacaaaa tttgagaagg accatatgaa 15360
tgaaatttca gctctcatag gggatgacga tatcaatagt ttcataactg agtttctgct 15420
catagagcca agattattca ctatctactt gggccagtgt gcggccatca attgggcatt 15480
tgatgtacat tatcatagac catcagggaa atatcagatg ggtgagctgt tgtcatcgtt 15540
cctttctaga atgagcaaag gagtgtttaa ggtgcttgtc aatgctctaa gccacccaaa 15600
gatctacaag aaattctggc attgtggtat tatagagcct atccatggtc cttcacttga 15660
tgctcaaaac ttgcacacaa ctgtgtgcaa catggtttac acatgctata tgacctacct 15720
cgacctgttg ttgaatgaag agttagaaga gttcacattt ctcttgtgtg aaagcgacga 15780
ggatgtagta ccggacagat tcgacaacat ccaggcaaaa cacttatgtg ttctggcaga 15840
tttgtactgt caaccaggga cctgcccacc aattcgaggt ctaagaccgg tagagaaatg 15900
tgcagttcta accgaccata tcaaggcaga ggctatgtta tctccagcag gatcttcgtg 15960
gaacataaat ccaattattg tagaccatta ctcatgctct ctgacttatc tccggcgagg 16020
atcgatcaaa cagataagat tgagagttga tccaggattc attttcgacg ccctcgctga 16080
ggtaaatgtc agtcagccaa agatcggcag caacaacatc tcaaatatga gcatcaaggc 16140
tttcagaccc ccacacgatg atgttgcaaa attgctcaaa gatatcaaca caagcaagca 16200
caatcttccc atttcagggg gcaatctcgc caattatgaa atccatgctt tccgcagaat 16260
cgggttgaac tcatctgctt gctacaaagc tgttgagata tcaacattaa ttaggagatg 16320
ccttgagcca ggggaggacg gcttgttctt gggtgaggga tcgggttcta tgttgatcac 16380
ttataaagag atacttaaac taaacaagtg cttctataat agtggggttt ccgccaattc 16440
tagatctggt caaagggaat tagcacccta tccctccgaa gttggccttg tcgaacacag 16500
aatgggagta ggtaatattg tcaaagtgct ctttaacggg aggcccgaag tcacgtgggt 16560
aggcagtgta gattgcttca atttcatagt tagtaatatc cctacctcta gtgtggggtt 16620
tatccattca gatatagaga ccttgcctga caaagatact atagagaagc tagaggaatt 16680
ggcagccatc ttatcgatgg ctctgctcct gggcaaaata ggatcaatac tggtgattaa 16740
gcttatgcct ttcagcgggg attttgttca gggatttata agttatgtag ggtctcatta 16800
tagagaagtg aaccttgtat accctagata cagcaacttc atctctactg aatcttattt 16860
ggttatgaca gatctcaagg ctaaccggct aatgaatcct gaaaagatta agcagcagat 16920
aattgaatca tctgtgagga cttcacctgg acttataggt cacatcctat ccattaagca 16980

actaagctgc atacaagcaa ttgtgggaga cgcagttagt agaggtgata tcaatcctac 17040
tctgaaaaaa cttacaccta tagagcaggt gctgatcaat tgcgggttgg caattaacgg 17100
acctaagctg tgcaaagaat tgatccacca tgatgttgcc tcagggcaag atggattgct 17160
taattctata ctcatcctct acagggagtt ggcaagattc aaagacaacc aaagaagtca 17220
acaagggatg ttccacgctt accccgtatt ggtaagtagc aggcaacgag aacttatatc 17280
taggatcacc cgcaaattct gggggcacat tcttctttac tccgggaaca aaaagttgat 17340
aaataagttt atccagaatc tcaagtccgg ctatctgata ctagacttac accagaatat 17400
cttcgttaag aatctatcca agtcagagaa acagattatt atgacggggg gtttgaaacg 17460
tgagtgggtt tttaaggtaa cagtcaagga gaccaaagaa tggtataagt tagtcggata 17520
cagtgccctg attaaggact aattggttga actccggaac cctaatcctg ccctaggtgg 17580
ttaggcatta tttgcaatat attaaagaaa actttgaaaa tacgaagttt ctattcccag 17640
ctttgtctgg tggccggcat ggtcccagcc tcctcgctgg cgccggctgg gcaacattcc 17700
gaggggaccg tcccctcggt aatggcgaat gggacgcggc cgatccggct gctaacaaag 17760
cccgaaagga agctgagttg gctgctgcca ccgctgagca ataactagca taaccccttg 17820
gggcctctaa acgggtcttg aggggttttt tgctgaaagg aggaactata tccggatgcg 17880
gccgcgggcc ctatggtacc cagcttttgt tccctttagt gagggttaat tccgagcttg 17940
gcgtaatcat ggtcatagct gtttcctgtg tgaaattgtt atccgctcac aattccacac 18000
aacataggag ccggaagcat aaagtgtaaa gcctggggtg cctaatgagt gaggtaactc 18060
acattaattg cgttgcgctc actgcccgct ttccagtcgg gaaacctgtc gtgccagctg 18120
cattaatgaa tcggccaacg cgcggggaga ggcggtttgc gtattgggcg ctcttccgct 18180
tcctcgctca ctgactcgct gcgctcggtc gttcggctgc ggcgagcggt atcagctcac 18240
tcaaaggcgg taatacggtt atccacagaa tcaggggata acgcaggaaa gaacatgtga 18300
gcaaaaggcc agcaaaaggc caggaaccgt aaaaaggccg cgttgctggc gtttttccat 18360
aggctcggcc cccctgacga gcatcacaaa aatcgacgct caagtcagag gtggcgaaac 18420
ccgacaggac tataaagata ccaggcgttc ccccctggaa gctccctcgt gcgctctcct 18480
gttccgaccc tgccgcttac cggatacctg tccgcctttc tcccttcggg aagcgtggcg 18540
ctttctcaat gctcacgctg taggtatctc agttcggtgt aggtcgttcg ctccaagctg 18600
ggctgtgtgc acgaaccccc cgttcagccc gaccgctgcg ccttatccgg taactatcgt 18660
cttgagtcca acccggtaag acacgactta tcgccactgg cagcagccac tggtaacagg 18720
attagcagag cgaggtatgt aggcggtgct acagagttct tgaagtggtg gcctaactac 18780
ggctacacta gaaggacagt atttggtatc tgcgctctgc tgaagccagt taccttcgga 18840

aaaagagttg gtagctcttg atccggcaaa caaaccaccg ctggtagcgg tggttttttt 18900

gtttgcaagc agcagattac gcgcagaaaa aaaggatctc aagaagatcc tttgatcttt 18960

tctacggggt ctgacgctca gtggaacgaa aactcacgtt aagggatttt ggtcatgaga 19020

ttatcaaaaa ggatcttcac ctagatcctt ttaaattaaa aatgaagttt taaatcaatc 19080

taaagtatat atgagtaaac ttggtctgac agttaccaat gcttaatcag tgaggcacct 19140

atctcagcga tctgtctatt tcgttcatcc atagttgcct gactgcccgt cgtgtagata 19200

actacgatac gggagggctt accatctggc cccagtgctg caatgatacc gcgagaccca 19260

cgctcaccgg ctccagattt atcagcaata aaccagccag ccggaagggc cgagcgcaga 19320

agtggtcctg caactttatc cgcctccatc cagtctatta attgttgccg ggaagctaga 19380

gtaagtagtt cgccagttaa tagtttgcgc aacgttgttg ccattgctac aggcatcgtg 19440

gtgtcacgct cgtcgtttgg tatggcttca ttcagctccg gttcccaacg atcaaggcga 19500

gttacatgat cccccatgtt gtgaaaaaaa gcggttagct ccttcggtcc tccgatcgtt 19560

gtcagaagta agttggccgc agtgttatca ctcatgctta tggcagcact gcataattct 19620

cttactgtca tgccatccgt aagatgcttt tctgtgactg gtgagtactc aaccaagtca 19680

ttctgagaat agtgtatgcg gcgaccgagt tgctcttgcc cggcgtcaat acgggataat 19740

accgcgccac atagcagaac tttaaaagtg ctcatcattg gaaaacgttc ttcggggcga 19800

aaactctcaa ggatcttacc gctgttgaga tccagttcga tgtaacccac tcgtgcaccc 19860

aactgatctt cagcatcttt tactttcacc agcgtttctg ggtgagcaaa aacaggaagg 19920

caaaatgccg caaaaaaggg aataagggcg acacggaaat gttgaatact catactcttc 19980

ctttttcaat attattgaag catttatcag ggttattgtc tcatgagcgg atacatattt 20040

gaatgtattt agaaaaataa acaaataggg gttccgcgca catttccccg aaaagtgcca 20100

cctgaaattg taaacgttaa tattttgtta aaattcgcgt taaatttttg ttaaatcagc 20160

tcatttttta accaataggc cgaaatcggc aaaatccctt ataaatcaaa agaatagacc 20220

gagatagggt tgagtgttgt tccagtttgg aacaagagtc cactattaaa gaacgtggac 20280

tccaacgtca aagggcgaaa aaccgtctat cagggcgatg gcccactacg tgaaccatca 20340

ccctaatcaa gtttttttggg gtcgaggtgc cgtaaagcac taaatcggaa ccctaaaggg 20400

agcccccgat ttagagcttg acggggaaag ccggcgaacg tggcgagaaa ggaagggaag 20460

aaagcgaaag gagcgggcgc tagggcgctg gcaagtgtag cggtcacgct gcgcgtaacc 20520

accacacccg ccgcgcttaa tgcgccgcta cagggcgcgt cccattcgcc attcaggctg 20580

cgcaactgtt gggaagggcg atcggtgcgg gcctcttcgc tattacgcca gccaccgcgg 20640

tgatccttat aaagctagat g 20661

<210> 7
<211> 20896
<212> DNA
<213> Artificial Sequence

<220>
<223> MV Zika sE

<400> 7
tcatgaacgc ggccgctaat acgactcact atagggccaa ctttgtttgg tctgatgagt 60

ccgtgaggac gaaacccgga gtcccgggtc accaaacaaa gttgggtaag gatagttcaa 120

tcaatgatca tcttctagtg cacttaggat tcaagatcct attatcaggg acaagagcag 180

gattagggat atccgagatg gccacacttt taaggagctt agcattgttc aaaagaaaca 240

aggacaaacc acccattaca tcaggatccg gtggagccat cagaggaatc aaacacatta 300

ttatagtacc aatccctgga gattcctcaa ttaccactcg atccagactt ctggaccggt 360

tggtgaggtt aattggaaac ccggatgtga gcgggcccaa actaacaggg gcactaatag 420

gtatattatc cttatttgtg gagtctccag gtcaattgat tcagaggatc accgatgacc 480

ctgacgttag cataaggctg ttagaggttg tccagagtga ccagtcacaa tctggcctta 540

ccttcgcatc aagaggtacc aacatggagg atgaggcgga ccaatacttt tcacatgatg 600

atccaattag tagtgatcaa tccaggttcg gatggttcgg gaacaaggaa atctcagata 660

ttgaagtgca agaccctgag ggattcaaca tgattctggg taccatccta gcccaaattt 720

gggtcttgct cgcaaaggcg gttacggccc cagacacggc agctgattcg gagctaagaa 780

ggtggataaa gtacacccaa caaagaaggg tagttggtga atttagattg gagagaaaat 840

ggttggatgt ggtgaggaac aggattgccg aggacctctc cttacgccga ttcatggtcg 900

ctctaatcct ggatatcaag agaacacccg gaaacaaacc caggattgct gaaatgatat 960

gtgacattga tacatatatc gtagaggcag gattagccag ttttatcctg actattaagt 1020

ttgggataga aactatgtat cctgctcttg gactgcatga atttgctggt gagttatcca 1080

cacttgagtc cttgatgaac ctttaccagc aaatggggga aactgcaccc tacatggtaa 1140

tcctggagaa ctcaattcag aacaagttca gtgcaggatc ataccctctg ctctggagct 1200

atgccatggg agtaggagtg gaacttgaaa actccatggg aggtttgaac tttggccgat 1260

cttactttga tccagcatat tttagattag ggcaagagat ggtaaggagg tcagctggaa 1320

aggtcagttc cacattggca tctgaactcg gtatcactgc cgaggatgca aggcttgttt 1380

cagagattgc aatgcatact actgaggaca agatcagtag agcggttgga cccagacaag 1440

cccaagtatc atttctacac ggtgatcaaa gtgagaatga gctaccgaga ttgggggggca 1500

aggaagatag gagggtcaaa cagagtcgag gagaagccag ggagagctac agagaaaccg 1560

ggcccagcag agcaagtgat gcgagagctg cccatcttcc aaccggcaca cccctagaca 1620

ttgacactgc aacggagtcc agccaagatc cgcaggacag tcgaaggtca gctgacgccc 1680
tgcttaggct gcaagccatg gcaggaatct cggaagaaca aggctcagac acggacaccc 1740
ctatagtgta caatgacaga aatcttctag actaggtgcg agaggccgag ggccagaaca 1800
acatccgcct accatccatc attgttataa aaaacttagg aaccaggtcc acacagccgc 1860
cagcccatca accatccact cccacgattg gagccaatgg cagaagagca ggcacgccat 1920
gtcaaaaacg gactggaatg catccgggct ctcaaggccg agcccatcgg ctcactggcc 1980
atcgaggaag ctatggcagc atggtcagaa atatcagaca acccaggaca ggagcgagcc 2040
acctgcaggg aagagaaggc aggcagttcg ggtctcagca aaccatgcct ctcagcaatt 2100
ggatcaactg aaggcggtgc acctcgcatc cgcggtcagg gacctggaga gagcgatgac 2160
gacgctgaaa ctttgggaat ccccccaaga aatctccagg catcaagcac tgggttacag 2220
tgttattacg tttatgatca cagcggtgaa gcggttaagg gaatccaaga tgctgactct 2280
atcatggttc aatcaggcct tgatggtgat agcaccctct caggaggaga caatgaatct 2340
gaaaacagcg atgtggatat tggcgaacct gataccgagg gatatgctat cactgaccgg 2400
ggatctgctc ccatctctat ggggttcagg gcttctgatg ttgaaactgc agaaggaggg 2460
gagatccacg agctcctgag actccaatcc agaggcaaca actttccgaa gcttgggaaa 2520
actctcaatg ttcctccgcc cccggacccc ggtagggcca gcacttccgg gacacccatt 2580
aaaaagggca cagacgcgag attagcctca tttggaacgg agatcgcgtc tttattgaca 2640
ggtggtgcaa cccaatgtgc tcgaaagtca ccctcggaac catcagggcc aggtgcacct 2700
gcggggaatg tccccgagtg tgtgagcaat gccgcactga tacaggagtg gacacccgaa 2760
tctggtacca caatctcccc gagatcccag aataatgaag aagggggaga ctattatgat 2820
gatgagctgt tctctgatgt ccaagatatt aaaacagcct tggccaaaat acacgaggat 2880
aatcagaaga taatctccaa gctagaatca ctgctgttat tgaagggaga agttgagtca 2940
attaagaagc agatcaacag gcaaaatatc agcatatcca ccctggaagg acacctctca 3000
agcatcatga tcgccattcc tggacttggg aaggatccca acgaccccac tgcagatgtc 3060
gaaatcaatc ccgacttgaa acccatcata ggcagagatt caggccgagc actggccgaa 3120
gttctcaaga aacccgttgc cagccgacaa ctccaaggaa tgacaaatgg acggaccagt 3180
tccagaggac agctgctgaa ggaatttcag ctaaagccga tcgggaaaaa gatgagctca 3240
gccgtcgggt ttgttcctga caccggccct gcatcacgca gtgtaatccg ctccattata 3300
aaatccagcc ggctagagga ggatcggaag cgttacctga tgactctcct tgatgatatc 3360
aaaggagcca atgatcttgc caagttccac cagatgctga tgaagataat aatgaagtag 3420
ctacagctca acttacctgc caaccccatg ccagtcgacc caactagcct accctccatc 3480
attgttataa aaaacttagg aaccaggtcc acacagccgc cagcccatca acgcgtacgg 3540

ccaccatggc acgcagagga gcggatacgt cagttggcat tgtgggcttg ctcctcacca 3600

ccgctatggc cgcagaagtt acaagaagag ggtctgccta ctacatgtat ctggacagga 3660

acgatgctgg cgaagccatc tcctttccca caaccctggg gatgaacaag tgctacattc 3720

agatcatgga tcttggtcac atgtgcgatg ccacaatgtc ctatgagtgt cccatgctcg 3780

atgagggcgt cgagcctgac gacgttgatt gctggtgtaa caccaccagc acctgggtgg 3840

tctatggcac ttgccatcac aagaagggtg aagcacggag gtcacgacgg gcagtgacat 3900

tgccaagtca tagcactagg aaactgcaga ctcgctccca gacctggctt gaatcccgag 3960

agtacacgaa acacctcatt cgtgtggaga actggatctt ccggaatccc gggtttgccc 4020

tggctgctgc cgctattgcc tggttgctcg gctctagcac atcacagaaa gtcatctacc 4080

tggtgatgat cttgctcatt gctccagcct actctatcag atgcatcgga gtttccaatc 4140

gtgactttgt cgaagggatg tccggaggca cctgggttga cgtggtgctt gagcatggtg 4200

gctgcgtaac ggtaatggct caggacaaac ccacggttga catagaactg gtgaccacga 4260

ctgtcagtaa tatggccgaa gtgcgaagct actgctatga ggcctctatc tctgacatgg 4320

catcagacag ccggtgtcca actcaaggcg aagcttatct ggacaagcaa tccgatacac 4380

agtacgtgtg caagcggact ctcgtggata gaggttgggg caatgggtgt ggtctgtttg 4440

ggaaaggcag tctggtaaca tgcgcaaagt tcgcatgttc caagaaaatg acaggaaaga 4500

gcattcagcc ggaaaatctg gagtatagga ttatgctcag cgtgcatggg agccagcaca 4560

gtggcatgat tgtcaatgat acaggccacg aaaccgacga gaacagagcg aaggttgaga 4620

tcactcccaa ttcacctcgc gcagaggcca ctctgggagg gtttggaagt ctcggtctgg 4680

actgtgaacc tcgcactggc ctggacttca gcgatctgta ttacctgacg atgaacaaca 4740

agcattggct ggtccataag gagtggttcc atgatattcc gcttccttgg catgccggag 4800

ccgataccgg aacaccccac tggaacaaca aagaggcttt ggtggagttc aaagatgcac 4860

acgctaagcg ccaaaccgtc gtggtcctgg gtagccaaga aggagcggtt cacactgcgc 4920

ttgccggagc cctggaggcg gaaatggatg gggccaaagg gcgcctttcc agtggacacc 4980

tgaagtgtcg gctgaaaatg gacaagctga gactgaaagg tgtgtcttac agcctgtgca 5040

ccgcagcctt cactttcacc aaaatccctg ctgaaacctt gcacggaaca gtgacagtag 5100

aggtgcagta tgcaggcaca gatggcccat gtaaggtgcc agctcagatg gcagtggaca 5160

tgcagaccct gactccagtg ggaaggctca taactgccaa tccggtcatc acagagtcta 5220

ccgagaactc aaagatgatg ctggagctgg accctccctt tggggactct tacatagtca 5280

taggggtagg cgagaagaaa atcacccatc actggcacag gagctaatga taagcgcgca 5340

gcgcttagac gtctcgcgat cgatactagt acaacctaaa tccattataa aaaacttagg 5400

agcaaagtga ttgcctccca aggtccacaa tgacagagac ctacgacttc gacaagtcgg 5460
catgggacat caaagggtcg atcgctccga tacaacccac cacctacagt gatggcaggc 5520
tggtgcccca ggtcagagtc atagatcctg gtctaggcga caggaaggat gaatgcttta 5580
tgtacatgtt tctgctgggg gttgttgagg acagcgattc cctagggcct ccaatcgggc 5640
gagcatttgg gttcctgccc ttaggtgttg gcagatccac agcaaagccc gaaaaactcc 5700
tcaaagaggc cactgagctt gacatagttg ttagacgtac agcagggctc aatgaaaaac 5760
tggtgttcta caacaacacc ccactaactc tcctcacacc ttggagaaag gtcctaacaa 5820
cagggagtgt cttcaacgca aaccaagtgt gcaatgcggt taatctgata ccgctcgata 5880
ccccgcagag gttccgtgtt gtttatatga gcatcacccg tctttcggat aacgggtatt 5940
acaccgttcc tagaagaatg ctggaattca gatcggtcaa tgcagtggcc ttcaacctgc 6000
tggtgaccct taggattgac aaggcgatag gccctgggaa gatcatcgac aatacagagc 6060
aacttcctga ggcaacattt atggtccaca tcgggaactt caggagaaag aagagtgaag 6120
tctactctgc cgattattgc aaaatgaaaa tcgaaaagat gggcctggtt tttgcacttg 6180
gtgggatagg gggcaccagt cttcacatta gaagcacagg caaaatgagc aagactctcc 6240
atgcacaact cgggttcaag aagaccttat gttacccgct gatggatatc aatgaagacc 6300
ttaatcgatt actctggagg agcagatgca agatagtaag aatccaggca gttttgcagc 6360
catcagttcc tcaagaattc cgcatttacg acgacgtgat cataaatgat gaccaaggac 6420
tattcaaagt tctgtagacc gtagtgccca gcaatgcccg aaaacgaccc ccctcacaat 6480
gacagccaga aggcccggac aaaaaagccc cctccgaaag actccacgga ccaagcgaga 6540
ggccagccag cagccgacgg caagcgcgaa caccaggcgg ccccagcaca gaacagccct 6600
gacacaaggc caccaccagc caccccaatc tgcatcctcc tcgtgggacc cccgaggacc 6660
aacccccaag gctgcccccg atccaaacca ccaaccgcat ccccaccacc cccgggaaag 6720
aaacccccag caattggaag gcccctcccc ctcttcctca acacaagaac tccacaaccg 6780
aaccgcacaa gcgaccgagg tgacccaacc gcaggcatcc gactccctag acagatcctc 6840
tctccccggc aaactaaaca aaacttaggg ccaaggaaca tacacaccca acagaaccca 6900
gaccccggcc cacggcgccg cgcccccaac cccgacaac cagagggagc ccccaaccaa 6960
tcccgccggc tcccccggtg cccacaggca gggacaccaa cccccgaaca gacccagcac 7020
ccaaccatcg acaatccaag acgggggggc ccccccaaaa aaaggccccc aggggccgac 7080
agccagcacc gcgaggaagc ccacccaccc cacacacgac cacggcaacc aaaccagaac 7140
ccagaccacc ctgggccacc agctcccaga ctcggccatc accccgcaga aaggaaaggc 7200
cacaacccgc gcaccccagc cccgatccgg cggggagcca cccaacccga accagcaccc 7260
aagagcgatc cccgaaggac ccccgaaccg caaaggacat cagtatccca cagcctctcc 7320

aagtcccccg gtctcctcct cttctcgaag ggaccaaaag atcaatccac cacacccgac 7380
gacactcaac tccccacccc taaaggagac accgggaatc ccagaatcaa gactcatcca 7440
atgtccatca tgggtctcaa ggtgaacgtc tctgccatat tcatggcagt actgttaact 7500
ctccaaacac ccaccggtca aatccattgg ggcaatctct ctaagatagg ggtggtagga 7560
ataggaagtg caagctacaa agttatgact cgttccagcc atcaatcatt agtcataaaa 7620
ttaatgccca atataactct cctcaataac tgcacgaggg tagagattgc agaatacagg 7680
agactactga gaacagtttt ggaaccaatt agagatgcac ttaatgcaat gacccagaat 7740
ataagaccgg ttcagagtgt agcttcaagt aggagacaca agagatttgc gggagtagtc 7800
ctggcaggtg cggccctagg cgttgccaca gctgctcaga taacagccgg cattgcactt 7860
caccagtcca tgctgaactc tcaagccatc gacaatctga gagcgagcct ggaaactact 7920
aatcaggcaa ttgagacaat cagacaagca gggcaggaga tgatattggc tgttcagggt 7980
gtccaagact acatcaataa tgagctgata ccgtctatga accaactatc ttgtgattta 8040
atcggccaga agctcgggct caaattgctc agatactata cagaaatcct gtcattattt 8100
ggccccagtt tacgggaccc catatctgcg gagatatcta tccaggcttt gagctatgcg 8160
cttggaggag acatcaataa ggtgttagaa aagctcggat acagtggagg tgatttactg 8220
ggcatcttag agagcggagg aataaaggcc cggataactc acgtcgacac agagtcctac 8280
ttcattgtcc tcagtatagc ctatccgacg ctgtccgaga ttaagggggt gattgtccac 8340
cggctagagg gggtctcgta caacataggc tctcaagagt ggtataccac tgtgcccaag 8400
tatgttgcaa cccaagggta ccttatctcg aattttgatg agtcatcgtg tactttcatg 8460
ccagagggga ctgtgtgcag ccaaaatgcc ttgtacccga tgagtcctct gctccaagaa 8520
tgcctccggg ggtacaccaa gtcctgtgct cgtacactcg tatccgggtc ttttgggaac 8580
cggttcattt tatcacaagg gaacctaata gccaattgtg catcaatcct ttgcaagtgt 8640
tacacaacag gaacgatcat taatcaagac cctgacaaga tcctaacata cattgctgcc 8700
gatcactgcc cggtagtcga ggtgaacggc gtgaccatcc aagtcgggag caggaggtat 8760
ccagacgctg tgtacttgca cagaattgac ctcggtcctc ccatatcatt ggagaggttg 8820
gacgtaggga caaatctggg gaatgcaatt gctaagttgg aggatgccaa ggaattgttg 8880
gagtcatcgg accagatatt gaggagtatg aaaggtttat cgagcactag catagtctac 8940
atcctgattg cagtgtgtct tggagggttg atagggatcc ccgctttaat atgttgctgc 9000
agggggcgtt gtaacaaaaa gggagaacaa gttggtatgt caagaccagg cctaaagcct 9060
gatcttacgg gaacatcaaa atcctatgta aggtcgctct gatcctctac aactcttgaa 9120
acacaaatgt cccacaagtc tcctcttcgt catcaagcaa ccaccgcacc cagcatcaag 9180

```
cccacctgaa attatctccg gcttccctct ggccgaacaa tatcggtagt taatcaaaac     9240
ttagggtgca agatcatcca caatgtcacc acaacgagac cggataaatg ccttctacaa     9300
agataacccc catcccaagg gaagtaggat agtcattaac agagaacatc ttatgattga     9360
tagaccttat gttttgctgg ctgttctgtt tgtcatgttt ctgagcttga tcgggttgct     9420
agccattgca ggcattagac ttcatcgggc agccatctac accgcagaga tccataaaag     9480
cctcagcacc aatctagatg taactaactc aatcgagcat caggtcaagg acgtgctgac     9540
accactcttc aaaatcatcg gtgatgaagt gggcctgagg acacctcaga gattcactga     9600
cctagtgaaa ttaatctctg acaagattaa attccttaat ccggataggg agtacgactt     9660
cagagatctc acttggtgta tcaacccgcc agagagaatc aaattggatt atgatcaata     9720
ctgtgcagat gtggctgctg aagagctcat gaatgcattg gtgaactcaa ctctactgga     9780
gaccagaaca accaatcagt tcctagctgt ctcaaaggga aactgctcag ggcccactac     9840
aatcagaggt caattctcaa acatgtcgct gtccctgtta gacttgtatt taggtcgagg     9900
ttacaatgtg tcatctatag tcactatgac atcccaggga atgtatgggg gaacttacct     9960
agtggaaaag cctaatctga gcagcaaaag gtcagagttg tcacaactga gcatgtaccg    10020
agtgtttgaa gtaggtgtta tcagaaatcc gggtttgggg gctccggtgt tccatatgac    10080
aaactatctt gagcaaccag tcagtaatga tctcagcaac tgtatggtgg ctttggggga    10140
gctcaaactc gcagcccttt gtcacgggga agattctatc acaattccct atcagggatc    10200
agggaaaggt gtcagcttcc agctcgtcaa gctaggtgtc tggaaatccc caaccgacat    10260
gcaatcctgg gtccccttat caacggatga tccagtgata gacaggcttt acctctcatc    10320
tcacagaggt gttatcgctg acaatcaagc aaaatgggct gtcccgacaa cacgaacaga    10380
tgacaagttg cgaatggaga catgcttcca acaggcgtgt aagggtaaaa tccaagcact    10440
ctgcgagaat cccgagtggg caccattgaa ggataacagg attccttcat acggggtctt    10500
gtctgttgat ctgagtctga cagttgagct taaaatcaaa attgcttcgg gattcgggcc    10560
attgatcaca cacggttcag ggatggacct atacaaatcc aaccacaaca atgtgtattg    10620
gctgactatc ccgccaatga agaacctagc cttaggtgta atcaacacat tggagtggat    10680
accgagattc aaggttagtc cctacctctt cactgtccca attaaggaag caggcgaaga    10740
ctgccatgcc ccaacatacc tacctgcgga ggtggatggt gatgtcaaac tcagttccaa    10800
tctggtgatt ctacctggtc aagatctcca atatgttttg gcaacctacg atacttccag    10860
ggttgaacat gctgtggttt attacgttta cagcccaagc cgctcatttt cttactttta    10920
tccttttagg ttgcctataa aggggtcccc catcgaatta caagtggaat gcttcacatg    10980
ggaccaaaaa ctctggtgcc gtcacttctg tgtgcttgcg gactcagaat ctggtggaca    11040
tatcactcac tctgggatgg tgggcatggg agtcagctgc acagtcaccc gggaagatgg    11100
```

aaccaatcgc agatagggct gctagtgaac caatcacatg atgtcaccca gacatcaggc 11160

atacccacta gtgtgaaata gacatcagaa ttaagaaaaa cgtagggtcc aagtggttcc 11220

ccgttatgga ctcgctatct gtcaaccaga tcttataccc tgaagttcac ctagatagcc 11280

cgatagttac caataagata gtagccatcc tggagtatgc tcgagtccct cacgcttaca 11340

gcctggagga ccctacactg tgtcagaaca tcaagcaccg cctaaaaaac ggattttcca 11400

accaaatgat tataaacaat gtggaagttg ggaatgtcat caagtccaag cttaggagtt 11460

atccggccca ctctcatatt ccatatccaa attgtaatca ggatttattt aacatagaag 11520

acaaagagtc aacgaggaag atccgtgaac tcctcaaaaa ggggaattcg ctgtactcca 11580

aagtcagtga taaggttttc caatgcttaa gggacactaa ctcacggctt ggcctaggct 11640

ccgaattgag ggaggacatc aaggagaaag ttattaactt gggagtttac atgcacagct 11700

cccagtggtt tgagcccttt ctgttttggt ttacagtcaa gactgagatg aggtcagtga 11760

ttaaatcaca aacccatact tgccatagga ggagacacac acctgtattc ttcactggta 11820

gttcagttga gttgctaatc tctcgtgacc ttgttgctat aatcagtaaa gagtctcaac 11880

atgtatatta cctgacattt gaactggttt tgatgtattg tgatgtcata gaggggaggt 11940

taatgacaga gaccgctatg actattgatg ctaggtatac agagcttcta ggaagagtca 12000

gatacatgtg gaaactgata gatggtttct tccctgcact cgggaatcca acttatcaaa 12060

ttgtagccat gctggagcct ctttcacttg cttacctgca gctgagggat ataacagtag 12120

aactcagagg tgctttcctt aaccactgct ttactgaaat acatgatgtt cttgaccaaa 12180

acgggttttc tgatgaaggt acttatcatg agttaactga agctctagat tacattttca 12240

taactgatga catacatctg acaggggaga ttttctcatt tttcagaagt ttcggccacc 12300

ccagacttga agcagtaacg gctgctgaaa atgttaggaa atacatgaat cagcctaaag 12360

tcattgtgta tgagactctg atgaaaggtc atgccatatt ttgtggaatc ataatcaacg 12420

gctatcgtga caggcacgga ggcagttggc caccgctgac cctccccctg catgctgcag 12480

acacaatccg gaatgctcaa gcttcaggtg aagggttaac acatgagcag tgcgttgata 12540

actggaaatc ttttgctgga gtgaaatttg gctgctttat gcctcttagc ctggatagtg 12600

atctgacaat gtacctaaag gacaaggcac ttgctgctct ccaaagggaa tgggattcag 12660

tttacccgaa agagttcctg cgttacgacc ctcccaaggg aaccgggtca cggaggcttg 12720

tagatgtttt ccttaatgat tcgagctttg acccatatga tgtgataatg tatgttgtaa 12780

gtggagctta cctccatgac cctgagttca acctgtctta cagcctgaaa gaaaaggaga 12840

tcaaggaaac aggtagactt tttgctaaaa tgacttacaa aatgagggca tgccaagtga 12900

ttgctgaaaa tctaatctca aacgggattg gcaaatattt taaggacaat gggatggcca 12960

aggatgagca cgatttgact aaggcactcc acactctagc tgtctcagga gtccccaaag 13020

atctcaaaga aagtcacagg gggggccag tcttaaaaac ctactcccga agcccagtcc 13080

acacaagtac caggaacgtg agagcagcaa aagggtttat agggttccct caagtaattc 13140

ggcaggacca agacactgat catccggaga atatggaagc ttacgagaca gtcagtgcat 13200

ttatcacgac tgatctcaag aagtactgcc ttaattggag atatgagacc atcagcttgt 13260

ttgcacagag gctaaatgag atttacggat tgccctcatt tttccagtgg ctgcataaga 13320

ggcttgagac ctctgtcctg tatgtaagtg accctcattg cccccccgac cttgacgccc 13380

atatcccgtt atataaagtc cccaatgatc aaatcttcat taagtaccct atgggaggta 13440

tagaagggta ttgtcagaag ctgtggacca tcagcaccat tccctatcta tacctggctg 13500

cttatgagag cggagtaagg attgcttcgt tagtgcaagg ggacaatcag accatagccg 13560

taacaaaaag ggtacccagc acatggccct acaaccttaa gaaacgggaa gctgctagag 13620

taactagaga ttactttgta attcttaggc aaaggctaca tgatattggc catcacctca 13680

aggcaaatga gacaattgtt tcatcacatt tttttgtcta ttcaaaagga atatattatg 13740

atgggctact tgtgtcccaa tcactcaaga gcatcgcaag atgtgtattc tggtcagaga 13800

ctatagttga tgaaacaagg gcagcatgca gtaatattgc tacaacaatg gctaaaagca 13860

tcgagagagg ttatgaccgt taccttgcat attccctgaa cgtcctaaaa gtgatacagc 13920

aaattctgat ctctcttggc ttcacaatca attcaaccat gacccgggat gtagtcatac 13980

ccctcctcac aaacaacgac ctcttaataa ggatggcact gttgcccgct cctattgggg 14040

ggatgaatta tctgaatatg agcaggctgt ttgtcagaaa catcggtgat ccagtaacat 14100

catcaattgc tgatctcaag agaatgattc tcgcctcact aatgcctgaa gagaccctcc 14160

atcaagtaat gacacaacaa ccgggggact cttcattcct agactgggct agcgaccctt 14220

actcagcaaa tcttgtatgt gtccagagca tcactagact cctcaagaac ataactgcaa 14280

ggtttgtcct gatccatagt ccaaacccaa tgttaaaagg attattccat gatgacagta 14340

aagaagagga cgagggactg gcggcattcc tcatggacag gcatattata gtacctaggg 14400

cagctcatga aatcctggat catagtgtca caggggcaag agagtctatt gcaggcatgc 14460

tggataccac aaaaggcttg attcgagcca gcatgaggaa gggggggtta acctctcgag 14520

tgataaccag attgtccaat tatgactatg aacaattcag agcagggatg gtgctattga 14580

caggaagaaa gagaaatgtc ctcattgaca aagagtcatg ttcagtgcag ctggcgagag 14640

ctctaagaag ccatatgtgg gcgaggctag ctcgaggacg gcctatttac ggccttgagg 14700

tccctgatgt actagaatct atgcgaggcc accttattcg gcgtcatgag acatgtgtca 14760

tctgcgagtg tggatcagtc aactacggat ggtttttgt cccctcgggt tgccaactgg 14820

atgatattga caaggaaaca tcatccttga gagtcccata tattggttct accactgatg 14880

agagaacaga catgaagctt gccttcgtaa gagccccaag tcgatccttg cgatctgctg 14940
ttagaatagc aacagtgtac tcatgggctt acggtgatga tgatagctct tggaacgaag 15000
cctggttgtt ggctaggcaa agggccaatg tgagcctgga ggagctaagg gtgatcactc 15060
ccatctcaac ttcgactaat ttagcgcata ggttgaggga tcgtagcact caagtgaaat 15120
actcaggtac atcccttgtc cgagtggcga ggtataccac aatctccaac gacaatctct 15180
catttgtcat atcagataag aaggttgata ctaactttat ataccaacaa ggaatgcttc 15240
tagggttggg tgttttagaa acattgtttc gactcgagaa agataccgga tcatctaaca 15300
cggtattaca tcttcacgtc gaaacagatt gttgcgtgat cccgatgata gatcatccca 15360
ggatacccag ctcccgcaag ctagagctga gggcagagct atgtaccaac ccattgatat 15420
atgataatgc acctttaatt gacagagatg caacaaggct atacacccag agccatagga 15480
ggcaccttgt ggaatttgtt acatggtcca caccccaact atatcacatt ttagctaagt 15540
ccacagcact atctatgatt gacctggtaa caaaatttga gaaggaccat atgaatgaaa 15600
tttcagctct catagggat gacgatatca atagtttcat aactgagttt ctgctcatag 15660
agccaagatt attcactatc tacttgggcc agtgtgcggc catcaattgg gcatttgatg 15720
tacattatca tagaccatca gggaaatatc agatgggtga gctgttgtca tcgttccttt 15780
ctagaatgag caaaggagtg tttaaggtgc ttgtcaatgc tctaagccac ccaaagatct 15840
acaagaaatt ctggcattgt ggtattatag agcctatcca tggtccttca cttgatgctc 15900
aaaacttgca cacaactgtg tgcaacatgg tttacacatg ctatatgacc tacctcgacc 15960
tgttgttgaa tgaagagtta gaagagttca catttctctt gtgtgaaagc gacgaggatg 16020
tagtaccgga cagattcgac aacatccagg caaaacactt atgtgttctg gcagatttgt 16080
actgtcaacc agggacctgc ccaccaattc gaggtctaag accggtagag aaatgtgcag 16140
ttctaaccga ccatatcaag gcagaggcta tgttatctcc agcaggatct tcgtggaaca 16200
taaatccaat tattgtagac cattactcat gctctctgac ttatctccgg cgaggatcga 16260
tcaaacagat aagattgaga gttgatccag gattcatttt cgacgccctc gctgaggtaa 16320
atgtcagtca gccaaagatc ggcagcaaca acatctcaaa tatgagcatc aaggctttca 16380
gacccccaca cgatgatgtt gcaaaattgc tcaaagatat caacacaagc aagcacaatc 16440
ttcccatttc aggggcaat ctcgccaatt atgaaatcca tgctttccgc agaatcgggt 16500
tgaactcatc tgcttgctac aaagctgttg agatatcaac attaattagg agatgccttg 16560
agccagggga ggacggcttg ttcttgggtg agggatcggg ttctatgttg atcacttata 16620
aagagatact taaactaaac aagtgcttct ataatagtgg ggtttccgcc aattctagat 16680
ctggtcaaag ggaattagca ccctatccct ccgaagttgg ccttgtcgaa cacagaatgg 16740

gagtaggtaa tattgtcaaa gtgctcttta acgggaggcc cgaagtcacg tgggtaggca 16800
gtgtagattg cttcaatttc atagttagta atatccctac ctctagtgtg gggtttatcc 16860
attcagatat agagaccttg cctgacaaag atactataga gaagctagag gaattggcag 16920
ccatcttatc gatggctctg ctcctgggca aaataggatc aatactggtg attaagctta 16980
tgcctttcag cggggatttt gttcagggat ttataagtta tgtagggtct cattatagag 17040
aagtgaacct tgtataccct agatacagca acttcatctc tactgaatct tatttggtta 17100
tgacagatct caaggctaac cggctaatga atcctgaaaa gattaagcag cagataattg 17160
aatcatctgt gaggacttca cctggactta taggtcacat cctatccatt aagcaactaa 17220
gctgcataca agcaattgtg ggagacgcag ttagtagagg tgatatcaat cctactctga 17280
aaaaacttac acctatagag caggtgctga tcaattgcgg gttggcaatt aacggaccta 17340
agctgtgcaa agaattgatc caccatgatg ttgcctcagg gcaagatgga ttgcttaatt 17400
ctatactcat cctctacagg gagttggcaa gattcaaaga caaccaaaga agtcaacaag 17460
ggatgttcca cgcttacccc gtattggtaa gtagcaggca acgagaactt atatctagga 17520
tcacccgcaa attctggggg cacattcttc tttactccgg gaacaaaaag ttgataaata 17580
agtttatcca gaatctcaag tccggctatc tgatactaga cttacaccag aatatcttcg 17640
ttaagaatct atccaagtca gagaaacaga ttattatgac ggggggtttg aaacgtgagt 17700
gggtttttaa ggtaacagtc aaggagacca aagaatggta taagttagtc ggatacagtg 17760
ccctgattaa ggactaattg gttgaactcc ggaaccctaa tcctgcccta ggtggttagg 17820
cattatttgc aatatattaa agaaaacttt gaaaatacga agtttctatt cccagctttg 17880
tctggtggcc ggcatggtcc cagcctcctc gctggcgccg gctgggcaac attccgaggg 17940
gaccgtcccc tcggtaatgg cgaatgggac gcggccgatc cggctgctaa caaagcccga 18000
aaggaagctg agttggctgc tgccaccgct gagcaataac tagcataacc ccttggggcc 18060
tctaaacggg tcttgagggg ttttttgctg aaaggaggaa ctatatccgg atgcggccgc 18120
gggccctatg gtacccagct tttgttccct ttagtgaggg ttaattccga gcttggcgta 18180
atcatggtca tagctgtttc ctgtgtgaaa ttgttatccg ctcacaattc cacacaacat 18240
aggagccgga agcataaagt gtaaagcctg gggtgcctaa tgagtgaggt aactcacatt 18300
aattgcgttg cgctcactgc ccgctttcca gtcgggaaac ctgtcgtgcc agctgcatta 18360
atgaatcggc caacgcgcgg ggagaggcgg tttgcgtatt gggcgctctt ccgcttcctc 18420
gctcactgac tcgctgcgct cggtcgttcg gctgcggcga gcggtatcag ctcactcaaa 18480
ggcggtaata cggttatcca cagaatcagg ggataacgca ggaaagaaca tgtgagcaaa 18540
aggccagcaa aaggccagga accgtaaaaa ggccgcgttg ctggcgtttt tccataggct 18600
ccgcccccct gacgagcatc acaaaaatcg acgctcaagt cagaggtggc gaaacccgac 18660

aggactataa agataccagg cgtttccccc tggaagctcc ctcgtgcgct ctcctgttcc 18720
gaccctgccg cttaccggat acctgtccgc ctttctccct tcgggaagcg tggcgctttc 18780
tcaatgctca cgctgtaggt atctcagttc ggtgtaggtc gttcgctcca agctgggctg 18840
tgtgcacgaa ccccccgttc agcccgaccg ctgcgcctta tccggtaact atcgtcttga 18900
gtccaacccg gtaagacacg acttatcgcc actggcagca gccactggta acaggattag 18960
cagagcgagg tatgtaggcg gtgctacaga gttcttgaag tggtggccta actacggcta 19020
cactagaagg acagtatttg gtatctgcgc tctgctgaag ccagttacct tcggaaaaag 19080
agttggtagc tcttgatccg gcaaacaaac caccgctggt agcggtggtt tttttgtttg 19140
caagcagcag attacgcgca gaaaaaaagg atctcaagaa gatcctttga tcttttctac 19200
ggggtctgac gctcagtgga acgaaaactc acgttaaggg attttggtca tgagattatc 19260
aaaaaggatc ttcacctaga tccttttaaa ttaaaaatga agttttaaat caatctaaag 19320
tatatatgag taaacttggt ctgacagtta ccaatgctta atcagtgagg cacctatctc 19380
agcgatctgt ctatttcgtt catccatagt tgcctgactg ccccgtcgtgt agataactac 19440
gatacgggag ggcttaccat ctggccccag tgctgcaatg ataccgcgag acccacgctc 19500
accggctcca gatttatcag caataaacca gccagccgga agggccgagc gcagaagtgg 19560
tcctgcaact ttatccgcct ccatccagtc tattaattgt tgccgggaag ctagagtaag 19620
tagttcgcca gttaatagtt tgcgcaacgt tgttgccatt gctacaggca tcgtggtgtc 19680
acgctcgtcg tttggtatgg cttcattcag ctccggttcc caacgatcaa ggcgagttac 19740
atgatccccc atgttgtgaa aaaaagcggt tagctccttc ggtcctccga tcgttgtcag 19800
aagtaagttg gccgcagtgt tatcactcat gcttatggca gcactgcata attctcttac 19860
tgtcatgcca tccgtaagat gcttttctgt gactggtgag tactcaacca agtcattctg 19920
agaatagtgt atgcggcgac cgagttgctc ttgcccggcg tcaatacggg ataataccgc 19980
gccacatagc agaactttaa aagtgctcat cattggaaaa cgttcttcgg ggcgaaaact 20040
ctcaaggatc ttaccgctgt tgagatccag ttcgatgtaa cccactcgtg cacccaactg 20100
atcttcagca tcttttactt tcaccagcgt ttctgggtga gcaaaaacag gaaggcaaaa 20160
tgccgcaaaa aagggaataa gggcgacacg gaaatgttga atactcatac tcttcctttt 20220
tcaatattat tgaagcattt atcagggtta ttgtctcatg agcggataca tatttgaatg 20280
tatttagaaa aataaacaaa taggggttcc gcgcacattt ccccgaaaag tgccacctga 20340
aattgtaaac gttaatattt tgttaaaatt cgcgttaaat ttttgttaaa tcagctcatt 20400
ttttaaccaa taggccgaaa tcggcaaaat cccttataaa tcaaaagaat agaccgagat 20460
agggttgagt gttgttccag tttggaacaa gagtccacta ttaaagaacg tggactccaa 20520

```
cgtcaaaggg cgaaaaaccg tctatcaggg cgatggccca ctacgtgaac catcacccta    20580
atcaagtttt ttggggtcga ggtgccgtaa agcactaaat cggaacccta aagggagccc    20640
ccgatttaga gcttgacggg gaaagccggc gaacgtggcg agaaaggaag ggaagaaagc    20700
gaaaggagcg ggcgctaggg cgctggcaag tgtagcggtc acgctgcgcg taaccaccac    20760
acccgccgcg cttaatgcgc cgctacaggg cgcgtcccat tcgccattca ggctgcgcaa    20820
ctgttgggaa gggcgatcgg tgcgggcctc ttcgctatta cgccagccac cgcggtgatc    20880
cttataaagc tagatg                                                    20896
```